# EUROPEAN PATENT APPLICATION

(11) **EP 4 242 202 A1**
(43) Date of publication of application: **13.09.2023**
(21) Application number: 21897124.0
(22) Date of filing: 26.11.2021
(51) Int. Cl.: C07D 401/04, C07D 405/04, A61K 31/497, A61P 3/10

(54) **BENZIMIDAZOLE DERIVATIVE AND PREPARATION METHOD THEREFOR AND MEDICAL USE THEREOF**

(30) Priority: 27.11.2020 CN 202011357951; 16.12.2020 CN 202011488073; 08.02.2021 CN 202110181666; 17.04.2021 CN 202110415182; 19.05.2021 CN 202110548095; 23.06.2021 CN 202110702857; 19.11.2021 CN 202111373437
(71) Applicant: Shenzhen Salubris Pharmaceuticals Co., Ltd., Shenzhen, Guangdong 518017 (CN)
(72) Inventor: WU, Junjun, Shenzhen, Guangdong 518040 (CN); LU, Yinsuo, Shenzhen, Guangdong 518040 (CN); WU, Jianli, Shenzhen, Guangdong 518040 (CN); XIAO, Ying, Shenzhen, Guangdong 518040 (CN); XING, Wei, Shenzhen, Guangdong 518040 (CN)
(74) Representative: Zimmermann & Partner Patentanwälte mbB
(86) International application number: PCT/CN2021/133447
(87) International publication number: WO 2022/111624

(57) **Abstract**

Disclosed are a compound of formula (I), a preparation method therefor, and a medical application thereof. In particular, provided are the compound of formula (I) or a stereoisomer, tautomer, or pharmaceutically acceptable salt thereof. These compounds are agonists of a glucagon-like peptide-1 receptor (GLP-1R). The present invention also relates to a pharmaceutical composition containing these compounds and use of the compound in a drug for treating diseases such as diabetes.

## Description

### FIELD OF THE INVENTION

The present invention belongs to the technical field of chemical pharmaceuticals and provides a series of agonists of glucagon-like peptide-1 receptor (GLP-1R). The present invention also relates to pharmaceutical compositions containing the compounds, and use of the compounds in the manufacture of medicament for the treatment of diseases such as diabetes mellitus.

### BACKGROUND OF THE INVENTION

Diabetes mellitus affects millions of people worldwide and is considered as one of the leading threats to human mortality in the 21^{st} century. Over time, uncontrolled diabetes mellitus can damage body systems, including the heart, blood vessels, eyes, kidneys and nerves. Diabetes mellitus results in heavy socioeconomic burden all over the world.

There are two main types of diabetes mellitus, named type 1 and type 2, wherein type 2 diabetes mellitus (T2DM) accounts for more than 90% of all patients with diabetes mellitus worldwide. Type 1 diabetes mellitus is characterized by insulin deficiency, mainly caused by autoimmune-mediated destruction of pancreatic beta cells. Type 2 diabetes mellitus is characterized by abnormal insulin secretion and consequent insulin tolerance. To prevent ketoacidosis, patients with type 1 diabetes mellitus must ingest exogenous insulin for surviving. Patients with type 2 diabetes mellitus may require exogenous insulin to control their blood glucose levels, although they are not as dependent on exogenous insulin as those with type 1 diabetes mellitus.

Glucagon-like peptide-1 (GLP-1), an incretin, is secreted by intestinal epithelium L cells and exerts physiological effects through binding to its receptor. GLP-1 receptor (GLP-1R) belongs to the subfamily of G protein-coupled receptors. When GLP-1 binds to the GLP-1 receptor, a series of biological effects are triggered. It has been shown that GLP-1 promotes insulin secretion in a glucose-dependent manner, i.e., when blood glucose concentration increases in the body, GLP-1 stimulates islet cells to increase insulin secretion and thus lowers blood glucose. GLP-1 receptor agonist is a new type of hypoglycemic drug, which can effectively control blood glucose level without causing hypoglycemia and effectively reduce body mass to achieve weight control by increasing satiety, delaying gastric emptying, suppressing appetite, reducing fat accumulation, and the like.

Currently, GLP-1 receptor agonist-based peptides drugs such as Liraglutide, Exenatide and Semaglutide have been applied to obese patients with type 2 diabetes mellitus and to simply obese or overweight subjects, and all have shown significant body mass reduction effects. However, they are often accompanied by gastrointestinal adverse effects such as nausea and vomiting. Oral non-peptide drugs are attempted by research institutions for the treatment of type 2 diabetes mellitus and weight control, but the development of small molecules drugs for the glucagon-like peptide-1 receptor is limited by the difficulty of small molecules to mimic the receptor-peptide interactions.

Currently, patent applications disclosing non-peptide GLP-1 receptor agonists include WO2009/111700, WO2010/114824, WO2011/114271, WO2013/090454, WO2018/056453, WO2018/109607, WO2019239319, WO2019239371, WO2020103815, etc., wherein only vTv's TTP-273 and Pfizer's PF-06882961 have entered phase II clinical study.

Therefore, the present invention is intended to discover new non-peptide GLP-1 receptor agonists, especially new compounds that have good biological properties and can be safely applied to humans. The present invention also provides an approach for the prevention and/or treatment of GLP-l-related diseases, especially diabetes mellitus-related diseases.

### SUMMARY

The present invention provides a series of benzimidazole derivatives, preparation methods therefor and pharmaceutical use thereof.

Specifically, the present invention provides a compound of Formula (I), or a stereoisomer, s tautomer, or a pharmaceutically acceptable salt thereof, wherein all variables are as defined herein.

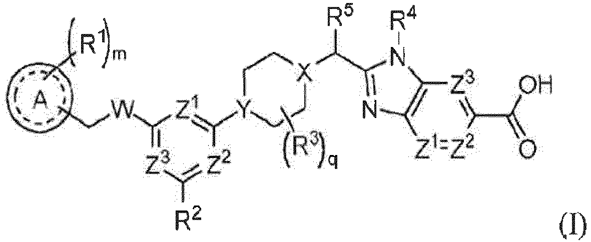

These compounds are agonists of glucagon-like peptide-1 receptor (GLP-1R). The present invention also relates to pharmaceutical compositions containing the compounds, and use of the compounds in the manufacture of a medicament for the treatment of diseases such as diabetes mellitus.

Specifically, the present invention is achieved by the following technical solutions.

A compound of Formula (I): 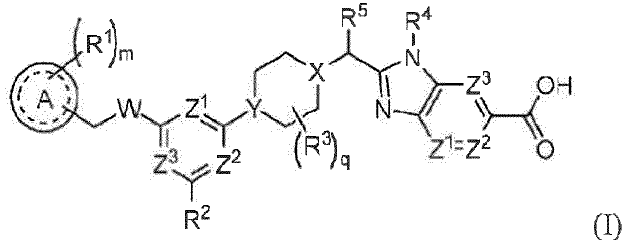
or a stereoisomer, tautomer, or pharmaceutically acceptable salt thereof, wherein,
A is selected from the group consisting of phenyl ring, heteroaromatic ring, and 8- to 10-membered fused aromatic ring;
R¹ is selected from the group consisting of hydrogen, halogen, cyano, alkyl, alkoxy, haloalkyl, haloalkoxy, alkoxyalkyl, cycloalkyl, heterocycloalkyl, alkyl substituted by one or more R⁷, aryl substituted by one or more R⁸, and heteroaryl substituted by one or more R⁸;
m is 0, 1, 2, or 3;
W is O or NH;
R² is selected from the group consisting of hydrogen, halogen, cyano, alkyl, haloalkyl, alkoxy, and alkoxyalkyl;
R³ is selected from the group consisting of fluorine, hydroxyl, cyano, oxo (=O), C₁₋₃ alkyl, OC₁₋₃ alkyl, C₃₋₄ cycloalkyl, and C₃₋₄ spiroalkyl formed by cyclization of two R³ together, wherein the C₁₋₃ alkyl, OC₁₋₃ alkyl, C₃₋₄ cycloalkyl, and C₃₋₄ spiroalkyl can be substituted by 0 to 3 fluorine atoms or 0 to 1 hydroxyl where the valence allows;
q is 0, 1, or 2;
X is CH or N;
Y is CH or N;
R⁵ is selected from the group consisting of halogen, hydroxyl, alkyl, haloalkyl, alkoxy, haloalkoxy, alkoxyalkyl, cycloalkyl, heterocycloalkyl, alkylcycloalkyl, and alkylheterocycloalkyl;
R⁴ is -C₁₋₃ alkyl, -C₀₋₃ alkylene-C₃₋₆ cycloalkyl, -C₀₋₃ alkylene-R⁹, or -C₁₋₃ alkylene-R⁶, wherein the alkyl can be substituted by 0 to 3 substituents independently selected from the group consisting of 0 to 3 F atoms or by 0 to 1 substituent selected from the group consisting of -C₀₋₁ alkylene-CN, C₀₋₁ alkylene-OR^{O} and -N(R^{N})₂ where the valence allows, and wherein the alkylene and cycloalkyl can be independently substituted by 0 to 2 substituents independently selected from the group consisting of 0 to 2 F atoms or by 0 to 1 substituent selected from the group consisting of -C₀₋₁ alkylene-CN, -C₀₋₁ alkylene-OR^{O} and -N(R^{N})₂ where the valence allows;
R⁹ is 4- to 6-membered heterocycloalkyl, wherein the heterocycloalkyl can be substituted by 0 to 2 substituents independently selected from the group consisting of the substituents below where the valence allows:
   0 to 1 oxo (=O),
   0 to 1 -CN,
   0 to 2 F atoms, and
   0 to 2 substituents independently selected from the group consisting of -C₁₋₃ alkyl and -OC₁₋₃ alkyl, wherein the alkyl of the -C₁₋₃ alkyl and -OC₁₋₃ alkyl can be substituted by 0 to 3 substituents independently selected from the group consisting of the substituents below where the valence allows:
      0 to 3 F atoms,
      0 to 1 -CN, and
      0 to 1 -OR^{O};
      R⁶ is 5- to 6-membered heteroaryl, wherein the heteroaryl can be substituted by 0 to 2 substituents independently selected from the group consisting of the substituents below where the valence allows:
         0 to 2 halogen,
         0 to 1 substituent selected from the group consisting of -OR^{O} and -N(R^{N})₂, and
         0 to 2 -C₁₋₃ alkyl, wherein the alkyl can be substituted by 0 to 3 substituents independently selected from the group consisting of the substituents below where the valence allows:
            0 to 3 F atoms, and
            0 to 1 -OR^{O};
            each R^{O} is independently H or -C₁₋₃ alkyl, wherein the C₁₋₃ alkyl can be substituted by 0 to 3 F atoms;
            each R^{N} is independently H or -C₁₋₃ alkyl;
            Z¹, Z², and Z³ are each independently -CR^{Z} or N, and each R^{Z} is independently H, F, Cl, or -CH₃;
            R⁷ is selected from the group consisting of halogen, cyano, hydroxyl, cycloalkyl, heterocycloalkyl, and sulfonyl; and
            R⁸ is selected from the group consisting of halogen, cyano, alkyl, haloalkyl, alkoxy, haloalkoxy, alkoxyalkyl, cycloalkyl, heterocycloalkyl, and sulfonyl.

As a preferred embodiment of the present invention, the compound or the stereoisomer, tautomer, or pharmaceutically acceptable salt thereof is shown as Formula (III): 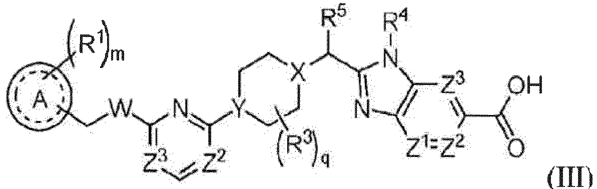 , wherein,
A is selected from the group consisting of phenyl ring, heteroaromatic ring, and 8- to 10-membered fused aromatic ring;
R¹ is selected from the group consisting of hydrogen, halogen, cyano, alkyl, alkoxy, haloalkyl, haloalkoxy, alkoxyalkyl, cycloalkyl, heterocycloalkyl, alkyl substituted by one or more R⁷, aryl substituted by one or more R⁸, and heteroaryl substituted by one or more R⁸;
m is 0, 1, 2, or 3;
W is O or NH;
R³ is selected from the group consisting of fluorine, hydroxyl, cyano, oxo (=O), C₁₋₃ alkyl, OC₁₋₃ alkyl, C₃₋₄ cycloalkyl, and C₃₋₄ spiroalkyl formed by cyclization of two R³ together, wherein the C₁₋₃ alkyl, OC₁₋₃ alkyl, C₃₋₄ cycloalkyl, and C₃₋₄ spiroalkyl can be substituted by 0 to 3 fluorine atoms or 0 to 1 hydroxyl where the valence allows;
q is 0, 1, or 2;
X is CH or N;
Y is CH or N;
R⁵ is selected from the group consisting of halogen, hydroxyl, alkyl, haloalkyl, alkoxy, haloalkoxy, alkoxyalkyl, cycloalkyl, heterocycloalkyl, alkylcycloalkyl, and alkylheterocycloalkyl;
R⁴ is -C₁₋₃ alkyl, -C₀₋₃ alkylene-C₃₋₆ cycloalkyl, -C₀₋₃ alkylene-R⁹, or -C₁₋₃ alkylene-R⁶, wherein the alkyl can be substituted by 0 to 3 substituents independently selected from the group consisting of 0 to 3 F atoms or by 0 to 1 substituent selected from the group consisting of -C₀₋₁ alkylene-CN, -C₀₋₁ alkylene-OR^{O} and -N(R^{N})₂ where the valence allows, and wherein the alkylene and cycloalkyl can be independently substituted by 0 to 2 substituents independently selected from the group consisting of 0 to 2 F atoms or by 0 to 1 substituent selected from the group consisting of -C₀₋₁ alkylene-CN, -C₀₋₁ alkylene-OR^{O} and -N(R^{N})₂ where the valence allows;
R⁹ is 4- to 6-membered heterocycloalkyl, wherein the heterocycloalkyl is substituted by 0 to 2 substituents independently selected from the group consisting of the substituents below where the valence allows:
   0 to 1 oxo (=O),
   0 to 1 -CN,
   0 to 2 F atoms, and
   0 to 2 substituents independently selected from the group consisting of -C₁₋₃ alkyl and -OC₁₋₃ alkyl, wherein the alkyl of the -C₁₋₃ alkyl and -OC₁₋₃ alkyl can be substituted by 0 to 3 substituents independently selected from the group consisting of the substituents below where the valence allows:
      0 to 3 F atoms,
      0 to 1 -CN, and
      0 to 1 -OR^{O};
      R⁶ is 5- to 6-membered heteroaryl, wherein the heteroaryl can be substituted by 0 to 2 substituents independently selected from the group consisting of the substituents below where the valence allows:
         0 to 2 halogen,
         0 to 1 substituent selected from the group consisting of -OR^{O} and -N(R^{N})₂, and
         0 to 2 -C₁₋₃ alkyl, wherein the alkyl can be substituted by 0 to 3 substituents independently selected from the group consisting of the substituents below where the valence allows:
            0 to 3 F atoms, and
            0 to 1 -OR^{O};
            each R^{O} is independently H or -C₁₋₃ alkyl, wherein the C₁₋₃ alkyl can be substituted by 0 to 3 F atoms;
            each R^{N} is independently H or -C₁₋₃ alkyl;
            Z¹, Z², and Z³ are each independently -CR^{Z} or N, and each R^{Z} is independently H, F, Cl, or -CH₃;
            R⁷ is selected from the group consisting of halogen, cyano, hydroxyl, cycloalkyl, heterocycloalkyl, and sulfonyl; and
            R⁸ is selected from the group consisting of halogen, cyano, alkyl, haloalkyl, alkoxy, haloalkoxy, alkoxyalkyl, cycloalkyl, heterocycloalkyl, and sulfonyl.

As a preferred embodiment of the present invention, the compound or the stereoisomer, tautomer, pharmaceutically acceptable salt thereof is shown as Formula (II): 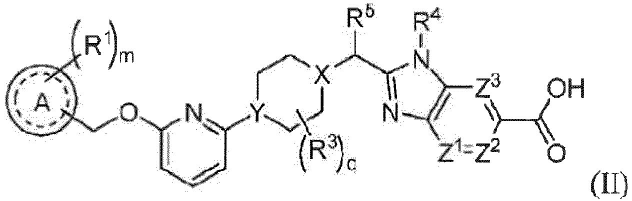 , wherein,
A is selected from the group consisting of phenyl ring, heteroaromatic ring, and 8- to 10-membered fused aromatic ring;
R¹ is selected from the group consisting of hydrogen, halogen, cyano, alkyl, alkoxy, haloalkyl, haloalkoxy, alkoxyalkyl, cycloalkyl, heterocycloalkyl, alkyl substituted by one or more R⁷, aryl substituted by one or more R⁸, and heteroaryl substituted by one or more R⁸;
m is 0, 1, 2, or 3;
R³ is selected from the group consisting of fluorine, hydroxyl, cyano, C₁₋₃ alkyl, OC₁₋₃ alkyl, C₃₋₄ cycloalkyl, and C₃₋₄ spiroalkyl formed by cyclization of two R³ together, wherein the C₁₋₃ alkyl, OC₁₋₃ alkyl, C₃₋₄ cycloalkyl, and C₃₋₄ spiroalkyl can be substituted by 0 to 3 fluorine atoms or 0 to 1 hydroxyl where the valence allows;
q is 0, 1, or 2;
X is CH or N;
Y is CH or N;
R⁵ is selected from the group consisting of halogen, hydroxyl, alkyl, haloalkyl, alkoxy, haloalkoxy, alkoxyalkyl, cycloalkyl, heterocycloalkyl, alkylcycloalkyl, and alkylheterocycloalkyl;
R⁴ is -C₁₋₃ alkyl, -C₀₋₃ alkylene-C₃₋₆ cycloalkyl, -C₀₋₃ alkylene-R⁹, or -C₁₋₃ alkylene-R⁶, wherein the alkyl can be substituted by 0 to 3 substituents independently selected from the group consisting of 0 to 3 F atoms or by 0 to 1 substituent selected from the group consisting of -C₀₋₁ alkylene-CN, -C₀₋₁ alkylene-OR^{O} and -N(R^{N})₂ where the valence allows, and wherein the alkylene and cycloalkyl can be independently substituted by 0 to 2 substituents independently selected from the group consisting of 0 to 2 F atoms or by 0 to 1 substituent selected from the group consisting of -C₀₋₁ alkylene-CN, -C₀₋₁ alkylene-OR^{O} and -N(R^{N})₂ where the valence allows;
R⁹ is 4- to 6-membered heterocycloalkyl, wherein the heterocycloalkyl can be substituted by 0 to 2 substituents independently selected from the group consisting of the substituents below where the valence allows:
   0 to 1 oxo (=O),
   0 to 1 -CN,
   0 to 2 F atoms, and
   0 to 2 substituents independently selected from the group consisting of -C₁₋₃ alkyl and -OC₁₋₃ alkyl, wherein the alkyl of the -C₁₋₃ alkyl and -OC₁₋₃ alkyl can be substituted by 0 to 3 substituents independently selected from the group consisting of the groups below where the valence allows:
      0 to 3 F atoms,
      0 to 1 -CN, and
      0 to 1 -OR^{O};
      R⁶ is 5- to 6-membered heteroaryl, wherein the heteroaryl can be substituted by 0 to 2 substituents independently selected from the group consisting of the substituents below where the valence allows:
         0 to 2 halogen,
         0 to 1 substituent selected from the group consisting of -OR^{O} and -N(R^{N})₂, and
         0 to 2 -C₁₋₃ alkyl, wherein the alkyl can be substituted by 0 to 3 substituents independently selected from the group consisting of the substituents below where the valence allows:
            0 to 3 F atoms, and
            0 to 1 -OR^{O};
            each R^{O} is independently H or -C₁₋₃ alkyl, wherein the C₁₋₃ alkyl can be substituted by 0 to 3 F atoms;
            each R^{N} is independently H or -C₁₋₃ alkyl;
            Z¹, Z², and Z³ are each independently -CR^{Z} or N, and each R^{Z} is independently H, F, Cl, or -CH₃;
            R⁷ is selected from the group consisting of halogen, cyano, hydroxyl, cycloalkyl, heterocycloalkyl, and sulfonyl; and
            R⁸ is selected from the group consisting of halogen, cyano, alkyl, haloalkyl, alkoxy, haloalkoxy, alkoxyalkyl, cycloalkyl, heterocycloalkyl, and sulfonyl.

As a preferred embodiment of the present invention, the alkyl refers to a linear, branched or cyclic saturated hydrocarbon chain containing a specific number of carbon atoms, preferably a C₁₋₆ alkyl selected from the group consisting of methyl, ethyl, propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl.

As a preferred embodiment of the present invention, the alkoxy refers to an alkyl ether radical, preferably a C₁₋₆ alkoxy selected from the group consisting of methoxy, ethoxy, propoxy, isopropoxy, n-butoxy, isobutoxy, sec-butoxy, tert-butoxy, n-pentyloxy, sec-pentyloxy, 1-ethylpropoxy, 2-methylbutoxy, tert-pentyloxy, 1,2-dimethylpropoxy, isopentyloxy, neopentyloxy, n-hexyloxy, isohexyloxy, sec-hexyloxy, tert-hexyloxy, neohexyloxy, 2-methylpentyloxy, 1,2-dimethylbutoxy, and 1-ethylbutoxy.

As a preferred embodiment of the present invention, the alkoxyalkyl refers to an alkyl on which one or more hydrogen atoms are substituted by alkoxy, preferably C₁₋₄ alkoxy-C₁₋₄ alkyl, further preferably methoxymethyl, methoxyethyl, methoxypropyl, methoxybutyl, ethoxymethyl, ethoxyethyl, ethoxypropyl, ethoxybutyl, propoxymethyl, propoxyethyl, propoxypropyl, propoxybutyl, butoxymethyl, butoxyethyl, butoxypropyl, butoxybutyl, and the like.

As a preferred embodiment of the present invention, the halogen is selected from the group consisting of fluorine, chlorine, bromine, and iodine; haloalkyl refers to an alkyl on which one or more of hydrogen atom is substituted by halogen; haloalkoxy refers to an alkoxy on which one or more of hydrogen atom is substituted by halogen; hydroxyalkyl refers to an alkyl on which one or more of hydrogen atom is substituted by hydroxyl; heterocycloalkyl refers to an alkyl on which one or more of hydrogen atom is substituted by heterocycle; and cycloalkylmethylene refers to an methyl on which one or more of hydrogen atom is substituted by cycloalkyl. As a preferred embodiment of the present invention, the fused aromatic ring is selected from the group consisting of naphthalene and fused heteroaromatic ring, wherein the fused heteroaromatic ring is formed by fusing an aromatic ring or a heteroaromatic ring with a heteroaromatic ring, and the heteroatom on the heteroaromatic ring is selected from the group consisting of nitrogen, oxygen, and sulfur, and there can be one or more heteroatoms.

As a preferred embodiment of the present invention, the fused heteroaromatic ring is selected from the group consisting of indazole, quinoline, isoquinoline, quinoxaline, indole, isoindole, cinnoline, quinazoline, phthalazine, purine, naphthyridine, pteridine, benzofuran, benzothiophene, benzoxazole, benzothiazole, benzisoxazole, benzisothiazole, benzoxadiazole, benzothiadiazole, benzotriazole, benzotriazine, benzimidazole, pyrazolopyrazine, pyrazinopyrimidine, pyrazinopyridazine, pyrazinotriazine, pyrazolopyrimidine, imidazolopyrimidine, triazolopyrimidine, pyrimidotriazine, pyrimidopyridazine, imidazolopyridazine, pyrazolopyridazine, triazolopyridazine, pyridazinotriazine, imidazolotriazine, pyrazolotriazine, triazolotriazine, imidazolopyridine, pyridopyridazine, pyrazolopyridine, pyridopyrimidine, pyridotriazine, oxazolopyridine, thiazolopyridine, isoxazolopyridine, isothiazolopyridine, oxadiazolopyridine, thiadiazolopyridine, furanopyridine, pyrrolopyridine, imidazolopyrazine, triazolopyrazine, oxazolopyrazine, thiazolopyrazine, isoxazolopyrazine, isothiazolopyrazine, oxadiazolopyrazine, thiadiazolopyrazine, furanopyrazine, pyrrolopyrazine, oxazolopyrimidine, thiazolopyrimidine, isoxazolopyrimidine, isothiazolopyrimidine, oxadiazolopyrimidine, thiadiazolopyrimidine, furanopyrimidine, pyrrolopyrimidine, oxazolopyridazine, thiazolopyridazine, isoxazolopyridazine, isothiazolopyridazine, oxadiazolopyridazine, thiadiazolopyridazine, furanopyridazine, pyrrolopyridazine, oxazolotriazine, thiazolotriazine, isoxazolotriazine, isothiazolotriazine, oxadiazolotriazine, thiadiazolotriazine, furanotriazine, and pyrrolotriazine. The naphthyridine is selected from the group consisting of 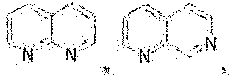 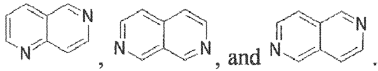 The imidazolopyridine is selected from the group consisting of 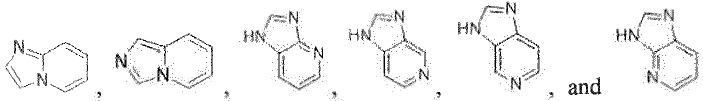 The imidazolopyrazine is selected from the group consisting of 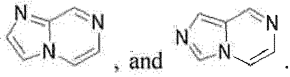 The triazolopyrazine is selected from the group consisting of 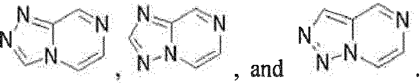 The pyrazolopyrimidine is selected from the group consisting of 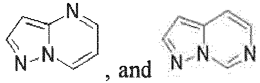 The imidazolopyrimidine is selected from the group consisting of 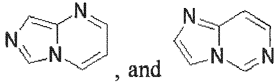 The triazolopyrimidine is selected from the group consisting of 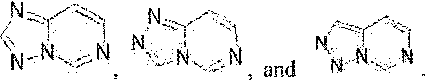 The imidazolopyridazine is selected from the group consisting of 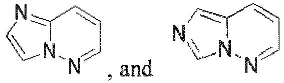 The thiazolopyridazine is selected from the group consisting of 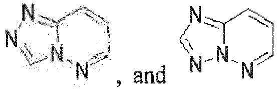 The imidazolotriazine is selected from the group consisting of 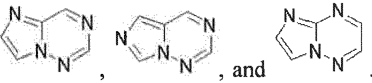 The pyridopyridazine is selected from the group consisting of 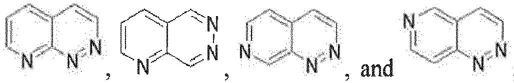 The pyrazolopyridine is selected from the group consisting of 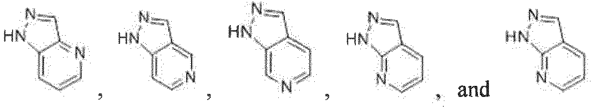 The pyridopyrimidine is selected from the group consisting of 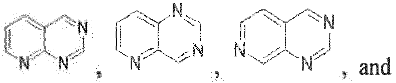 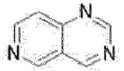 The pyridotriazine is selected from the group consisting of 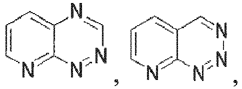 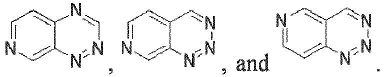 The pyrimidotriazine is selected from the group consisting of

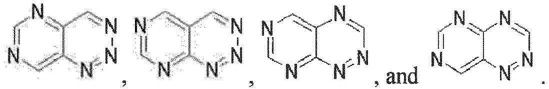

As a preferred embodiment of the present invention, the heterocycle of the heterocycloalkyl is selected from 4- to 10-membered heterocycle, wherein the 4- to 10-membered heterocycle may be selected from the group consisting of 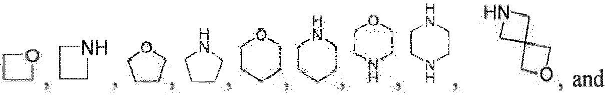 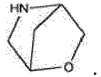 The aryl is selected from phenyl. The heteroaryl is selected from 5- to 12-membered heteroaryl, wherein the 5- to 12-membered heteroaryl may be selected from the group consisting of

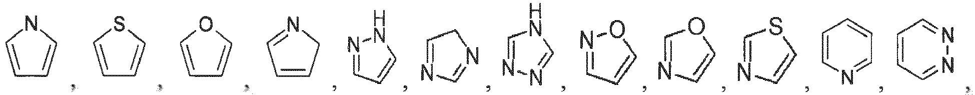

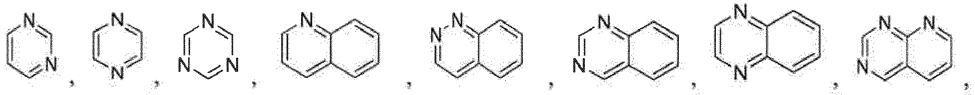

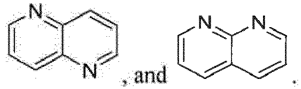

As a preferred embodiment of the present invention, the "alkylene" refers to an alkyl that can be independently substituted by 0 to 2 substituents where the valence allows; the "cyano" refers to -CN, the "hydroxyl" refers to -OH, and the "sulfonyl" refers to -SO₂.

As a preferred embodiment of the present invention, the "heterocycle" refers to a saturated or unsaturated heterocycle containing one or more heteroatoms (nitrogen, oxygen and sulfur). The "aromatic ring" refers to a phenyl ring. The "heteroaromatic ring" refers to a heterocycle with 4n+2 π electrons in a closed conjugated system, which may be, e.g., furan, thiophene, tetrahydropyrrole, dihydropyrazole, imidazole, thiazole, isothiazole, thiadiazole, oxazole, oxadiazole, isoxazole, piperidine, piperazine, pyridazine, pyrazine, triazine, pyrimidine, morpholine, or pyridine. The expression of "more" (elements) refers to two, three or four elements.

As a preferred embodiment of the present invention, the cycloalkyl is selected from C₃₋₆ cycloalkyl, wherein the C₃₋₆ cycloalkyl may be selected from the group consisting of cyclopropyl, cyclobutyl, cyclopentyl, and cyclohexyl.

As a preferred embodiment of the present invention, A is selected from the group consisting of phenyl ring,

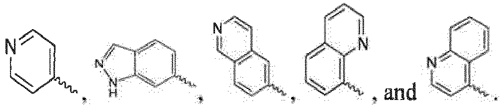

As a preferred embodiment of the present invention, the compound or stereoisomer, tautomer, pharmaceutically acceptable salt thereof is represented by Formula (I), wherein:

A is selected from the group consisting of phenyl ring, 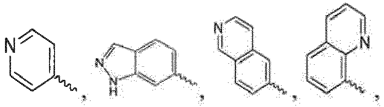 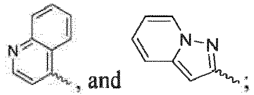
R¹ is selected from the group consisting of hydrogen, fluorine, chlorine, bromine, cyano, difluoroethyl, and trifluoroethyl;
m is 0, 1, or 2;
W is O or NH;
R² is selected from the group consisting of chlorine, cyano, trifluoromethyl, methoxy, and methoxymethyl;
q is 0 or 1;
R³ is fluorine, methyl, oxo (=O), hydroxyl, fluoromethyl, and methoxyethyl;
R⁴ is oxetan-2-ylmethyl;
X is CH or N;
Y is CH or N;
R⁵ is selected from the group consisting of methyl, ethyl, cyclopropylmethyl, fluoroethyl, and methoxyethyl;
Z¹ is CH;
Z² is CH or CF; and
Z³ is CH, N, or CF.

As a preferred embodiment of the present invention, the compound or stereoisomer, tautomer, pharmaceutically acceptable salt thereof is represented by Formula (III), wherein:

A is selected from the group consisting of phenyl ring, 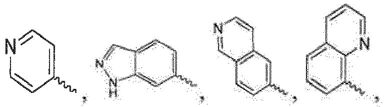 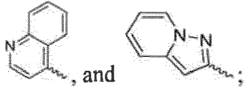
R¹ is selected from the group consisting of hydrogen, fluorine, chlorine, bromine, cyano, difluoroethyl, and trifluoroethyl;
m is 0, 1, or 2;
W is O or NH;
q is 0 or 1;
R³ is fluorine, methyl, oxo (=O), hydroxyl, fluoromethyl, or methoxyethyl;
R⁴ is oxetan-2-ylmethyl;
X is CH or N;
Y is CH or N;
R⁵ is methyl, ethyl, cyclopropylmethyl, fluoroethyl, or methoxyethyl;
Z¹ is CH;
Z² is CH or CF; and
Z³ is CH, N, or CF.

As a preferred embodiment of the present invention, the compound or stereoisomer, tautomer, pharmaceutically acceptable salt thereof is represented by Formula (II), wherein:

A is selected from the group consisting of phenyl ring, 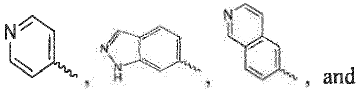 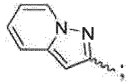
R¹ is selected from the group consisting of hydrogen, fluorine, chlorine, bromine, cyano, difluoroethyl, and trifluoroethyl;
m is 0, 1, or 2;
q is 0;
R⁴ is oxetan-2-ylmethyl;
X is CH or N;
Y is CH or N;
R⁵ is selected from the group consisting of methyl, ethyl, cyclopropylmethyl, fluoroethyl, and methoxyethyl;
Z¹ is CH;
Z² is CH or CF; and
Z³ is CH, N, or CF.

As a preferred embodiment of the present invention, in the compound or stereoisomer, tautomer, pharmaceutically acceptable salt thereof, when the carbon atom connected with R⁵ is a chiral carbon atom, the chiral carbon atom is in S configuration and/or R configuration, preferably in S configuration.

As a preferred embodiment of the present invention, the compound or pharmaceutically acceptable salt thereof is selected from the group consisting of:

| | **Structural formula** | | **Structural formula** |
|---|---|---|---|
| 1 | 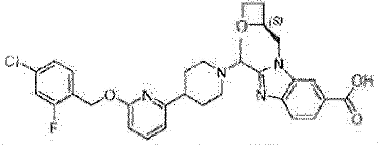 | 27 | 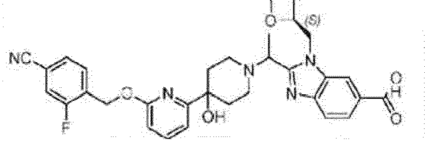 |
| 2 | 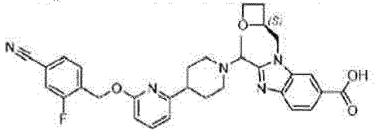 | 28 | 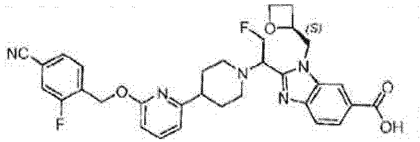 |
| 3 | 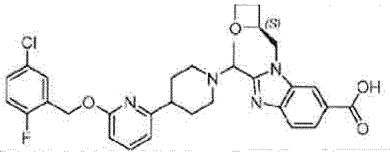 | 29 | 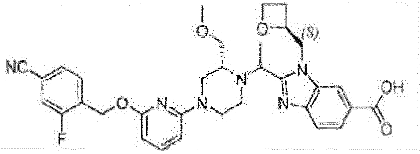 |
| 4 | 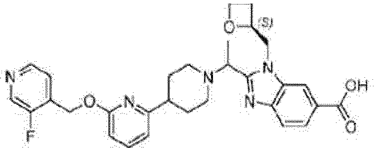 | 30 | 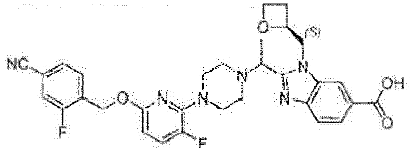 |
| 5 | 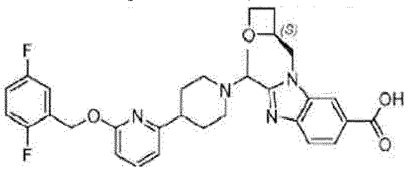 | 31 | 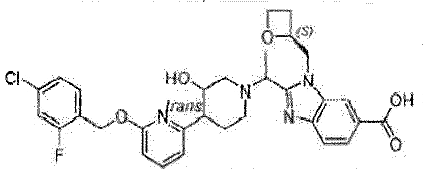 |
| 6 | 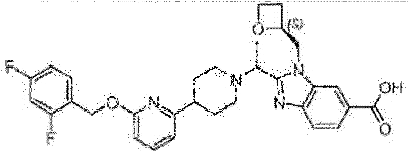 | 32 | 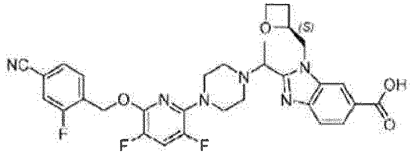 |
| 7 | 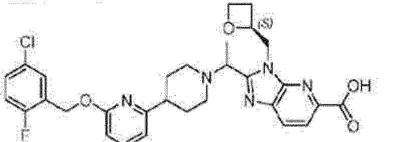 | 33 | 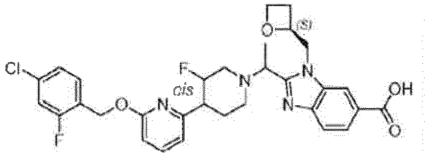 |
| 8 | 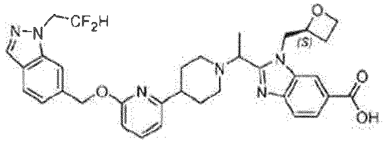 | 34 | 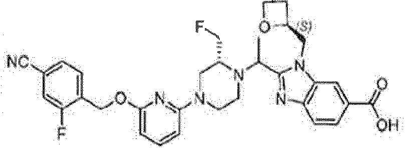 |
| 9 | 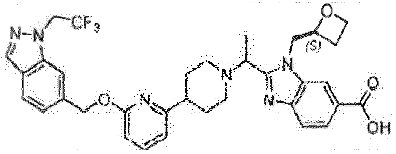 | 35 | 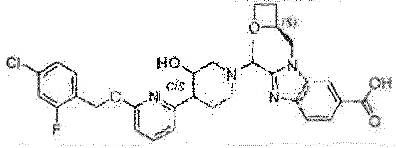 |
| 10 | 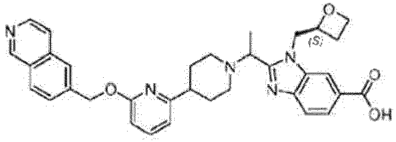 | 36 | 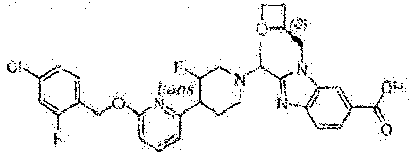 |
| 11 | 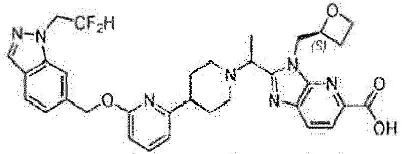 | 37 | 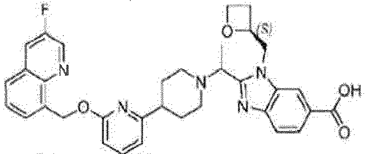 |
| 12 | 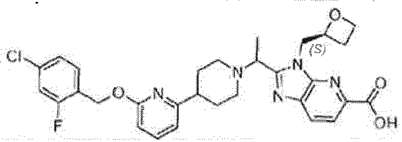 | 38 | 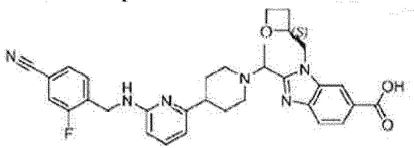 |
| 13 | 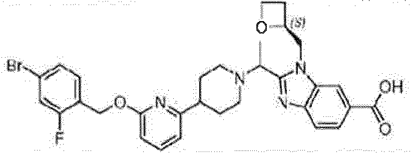 | 39 | 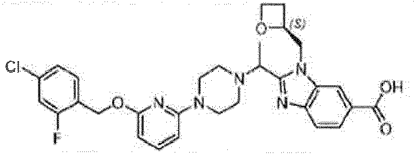 |
| 14 | 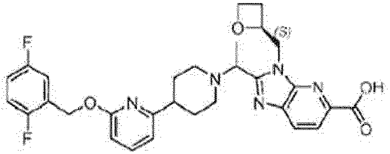 | 40 | 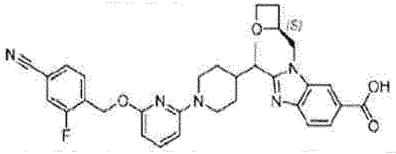 |
| 15 | 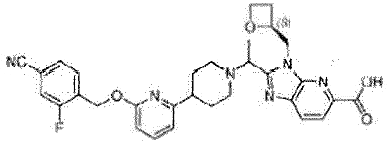 | 41 | 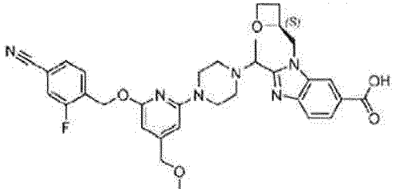 |
| 16 | 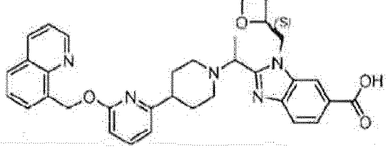 | 42 | 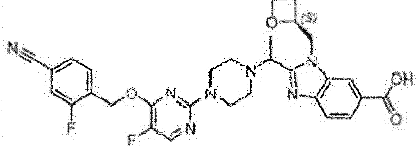 |
| 17 | 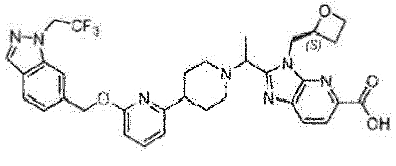 | 43 | 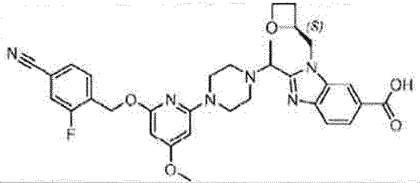 |
| 18 | 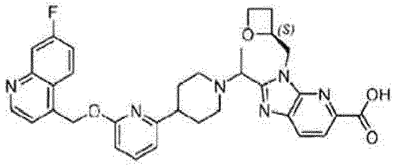 | 44 | 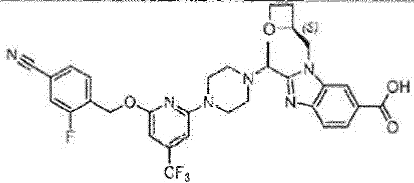 |
| 19 | 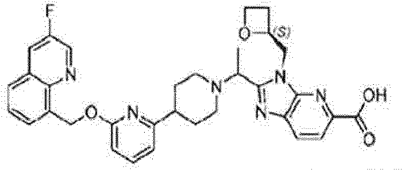 | 45 | 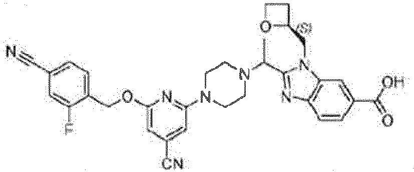 |
| 20 | 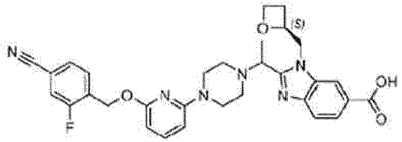 | 46 | 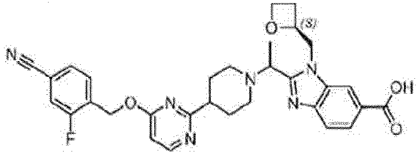 |
| 21 | 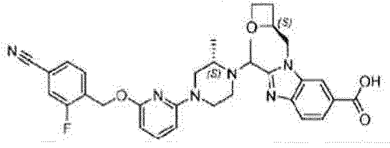 | 47 | 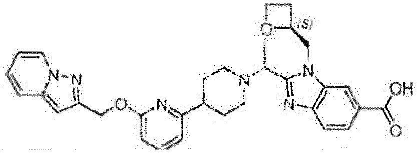 |
| 22 | 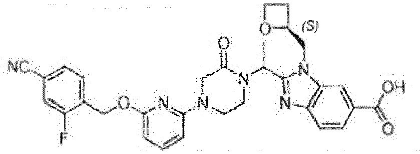 | 48 | 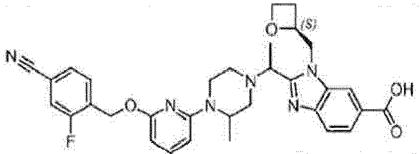 |
| 23 | 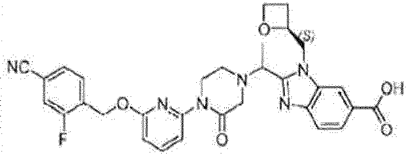 | 49 | 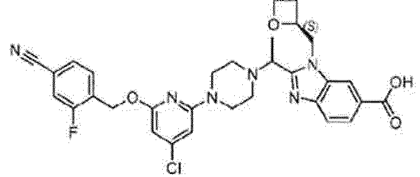 |
| 24 | 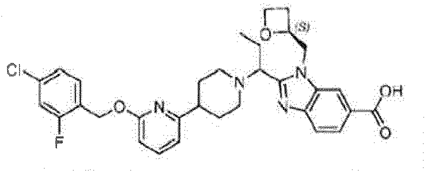 | 50 | 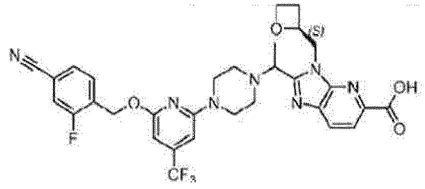 |
| 25 | 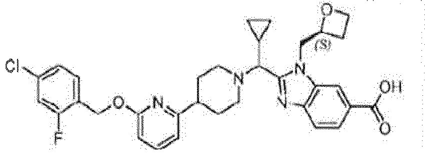 | 51 | 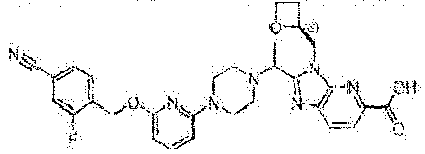 |
| 26 | 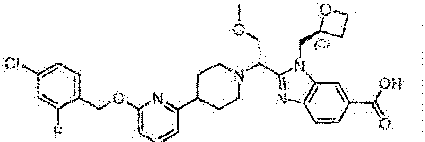 | 52 | 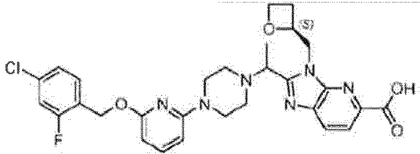 |

As a preferred embodiment of the present invention, the compound or pharmaceutically acceptable salt thereof is selected from the group consisting of:

| | **Structural formula** | | **Structural formula** |
|---|---|---|---|
| 1A | 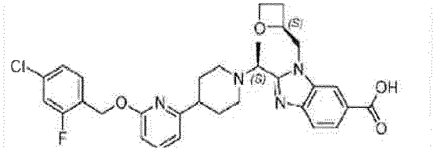 | 27 | 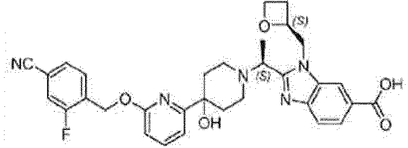 |
| 1B | 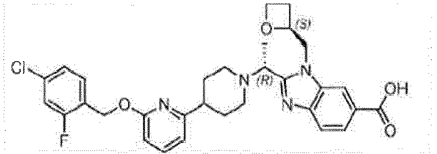 | 28 | 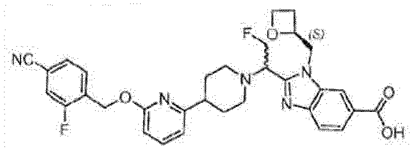 |
| 2A | 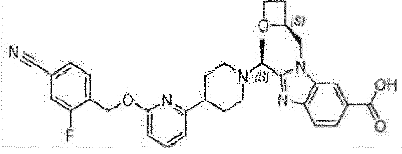 | 29 | 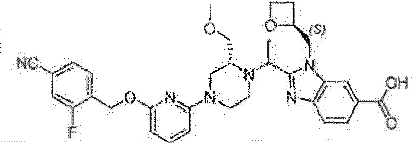 |
| 2B | 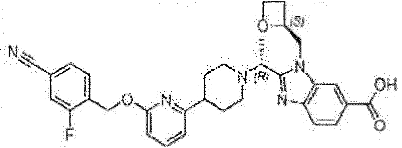 | 30 | 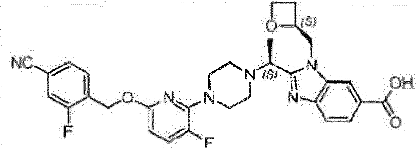 |
| 3 | 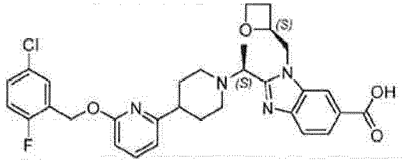 | 31 | 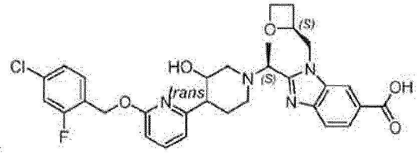 |
| 4 | 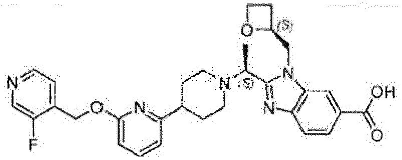 | 32 | 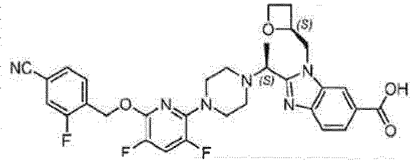 |
| 5 | 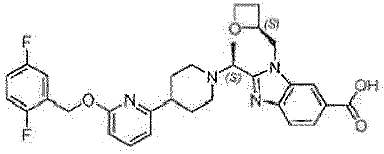 | 33 | 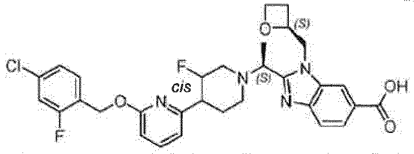 |
| 6 | 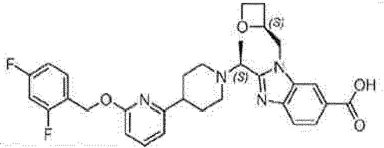 | 34 | 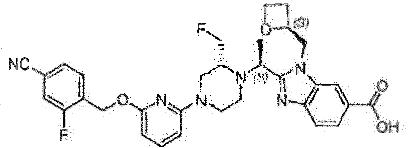 |
| 7 | 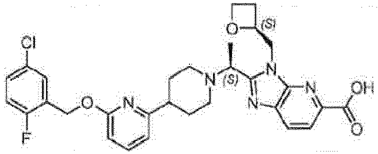 | 35 | 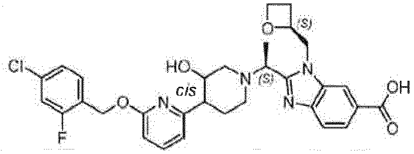 |
| 8 | 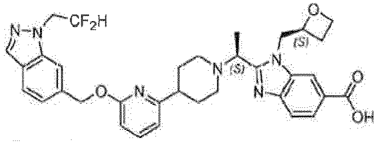 | 36 | 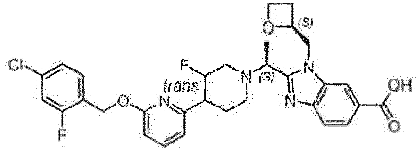 |
| 9 | 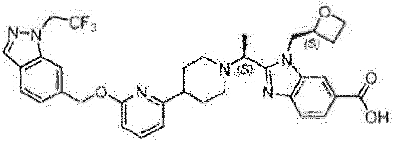 | 37 | 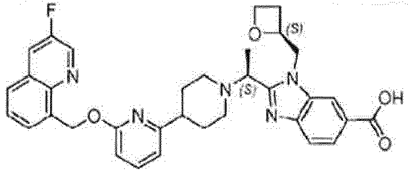 |
| 10 | 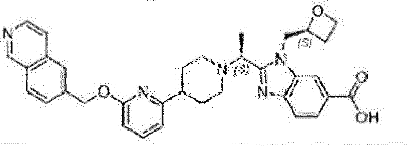 | 38 | 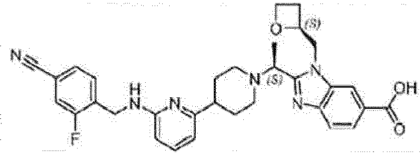 |
| 11 | 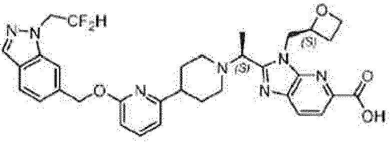 | 39A | 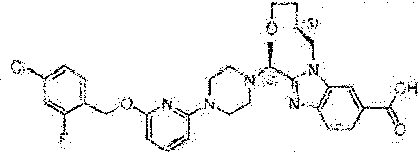 |
| 12 | 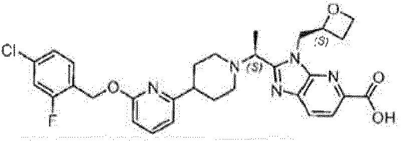 | 39B | 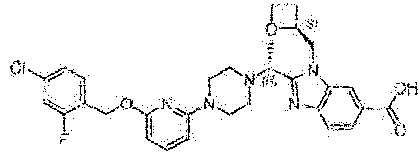 |
| 13 | 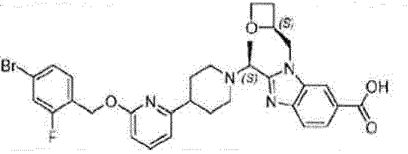 | 40 | 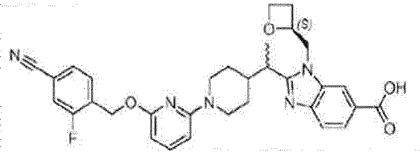 |
| 11 | 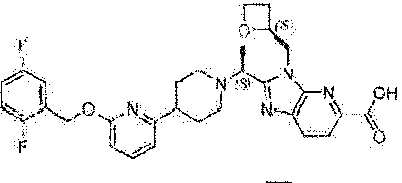 | 41 | 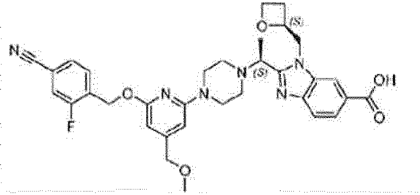 |
| 15 | 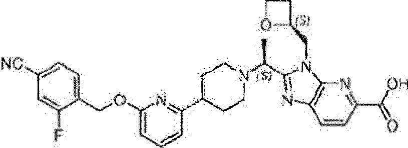 | 42 | 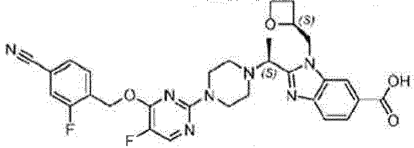 |
| 16 | 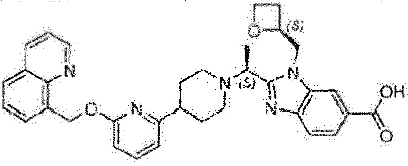 | 43 | 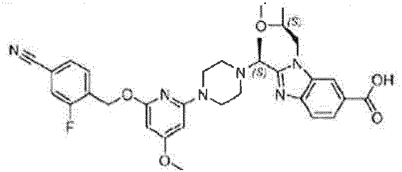 |
| 17 | 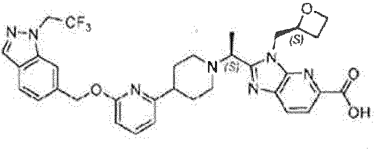 | 44 | 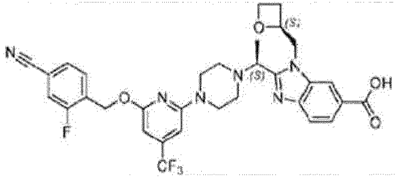 |
| 18 | 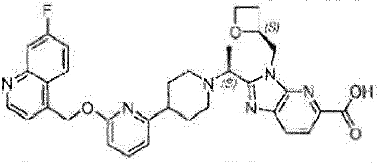 | 45 | 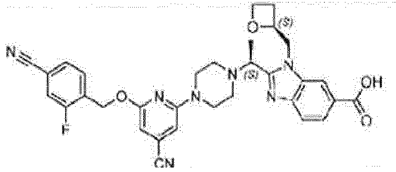 |
| 19 | 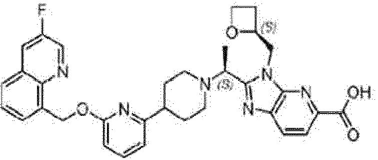 | 46 | 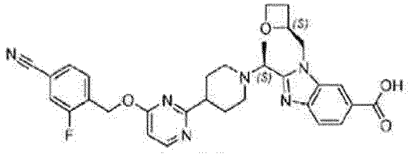 |
| 20A | 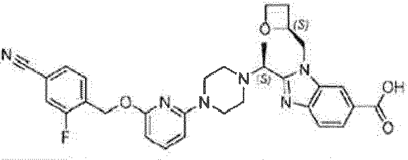 | 47 | 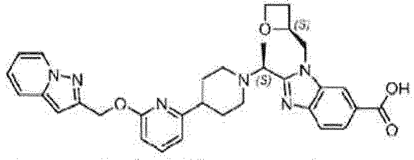 |
| 20B | 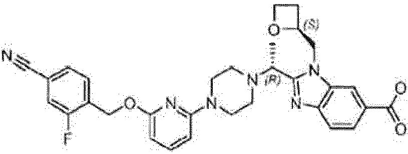 | 48A | 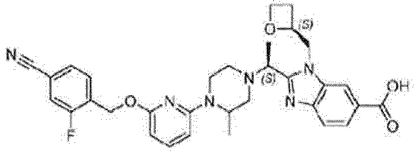 |
| 21 | 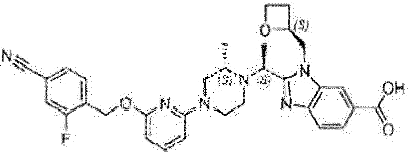 | 48B | 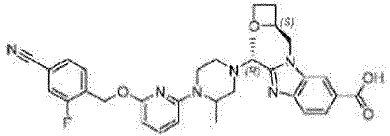 |
| 22 | 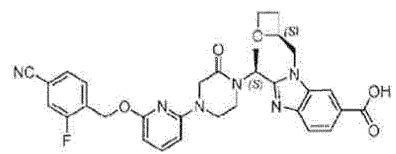 | 49 | 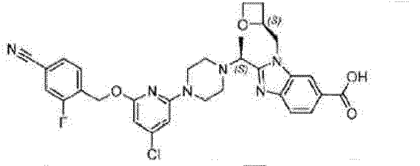 |
| 23 | 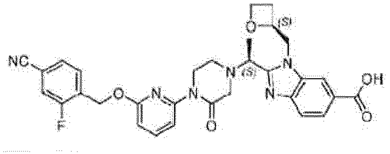 | 50 | 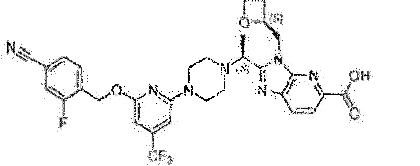 |
| 24A | 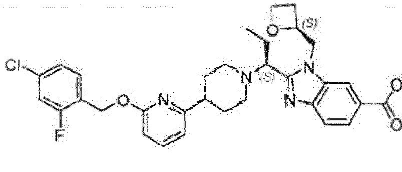 | 51A | 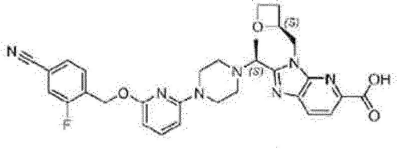 |
| 24B | 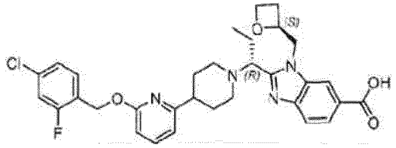 | 51B | 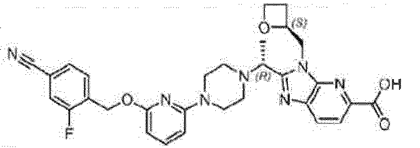 |
| 25 | 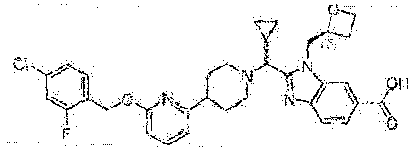 | 52A | 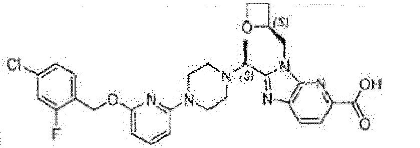 |
| 26 | 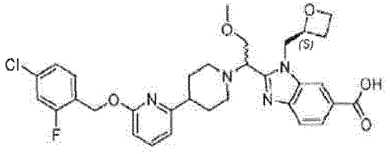 | 52B | 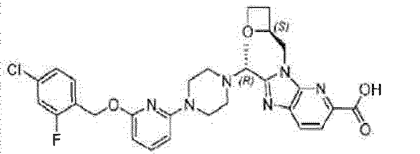 |

As a preferred embodiment of the present invention, the pharmaceutically acceptable salt refers to a salt that is prepared from the compound and a pharmaceutically acceptable acid or base.

As a preferred embodiment of the present invention, one or more hydrogen atoms of the compound are substituted with isotopic deuterium.

Another object of the present invention is to provide a pharmaceutical composition comprising the aforementioned compound of formula (I), or a stereoisomer, tautomer, or pharmaceutically acceptable salt thereof, and one or more pharmaceutically acceptable carriers.

Another object of the present invention is to provide the use of the compound of formula (I) or a stereoisomer, tautomer or pharmaceutically acceptable salt thereof in the manufacture of a medicament for treating GLP-1-related diseases, specifically diabetes mellitus-related diseases.

Unless otherwise stated, the following terms and phrases used herein are intended to have the following meanings. A particular term or phrase should not be considered indefinite or unclear in the absence of a particular definition, but should be understood according to its ordinary meaning. When a trade name appears herein, it is intended to refer to its corresponding product or its active ingredient. The term "pharmaceutically acceptable" as used herein refers to those compounds, materials, compositions, and/or dosage forms which are, within the scope of sound medical judgment, suitable for contact with the tissues of human beings or animals without excessive toxicity, irritation, allergic response, or other problems, or complications, and is commensurate with a reasonable benefit/risk ratio.

The term "pharmaceutically acceptable salt" refers to the salt of the compounds of the present invention prepared from the compounds having specific substituents of the present invention and pharmaceutically acceptable acids or bases.

In addition to the salt form, the compounds provided by the present invention also comprise the prodrug form. The prodrug of the compounds described here can easily undergo chemical changes under physiological conditions, thus being converted into the compounds of the present invention. Furthermore, the prodrug can be converted to the compounds of the present invention by chemical or biochemical methods *in vivo.*

Certain compounds of the present invention may be present in a nonsolvated form or in a solvated form, including a hydrated form. In general, the solvated form and the nonsolvated form both are included within the scope of the present invention.

The compounds of the present invention may exist in specific geometric or stereoisomeric forms. It is contemplated that all such compounds of the present invention, including cis and trans isomers, (-)-and (+)- enantiomers, (R)- and (S)- enantiomers, diastereoisomers, (D)-isomers, (L)-isomers, and racemic mixtures and other mixtures thereof, such as enantiomerically or diastereomerically enriched mixtures, are all within the scope of the present invention. Additional asymmetric carbon atom(s) may be present in a substituent such as an alkyl group. All these isomers, as well as mixtures thereof, are included within the scope of the present invention.

The optically active (R)- and (S)-isomers, as well as the D and L isomers, can be prepared by chiral synthesis or chiral reagents or other conventional techniques. If one wishes to obtain an enantiomer of a compound of the present invention, it can be prepared by asymmetric synthesis or derivatization with chiral auxiliaries, where the resulting diastereomeric mixture is separated and the auxiliary group is cleaved to provide the pure desired enantiomer. Alternatively, when the compound contains a basic functional group (e.g. amino) or an acidic functional group (e.g. carboxyl) in the molecule, the pure enantiomer can be obtained by forming a salt of the diastereoisomer by the molecule with a suitable optically active acid or base, then separating the diastereoisomer by conventional methods well known in the art, and subsequently recycling. Additionally, the separation of the enantiomers from the diastereoisomers is generally accomplished by chromatography, said chromatography employing a chiral stationary phase and optionally in combination with chemical derivatization (e.g., the generation of carbamate from amine).

The atoms in the molecules of the compounds of the present invention may be isotopes, and derivatization by isotopes can generally result in prolonged half-life, reduced clearance, improved metabolic stability, increased *in vivo* activity, and the like. Furthermore, the present disclosure further comprises an embodiment in which at least one atom is substituted by an atom having the same atomic number (proton number) and different mass numbers (sum of proton number and neutron number). Examples of isotopes included in the compounds of the present invention involve hydrogen atom, carbon atom, nitrogen atom, oxygen atom, phosphorus atom, sulfur atom, fluorine atom, chlorine atom, which include ²H, ³H, ¹³C, ¹⁴C, ¹⁵N, ¹⁷O, ¹⁸O, ³¹P, ³²P, ³⁵S, ¹⁸F, and ³⁶Cl, respectively. In particular, radioactive isotopes that emit radiation with their decay, e.g., ³H or ¹⁴C, can be used in the regional anatomy examination of pharmaceutical formulations or compounds *in vivo.* Stable isotopes neither decay or change with their amount, nor are they radioactive, so they can be used safely. When the atoms constituting the molecules of the compounds of the present invention are isotopes, the isotopic conversion can be performed according to common methods by replacing the reagent used in the synthesis with those containing the corresponding isotopes.

The compounds of the present invention may contain atomic isotopes in unnatural proportions at one or more of the atoms constituting the compound. For example, the compounds can be labeled with radioactive isotopes such as deuterium (²H), iodine-125 (¹²⁵I), or C-14 (¹⁴C). All transformations of the isotopic composition of the compounds of the present invention, whether radioactive or not, are included in the scope of the present invention.

Further, one or more hydrogen atoms of the compounds of the present invention may be substituted by isotope deuterium (²H), and the deuterium-substituted compounds of the present invention has the advantages of prolonged half-life, reduced clearance, improved metabolic stability, increased activity *in vivo,* and the like.

The preparation method of the isotopic derivatives generally includes phase transfer catalytic method. For example, preferred deuteration method employs phase transfer catalysts (e.g., tetraalkylammonium salts, NBu₄HSO₄). Higher deuterium can be introduced by using a phase transfer catalyst to exchange methylene protons of diphenylmethane compounds than using deuterated silane (such as deuterated triethyl silane) in the presence of acid (such as methanesulfonic acid) or using deuterated sodium borate with Lewis acid such as aluminum trichloride for reduction.

The term "pharmaceutically acceptable carrier" refers to any formulation carrier or medium that is capable of delivering an effective amount of the active substance of the invention, does not interfere with the biological activity of the active substance, and has no toxic side effects on the host or patient. Representative carriers include water, oil, vegetables and minerals, cream base, lotion base, ointment base, etc. These bases include suspending agent, tackifier, penetration enhancer, etc. Their formulations are well known to those skilled in the cosmetic or topical drugs field. For additional information on carriers, reference can be made to Remington: The Science and Practice of Pharmacy, 21st Ed., Lippincott, Williams & Wilkins (2005), the contents of which are incorporated herein by reference.

The term "excipient" generally refers to the carrier, diluent, and/or medium required for the formulation of an effective pharmaceutical composition.

For a drug or pharmacologically active agent, the term "effective amount" or "therapeutically effective amount" refers to a sufficient amount of a drug or agent that is non-toxic but capable of achieving the desired effect. For the oral dosage form of the present invention, the "effective amount" of an active substance in a composition refers to the amount required to achieve the desired effect when used in combination with another active substance in the composition. The determination of the effective amount varies from person to person, depending on the age and general conditions of the subject, as well as on the specific active substance. The appropriate effective amount in individual cases can be determined by those skilled in the art based on routine experiments.

The terms "active ingredient", "therapeutic agent", "active substance" or "active agent" refer to a chemical entity that can effectively treat disorders, diseases, or conditions of a subject.

The term "optional" or "optionally" refers to the situation where the subsequently described event or circumstance may, but is not required to, occur, and the description includes situations in which said event or circumstance occurs as well as situations in which said event or circumstance does not occur.

" 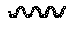 " indicates a linkage.

The compounds of the present invention can be prepared by a variety of synthetic methods known to those skilled in the art, including the specific embodiments recited below, embodiments resulting from their combination with other chemical synthesis methods, and equivalents known to those skilled in the art. Preferred embodiments include, but not limited to, embodiments of the present invention.

### BRIEF DESCRIPTION OF THE DRAWINGS

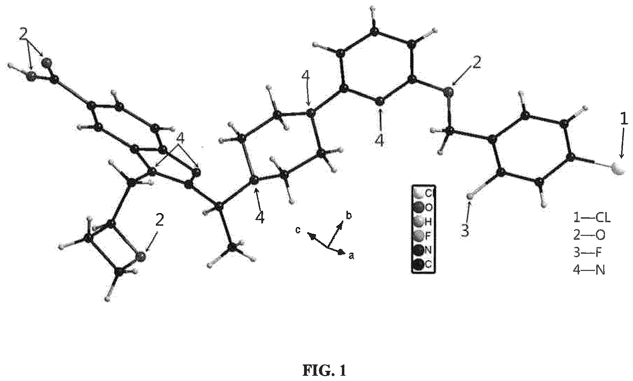

Figure 1 is a schematic diagram of the single crystal structure of 39A in Example 39.

### DETAILED DESCRIPTION

The invention is described in further detail below in conjunction with the embodiments, but the embodiments of the invention are not limited thereto.

The structure of the compounds are determined by nuclear magnetic resonance (NMR) or mass spectrometry (MS). The NMR shifts (δ) are given in 10⁻⁶ (ppm). NMR is measured using a Bruker AVANCE-III NMR spectrometer with deuterated dimethyl sulfoxide (DMSO-d₆) and deuterated chloroform (CDCl₃) as the solvent for determination and tetramethylsilane (TMS) as the internal standard.

MS is measured with an ISQ EC mass spectrometer (manufacturer: Thermo, model: ISQ EC).

High performance liquid chromatography (HPLC) analysis is performed using Thermo U3000 HPLC DAD HPLC high performance liquid chromatograph and Agilent 1260 high performance liquid chromatograph.

CombiFlash rapid preparation instrument is used with CombiFlash Rf+ LUMEN (TELEDYNE ISCO).

HSGF₂₅₄ or GF₂₅₄ silica gel plates from Yantai Yinlong are used as thin layer chromatography silica gel plate. The specification of silica gel plate used for thin layer chromatography (TLC) is 0.17-0.23 mm, and the specification of silica gel plate used for thin layer chromatography for the separation and purification of products is 0.4-0.5 mm.

The silica gel column chromatography generally uses 100-200 mesh silica gel from Rushan Shangbang silica gel as the carrier.

Agents: LDA, lithium diisopropylamide; THF, tetrahydrofuran; TEA, triethylamine; 1,4-dioxane; BH₃, borane; EDCl, 1-ethyl-3-(3-dimethyllaminopropyl)carbodiimide hydrochloride; HOBT, 1-hydroxybenzotriazole; DIPEA, *N*,*N-*diisopropylethylamine; DMF, *N*,*N-*dimethylformamide; K₂CO₃, potassium carbonate; DMSO, dimethyl sulfoxide; EtOH, ethanol; NaH, sodium hydride; toluene; r.t., room temperature; PdCl₂(dppf), 1,1'-bis(diphenylphosphino)ferrocene palladium dichloride; Pd(OAc)₂, palladium acetate; PhMe, toluene; HCl/dioxane, hydrochloric acid in dioxane; NaCO₃, sodium carbonate; MeOH, methanol; DCE, dichloroethane; NCS, N-chlorosuccinimide; TfOH, trifluoromethanesulfonic acid; TFA, trifluoroacetic acid; AcOH, acetic acid; (CH₂O)ₙ, polyformaldehyde; CDI, *N*,*N*'-carbonyldiimidazole; LAH, lithium aluminum hydride; PPA, polyphosphoric acid; TsOH, p-toluenesulfonic acid; NBS, N-bromosuccinimide; BPO, benzoyl peroxide; HATU, 2-(7-azabenzotriazol-1-yl)-*N*,*N*,*N',N*'-tetramethyluronium hexafluorophosphate; KOH.H₂O, potassium hydroxide monohydrate; NaOH, sodium hydroxide; CS₂CO₃, cesium carbonate; AIBN, azodiisobutyronitrile; MeCN, acetonitrile; DEAD, diethyl azodicarboxylate; DAST, diethylaminosulphur trifluoride; PPH₃, triphenylphosphine; DCM, dichloromethane; DMAP, 4-dimethylaminopyridine; TBD, 1,5,7-triazidobicyclo, 4,4,0) decan-5-ene; DIEA, *N,N-*diisopropylethylamine; BINAP, 2,2'-bis(diphenylphosphino)-1,1'-binaphthalene; NMP, N-methylpyrrolidone).

### Example 1. 2-((S)-1-(4-(6-((4-chloro-2-fluorobenzyl)oxy)pyridin-2-yl)piperidin-1-yl)ethyl)-1-(((S)-oxetan-2-yl)methyl)-1H-benzo[d]imidazol-6-carboarylic acid (1A) and 2-((R)-1-(4-(6-((4-chloro-2-fluorobenzyl)oxy)pyridin-2-yl)piperidin-1-yl)ethyl)-1-(((S)-oxetan-2-yl)methyl)-1H-benzo[d]imidazol-6-carboxylic acid (1B)

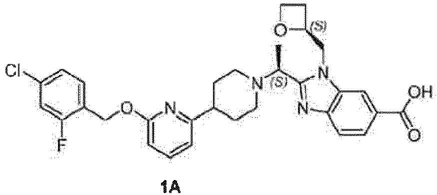

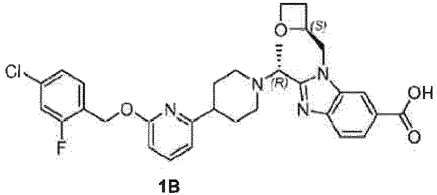

The specific synthetic route is as follows.

### Step A: Synthesis of 2-chloro-6-((4-chloro-2-fluorobenzyl)oxy)pyridine

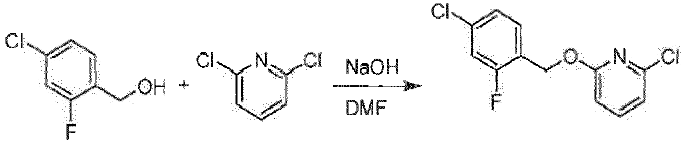

(4-Chloro-2-fluorophenyl)methanol (1.6 g, 10.0 mmol), 2,6-dichloropyridine (1.627 g, 11.0 mmol), and sodium hydroxide (1.2 g, 30.0 mmol) were dissolved in *N*,*N-*dimethylformamide (30.0 mL) and stirred at 120°C for 12 hours.

After the completion of the reaction, the reaction solution was poured into 100 mL of ice water. The mixed solution was extracted with ethyl acetate (50 mL × 3 times). The organic phases were combined, dried, and concentrated to give a crude product, which was purified by silica gel column chromatography (eluent: n-hexane/ethyl acetate = 10/1) to obtain 1.2 g of white solid 2-chloro-6-((4-chloro-2-fluorobenzyl)oxy)pyridine (yield: 44.2%). LC-MS: RT = 2.29 min, [M+H]⁺ = 272.04.

### Step B: Synthesis of tert-butyl 6-((4-chloro-2-fluorobenzyl)oxy)-3',6'-dihydro-[2,4'-bipyridin]-1'(2'H)-carboxylate

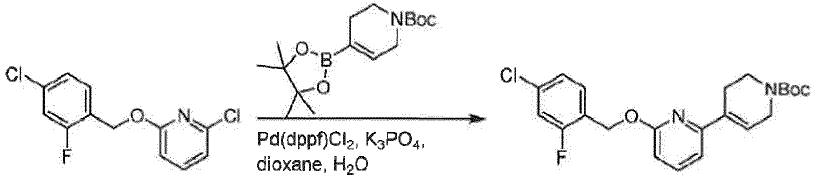

2-Chloro-6-((4-chloro-2-fluorobenzyl)oxy)pyridine (1.2 g, 4.4 mmol), tert-butyl 4-(4,4,5,5-tetramethyl-1,3,2-dioxaborinan-2-yl)-3,6-dihydropyridin-1(2*H*)-carboxylate (1.3 g, 4.4 mmol), [1,1-bis (diphenylphosphine)ferrocene]palladium dichloride (323.0 mg), potassium phosphate (1.8 g, 8.8 mmol) were dissolved in a mixture of dioxane (20 mL) and water (4 mL). The temperature was raised to 75°C and the mixed solution was stirred for 8 hours.

After the completion of the reaction, the reaction solution was poured into 20 mL of water. The mixed solution was extracted with ethyl acetate (20 mL × 3 times). The organic phases were combined, dried, and concentrated to give a crude product, which was purified by column chromatography (eluent: n-hexane/ethyl acetate = 10/1) to obtain 1.2 g of light yellow solid tert-butyl 6-((4-chloro-2-fluorobenzyl)oxy)-3',6'-dihydro-[2,4'-bipyridin]-1'(2'*H*)-carboxylate (yield: 65.2%). LC-MS: RT = 2.43 min, [M+H]⁺ = 419.24.

### Step C: Synthesis of tert-butyl 4-(6-((4-chloro-2-fluorobenzyl)oxy)pyridin-2-yl)piperidin-1-carboxylate

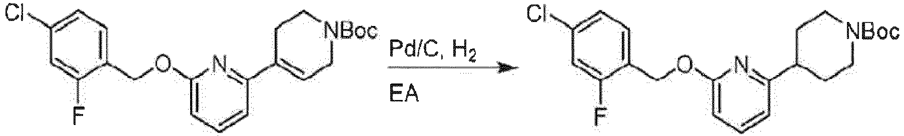

Tert-butyl 6-((4-chloro-2-fluorobenzyl)oxy)-3',6'-dihydro-[2,4'-bipyridin]-1'(2'*H*)-carboxylate (1.1 g, 2.6 mmol) was dissolved in ethyl acetate (55.0 mL), and palladium/carbon (275.0 mg) was added to the mixed solution. The reaction was carried out at room temperature under hydrogen (one standard atmospheric pressure) for 6 hours.

After the completion of the reaction, the reaction solution was filtered and the solid was spin-dried to obtain 1.2 g of colorless oily product tert-butyl 4-(6-((4-chloro-2-fluorobenzyl)oxy)pyridin-2-yl)piperidin-1-carboxylate (yield: 80.0%). LC-MS: RT = 2.43 min, [M+H-56]⁺ = 365.20.

### Step D: Synthesis of 2-((4-chloro-2-fluorobenzyl)oxy)-6-(piperidin-4-yl)pyridine

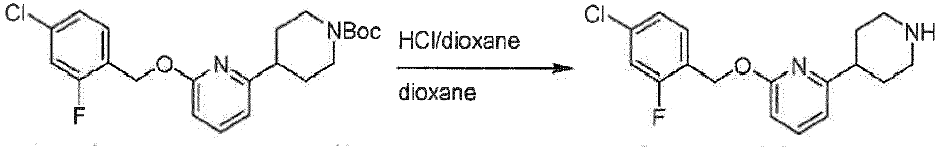

Tert-butyl 4-(6-((4-chloro-2-fluorobenzyl)oxy)pyridin-2-yl)piperidin-1-carboxylate (1.1 g, 4.5 mmol) was dissolved in dioxane (5.0 mL), and hydrochloric acid in dioxane (35.0 mL) was added to the mixed solution under ice bath. The reaction was carried out at room temperature for 2 hours.

After the completion of the reaction, a solid was precipitated and the reaction solution was filtered and the solid was spin-dried to obtain 500 mg of light yellow solid 2-((4-chloro-2-fluorobenzyl)oxy)-6-(piperidin-4-yl)pyridine.

### Step E: Synthesis of ethyl 2-(4-(6-((4-chloro-2-fluorobenzyl)oxy)pyridin-2-yl)piperidin-1-yl)propionate

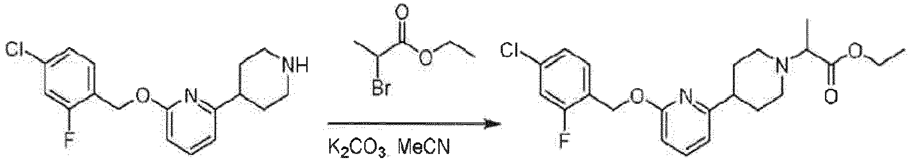

2-((4-Chloro-2-fluorobenzyl)oxy)-6-(piperidin-4-yl)pyridine (500.0 mg, 1.6 mmol), ethyl 2-bromopropionate (309.3 mg, 1.7 mmol), and potassium carbonate (441.0 mg, 3.2 mmol) were dissolved in acetonitrile (8.0 mL) at room temperature, and the mixed solution was stirred at room temperature overnight.

After the completion of the reaction, the reaction was quenched with saturated sodium chloride, and the reaction solution was extracted with ethyl acetate (30 mL × 3 times). The organic phases were combined, dried, and concentrated to give a crude product, which was purified by silica gel column chromatography (eluent: ethyl acetate/n-hexane = 1/3) to obtain 353.0 mg of white solid ethyl 2-(4-(6-((4-chloro-2-fluorobenzyl)oxy)pyridin-2-yl)piperidin-1-yl)propionate (yield: 52.5%). LC-MS: RT = 1.82 min, [M+H]⁺ = 421.25.

### Step F: Synthesis of 2-(4-(6-((4-chloro-2-fluorobenzyl)oxy)pyridin-2-yl)piperidin-1-yl)propionic acid

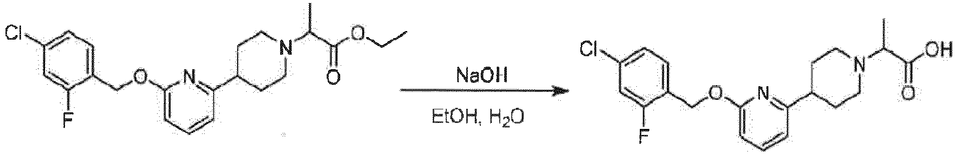

Ethyl 2-(4-(6-((4-chloro-2-fluorobenzyl)oxy)pyridin-2-yl)piperidin-1-yl)propionate (323 mg, 0.7 mmol) was dissolved in a solvent mixture (3.6 mL, ethanol/water = 5/1) at room temperature, and sodium hydroxide (121.6 mg, 3.0 mmol) was added to the mixed solution, which was then stirred at room temperature for 12 hours.

After the completion of the reaction, 0.5 N of diluted hydrochloric acid was added to the reaction solution, and the pH was adjusted to 2 to precipitate a white solid. The reaction solution was filtered and the solid was spin-dried to obtain 302.0 mg of white solid 2-(4-(6-((4-chloro-2-fluorobenzyl)oxy)pyridin-2-yl)piperidin-1-yl)propionic acid. LC-MS: RT= 1.84 min, [M+H]⁺ = 393.21.

### Step G: Synthesis of methyl 4-(2-(4-(6-((4-chloro-2-fluorobenzyl)oxy)pyridin-2-yl)piperidin-1-yl)propionamido)-3-((((S)-oxetan-2-yl)methyl)amino)benzoate

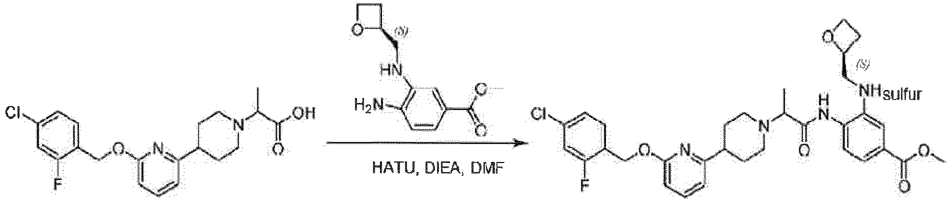

2-(4-(6-((4-Chloro-2-fluorobenzyl)oxy)pyridin-2-yl)piperidin-1-yl)propionic acid (262.0 mg, 0.7 mmol) was dissolved in *N*,*N-*dimethylformamide (2.5 mL), followed by adding 2-(7-azabenzotriazol-1-yl)-*N*;*N*;*N*',*N*'-tetramethyluronium hexafluorophosphate (380.0 mg, 1.0 mmol) and *N,N-*diisopropylethylamine (0.5 mL) at room temperature. The mixed solution was stirred for 0.5 hour, added with methyl (S)-4-amino-3-(((oxetan-2-yl)methyl)amino)benzoate (158.0 mg, 0.6 mmol), and then stirred at room temperature overnight.

After the completion of the reaction, the reaction was quenched with saturated sodium chloride, and the reaction solution was extracted with ethyl acetate (30 mL × 3 times). The organic phases were combined, dried with anhydrous sodium sulfate, filtered, and concentrated to give a crude product, which was purified by silica gel column chromatography (eluent: ethyl acetate/n-hexane = 1/1) to obtain 395.0 mg of light yellow solid methyl 4-(2-(4-(6-((4-chloro-2-fluorobenzyl)oxy)pyridin-2-yl)piperidin-1-yl)propionamido)-3-((((S)-oxetan-2-yl)methyl)amino)benzoate (yield: 92.5%). LC-MS: RT = 1.82 min, [M+H]⁺ = 611.31.

### Step H: Synthesis of methyl 2-(1-(4-(6-((4-chloro-2-fluorobenzyl)oxy)pyridin-2-yl)piperidin-1-yl)ethyl)-1-(((S)-oxetan-2-yl)methyl)-1H-benzo[d]imidazol-6-carboxylate

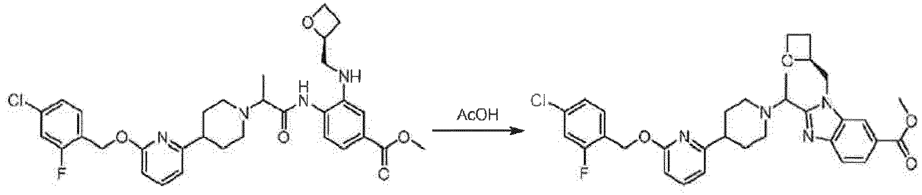

Methyl 4-(2-(4-(6-((4-chloro-2-fluorobenzyl)oxy)pyridin-2-yl)piperidin-1-yl)propionamido)-3-((((S)-oxetan-2-yl)methyl)amino)benzoate (355.0 mg, 0.6 mmol) was dissolved in acetic acid (4.0 mL). The temperature was raised to 60°C, and the reaction was carried out for 5 hours.

After the completion of the reaction, the reaction solution was poured into 50.0 mL of saturated aqueous sodium bicarbonate solution, and the reaction solution was extracted with ethyl acetate (30 mL × 3 times). The organic phases were combined, dried with anhydrous sodium sulfate, filtered, and concentrated to give a crude product, which was separated by Prep-TLC (developing solvent: dichloromethane/methanol = 13/1) to obtain 118.0 mg of light yellow solid methyl 2-(1-(4-(6-((4-chloro-2-fluorobenzyl)oxy)pyridin-2-yl)piperidin-1-yl)ethyl)-1-(((S)-oxetan-2-yl)methyl)-1*H-*benzo[*d*]imidazol-6-carboxylate (yield: 33.0%). LC-MS: RT= 1.84 min, [M+H]⁺ = 593.31.

### Step I: Synthesis of 2-((S)-1-(4-(6-((4-chloro-2-fluorobenzyl)oxy)pyridin-2-yl)piperidin-1-yl)ethyl)-1-(((S)-oxetan-2-yl)methyl)-1H-benzo[d]imidazol-6-carboxylic acid (1A) and 2-((R)-1-(4-(6-((4-chloro-2-fluorobenzyl)oxy)pyridin-2-yl)piperidin-1-yl)ethyl)-1-(((S)-oxetan-2-yl)methyl)-1H-benzo[d]imidazol-6-carboxylic acid (1B)

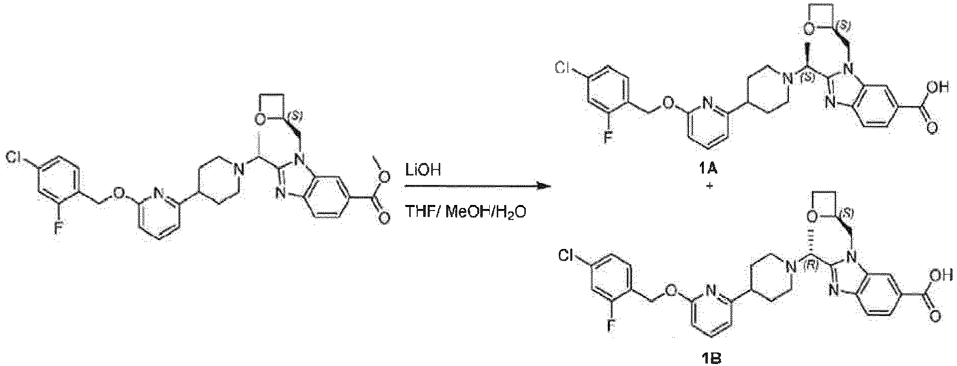

Methyl 2-(1-(4-(6-((4-chloro-2-fluorobenzyl)oxy)pyridin-2-yl)piperidin-1-yl)ethyl)-1-(((S)-oxetan-2-yl)methyl)-1*H*-benzo[*d*]imidazol-6-carboxylate (70.0 mg, 0.2 mmol) was dissolved in a solvent mixture (2.5 mL, tetrahydrofuran/methanol/water = 1/3/1) at room temperature, followed by adding lithium hydroxide (48.0 mg, 1.1 mmol), and the reaction was carried out at room temperature for 24 hours.

After the completion of the reaction, the reaction was quenched with saturated ammonium chloride, and the reaction solution was extracted with a solvent mixture (15 mL × 3 times, dichloromethane/methanol = 15/1). The organic phases were combined, dried with anhydrous sodium sulfate, filtered, and concentrated to give a crude product, which was separated by reversed-phase HPLC (eluate concentration, lyophilization) under the following separation conditions: chromatographic column: Agilent 5 Prep-C18 100 mm×30 mm 5 µM; mobile phase: water (containing 0.1% ammonia) and acetonitrile elution; flow rate: 20 mL/min; gradient: 27% acetonitrile, compounds 1A and 1B are eluted at 9.07 min and 12.00 min, respectively; detection wavelength: 254 nm.

8.0 mg of white solid 2-((S)-1-(4-(6-((4-chloro-2-fluorobenzyl)oxy)pyridin-2-yl)piperidin-1-yl)ethyl)-1-(((S)-oxetan-2-yl)methyl)-1*H-*benzo[*d*]imidazol-6-carboxylic acid **(1A)** (yield: 7.0%) and 4.0 mg of 2-((R)-1 -(4-(6-((4-chloro-2-fluorobenzyl)oxy)pyridin-2-yl)piperidin-1 -yl)ethyl)-1 -(((S)-oxetan-2-yl)methyl)-1*H-*benzo[*d*]imidazol-6-carboxylic acid **(1B)** (yield: 3.5%) were obtained successively.

Compound 1A: HPLC: RT = 9.07 min.

LC-MS: RT = 1.82 min, [M+H]⁺ = 579.29_{∘} ¹H NMR (400 MHz, DMSO) δ 8.20 (brs, 1H), 7 80 (dd, *J =* 8.4, 1 3 Hz, 1H), 7.63-7.53 (m, 3H), 7.45 (dd, *J* = 10.0, 1.9 Hz, 1H), 7.28 (dd, *J* = 8.2, 2.0 Hz, 1H), 6.84 (d, *J* = 7.3 Hz, 1H), 6.66 (d, *J* = 8.2 Hz, 1H), 5.36 (s, 2H), 5.22-5.16 (m, 1H), 4.87-4.78 (m, 1H), 4.62 (dd, *J* = 15.2, 5.2 Hz, 1H), 4.49-4.41 (m, 2H), 4.18 (dt, *J* = 9.4, 5.9 Hz, 1H), 2.98-2.96 (m, 1H), 2,65-2.60 (m, 2H), 2.59-2.49 (m, 2H), 2.38-2.21 (m, 2H), 1.86-1.68 (m, 3H), 1.58-1.48 (m, 1H), 1.47 (d, *J* = 6.7 Hz, 3H).

Compound **1B:** HPLC: RT = 12.00 min.

LC-MS: RT = 1.84min, [M+H]⁺ = 579.31_{∘} ¹H NMR (400 MHz, DMSO) δ 8.38 (s, 1H), 7.90 (d, *J* = 7.8 Hz, 1H), 7 79 (d, *J* = 8.1 Hz, 1H), 7.70 7.57 (m, 2H), 7.47 (d, *J* = 9.8 Hz, 1H), 7.36-7.26 (m, 1H), 6.91-6.89 (m, 1H), 6.74-6.72 (m, 1H), 5.38 (s, 2H), 5.14-4.91 (m, 2H), 4.71-4.46 (m, 2H), 3.60-3.15 (m, 3H), 2.90-2.75 (m, 1H), 2.50-2.39 (m, 1H), 2.14-1.92 (m, 4H), 1.79-1.62 (m, 3H), 1.28-1.22 (m, 4H).

### Example 2 2-((S)-1-(4-(6-((4-cyano-2-fluorobenzyl)oxy)pyridin-2-yl)piperidin-1-yl)ethyl)-1-(((S)-oxetan-2-yl)methyl)-1H-benzo[d]imidazol-6-carboxylic acid (2A) and 2-((R)-1-(4-(6-((4-cyano-2-fluorobenzyl)oxy)pyridin-2-yl)piperidin-1-yl)ethyl)-1-(((S)-oxetan-2-yl)methyl)-1H-benzo[d]imidazol-6-carboxylic acid (2B)

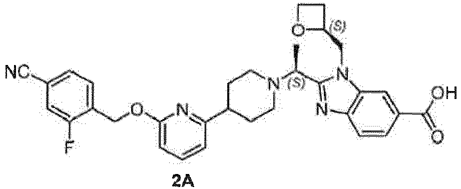

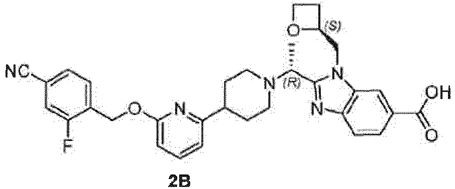

The specific synthetic route is as follows.

### Step A: Synthesis of tert-butyl 4-(6-((4-cyano-2-fluorobenzyl)oxy)pyridin-2-yl)piperidin-1-carboxylate

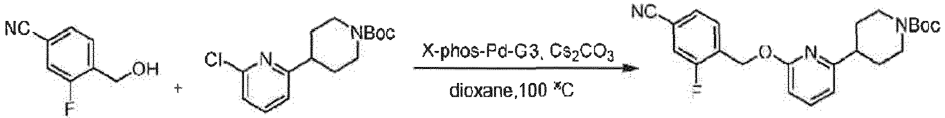

3-Fluoro-4-(hydroxymethyl)benzonitrile (3.45 g, 22.88 mmol), tert-butyl 4-(6-chloropyridin-2-yl)piperidin-1-carboxylate (4.50 g, 15.25 mmol), palladium catalyst (644 mg), and cesium carbonate (10 g, 30.50 mmol) were dissolved in dioxane (80 mL). The temperature was raised to 100°C and the mixed solution was stirred for 8 hours.

After the completion of the reaction, the reaction solution was vacuum filtered through diatomaceous earth and drip-washed with dichloromethane. The filtrate was concentrated to give a crude product, which was purified by column chromatography (eluent: ethyl acetate/n-hexane = 10/1) to obtain 5 g of a light yellow oily liquid tert-butyl 4-(6-((4-cyano-2-fluorobenzyl)oxy)pyridin-2-yl)piperidin-1-carboxylate (yield: 80%). LC-MS: RT = 2.27 min, [M-55]⁺ = 356.21.

### Step B: Synthesis of 3-fluoro-4-(((6-(piperidin-4-yl)pyridin-2-yl)oxy)methyl)benzonitrile

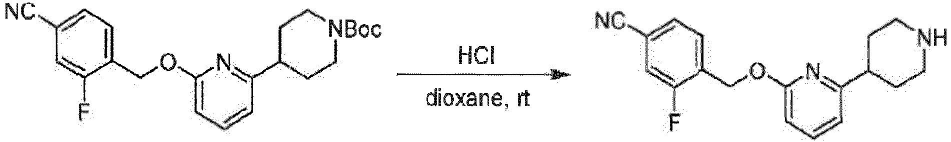

Tert-butyl 4-(6-((4-cyano-2-fluorobenzyl)oxy)pyridin-2-yl)piperidin-1-carboxylate (5.00 g) was dissolved in dioxane in hydrochloric acid (40.0 mL), and the mixed solution was stirred at room temperature.

After the completion of the reaction, the reaction solution was spin-dried under reduced pressure to obtain 4.5 g of light yellow solid 3-fluoro-4-(((6-(piperidin-4-yl)pyridin-2-yl)oxy)methyl)benzonitrile. LC-MS: RT= 1.65 min, [M+H]⁺ = 312.21.

### Step C: Synthesis of ethyl 2-(4-(6-((4-cyano-2-fluorobenzyl)oxy)pyridin-2-yl)piperidin-1-yl)propionate

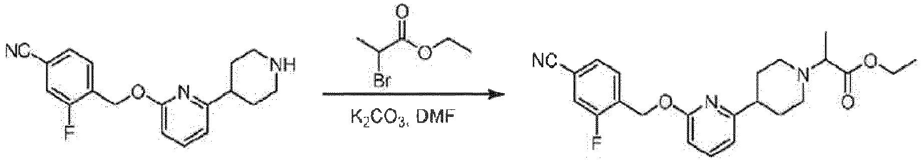

3-Fluoro-4-(((6-(piperidin-4-yl)pyridin-2-yl)oxy)methyl)benzonitrile (4.50 g, 14.46 mmol) and potassium carbonate (4.00 g, 28.92 mmol) were dissolved in *N*,*N-*dimethylformamide (50 mL) at room temperature, followed by adding ethyl 2-bromopropionate (3.16 mg, 17.35 mmol), and the mixed solution was stirred at room temperature.

After the completion of the reaction, the reaction was quenched with saturated sodium chloride, and the reaction solution was extracted with ethyl acetate (30 mL × 3 times). The organic phases were combined, dried, and concentrated to give a crude product, which was purified by silica gel column chromatography (eluent: ethyl acetate/n-hexane = 1/3) to obtain 3.7 g of white solid ethyl 2-(4-(6-((4-cyano-2-fluorobenzyl)oxy)pyridin-2-yl)piperidin-1-yl)propionate (yield: 62%). LC-MS: RT = 1.70 min, [M+H]⁺ = 412.25.

### Step D: Synthesis of 2-(4-(6-((4-cyano-2-fluorobenzyl)oxy)pyridin-2-yl)piperidin-1-yl)propionic acid

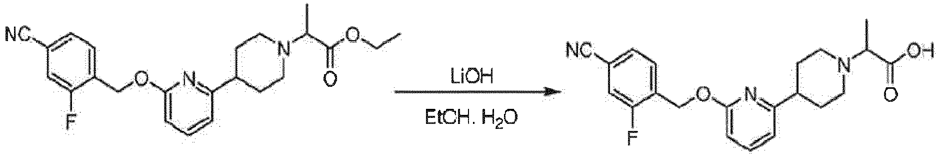

Ethyl 2-(4-(6-((4-cyano-2-fluorobenzyl)oxy)pyridin-2-yl)piperidin-1-yl)propionate (1.00 g, 2.43 mmol) was dissolved in a solvent mixture (22 mL, ethanol/water= 5/1) at room temperature, and lithium hydroxide (185 mg, 4.87 mmol) was added to the mixed solution, which was stirred at room temperature for 48 hours.

After the completion of the reaction, the reaction was quenched with saturated sodium chloride, and the reaction solution was extracted with ethyl acetate (20 mL × 3 times). The organic phases were combined, dried with anhydrous sodium sulfate, filtered, and concentrated to give a crude product, which was purified by silica gel column chromatography (eluent: ethyl acetate/n-hexane = 1/1) to obtain 200 mg of white solid 2-(4-(6-((4-cyano-2-fluorobenzyl)oxy)pyridin-2-yl)piperidin-1-yl)propionic acid (yield: 21%). LC-MS: RT= 1.73 min, [M+H]⁺ = 384.27.

### Step E: Synthesis of methyl 4-(2-(4-(6-((4-cyano-2-fluorobenzyl)oxy)pyridin-2-yl)piperidin-1-yl)propionamido)-3-((((S)-oxetan-2-yl)methyl)amino)benzoate

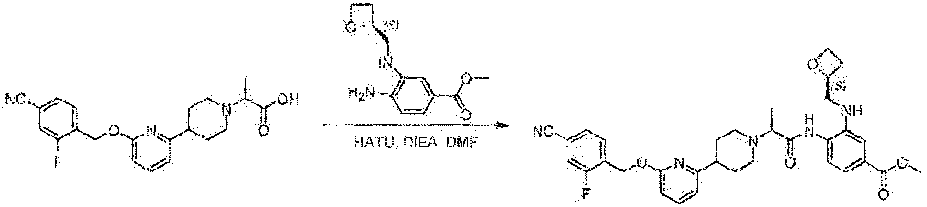

2-(4-(6-((4-Cyano-2-fluorobenzyl)oxy)pyridin-2-yl)piperidin-1-yl)propionic acid (200 mg, 0.51 mmol) was dissolved in *N*,*N-*dimethylformamide (10 mL) at room temperature, followed by adding HATU (388 mg, 1.02 mmol) and DIEA (2 mL). The mixed solution was stirred for 0.5 hour, added with methyl (S)-4-amino-3-(((oxetan-2-yl)methyl)amino)benzoate (144 mg, 0.61 mmol), and then stirred at room temperature.

After the completion of the reaction, the reaction was quenched with saturated sodium chloride, and the reaction solution was extracted with ethyl acetate (20 mL × 3 times). The organic phases were combined, dried with anhydrous sodium sulfate, filtered, and concentrated to give a crude product, which was purified by silica gel column chromatography (eluent: ethyl acetate/n-hexane = 1/1) to obtain 183 mg of light yellow solid methyl 4-(2-(4-(6-((4-cyano-2-fluorobenzyl)oxy)pyridin-2-yl)piperidin-1-yl)propionamido)-3-((((S)-oxetan-2-yl)methyl)amino)benzoate (yield: 60%). LC-MS: RT = 1.77 min, [M+H]⁺ = 602.32.

### Step F: Synthesis of methyl 2-(1-(4-(6-((4-cyano-2-fluorobenzyl)oxy)pyridin-2-yl)piperidin-1-yl)ethyl)-1-(((S)-oxetan-2-yl)methyl)-1H-benzo[d]imidazol-6-carboxylate

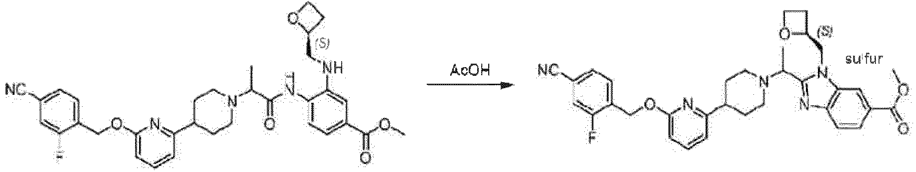

Methyl 4-(2-(4-(6-((4-cyano-2-fluorobenzyl)oxy)pyridin-2-yl)piperidin-1-yl)propionamido)-3-((((S)-oxetan-2-yl)methyl)amino)benzoate (183 mg, 0.30 mmol) was dissolved in acetic acid (2 mL), and the reaction was carried out under a temperature of 60°C.

After the completion of the reaction, a crude product was obtained by direct concentration, and was purified by silica gel column chromatography (eluent: ethyl acetate/n-hexane = 7/1) to give 150 mg of brownish-yellow solid methyl 2-(1-(4-(6-((4-cyano-2-fluorobenzyl)oxy)pyridin-2-yl)piperidin-1-yl)ethyl)-1-(((S)-oxetan-2-yl)methyl)-1*H-*benzo[*d*]imidazol-6-carboxylate (yield: 86%). LC-MS: RT = 1.79 min, [M+H]⁺ = 584.32.

### Step G: Synthesis of 2-((S)-1-(4-(6-((4-eyano-2-fluorobenzyl)oxy)pyridin-2-yl)piperidin-1-yl)ethyl)-1-(((S)-oxetan-2-yl)methyl)-1H-benzo[d]imidazol-6-carboxylic acid (2A) and 2-((R)-1-(4-(6-((4-cyano-2-fluorobenzyl)oxy)pyridin-2-yl)piperidin-1-yl)ethyl)-1-(((S)-oxetan-2-yl)methyl)-1H-benzo[d]imidazol-6-carboxylic acid (2B)

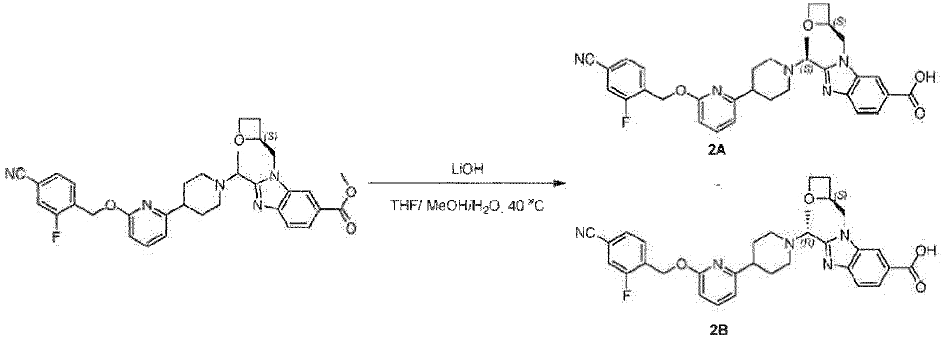

Methyl 2-(1-(4-(6-((4-cyano-2-fluorobenzyl)oxy)pyridin-2-yl)piperidin-1-yl)ethyl)-1-(((S)-oxetan-2-yl)methyl)-1*H*-benzo[*d*]imidazol-6-carboxylate (150 mg, 0.26 mmol) was dissolved in a solvent mixture (5 mL, tetrahydrofuran/methanol/water = 2.5/2.5/1) at room temperature, followed by adding lithium hydroxide (40 mg, 1.05 mmol), and the reaction was carried out under a temperature of 40°C.

After the completion of the reaction, the reaction was quenched with saturated ammonium chloride and the reaction solution was sequentially extracted with ethyl acetate (10 mL × 2 times) and a solvent mixture (10 mL × 2 times, dichloromethane/methanol = 30/1). The organic phases were combined, dried with anhydrous sodium sulfate, filtered, and concentrated to give a crude product, which was separated by reversed-phase HPLC (eluate concentration, lyophilization) under the following separation conditions: chromatographic column: Agilent 5 Prep-C18 100 mm×30 mm 5 µM; mobile phase: water (containing 0.1% ammonia) and acetonitrile elution; flow rate: 20 mL/min; gradient: 23% acetonitrile, compound 2A and 2B are eluted at 13.77 min and 15.7 min, respectively; detection wavelength: 254 nm. 18.93 mg of white solid 2-((S)-1-(4-(6-((4-cyano-2-fluorobenzyl)oxy)pyridin-2-yl)piperidin-1-yl)ethyl)-1-(((S)-oxetan-2-yl)methyl)-1*H*-benzo[*d*]imidazol-6-carboxylic acid **(2A)** (yield: 13%) and 31.15 mg of white solid 2-((R)-1-(4-(6-((4-cyano-2-fluorobenzyl)oxy)pyridin-2-yl)piperidin-1-yl)ethyl)-1 -(((S)-oxetan-2-yl)methyl)-1*H*-benzo[*d*]imidazol-6-carboxylic acid **(2B)** (yield: 21%) were obtained successively.

Compound **2A:** HPLC: RT = 13.77 min.

LC-MS : RT = 1 77 min, [M+H]⁻ = 570.31_{∘} ¹H NMR (400 MHz, DMSO) δ 12.77 (brs, 1H), 8.29-8.28 (m, 1H), 7.87 (d, *J* = 10 4 Hz, 1H), 7.80 (dd, *J* = 8.4, 1.6 Hz, 1H), 7.70-7.66 (m, 3H), 7.62 (dd, *J* = 8.2, 7.4 Hz, 1H), 6.84 (d, *J* = 7.1 Hz, 1H), 6.70 (d, *J* = 7.7 Hz, 1H), 5.44 (brs, 2H), 5.19-5.14 (m, 1H), 4.83 (dd, *J* = 15.5, 3.1 Hz, 1H), 4.68 (dd, *J* = 15 4, 5.6 Hz, 1H), 4.50-4.39 (m, 2H), 4 18 (dt, *J* = 9.1, 5.7 Hz, 1H), 2.98-2.94 (m, 1H), 2.67-2.45 (m, 4H), 2.37-2.19 (m, 2H), 1.84-1.61 (m, 3H), 1.46 (d, *J* = 6.8 Hz, 3H), 1.54-1.43 (m, 1H).

Compound **2B:** HPLC: RT= 15.74 min.

LC-MS: RT = 1 77 min, [M+H]⁺ = 570.35_{∘} ¹H NMR (400 MHz, DMSO-d₆) *δ* 8 24 (s, 1H), 7.87 (d, *J* = 10.1 Hz, 1H), 7.78 *(dd, J =* 8.4, 1.4 Hz, 1H), 7.72-7.67 (m, 2H), 7.63 (dd, *J* = 8 0, 6.2 Hz, 2H), 6.86 (d, *J* = 7.3 Hz, 1H), 6.70 (d, *J* = 8.1 Hz, 1H), 5.46 (s, 2H), 5.12-5.00 (m, 1H), 4.89 (dd, *J* = 15.1, 8.6 Hz, 1H), 4.63-4.40 (m, 4H), 3 04 (d, *J* = 10.8 Hz, 1H), 2 78 2.69 (m, 1H), 2.61 (t, *J* = 10.4 Hz, 1H), 2 55 (s, 2H), 2.26 (t, *J* = 10.7 Hz, 1H), 1.96-1.59 (m, 4H), 1.55-1.39 (m, 4H).

### Example 3 2-((S)-1-(4-(6-((5-chloro-2-fluorobenzyl)oxy)pyridin-2-yl)piperidin-1-yl)ethyl)-1-(((S)-oxetan-2-yl)methyl)-1H-benzo[d]imidazol-6-carboarylic acid

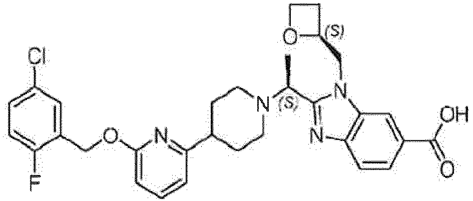

The specific synthetic route is as follows.

### Step A: Synthesis of 5-chloro-2-fluorobenzyl methanol

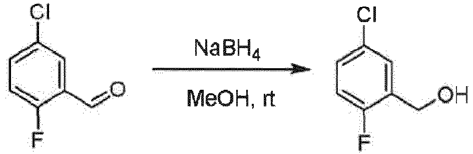

5-Chloro-2-fluorobenzaldehyde (500 mg, 3.16 mmol) was dissolved in methanol (15 mL) at room temperature, and the mixed solution was cooled to 0°C in an ice bath, followed by adding sodium borohydride (234 mg, 6.33 mmol). The solution was stirred at room temperature.

After the completion of the reaction, the reaction was quenched with saturated sodium chloride. The reaction solution was extracted with dichloromethane (30 mL), washed twice with water, dried with anhydrous sodium sulfate, filtered, and concentrated to give a crude product, which was purified by silica gel column chromatography (eluent: ethyl acetate/n-hexane = 1/3) to obtain 410 mg of colorless oily liquid 5-chloro-2-fluorobenzyl methanol (yield: 81%). LC-MS: RT = 1.78 min.

### Step B: Synthesis of tert-butyl 4-(6-((5-chloro-2-fluorobenzyl)oxy)pyridin-2-yl)piperidin-1-carboxylate

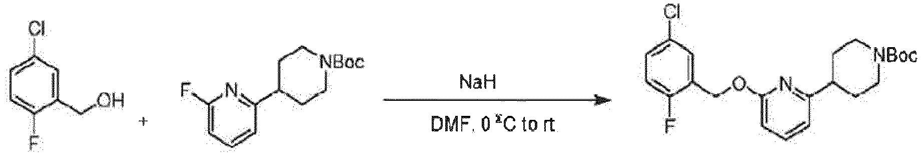

5-Chloro-2-fluorobenzyl methanol (410 mg, 2.56 mmol) was dissolved in *N*,*N*-dimethylformamide (10 mL), and the mixed solution was cooled to 0°C in an ice bath, followed by adding sodium hydride (270 mg, 5.12 mmol). The mixture was stirred for 0.5 hour under room temperature, and tert-butyl 4-(6-fluoropyridin-2-yl)piperidin-1-carboxylate (718 mg, 2.56 mmol) was added and the reaction was carried out at room temperature.

After the completion of the reaction, the reaction was quenched with saturated sodium chloride, and the reaction solution was extracted with ethyl acetate (30 mL), washed twice with water, dried with anhydrous sodium sulfate, filtered, and concentrated to give a crude product, which was purified by silica gel column chromatography (eluent: ethyl acetate/n-hexane=1/2) to obtain 550 mg of colorless oily liquid tert-butyl 4-(6-((5-chloro-2-fluorobenzyl)oxy)pyridin-2-yl)piperidin-1-carboxylate (yield: 51%). LC-MS: RT = 2.41 min, [M-55]⁺ = 365.17.

### Step C: Synthesis of 2-((5-chloro-2-fluorobenzyl)oxy)-6-(piperidin-4-yl)pyridine

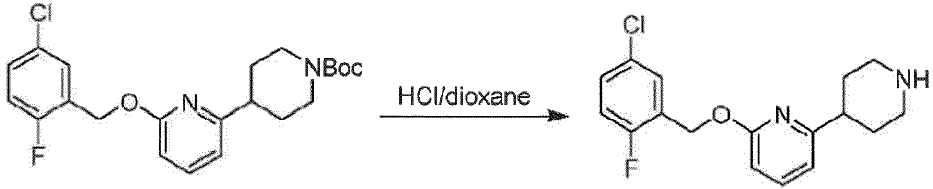

Tert-butyl 4-(6-((5-chloro-2-fluorobenzyl)oxy)pyridin-2-yl)piperidin-1-carboxylate (550 mg, 1.31 mmol) was dissolved in dioxane in hydrochloric acid (10 mL), and the mixed solution was stirred at room temperature. After the completion of the reaction, 520 mg of white solid 2-((5-chloro-2-fluorobenzyl)oxy)-6-(piperidin-4-yl)pyridine was obtained after concentration under reduced pressure. LC-MS: RT= 1.72 min, [M+H]⁺ = 321.18.

### Step D: Synthesis of methyl 2-((S)-1-(4-(6-((5-chloro-2-fluorobenzyl)oxy)pyridin-2-yl)piperidin-1-yl)ethyl)-1-(((S)-oxetan-2-yl)methyl)-1H-benzo[d]imidazol-6-carboxylate

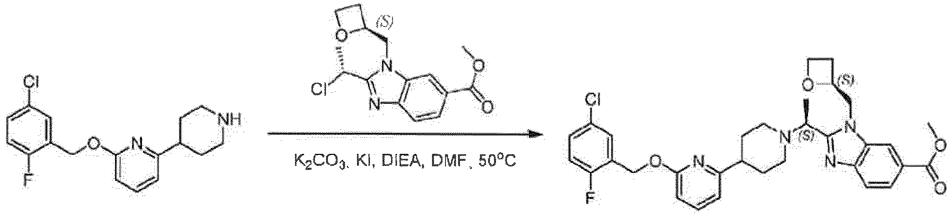

2-((5-Chloro-2-fluorobenzyl)oxy)-6-(piperidin-4-yl)pyridine (150 mg, 0.47 mmol), DIEA (1 mL), potassium iodide (156 mg, 0.94 mmol), and potassium carbonate (130 g, 0.94 mmol) were dissolved in *N*,*N-*dimethylformamide (10 mL) at room temperature, followed by adding methyl 2-((R)-1-chloroethyl)-1-(((S)-oxetan-2-yl)methyl)-1*H-*benzo[*d*]imidazol-6-carboxylate (144 mg, 0.47 mmol), and the mixed solution was heated up to 50°C.

After the completion of the reaction, the reaction was quenched with saturated sodium chloride, and the reaction solution was extracted with ethyl acetate (20 mL × 2 times). The organic phases were combined, dried, and concentrated to give a crude product, which was purified by silica gel column chromatography (eluent: ethyl acetate/n-hexane = 1/8) to obtain 90 mg of white solid compound methyl 2-((S)-1-(4-(6-((5-chloro-2-fluorobenzyl)oxy)pyridin-2-yl)piperidin-1-yl)ethyl)-1-(((S)-oxetan-2-yl)methyl)-1*H*-benzo[*d*]imidazol-6-carboxylate (yield: 32%). LC-MS: RT = 1.86 min, [M+H]⁺ = 593.31.

### Step E: Synthesis of 2-((S)-1-(4-(6-((5-chloro-2-fluorobenzyl)oxy)pyridin-2-yl)piperidin-1-yl)ethyl)-1-(((S)-oxetan-2-yl)methyl)-1H-benzo[d]imidazol-6-carboxylic acid

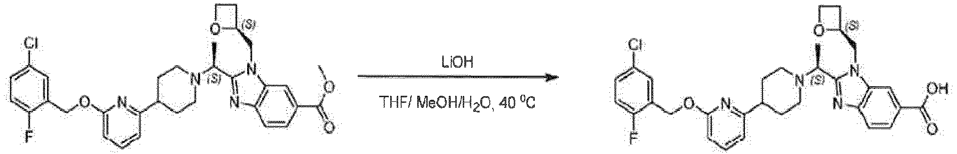

Compound methyl 2-((S)-1-(4-(6-((5-chloro-2-fluorobenzyl)oxy)pyridin-2-yl)piperidin-1-yl)ethyl)-1-(((S)-oxetan-2-yl)methyl)-1*H-*benzo[*d*]imidazol-6-carboxylate (90.0 mg, 0.15 mmol) was dissolved in a solvent mixture (5 mL, tetrahydrofuran/methanol/water = 2.5/2.5/1) at room temperature, followed by adding lithium hydroxide (23.0 mg, 0.61 mmol), and the reaction was carried out under a temperature of 40°C.

After the completion of the reaction, the reaction was quenched with saturated ammonium chloride, and the reaction solution was sequentially extracted with ethyl acetate (10 mL × 2 times) and a solvent mixture (10 mL × 2 times, dichloromethane/methanol = 30/1). The organic phases were combined, dried with anhydrous sodium sulfate, filtered, and concentrated to give a crude product, which was purified by silica gel column chromatography (eluent: dichloromethane/methanol = 20/1) to obtain 31.88 mg of white solid 2-((S)-1-(4-(6-((5-chloro-2-fluorobenzyl)oxy)pyridin-2-yl)piperidin-1-yl)ethyl)-1-(((S)-oxetan-2-yl)methyl)-1*H*-benzo[*d*]imidazol-6-carboxylic acid (yield: 21%).
LC-MS: RT = 1.81min, [M+H]⁺ = 579.30. ¹H NMR (400 MHz, DMSO-d₆) *δ* 12.72 (s, 1H), 8.28 (s, 1H), 779 (s, 1H), 7.64 (dd, *J* = 30_9, 7.9 Hz, 3H), 7.41 (d, *J* = 3.6 Hz, 1 H), 7.26 (t, *J* = 9.2 Hz, 1H), 6 83 (d, *J* = 74 Hz, 1H), 6.67 (d, *J* = 8.0 Hz, 1H), 5.36 (d, *J* = 9.1 Hz, 2H), 5 16 (s, 1H), 4.84 (d, *J* = 13.6 Hz, 1H), 4.69 (s, 1H), 4.54-4.31 (m, 2H), 4.17 (dt, *J* = 11.4, 5.7 Hz, 1H), 2.97 (s, 1H), 2.62 (d, *J* = 6.0 Hz, 2H), 224 (d, *J* = 54.1 Hz, 3H), 1.73 (dd, *J* = 28.8, 21.5 Hz, 4H), 1.59-1.35 (m, 4H).

### Example 4 2-((S)-1-(4-(6-((3-fluoropyridin-4-yl)methoxy)pyridin-2-yl)piperidin-1-yl)ethyl)-3-(((S)-oxetan-2-yl)methyl)-3H-imidazolo[4,5-b]pyridin-5-carboxylic acid

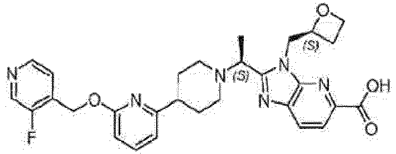

The specific synthetic route is as follows.

### Step A: Synthesis of tert-butyl 4-(6-((3-fluoropyridin-4-yl)methoxy)pyridin-2-yl)piperidin-1-formate

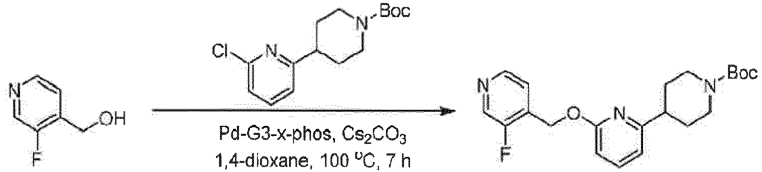

(3-Fluoropyridin-4-yl)methanol (86 mg, 0.68 mmol) and tert-butyl 4-(6-chloropyridin-2-yl)piperidin-1-formate (201 mg, 0.68 mmol) were dissolved in 4 mL of anhydrous dioxane, followed by adding cesium carbonate (443 mg, 1.36 mmol) and methanesulfonato(2-dicyclohexylphosphino-2',4',6'-triisopropyl-1,1'-biphenyl)(2'-amino-1,1'-biphenyl-2-yl)palladium(II) (58 mg, 0.068 mmol). Nitrogen replacement was performed 3 times and the reaction was carried out at 100°C for 7 hours.

After the completion of the reaction, the reaction solution was diluted with water and extracted with ethyl acetate (50 mL × 2 times). The organic phases were combined, washed with saturated saline (20 mL × 2 times), dried over anhydrous sodium sulfate, and concentrated under reduced pressure to give a crude product, which was separated by column chromatography (n-hexane/ethyl acetate = 4/1) to obtain 101 mg of light yellow solid tert-butyl 4-(6-((3-fluoropyridin-4-yl)methoxy)pyridin-2-yl)piperidin-1-formate (yield: 38.3%).

### Step B: Synthesis of 4-(6-((3-fluoropyridin-4-yl)methoxy)pyridin-2-yl)piperidine

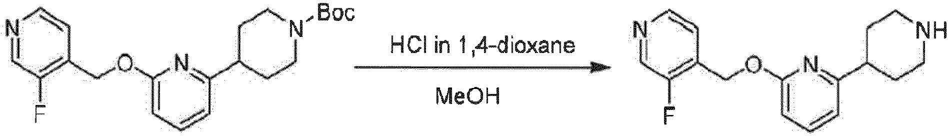

Tert-butyl 4-(6-((3-fluoropyridin-4-yl)methoxy)pyridin-2-yl)piperidin-1-formate (101 mg, 0.26 mmol) was dissolved in 3 mL of methanol, and 4 M hydrochloric acid/dioxane solution (3 mL) was added at 0°C. The mixed solution was heated to room temperature and the reaction was carried out for 40 min.

After the completion of the reaction, the reaction solution was spin-dried directly to give 98 mg of white solid 4-(6-((3-fluoropyridin-4-yl)methoxy)pyridin-2-yl)piperidine, which was used directly in the next reaction step. LC-MS: RT = 1.56 min, [M+H]⁺ = 288.22.

### Step C: Synthesis of methyl 4-((S)-2-chloropropionamido)-3-((((S)-oxetan-2-yl)methyl)amino)benzoate

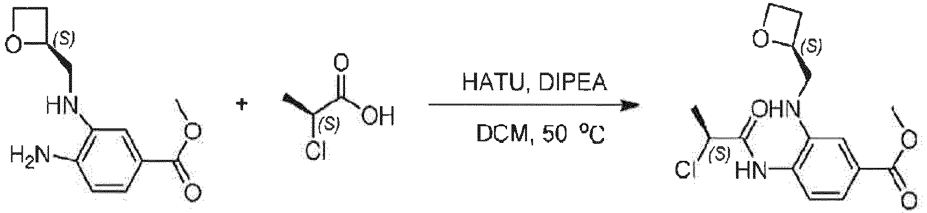

Methyl (S)-4-amino-3-(((oxetan-2-yl)methyl)amino)benzoate (500.0 mg, 2.25 mmol), (S)-2-chloropropionic acid (243.0 mg, 2.25 mmol) were dissolved in dichloromethane (10.0 mL) at room temperature, followed by the addition of 2-(7-azabenzotriazol-l-yl)-*N,N,N*',*N*'-tetramethyluronium hexafluorophosphate (1.28 g, 3.38 mmol) and *N*,*N-*diisopropylethylamine (3 mL). The reaction was carried out at 50°C for 2.0 hours under N₂ protection.

After the completion of the reaction, the reaction was quenched with water and the reaction solution was extracted with ethyl acetate (50 mL × 3 times). The organic phases were combined, washed with saturated saline (30 mL × 3 times), dried with anhydrous sodium sulfate, and finally purified by silica gel column chromatography (eluent: n-hexane/ethyl acetate = 2/1) to obtain 580.0 mg of light yellow solid methyl 4-((S)-2-chloropropionamido)-3-((((S)-oxetan-2-yl)methyl)amino)benzoate (yield: 79.1%). LC-MS: RT= 1.82 min, [M+H]⁺ = 327.18.

### Step D: Synthesis of methyl 2-((S)-1-chloroethyl)-1-(((S)-oxetan-2-yl)methyl)-1H-benzo[d]imidazol-6-carboxylate

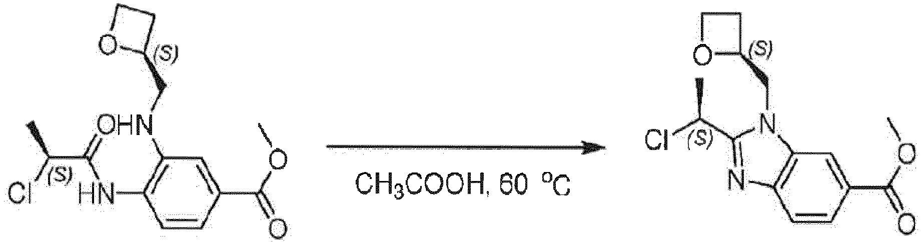

Methyl 4-((S)-2-chloropropionamido)-3-((((S)-oxetan-2-yl)methyl)amino)benzoate (580.0 mg, 1.78 mmol) was dissolved in acetic acid (10.0 mL) at room temperature, and the reaction was carried out at 60°C for 12.0 hours under N₂ protection.

After the completion of the reaction, the reaction was quenched with water, and the pH of the reaction solution was adjusted to 6 with sodium bicarbonate solution (0.5 mol/L). The reaction solution was extracted with dichloromethane (30 mL × 3 times), and the organic phases was combined, washed with saturated saline (30 mL × 2 times), dried with anhydrous sodium sulfate, and finally purified by silica gel column chromatography (eluent: n-hexane: ethyl acetate = 4:1) to give 520.0 mg of light red oily methyl 2-((S)-1-chloroethyl)-1-(((S)-oxetan-2-yl)methyl)-1*H-*benzo[*d*]imidazol-6-carboxylate (yield: 94.8%). LC-MS: RT = 1.86 min, [M+H]⁺ = 309.23.

### Step E: Synthesis of methyl 2-((S)-1-(4-(6-((3-fluoropyridin-4-yl)methoxy)pyridin-2-yl)piperidin-1-yl)ethyl)-3-(((S)-oxetan-2-yl)methyl)-3H-imidazolo[4,5-b]pyridin-5-carboxylate

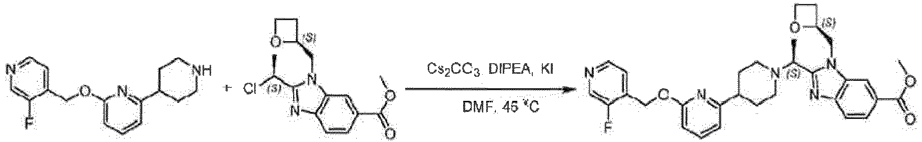

Methyl 4-(6-((3-fluoropyridin-4-yl)methoxy)pyridin-2-yl)piperidine (55.0 mg, 0.19 mmol), methyl 2-((S)-1-chloroethyl)-1-(((S)-oxetan-2-yl)methyl)-1*H-*benzo[*d*]imidazol-6-carboxylate (58.5 mg, 0.19 mmol), potassium iodide (31.5 mg, 0.19 mmol), and cesium carbonate (123.1 mg, 0.38 mmol) were added to *N*,*N-*dimethylformamide (10.0 mL) at room temperature, followed by the addition of *N,N-*diisopropylethylamine (1.0 mL). The reaction was carried out at 45°C for 3.0 hours under N₂ protection.

After the completion of the reaction, the reaction was quenched with water and the reaction solution was extracted with ethyl acetate (50 mL × 3 times). The organic phases were combined, washed with saturated saline (30 mL × 3 times), dried with anhydrous sodium sulfate, and finally purified by silica gel column chromatography (eluent: dichloromethane/methanol = 20/1) to give 65.0 mg of a light yellow solid methyl 2-((S)-1-(4-(6-((3-fluoropyridin-4-yl)methoxy)pyridin-2-yl)piperidin-1-yl)ethyl)-3-(((S)-oxetan-2-yl)methyl)-3*H-*imidazolo[4,5-*b*]pyridin-5-carboxylate (yield: 58.6%). LC-MS: RT = 1.73 min, [M+H]⁺ = 560.34.

### Step F: Synthesis of 2-((S)-1-(4-(6-((3-fluoropyridin-4-yl)methoxy)pyridin-2-yl)piperidin-1-yl)ethyl)-3-(((S)-oxetan-2-yl)methyl)-3H-imidazolo[4,5-b]pyridin-5-carboxylic acid

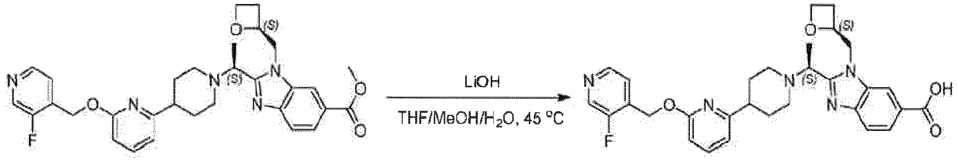

Lithium hydroxide (20.0 mg, 0.48 mmol) in water (1.0 mL) was added dropwise to tetrahydrofuran (3.0 mL) containing methyl 2-((S)-1-(4-(6-((3-fluoropyridin-4-yl)methoxy)pyridin-2-yl)piperidin-1-yl)ethyl)-3-(((S)-oxetan-2-yl)methyl)-3*H-*imidazolo[4,5-*b*]pyridin-5-carboxylate (65.0 mg, 0.11 mmol) at room temperature, followed by the addition of 1.0 mL of methanol. The reaction was carried out at 45°C for 3.0 hours.

The reaction was quenched with water, and the pH was adjusted to 6 with aqueous ammonium chloride solution (0.5 mol/L). The reaction solution was extracted with dichloromethane (30 mL × 3 times), and the organic phases were combined, washed with aqueous ammonium chloride solution (30 mL × 2 times), dried with anhydrous sodium sulfate to give a crude product, which was purified by column separation (eluent: dichloromethane/methanol = 15/1) to obtain 32 mg of a light yellow solid 2-((S)-1-(4-(6-((3-fluoropyridin-4-yl)methoxy)pyridin-2-yl)piperidin-1-yl)ethyl)-3-(((S)-oxetan-2-yl)methyl)-3*H-*imidazolo[4,5-*b*]pyridin-5-carboxylic acid (yield: 51.1%). LC-MS: RT = 1.68 min, [M+H]⁺ = 546.31.

### Example 5 2-((S)-1-(4-(6-((2,5-difluorobenzyl)oxy)pyridin-2-yl)piperidin-1-yl)ethyl)-1-(((S)-oxetan-2-yl)methyl)-1H-benzo[d]imidazol-6-carboxylic acid

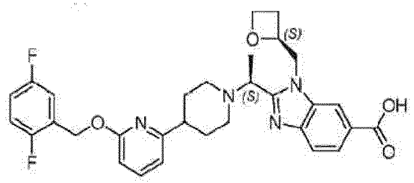

The specific synthetic route is as follows.

### Step A: Synthesis of (2,5-difluorophenyl)methanol

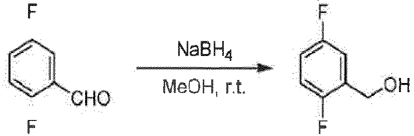

2,5-Difluorobenzaldehyde (500.0 mg, 3.52 mmol) was added to methanol (30.0 mL) under an ice bath, followed by the addition of sodium borohydride (200.6 mg, 5.28 mmol) in batches. The reaction was carried out with stirring at room temperature for 1.0 hour under N₂ protection.

After the completion of the reaction, the reaction was quenched with water, and the reaction solution was extracted with ethyl acetate (30 mL × 3 times). The organic phases were combined, washed with saturated saline (20 mL × 2 times), dried with anhydrous sodium sulfate, and finally concentrated under reduced pressure to give 402.0 mg of colorless oily (2,5-difluorophenyl)methanol (yield: 80.4%).

### Step B: Synthesis of methyl 4-((R)-2-chloropropionamido)-3-((((S)-oxetan-2-yl)methyl)amino)benzoate

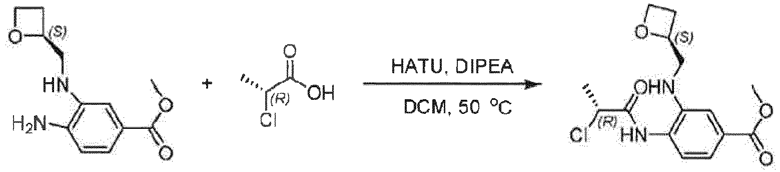

Methyl (S)-4-amino-3-(((oxetan-2-yl)methyl)amino)benzoate (500.0 mg, 2.25 mmol), (R)-2-chloropropionic acid (243.0 mg, 2.25 mmol) were dissolved in dichloromethane (10.0 mL) at room temperature, followed by the addition of 2-(7-azabenzotriazol-1-yl)-*N*,*N*,*N*',*N*'-tetramethyluronium hexafluorophosphate (1.28 g, 3.38 mmol) and *N*,*N-*diisopropylethylamine (3 mL). The reaction was carried out at 50°C for 2.0 hours under N₂ protection.

After the completion of the reaction, the reaction was quenched with water, and the reaction solution was extracted with ethyl acetate (50 mL × 3 times). The organic phases were combined, washed with saturated saline (30 mL × 3 times), dried with anhydrous sodium sulfate, and finally purified by silica gel column chromatography (eluent: n-hexane/ethyl acetate = 2:1) to obtain 586.0 mg of light yellow solid methyl 4-((R)-2-chloropropionamido)-3-((((S)-oxetan-2-yl)methyl)amino)benzoate (yield: 79.8%). LC-MS: RT = 1.82 min, [M+H]⁺ = 327.17.

### Step C: Synthesis of methyl 2-((R)-1-chloroethyl)-1-(((S)-oxetan-2-yl)methyl)-1H-benzo[d]imidazol-6-carboxylate

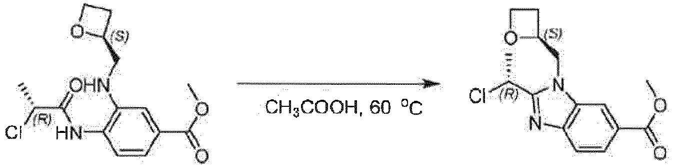

Methyl 4-((R)-2-chloropropionamido)-3-((((S)-oxetan-2-yl)methyl)amino)benzoate (586.0 mg, 1.80 mmol) was dissolved in acetic acid (10.0 mL) at room temperature, and the reaction was carried out at 60°C for 12.0 hours under N₂ protection.

After the completion of the reaction, the reaction was quenched with water and the pH of the reaction solution was adjusted to 6 with sodium bicarbonate solution (0.5 mol/L). The reaction solution was extracted with dichloromethane (30 mL × 3 times), and the organic phases was combined, washed with saturated saline (30 mL × 2 times), dried with anhydrous sodium sulfate, and finally purified by silica gel column chromatography (eluent: n-hexane: ethyl acetate = 4:1) to give 408.0 mg of light red oily methyl 2-((R)-1-chloroethyl)-1-(((S)-oxetan-2-yl)methyl)-1*H-*benzo[*d*]imidazol-6-carboxylate (yield: 73.5%). LC-MS: RT = 1.84 min, [M+H]⁺ = 309.23.

### Step D: Synthesis of tert-butyl 4-(6-((2,5-difluorobenzyl)oxy)pyridin-2-yl)piperidin-1-formate

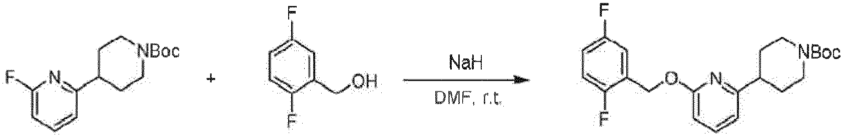

(2,5-Difluorophenyl)methanol (50.0 mg, 0.35 mmol) was added to *N*,*N-*dimethylformamide (4.0 mL) under an ice bath, followed by the addition of 60 wt. % sodium hydride (28.0 mg, 0.70 mmol) in batches. After stirring for 0.5 hour at room temperature under N₂ protection, tert-butyl 4-(6-fluoropyridin-2-yl)piperidin-1-formate (98.0 mg, 0.35 mmol) was added and the reaction was carried out for 1.0 hour at room temperature.

After the completion of the reaction, the reaction was quenched with water, and the reaction solution was extracted with ethyl acetate (30 mL × 3 times). The organic phases were combined, washed with saturated saline (20 mL × 2 times), dried with anhydrous sodium sulfate, and finally concentrated under reduced pressure. The resulting residue was purified by silica gel column chromatography (eluent: ethyl acetate/n-hexane = 50/1) to obtain 88.0 mg of white solid tert-butyl 4-(6-((2,5-difluorobenzyl)oxy)pyridin-2-yl)piperidin-1-formate (yield: 62.2%). LC-MS: RT = 2.33 min, [M+H-56]⁺ = 349.33.

### Step E: Synthesis of 2-((2,5-fluorobenzyl)oxy)-6-(piperidin-4-yl)pyridine

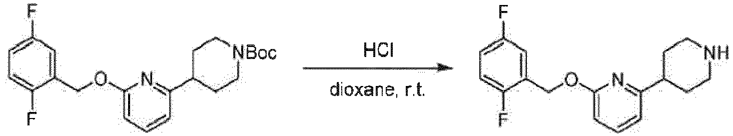

Tert-butyl 4-(6-((2,5-difluorobenzyl)oxy)pyridin-2-yl)piperidin-1-formate (88.0 mg, 0.22 mmol) was added to hydrochloric acid in dioxane (1.00 mmol/mL, 3 mL) at room temperature, and the reaction was carried out at room temperature for 1 hour under N₂ protection.

After the completion of the reaction, the reaction solution was concentrated under reduced pressure. 58.0 mg of milky white solid 2-((2,5-fluorobenzyl)oxy)-6-(piperidin-4-yl)pyridine was obtained (yield: 87.4%), which was used directly in the next reaction step. LC-MS: RT = 1.68 min, [M+H]⁺ = 305.22.

### Step F: Synthesis of methyl 2-((S)-1-(4-(6-(2,5-difluorobenzyl)oxy)pyridin-2-yl)piperidin-1-yl)ethyl)-1-(((S)-oxetan-2-yl)methyl)-1H-benzo[d]imidazol-6-carboxylate

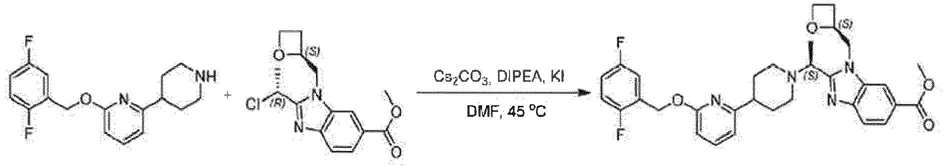

2-((2,5-Fluorobenzyl)oxy)-6-(piperidin-4-yl)pyridine (58.0 mg, 0.19 mmol), methyl 2-((R)-1-chloroethyl)-1-(((S)-oxetan-2-yl)methyl)-1*H-*benzo[*d*]imidazol-6-carboxylate (58.5 mg, 0.19 mmol), potassium iodide (31.5 mg, 0.19 mmol), and cesium carbonate (123.1 mg, 0.38 mmol) were added to *N*,*N-*dimethylformamide (10.0 mL) at room temperature, followed by the addition of *N,N-*diisopropylethylamine (1.0 mL). The reaction was carried out at 45°C for 3.0 hours under N₂ protection.

After the completion of the reaction, the reaction was quenched with water, and the reaction solution was extracted with ethyl acetate (50 mL × 3 times). The organic phases were combined, washed with saturated saline (30 mL × 3 times), dried with anhydrous sodium sulfate, and finally purified by silica gel column chromatography (eluent: dichloromethane/methanol = 20/1) to give 68.0 mg of light yellow solid methyl 2-((S)-1-(4-(6-((2,5-difluorobenzyl)oxy)pyridin-2-yl)piperidin-1-yl)ethyl)-1-(((S)-oxetan-2-yl)methyl)-1*H*-benzo[*d*]imidazol-6-carboxylate (yield: 62.1%). LC-MS: RT = 1.85 min, [M+H]⁺ = 577.39.

### Step G: Synthesis of 2-((S)-1-(4-(6-((2,5-difluorobenzyl)oxy)pyridin-2-yl)piperidin-1-yl)ethyl)-1-(((S)-oxetan-2-yl)methyl)-1H-benzo[d]imidazol-6-carboxylic acid

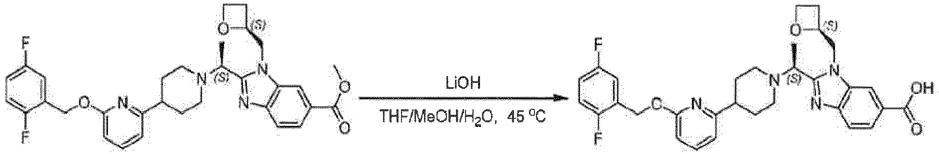

Lithium hydroxide (20.0 mg, 0.48 mmol) in water (1.0 mL) was added dropwise to tetrahydrofuran (3.0 mL) containing methyl 2-((S)-1-(4-(6-((2,5-difluorobenzyl)oxy)pyridin-2-yl)piperidin-1-yl)ethyl)-1-(((S)-oxetan-2-yl)methyl)-1*H-*benzo[*d*]imidazol-6-carboxylate (68.0 mg, 0.12 mmol) at room temperature, followed by the addition of 1.0 mL of methanol. The reaction was carried out at 45°C for 3.0 hours.

After the completion of the reaction, the reaction was quenched with water and the pH was adjusted to 6 with aqueous ammonium chloride solution (0.5 mol/L). The reaction solution was extracted with dichloromethane (30 mL × 3 times), and the organic phases was combined, washed with aqueous ammonium chloride solution (30 mL × 2 times), and dried with anhydrous sodium sulfate to give a crude product, which was purified by preparative high performance liquid chromatography. The purification conditions are as follows: column: Agilent 5 Prep-C18 100 mm×30 mm 5 µM; mobile phase: water (containing 0.05% ammonia) and acetonitrile; flow rate: 20 mL/min; gradient: 28% acetonitrile, eluted at 8.00 min; detection wavelength: 254 nm. After purification and lyophilization, 24.0 mg of the white solid compound 2-((S)-1-(4-(6-((2,5-difluorobenzyl)oxy)pyridin-2-yl)piperidin-1-yl)ethyl)-1-(((S)-oxetan-2-yl)methyl)-1*H*-benzo[*d*]imidazol-6-carboxylic acid was obtained (yield: 35.8%).

LC-MS: RT = 1.79 min, [M+H]+ = 563.32_{∘} ¹H NMR (400 MHz, DMSO) δ 12.70 (brs, 1H), 8.30-8.29 (m, 1H), 7 83-7.79 (m, 1H), 7.69 (d, *J* - 8.5 Hz, 1H), 7.65-7.60 (m, 1H), 7.38-7.34 (m, 1H), 7.31-7.19 (m, 2H), 6.85 (d, *J* = 7.2 Hz, 1H), 6.69 (d, *J* = 8.2 Hz, 1H), 5.36 (brs, 2H), 5.20-5.15 (m, 1H), 4.89-4.82 (m, 1H), 4.68 (dd, *J* = 15.1, 5.2 Hz, 1H), 4.50-4.42 (m, 2H), 4.19 (dt, *J* = 9.0, 5.7 Hz, 1H), 2 99 -2.96 (m, 1H), 2.70-2.49 (m, 4H), 2.38 -2.21 (m, 2H), 1.86- 1.68 (m, 3H), 1.58-1 45 (m, 1H), 1.48 (d, *J =* 6.6 Hz, 3H),

### Example 6 2-((S)-1-(4-(6-((2,4-difluorobenzyl)oxy)pyridin-2-yl)piperidin-1-yl)ethyl)-1-(((S)-oxetan-2-yl)methyl)-1H-benzo[d]imidazol-6-carboxylic acid

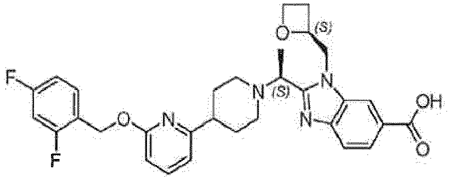

The specific synthetic route is as follows.

### Step A: Synthesis of tert-butyl 4-(6-((2,4-difluorobenzyl)oxy)pyridin-2-yl)piperidin-1-formate

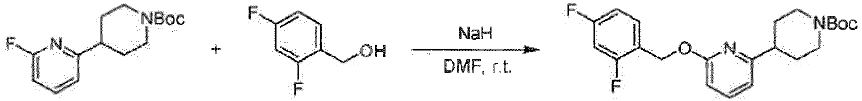

(2,4-Difluorophenyl)methanol (50.0 mg, 0.35 mmol) was added to *N*,*N-*dimethylformamide (4.0 mL) under an ice bath, followed by the addition of 60 wt. % sodium hydride (28.0 mg, 0.70 mmol) in batches. After stirring for 0.5 hour at room temperature under N₂ protection, tert-butyl 4-(6-fluoropyridin-2-yl)piperidin-1-formate (98.0 mg, 0.35 mmol) was added, and the reaction was carried out for 1.0 hour at room temperature.

After the completion of the reaction, the reaction was quenched with water, and the reaction solution was extracted with ethyl acetate (30 mL × 3 times). The organic phases were combined, washed with saturated saline (20 mL × 2 times), dried with anhydrous sodium sulfate, and finally concentrated under reduced pressure. The resulting residue was purified by silica gel column chromatography (eluent: ethyl acetate/n-hexane = 50/1) to obtain 76.0 mg of white solid tert-butyl 4-(6-((2,4-difluorobenzyl)oxy)pyridin-2-yl)piperidin-1-formate (yield: 54.2%). LC-MS: RT = 2.33 min, [M+H-56]⁺ = 349.33.

### Step B: Synthesis of 2-((2,4-difluorobenzyl)oxy)-6-(piperidin-4-yl)pyridine

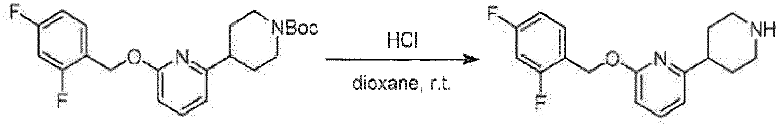

Tert-butyl 4-(6-((2,4-difluorobenzyl)oxy)pyridin-2-yl)piperidin-1-formate (76.0 mg, 0.19 mmol) was added to hydrochloric acid in dioxane (1.00 mmol/mL, 3 mL) at room temperature, and the reaction was carried out at room temperature for 1 hour under N₂ protection.

After the completion of the reaction, the reaction solution was concentrated under reduced pressure. 43.0 mg of milky white solid 2-((2,4-difluorobenzyl)oxy)-6-(piperidin-4-yl)pyridine (yield: 74.4%) was obtained, which was used directly in the next reaction step. LC-MS: RT= 1.68 min, [M+H]⁺ = 305.22.

### Step C: Synthesis of methyl 2-((S)-1-(4-(6-((2,4-difluorobenzyl)oxy)pyridin-2-yl)piperidin-1-yl)ethyl)-1-(((S)-oxetan-2-yl)methyl)-1H-benzo[d]imidazol-6-carboxylate

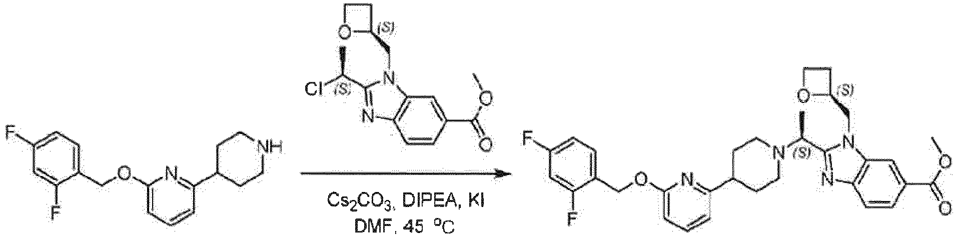

2-((2,4-Difluorobenzyl)oxy)-6-(piperidin-4-yl)pyridine (43.0 mg, 0.14 mmol), methyl 2-((S)-1-chloroethyl)-1-(((S)-oxetan-2-yl)methyl)-1*H-*benzo[*d*]imidazol-6-carboxylate (43.5 mg, 0.14 mmol), potassium iodide (23.2 mg, 0.14 mmol), and cesium carbonate (91.3 mg, 0.28 mmol) were added to *N*,*N*-dimethylformamide (10.0 mL) at room temperature, followed by the addition of *N,N-*diisopropylethylamine (1.0 mL). The reaction was carried out at 45°C for 3.0 hours under N₂ protection.

After the completion of the reaction, the reaction was quenched with water, and the reaction solution was extracted with ethyl acetate (50 mL × 3 times). The organic phases were combined, washed with saturated saline (30 mL × 3 times), dried with anhydrous sodium sulfate, and finally purified by silica gel column chromatography (eluent: dichloromethane/methanol = 20/1) to give 59.0 mg of a light yellow solid methyl 2-((S)-1-(4-(6-((2,4-difluorobenzyl)oxy)pyridin-2-yl)piperidin-1-yl)ethyl)-1-(((S)-oxetan-2-yl)methyl)-1*H-*benzo[*d*]imidazol-6-carboxylate (yield: 73.2%). LC-MS: RT = 1.85 min, [M+H]⁺ = 577.39.

### Step D: Synthesis of 2-((S)-1-(4-(6-((2,4-difluorobenzyl)oxy)pyridin-2-yl)piperidin-1-yl)ethyl)-1-(((S)-oxetan-2-yl)methyl)-1H-benzo[d]imidazol-6-carboxylic acid

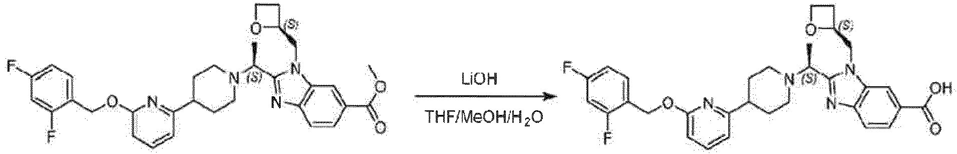

Lithium hydroxide (16.8 mg, 0.40 mmol) in water (1.0 mL) was added dropwise to tetrahydrofuran (3.0 mL) containing methyl 2-((S)-1-(4-(6-((2,4-difluorobenzyl)oxy)pyridin-2-yl)piperidin-1-yl)ethyl)-1-(((S)-oxetan-2-yl)methyl)-1*H*-benzo[*d*]imidazol-6-carboxylate (59.0 mg, 0.10 mmol) at room temperature, followed by the addition of 1.0 mL of methanol. The reaction was carried out at 45°C for 3.0 hours.

The reaction was quenched with water and the pH was adjusted to 6 with aqueous ammonium chloride solution (0.5 mol/L). The reaction solution was extracted with dichloromethane (30 mL × 3 times), and the organic phases were combined, washed with aqueous ammonium chloride solution (30 mL × 2 times), dried with anhydrous sodium sulfate to give a crude product, which was purified by silica gel column chromatography (eluent: dichloromethane/methanol = 10/1) to obtain 17.3 mg of a light yellow solid 2-((S)-1-(4-(6-((2,4-difluorobenzyl)oxy)pyridin-2-yl)piperidin-1-yl)ethyl)-1-(((S)-oxetan-2-yl)methyl)-1*H*-benzo[*d*]imidazol-6-carboxylic acid (yield: 32.0%). LC-MS: RT = 1.79 min, [M+H]⁺ = 563.32.

1H NMR (500 MHz, DMSO) δ 8.29 (s, 1H), 8.04 (s, 1H), 7.81 (dd, *J* = 8.4, 1.6 Hz, 1H), 7.69 (d, *J* = 8 5 Hz, 1H), 7.10-72.1 (m, 1H), 7.11-7.04 (m, 1H), 6.84 (d, *J* = 7.2 Hz, 1H), 6 65 (d, *J* = 7.7 Hz, 1H), 6.15 (t, *J* = 6.7 Hz, 1*H*), 5.35 (s, 2H), 5.27 (d, *J* = 4.2 Hz, 1H), 5.04 (t, *J* = 5.2 Hz, 1H), 4.85 (dd, *J* = 155, 3.1 Hz, 1H), 4.69 (dd, *J* = 15.8, 5.8 Hz, 1H), 4.49-4.42 (m, 3H), 4.07 (d, *J* = 2.1 Hz, 3H), 3.80-3.77 (m, 1H), 2.11 (dd, *J* = 8.4, 6.8 Hz, 3H), 1.72 (dd, *J* = 16.0, 7.2 Hz, 3H), 1.48 (d, *J =* 6.7 Hz, 3H).

### Example 7 2-((S)-1-(4-(6-((5-chloro-2-fluorobenzyl)oxy)pyridin-2-yl)piperidin-1-yl)ethyl)-3-(((S)-oxetan-2-yl)methyl)-3H-imidazolo[4,5-b]pyridin-5-carboxylic acid

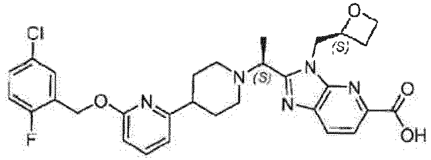

The specific synthetic route is as follows.

### Step A: Synthesis of methyl 5-((S)-2-chloropropionamido)-6-((((S)-oxetan-2-yl)methyl)amino)picolinate

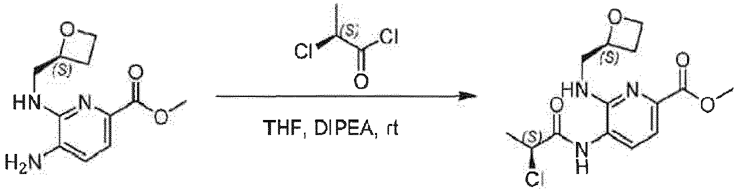

Methyl (S)-5-amino-6-(((oxetan-2-yl)methyl)amino)picolinate (0.8 g, 2.3 mmol) was dissolved in tetrahydrofuran (30.0 mL), followed by the addition of triethylamine (1.0 mL, 8.0 mmol). The mixed solution was stirred at 0° C for 15 min, followed by a slow dropwise addition of a tetrahydrofuran solution (10.0 mL) of (S)-2-chloropropionyl chloride (350.0 mg, 2.76 mmol). The solution was stirred at room temperature for 1 hour and the reaction was monitored by LC-MS until the completion.

Water (20.0 mL) was added to the reaction solution, which was then extracted with ethyl acetate (40 mL × 3 times). The organic phases were combined, washed with saturated saline (25.0 mL × 3 times), dried with anhydrous sodium sulfate, and concentrated under reduced pressure to give a residue, which was purified by silica gel column chromatography to obtain 600 mg yellow solid methyl 5-((S)-2-chloropropionamido)-6-((((S)-oxetan-2-yl)methyl)amino)picolinate (yield: 62.2%). LC-MS: RT = 1.73 min, [M+H] = 328.21.

### Step B: Synthesis of methyl 2-((S)-1-chloroethyl)-3-(((S)-oxetan-2-yl)methyl)-3H-imidazolo[4,5-b]pyridin-5-carboxylate

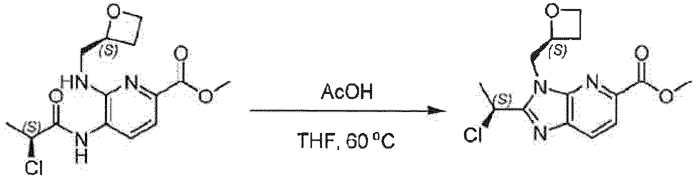

Methyl 5-((S)-2-chloropropionamido)-6-((((S)-oxetan-2-yl)methyl)amino)picolinate (0.4 g, 1.23 mmol) was dissolved in tetrahydrofuran (10.0 mL), followed by the addition of acetic acid (1.0 mL). The mixed solution was stirred at 60°C for 2 hours, and the reaction was monitored by LC-MS until the completion.

Water (20.0 mL) was added to the reaction solution, which was then extracted with ethyl acetate (40 mL × 3 times). The organic phases were combined, washed with saturated saline (25.0 mL × 3 times), dried with anhydrous sodium sulfate, and concentrated under reduced pressure to give a residue, which was purified by silica gel column chromatography to obtain 300 mg yellow solid methyl 2-((S)-1-chloroethyl)-3-(((S)-oxetan-2-yl)methyl)-3*H*-imidazolo[4,5-*b*]pyridin-5-carboxylate (yield: 78.3%). LC-MS: RT = 1.79 min, [M+H]⁺ = 310.11.

### Step C: Synthesis of methyl 2-((S)-1-(4-(6-((5-chloro-2-fluorobenzyl)oxy)pyridin-2-yl)piperidin-1-yl)ethyl)-3-(((S)-oxetan-2-yl)methyl)-3H-imidazolo[4,5-b]pyridin-5-carboxylate

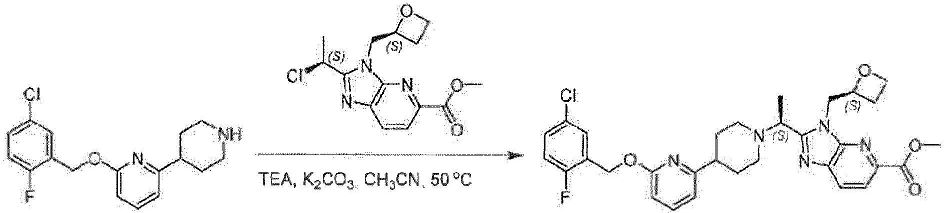

2-((5-Chloro-2-fluorobenzyl)oxy)-6-(piperidin-4-yl)pyridine (100.0 mg, 0.28 mmol) was dissolved in acetonitrile (10.0 mL) and triethylamine (2.0 mL), followed by the addition of potassium carbonate (77.0 mg, 0.56 mmol), potassium iodide (93.0 mg, 0.56 mmol), and methyl 2-((S)-1-chloroethyl)-3-(((S)-oxetan-2-yl)methyl)-3*H-*imidazolo[4,5-*b*]pyridin-5-carboxylate (82.0 mg, 0.28 mmol). The mixed solution was stirred at 50°C for 2 hours and the reaction was monitored by TLC until the completion.

Water (30.0 mL) was added to the reaction solution, which was then extracted with ethyl acetate (40 mL × 3 times). The organic phases were combined, washed with saturated saline (25.0 mL × 3 times), dried with anhydrous sodium sulfate, and concentrated under reduced pressure to obtain 120 mg yellow solid methyl 2-((S)-1-(4-(6-((5-chloro-2-fluorobenzyl)oxy)pyridin-2-yl)piperidin-1-yl)ethyl)-3-(((S)-oxetan-2-yl)methyl)-3*H*-imidazolo[4,5-*b*]pyridin-5-carboxylate (yield: 72.3%). LC-MS: RT = 1.81 min, [M+H]⁺ = 594.21.

### Step D: Synthesis of 2-((S)-1-(4-(6-((5-chloro-2-fluorobenzyl)oxy)pyridin-2-yl)piperidin-1-yl)ethyl)-3-(((S)-oxetan-2-yl)methyl)-3H-imidazolo[4,5-b]pyridin-5-carboxylic acid

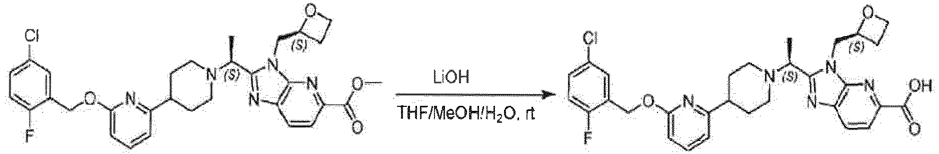

Methyl 2-((S)-1-(4-(6-((5-chloro-2-fluorobenzyl)oxy)pyridin-2-yl)piperidin-1-yl)ethyl)-3-(((S)-oxetan-2-yl)methyl)-3*H-*imidazolo[4,5-*b*]pyridin-5-carboxylate (120.0 mg, 0.73 mmol) was dissolved in tetrahydrofuran (30.0 mL) and methanol (10.0 mL), followed adding a solution of lithium hydroxide (84.0 mg, 2.0 mmol) in water (10.0 mL), and the mixed solution was stirred at room temperature for 2 hours.

After the completion of the reaction as monitored by LC-MS, the reaction solution was extracted with dichloromethane/methanol (9/1, 20.0 mL × 3 times). The organic phases were combined, washed with saturated saline (15.0 mL × 3 times), dried with anhydrous sodium sulfate, and concentrated under reduced pressure. The resulting residue was purified by silica gel column chromatography to obtain 46 mg of white solid 2-((S)-1-(4-(6-((5-chloro-2-fluorobenzyl)oxy)pyridin-2-yl)piperidin-1-yl)ethyl)-3-(((S)-oxetan-2-yl)methyl)-3*H*-imidazolo[4,5-*b*]pyridin-5-carboxylic acid (yield: 63.8%).

LC-MS: RT = 1.77 min, [M-H]⁺= 580.31. ¹H NMR (400 MHz, DMSO) δ 8.17 (d, *J* = 8.2 Hz, 1H), 7.99 (d, *J* = 8.2 Hz, 1H), 7.61 (dd, *J* = 10.8, 4 9 Hz, 2H), 7.45-7 38 (m, 1H), 7 27 (t, *J* = 9.3 Hz, 1H), 6.84 (d, *J* = 7.3 Hz, 1H), 6 67 (d, *J* = 8.2 Hz, 1H), 5.36 (s, 2H), 5.31 (s, 1H), 4.85 (dd, *J* = 15.0, 4.3 Hz, 1H), 4.79-4.68 (m, 1H), 4.61 (d, *J =* 6 6 Hz, 1H), 4 41 (dd, *J =* 13.7, 7.7 Hz, 1H), 4.13-4.02 (m, 1H), 3.00 (d, *J* = 11.4 Hz, 1H), 2 60 (dd, *J* = 22.6, 10.7 Hz, 4H), 2.36 (s, 1H), 2,30-2.21 (m, 1H), 1 80 (d, *J* = 23.1 Hz, 2H), 1.73-1.63 (m, 1H), 1 56 (d, *J* = 86 Hz, 1H), 1.48 (d, *J* = 6 6 Hz, 3H).

### Example 8 2-((S)-1-(4-(6-((1-(2,2-difluoroethyl)-1H-indazol-6-yl)methoxy)pyridin-2-yl)piperidin-1-yl)ethyl)-1-(((S)-oxetan-2-yl)methyl)-1H-benzo[d]imidazol-6-carboxylic acid

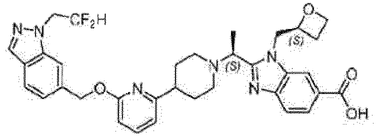

The specific synthetic route is as follows.

### Step A: Synthesis of methyl 2-((S)-1-(4-(6-((1-(2,2-difluoroethyl)-1H-indazol-6-yl)methoxy)pyridin-2-yl)piperidin-1-yl)ethyl)-1-(((S)-oxetan-2-yl)methyl)-1H-benzo[d]imidazol-6-carboxylate

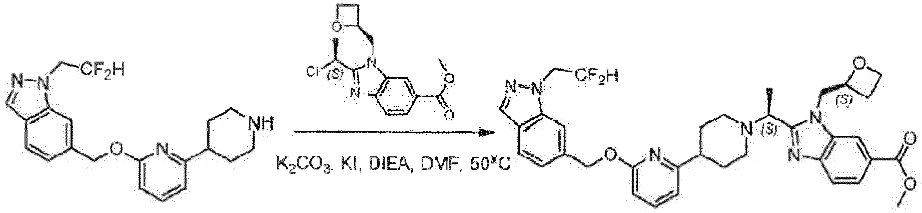

1-(2,2-Difluoroethyl)-6-(((6-(piperidin-4-yl)pyridin-2-yl)oxy)methyl)-1*H-*indazole (150 mg, 0.40 mmol), DIEA (1 mL), potassium iodide (133 mg, 0.80 mmol) and potassium carbonate (110 mg, 0.80 mmol) were dissolved in *N*,*N-*dimethylformamide (10 mL) at room temperature, followed by the addition of methyl 2-((S)-1-chloroethyl)-1-(((S)-oxetan-2-yl)methyl)-1*H-*benzo[*d*]imidazol-6-carboxylate (124 mg, 0.40 mmol), and the reaction was carried out at a temperature of 50°C.

After the completion of the reaction, the reaction was quenched with saturated sodium chloride, and the reaction solution was extracted with ethyl acetate (20 mL × 2 times). The organic phases were combined, dried, and concentrated to give a crude product, which was purified by silica gel column chromatography (eluent: ethyl acetate/n-hexane = 1/8) to obtain 69.0 mg of white solid methyl 2-((S)-1-(4-(6-((1-(2,2-difluoroethyl)-1*H-*indazol-6-yl)methoxy)pyridin-2-yl)piperidin-1-yl)ethyl)-1-(((S)-oxetan-2-yl)methyl)-1*H*-benzo[*d*]imidazol-6-carboxylate (yield: 27%). LC-MS: RT = 1.79 min, [M+H]⁺ = 645.42.

### Step B: Synthesis of 2-((S)-1-(4-(6-((1-(2,2-difluoroethyl)-1H-indazol-6-yl)methoxy)pyridin-2-yl)piperidin-1-yl)ethyl)-1-(((S)-oxetan-2-yl)methyl)-1H-benzo[d]imidazol-6-carboxylic acid

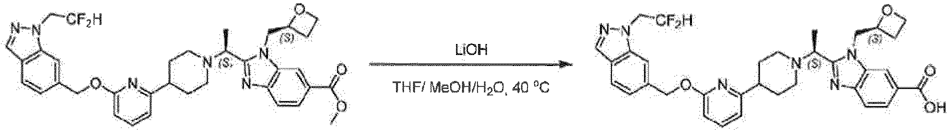

Methyl 2-((S)-1-(4-(6-((1-(2,2-difluoroethyl)-1*H-*indazol-6-yl)methoxy)pyridin-2-yl)piperidin-1-yl)ethyl)-1-(((S)-oxetan-2-yl)methyl)-1*H-*benzo[*d*]imidazol-6-carboxylate (69.0 mg, 0.11 mmol) was dissolved in a solvent mixture (5 mL, tetrahydrofuran/methanol/water = 2.5/2.5/1) at room temperature, followed by the addition of lithium hydroxide (16.0 mg, 0.44 mmol), and the reaction was carried out at a temperature of 40°C.

After the completion of the reaction, the reaction was quenched with saturated ammonium chloride, and the reaction solution was sequentially extracted with ethyl acetate (10 mL × 2 times) and a solvent mixture (10 mL × 2 times, dichloromethane/methanol = 30/1). The organic phases were combined, dried with anhydrous sodium sulfate, filtered, and concentrated to give a crude product, which was purified by silica gel column chromatography (eluent: dichloromethane/methanol = 20/1) to obtain 19.73 mg of white solid 2-((S)-1-(4-(6-((1-(2,2-difluoroethyl)-1*H-*indazol-6-yl)methoxy)pyridin-2-yl)piperidin-1-yl)ethyl)-1-(((S)-oxetan-2-yl)methyl)-1*H-*benzo[*d*]imidazol-6-carboxylic acid (yield: 29%).

LC-MS: RT = 1.75 min, [M+H]⁺ = 631.40. ¹H NMR (400 MHz, DMSO-d₆) *δ* 12 73 (s, 1H), 8 27 (s, 1H), 8 12 (s, 1H), 7.77 (dd, *J* = 18.7, 7.8 Hz, 3H), 7.70-7.56 (m, 2H), 7.26 (d, *J* = 8.3 Hz, 1H), 6 82 (d, *J* = 7.4 Hz, 1H), 6.67 (d, *J* = 8.1 Hz, 1H), 6.52 (s, 0.3 1H), 6.39 (t, *J* = 3.4 Hz, 0.47H), 6.25 (s, 0.29H), 5.45 (s, 2H), 5.16 (s, 1H), 4.90 (d, *J*= 12.1 Hz, 1H), 4.66 (d, *J*= 10.9 Hz, 1H), 4.52-4.37 (m, 2H), 4.22-4.11 (m, 1H), 2.96 (s, 1H), 2.62 (s, 2H), 2.39-2.11 (m, 3H), 1.77 (dd, *J* = 33.1, 20.9 Hz, 4H), 1.51 (dd, *J* = 35.0, 7.7 Hz, 4H).

### Example 9 1-(((S)-oxetan-2-yl)methyl)-2-((S)-1-(4-(6-((1-(2,2,2-trifluoroethyl)-1H-indazol-6-yl)methoxy)pyridin-2-yl)piperidin-1-yl)ethyl)-1H-benzo[d]imidazol-6-carboxylic acid

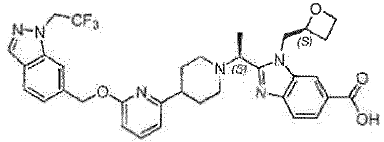

The specific synthetic route is as follows.

### Step A: Synthesis of methyl 1-(((S)-oxetan-2-yl)methyl)-2-((S)-1-(4-(6-((1-(2,2,2-trifluoroethyl)-1H-indazol-6-yl)methoxy)pyridin-2-yl)piperidin-1-yl)ethyl)-1H-benzo[d]imidazol-6-carboxylate

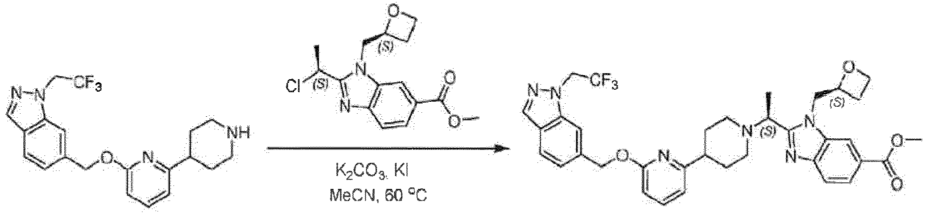

6-(((6-(Piperidin-4-yl)pyridin-2-yl)oxy)methyl)-1-(2,2,2-trifluoroethyl)-1*H-*indazole (75 mg, 0.176 mmol), potassium carbonate (48.6 mg, 0.352 mmol) and potassium iodide (58 mg, 0.352 mmol) were dissolved in 5 mL of anhydrous acetonitrile, followed by the addition of methyl 2-(S)-1-chloroethyl)-1-(((S)-oxetan-2-yl)methyl)-1*H-*benzo[*d*]imidazol-6-carboxylate (54 mg, 0.176 mmol), and the reaction was carried out at 60°C for 2 hours.

After the completion of the reaction, the reaction solution was diluted with water and extracted with ethyl acetate (30 mL × 2 times). The organic phases were combined, washed with saturated saline (20 mL × 2 times), dried with anhydrous sodium sulfate, and finally concentrated under reduced pressure to give 96 mg of a light yellow solid methyl 1-(((S)-oxetan-2-yl)methyl)-2-((S)-1-(4-(6-((1-(2,2,2-trifluoroethyl)- 1*H-*indazol-6-yl)methoxy)pyridin-2-yl)piperidin-1-yl)ethyl)-1*H-*benzo[*d*]imidazol-6-carboxylate. LC-MS: RT = 1.86 min, [M+H]⁺ = 663.38.

### Step B: Synthesis of 1-(((S)-oxetan-2-yl)methyl)-2-((S)-1-(4-(6-((1-(2,2,2-trifluoroethyl)-1H-indazol-6-yl)methoxy)pyridin-2-yl)piperidin-1-yl)ethyl)-1H-benzo[d]imidazol-6-carboxylic acid

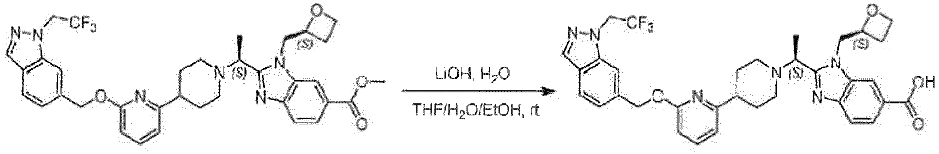

Methyl 1-(((S)-oxetan-2-yl)methyl)-2-((S)-1 -(4-(6-((1-(2,2,2-trifluoroethyl)-1*H*-indazol-6-yl)methoxy)pyridin-2-yl)piperidin-1-yl)ethyl)-1*H-*benzo[*d*]imidazol-6-carboxylate (96 mg, 0.145 mmol) was dissolved in 4 mL of tetrahydrofuran, followed by the addition of lithium hydroxide monohydrate (31 mg, 0.723 mmol), 1 mL of water, and 1 mL of ethanol. The reaction was carried out at room temperature for 50 min.

After the completion of the reaction, the reaction solution was neutralized with 20 mL of saturated ammonium chloride solution and extracted with ethyl acetate (30 mL × 2 times). The organic phases were combined, washed with saturated saline (20 mL × 2 times), dried with anhydrous sodium sulfate, and finally concentrated under reduced pressure to give a crude product, which was purified by column chromatography to obtain 42 mg of light yellow solid 1-(((S)-oxetan-2-yl)methyl)-2-((S)-1-(4-(6-((1-(2,2,2-trifluoroethyl)-1*H-*indazol-6-yl)methoxy)pyridin-2-yl)piperidin-1-yl)ethyl)-1*H-*benzo[d]imidazol-6-carboxylic acid.

¹H NMR (500 MHz, DMSO) δ 12.65 (s, 1H), 8.29 (s, 1H), 8.19 (d, *J* = 0.9 Hz, 1H), 7.88 (s, 1H), 7.77-7.83 (m, 2H), 7.69 (d, *J* = 3.4 Hz, 1H), 7.59-7.65 (m, 1H), 7.31 (dd, *J* = 8.3, 1.2 Hz, 1H), 6.84 (d, *J* = 7.3 Hz, 1H), 6.69 (d, *J* = 8.2 Hz, 1H), 5.48 (s, 2H), 5.42 (q, *J* = 9.2 Hz, 2H), 5.12-5.18 (m,1H), 4.80-4.84 (m, 1H), 4.66-4.70 (m, 1H), 4.40-4.49 (m, 2H), 4.16-4.20 (m, 1H), 2.95-3.02 (m, 1H), 2.54-2.69 (m, 4H), 2.22-2.35 (m, 2H), 1.85-1.90(m, 1H), 1.72-1.78 (m, 2H), 1.53-1.60 (m, 1H), 1.48 (d, *J*= 6.5 Hz, 3H) LC-MS: RT =1.77 min, [M+H]⁻ = 649.37.

### Example 10 2-((S)-1-(4-(6-(isoquinolin-6-yl-methoxy)pyridin-2-yl)piperidin-1-yl)ethyl)-1-(((S)-oxetan-2-yl)methyl)-1H-benzo[d]imidazol-6-carboxylic acid

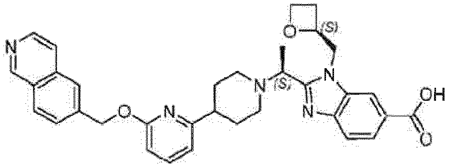

The specific synthetic route is as follows.

### Step A: Synthesis of methyl 2-((S)-1-(4-(6-(isoquinolin-6-yl-methoxy)pyridin-2-yl)piperidin-1-yl)ethyl)-1-(((S)-oxetan-2-yl)methyl)-1H-benzo[d]imidazol-6-carboxylate

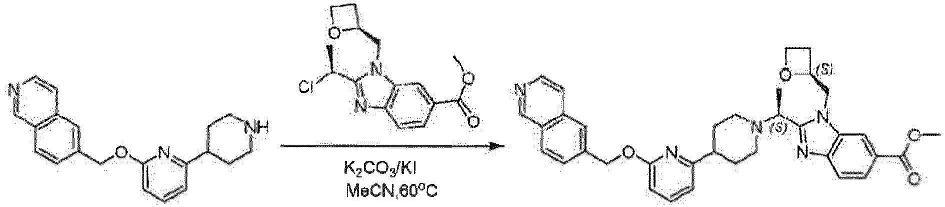

6-(((6-(Piperidin-4-yl)pyridin-2-yl)oxy)methyl)isoquinoline (100 mg, 0.281 mmol), potassium carbonate (73.1 mg, 0.563 mmol), and potassium iodide (92 mg, 0.563 mmol) were dissolved in 5 mL of anhydrous acetonitrile, followed by the addition of methyl 2-(S)-1-chloroethyl)-1-(((S)-oxetan-2-yl)methyl)-1*H*-benzo[*d*]imidazol-6-carboxylate (86 mg, 0.281 mmol), and the reaction was carried out at 60°C for 2 hours. After the completion of the reaction, the reaction solution was diluted with water and extracted with ethyl acetate (30 mL × 2 times). The organic phases were combined, washed with saturated saline (20 mL × 2 times), dried with anhydrous sodium sulfate, and finally concentrated under reduced pressure to give 78 mg of a light yellow solid methyl 2-((S)-1-(4-(6-(isoquinolin-6-yl-methoxy)pyridin-2-yl)piperidin-1-yl)ethyl)-1-(((S)-oxetan-2-yl)methyl)-1*H-*benzo[*d*]imidazol-6-carboxylate. LC-MS: RT = 1.71 min, [M+H]⁺ = 592.28.

### Step B: Synthesis of 2-((S)-1-(4-(6-(isoquinolin-6-yl-methoxy)pyridin-2-yl)piperidin-1-yl)ethyl)-1-(((S)-oxetan-2-yl)methyl)-1H-benzo[d]imidazol-6-carboxylic acid

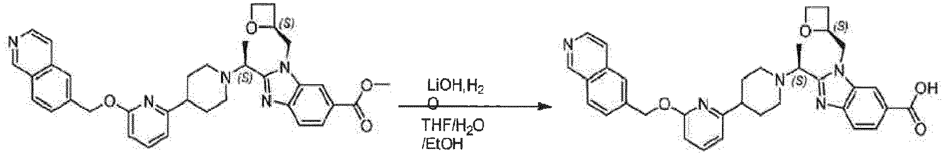

Methyl 2-((S)-1-(4-(6-(isoquinolin-6-yl-methoxy)pyridin-2-yl)piperidin-1-yl)ethyl)-1-(((S)-oxetan-2-yl)methyl)-1*H-*benzo[*d*]imidazol-6-carboxylate (78 mg, 0.131 mmol) was dissolved in 4 mLof tetrahydrofuran, followed by the addition of lithium hydroxide monohydrate (28 mg, 0.655 mmol), 1 mL of water, and 1 mL of ethanol. The reaction was carried out at room temperature for 50 min.

After the completion of the reaction, the reaction solution was neutralized with 20 mL of saturated ammonium chloride solution, and extracted with ethyl acetate (30 mL × 2 times). The organic phases were combined, washed with saturated saline (20 mL × 2 times), dried with anhydrous sodium sulfate, and finally concentrated under reduced pressure to give a crude product, which was purified by column chromatography to obtain 38 mg of light yellow solid 2-((S)-1-(4-(6-(isoquinolin-6-yl-methoxy)pyridin-2-yl)piperidin-1-yl)ethyl)-1-(((S)-oxetan-2-yl)methyl)-1*H-*benzo[*d*]imidazol-6-carboxylic acid.

¹H NMR (400 MHz, DMSO) δ 12 20 (s, 1H), 9.27 (s, 1H), 8.40 (d, *J* = 5.5 Hz, 1H), 8.28-8.33 (m, 1H), 8.10 (d, *J=* 8.5 Hz, 1H), 7.89 -8.04 (m, 1H), 7.80-7.85 (m, 1H), 7.68-7.78 (m, 3H), 7.60-7.67 (m, 1H), 6.84 (d, *J* = 7.1 Hz, 1H), 6 73 (d, *J* = 8.0 Hz, 1H), 5.56 (s, 2H), 5.15-5.20 (m, 1H), 4.81-4.86 (m, 1H), 4.64-4.74 (m, 1H), 4.38-4.51 (m, 2H), 4.15-4.20 (m, 1H), 2.94-3.00 (m, 1H), 2.57-2.66 (m, 2H), 2.14-2.35 (m, 4H), 1.77-1.85 (m, 2H), 1.66-1.75 (m, 2H), 1.45-1.52 (m, 3H). LC-MS: RT =1.60 min, [M-H]⁻ =578.39.

### Example 11 2-((S)-1-(4-(6-((1-(2,2-difluoroethyl)-1H-indazol-6-yl)methoxy)pyridin-2-yl)piperidin-1-yl)ethyl)-3-(((S)-oxetan-2-yl)methyl)-3H-imidazo[4,5-b]pyridin-5-carboxylic acid

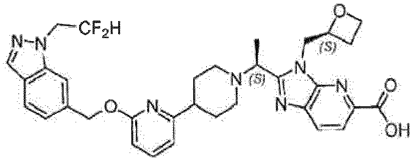

The specific synthetic route is as follows.

### Step A: Synthesis of methyl 2-((S)-1-(4-(6-((1-(2,2-difluoroethyl)-1H-indazol-6-yl)methoxy)pyridin-2-yl)piperidin-1-yl)ethyl)-3-(((S)-oxetan-2-yl)methyl)-3H-imidazo[4,5-b]pyridin-5-carboxylate

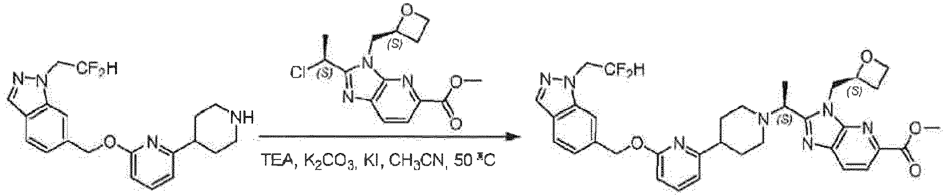

1-(2,2-difluoroethyl)-6-(((6-(piperidin-4-yl)pyridin-2-yl)oxy)methyl)-1*H-*indazole (150.0 mg, 0.37 mmol) was dissolved in acetonitrile (10.0 mL) and triethylamine (2.0 mL), followed by the addition of potassium carbonate (102.0 mg, 0.74 mmol), potassium iodide (123.0 mg, 0.74 mmol), and methyl 2-((S)-1-chloroethyl)-3-(((S)-oxetan-2-yl)methyl)-3*H-*imidazolo[4,5-*b*]pyridin-5-carboxylate (114.0 mg, 0.37 mmol). The mixed solution was stirred at 50°C for 2 hours.

After the reaction was completed as monitored by TLC, the reaction solution was diluted with water (30.0 mL) and extracted with ethyl acetate (40 mL × 3 times). The organic phases were combined, washed with saturated saline (25.0 mL × 3 times), dried with anhydrous sodium sulfate, and concentrated under reduced pressure to obtain 180 mg yellow solid methyl 2-((S)-1-(4-(6-((1-(2,2-difluoroethyl)-1*H-*indazol-6-yl)methoxy)pyridin-2-yl)piperidin-1-yl)ethyl)-3-(((S)-oxetan-2-yl)methyl)-3*H*-imidazo[4,5-*b*]pyridin-5-carboxylate. LC-MS: RT = 1.78 min, [M+H]⁺ = 646.34.

### Step B: Synthesis of 2-((S)-1-(4-(6-((1-(2,2-difluoroethyl)-1H-indazol-6-yl)methoxy)pyridin-2-yl)piperidin-1-yl)ethyl)-3-(((S)-oxetan-2-yl)methyl)-3H-imidazo[4,5-b]pyridin-5-carboxylic acid

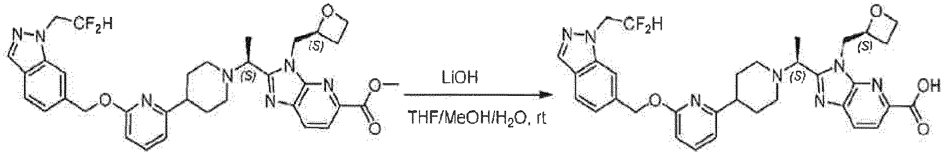

Methyl 2-((S)-1-(4-(6-((1-(2,2-difluoroethyl)-1*H-*indazol-6-yl)methoxy)pyridin-2-yl)piperidin-1-yl)ethyl)-3-(((S)-oxetan-2-yl)methyl)-3*H-*imidazo[4,5-*b*]pyridin-5-carboxylate (180.0 mg, 0.28 mmol) was dissolved in tetrahydrofuran (6.0 mL) and methanol (2.0 mL), followed by the addition of lithium hydroxide (21.0 mg, 0.5 mmol) in water (2.0 mL), and the mixed solution was stirred at room temperature for 1 hour.

After the completion of the reaction as monitored by LC-MS, the reaction solution was extracted with dichloromethane/methanol (9/1, 20.0 mL × 3 times). The organic phases were combined, washed with saturated saline (15.0 mL × 3 times), dried with anhydrous sodium sulfate, and finally concentrated under reduced pressure. The resulting residue was purified by silica gel column chromatography to obtain 64 mg of white solid 2-((S)-1-(4-(6-((1-(2,2-difluoroethyl)-1*H-*indazol-6-yl)methoxy)pyridin-2-yl)piperidin-1-yl)ethyl)-3-(((S)-oxetan-2-yl)methyl)-3*H-*imidazo[4,5-*b*]pyridin-5-carboxylic acid (yield: 36.2%).

LC-MS: RT = 1.73 min, [M+H]⁺ =632.35. ¹H NMR (400 MHz, DMSO) δ 8.14 (s, 1H), 8 06 (d, *J=* 8.1 Hz, 1H), 7.95 (d, *J* = 8.1 Hz, 1H), 7.81 (s, 1H), 7.75 (d, *J* = 8.3 Hz, 1H), 7.61 (t, *J* = 7.8 Hz, 1H), 7.26 (d, *J* = 8.2 Hz, 1H), 6.82 (d, *J* = 7 3 Hz, 1H), 6.67 (d, *J=* 8.2 Hz, 1H), 6.54 (t, *J* = 3.8 Hz, 0.27H), 6.40 (m, 0.49H), 6.27 (m, 0.28H), 5.45 (d, *J=* 3.3 Hz, 2H), 5.20 (s, 1H), 4.89 (td, *J* = 15.4, 3.5 Hz, 2H), 4.76 (s, 2H), 4.65-4.57 (m, 1H), 4.34 (dd, J= 13.5, 7 8 Hz, 1H), 4.06-4.01 (m, 1H), 4.00-3.93 (m, 1H), 2.98 (d, *J* = 10.5 Hz, 1H), 2.61 (d, *J* = 11.0 Hz, 2H), 2.23 (t, *J* = 10.8 Hz, 1H), 2.10 (d, *J* = 8.7 Hz, 1H), 1.99 (s, 1H), 1.92-1.67 (m, 3H), 1.62-1.50 (m, 1H), 1.47 (d, *J* = 6.7 Hz, 3H), 1.17 (t, *J* = 7.1 Hz, 1H), 1.17 (t, *J* = 7.1 Hz, I H).

### Example 12 2-((S)-1-(4-(6-((4-chloro-2-fluorobenzyl)oxy)pyridin-2-yl)piperidin-1-yl)ethyl)-3-(((S)-oxetan-2-yl)methyl)-3H-imidazolo[4,5-b]pyridin-5-carboxylic acid

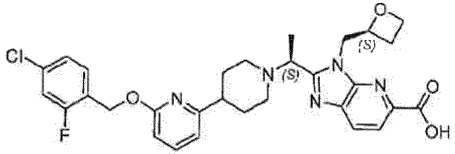

The specific synthetic route is as follows.

### Step A: Synthesis of methyl 2-((S)-1-(4-(6-((4-chloro-2-fluorobenzyl)oxy)pyridin-2-yl)piperidin-1-yl)ethyl)-3-(((S)-oxetan-2-yl)methyl)-3H-imidazolo[4,5-b]pyridin-5-carboxylate

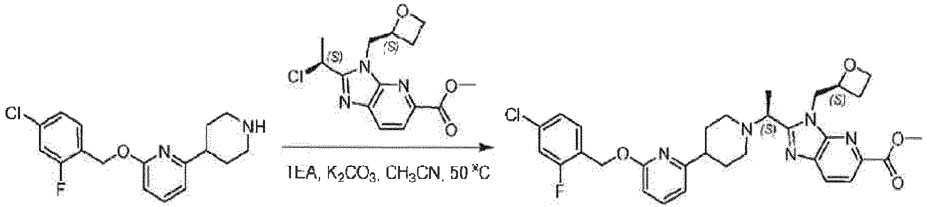

2-((4-Chloro-2-fluorobenzyl)oxy)-6-(piperidin-4-yl)pyridine (100 mg, 0.28 mmol) was dissolved in acetonitrile (10.0 mL) and triethylamine (2.0 mL), followed by the addition of potassium carbonate (77.0 mg, 0.56 mmol), potassium iodide (93.0 mg, 0.56 mmol), and methyl 2-((S)-1-chloroethyl)-3-(((S)-oxetan-2-yl)methyl)-3*H-*imidazolo[4,5-*b*]pyridin-5-carboxylate (82.0 mg, 0.28 mmol). The mixed solution was stirred at 50°C for 2 hours.

After the reaction was completed as monitored by TLC, the reaction solution was diluted with water (30.0 mL) and extracted with ethyl acetate (40.0 mL × 3 times). The organic phases were combined, washed with saturated saline (25.0 mL × 3 times), dried with anhydrous sodium sulfate, and concentrated under reduced pressure to obtain 120 mg yellow solid methyl 2-((S)-1-(4-(6-(4-chloro-2-fluorobenzyl)oxy)pyridin-2-yl)piperidin-l-yl)ethyl)-3-(((S)-oxetan-2-yl)methyl)-3*H*-imidazolo[4,5-*b*]pyridin-5-carboxylate (yield: 72.3%). LC-MS: RT = 1.83 min, [M+H]⁺ = 594.21.

### Step B: Synthesis of 2-((S)-1-(4-(6-((4-chloro-2-fluorobenzyl)oxy)pyridin-2-yl)piperidin-1-yl)ethyl)-3-(((S)-oxetan-2-yl)methyl)-3H-imidazolo[4,5-b]pyridin-5-carboxylic acid

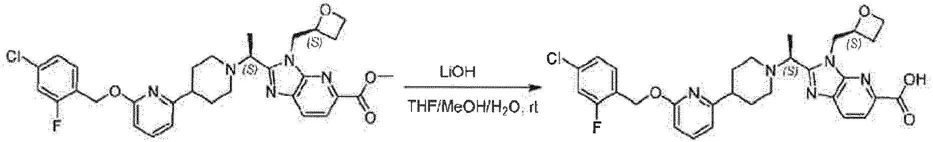

Methyl 2-((S)-1-(4-(6-((4-chloro-2-fluorobenzyl)oxy)pyridin-2-yl)piperidin-1-yl)ethyl)-3-(((S)-oxetan-2-yl)methyl)-3*H*-imidazolo[4,5-*b*]pyridin-5-carboxylate (120 mg, 0.73 mmol) was dissolved in tetrahydrofuran (30.0 mL) and methanol (10 mL), followed by the addition of lithium hydroxide (84 mg, 2.0 mmol) in water (10.0 mL), and the mixed solution was stirred at room temperature for 5 hours.

After the completion of the reaction as monitored by LC-MS, the reaction solution was extracted with dichloromethane/methanol (9/1, 20.0 mL × 3 times). The organic phases were combined, washed with saturated saline (15.0 mL × 3 times), dried with anhydrous sodium sulfate, and finally concentrated under reduced pressure. The resulting residue was purified by silica gel column chromatography to obtain 16 mg of white solid 2-((S)-1-(4-(6-((4-chloro-2-fluorobenzyl)oxy)pyridin-2-yl)piperidin-1-yl)ethyl)-3-(((S)-oxetan-2-yl)methyl)-3*H-*imidazolo[4,5-*b*]pyridin-5-carboxylic acid (yield: 23.8%). LC-MS: RT = 1.81 min, [M+H]⁺ = 580.31.

### Example 13 2-((S)-1-(4-(6-((4-bromo-2-fluorobenzyl)oxy)pyridin-2-yl)piperidin-1-yl)ethyl)-1-(((S)-oxetan-2-yl)methyl)-1H-benzo[d]imidazol-6-carboxylic acid

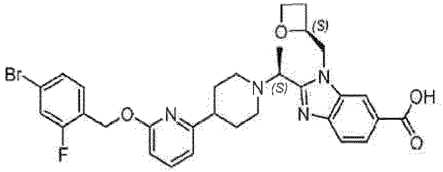

The specific synthetic route is as follows.

### Step A: Synthesis of (2,5-difluorophenyl)methanol

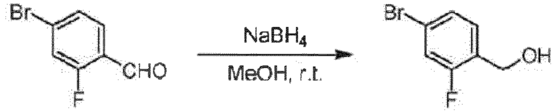

2,5-Difluorobenzaldehyde (500.0 mg, 2.47 mmol) was added to methanol (25.0 mL) in an ice bath, followed by the addition of sodium borohydride (140.6 mg, 3.70 mmol) in batches. The reaction was carried out under stirring at room temperature for 1.0 hour under N₂ protection.

After the completion of the reaction, the reaction was quenched with water, and the reaction solution was extracted with ethyl acetate (30 mL × 3 times). The organic phases were combined, washed with saturated saline (20 mL × 2 times), dried with anhydrous sodium sulfate, and finally concentrated under reduced pressure to give 380.3 mg of yellow oily (2,5-difluorophenyl)methanol (yield: 76.0%).

### Step B: Synthesis of tert-butyl 4-(6-((4-bromo-2-fluorobenzyl)oxy)pyridin-2-yl)piperidin-1-carboxylate

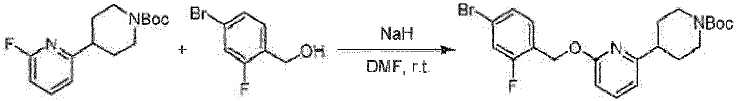

(4-bromo-2-fluorophenyl)methanol (60.0 mg, 0.29 mmol) was added to *N*,*N-*dimethylformamide (3.0 mL) in an ice bath, followed by the addition of 60 wt. % sodium hydride (23.2 mg, 0.58 mmol) in batches. After stirring for 0.5 hour at room temperature under N₂ protection, tert-butyl 4-(6-fluoropyridin-2-yl)piperidin-1-formate (81.2 mg, 0.29 mmol) was added and the reaction was carried out for 1.0 hour at room temperature.

After the completion of the reaction, the reaction was quenched with water, and the reaction solution was extracted with ethyl acetate (30 mL × 3 times). The organic phases were combined, washed with saturated saline (20 mL × 2 times), dried with anhydrous sodium sulfate, and finally concentrated under reduced pressure. The resulting residue was purified by silica gel column chromatography (eluent: ethyl acetate/n-hexane = 50/1) to obtain 82.0 mg of white solid tert-butyl 4-(6-((4-bromo-2-fluorobenzyl)oxy)pyridin-2-yl)piperidin-1-carboxylate (yield: 61.0%). LC-MS: RT = 2.45 min, [M+H-56]⁺ = 409.17.

### Step C: Synthesis of 2-((4-bromo-2-fluorobenzyl)oxy)-6-(piperidin-4-yl)pyridine

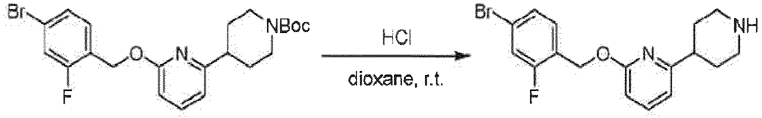

Tert-butyl 4-(6-((4-bromo-2-fluorobenzyl)oxy)pyridin-2-yl)piperidin-1-carboxylate (82.0 mg, 0.18 mmol) was added to hydrochloric acid in dioxane (1.00 mmol/mL, 3 mL) at room temperature, and the reaction was carried out at room temperature for 1 hour under N₂ protection.

After the completion of the reaction, the reaction solution was concentrated under reduced pressure. 50.2 mg of milky white solid 2-((4-bromo-2-fluorobenzyl)oxy)-6-(piperidin-4-yl)pyridine (yield: 76.4%) was obtained, which was used directly in the next reaction step. LC-MS: RT = 1.77 min, [M+H]⁺ = 365.16.

### Step D: Synthesis of methyl 2-((S)-1-(4-(6-((4-bromo-2-fluorobenzyl)oxy)pyridin-2-yl)piperidin-1-yl)ethyl)-1-(((S)-oxetan-2-yl)methyl)-1H-benzo[d]imidazol-6-carboxylate

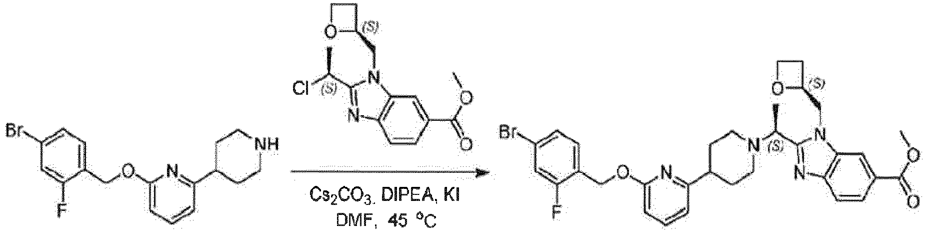

2-((4-Bromo-2-fluorobenzyl)oxy)-6-(piperidin-4-yl)pyridine (50.2 mg, 0.14 mmol), methyl 2-((S)-1-chloroethyl)-1-(((S)-oxetan-2-yl)methyl)-1*H-*benzo[*d*]imidazol-6-carboxylate (43.5 mg, 0.14 mmol), potassium iodide (23.2 mg, 0.14 mmol), and cesium carbonate (91.3 mg, 0.28 mmol) were added to *N*,*N-*dimethylformamide (10.0 mL) at room temperature, followed by the addition of *N*,*N-*diisopropylethylamine (1.0 mL). The reaction was carried out at 45°C for 3.0 hours under N₂ protection.

After the completion of the reaction, the reaction was quenched with water, and the reaction solution was extracted with ethyl acetate (50 mL × 3 times). The organic phases were combined, washed with saturated saline (30 mL × 3 times), dried with anhydrous sodium sulfate, and finally purified by silica gel column chromatography (eluent: dichloromethane/methanol = 20/1) to give 66.3 mg of a light yellow solid methyl 2-((S)-1-(4-(6-((4-bromo-2-fluorobenzyl)oxy)pyridin-2-yl)piperidin-1-yl)ethyl)-1-(((S)-oxetan-2-yl)methyl)-1*H-*benzo[*d*]imidazol-6-carboxylate (yield: 74.4%). LC-MS: RT = 1.88 min, [M+H]⁺ = 637.26.

### Step E: Synthesis of 2-((S)-1-(4-(6-((4-bromo-2-fluorobenzyl)oxy)pyridin-2-yl)piperidin-1-yl)ethyl)-1-(((S)-oxetan-2-yl)methyl)-1H-benzo[d]imidazol-6-carboxylic acid

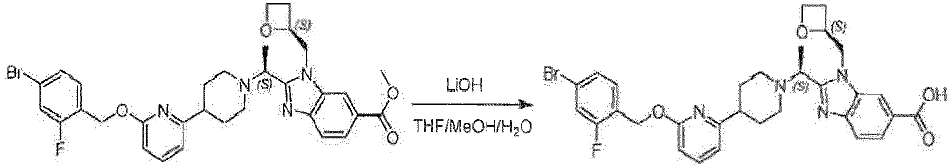

Lithium hydroxide (16.8 mg, 0.40 mmol) in water (1.0 mL) was added dropwise to tetrahydrofuran (3.0 mL) containing methyl 2-((S)-1-(4-(6-((4-bromo-2-fluorobenzyl)oxy)pyridin-2-yl)piperidin-1-yl)ethyl)-1-(((S)-oxetan-2-yl)methyl)-1*H-*benzo[*d*]imidazol-6-carboxylate (66.3 mg, 0.10 mmol) at room temperature, followed by the addition of 1.0 mL of methanol. The reaction was carried out at 45°C for 3.0 hours.

After the completion of the reaction, the reaction was quenched with water, and the pH was adjusted to 6 with aqueous ammonium chloride solution (0.5 mol/L). The reaction solution was extracted with dichloromethane (30 mL × 3 times), and the organic phases was combined, washed with aqueous ammonium chloride solution (30 mL × 2 times), and dried with anhydrous sodium sulfate to give a crude product, which was purified by preparative high performance liquid chromatography. The purification conditions are as follows: column: Agilent 5 Prep-C18 100 mm×30 mm 5 µM; mobile phase: water (containing 0.05% ammonia) and acetonitrile; flow rate: 20 mL/min; gradient: 25% acetonitrile, eluted at 7.50 min; detection wavelength: 254 nm. After purification and lyophilization, 19.3 mg of a white solid 2-((S)-1-(4-(6-((4-bromo-2-fluorobenzyl)oxy)pyridin-2-yl)piperidin-1-yl)ethyl)-1-(((S)-oxetan-2-yl)methyl)-1*H-*benzo[*d*]imidazol-6-carboxylic acid was obtained (yield: 30.0%). LC-MS: RT = 1.84 min, [M+H]⁺ = 623.28.

### Example 14 2-((S)-1-(4-(6-((2,5-difluorobenzyl)oxy)pyridin-2-yl)piperidin-1-yl)ethyl)-1-(((S)-oxetan-2-yl)methyl)-1H-benzo[d]imidazol-6-carboxylic acid

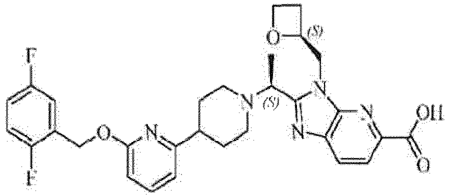

The specific synthetic route is as follows.

### Step A: Synthesis of (2,5-difluorophenyl)methanol

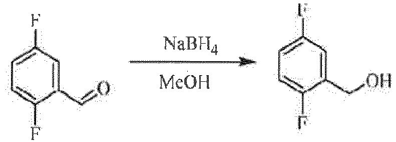

2,5-Difluorobenzaldehyde (300 mg, 2.11 mmol) was dissolved in methanol (15 mL) at room temperature, followed by the addition of sodium borohydride (156 mg, 4.23 mmol) at 0°C. The reaction was carried out at room temperature with stirring. After the completion of the reaction, the reaction was quenched with saturated sodium chloride solution. The reaction solution was extracted with dichloromethane (30 mL) and washed twice with water. The organic phases was combined, dried with anhydrous sodium sulfate, filtered, and concentrated to give a crude product, which was purified by silica gel column chromatography (eluent: ethyl acetate/n-hexane = 1/3) to obtain 240 mg of colorless oily liquid (2,5-difluorophenyl)methanol (yield: 80%). LC-MS: RT = 1.78 min.

### Step B: Synthesis of tert-butyl 4-(6-((2,5-difluorobenzyl)oxy)pyridin-2-yl)piperidin-1-carboxylate

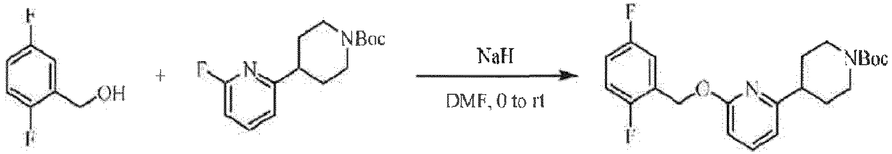

(2,5-Difluorophenyl)methanol (240 mg, 1.67 mmol) was dissolved in *N*,*N-*dimethylformamide (10 mL). The mixed solution was cooled to 0°C, and added with sodium hydride (80 mg, 3.34 mmol). The reaction solution was stirred at room temperature for 0.5 hour, and was added with tert-butyl 4-(6-fluoropyridin-2-yl)piperidin-1-carboxylate (476 mg, 1.67 mmol). The reaction was carried out at room temperature. After the completion of the reaction, the reaction was quenched with saturated sodium chloride. The reaction solution was extracted with ethyl acetate (30 mL) and washed twice with water. The organic phases was combined, dried with anhydrous sodium sulfate, filtered, and concentrated to give a crude product, which was purified by silica gel column chromatography (eluent: ethyl acetate/n-hexane = 1/2) to obtain 280 mg of colorless oily liquid tert-butyl 4-(6-((2,5-difluorobenzyl)oxy)pyridin-2-yl)piperidin-1-carboxylate (yield: 48%). LC-MS: RT = 2.32 min, [M-55]⁺ = 349.25.

### Step C: Synthesis of 2-((2,5-difluorobenzyl)oxy)-6-(piperidin-4-yl)pyridine

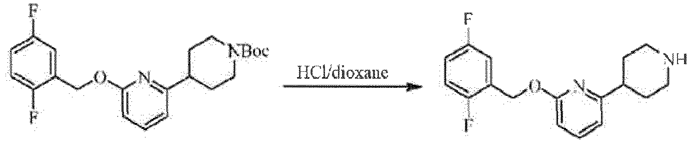

Tert-butyl 4-(6-((2,5-difluorobenzyl)oxy)pyridin-2-yl)piperidin-1-carboxylate (280 mg, 0.69 mmol) was dissolved in hydrochloric acid in dioxane (5 mL), and the mixed solution was stirred at room temperature. After the completion of the reaction, 300 mg of crude product was obtained after concentration under reduced pressure, which is a white solid 2-((2,5-difluorobenzyl)oxy)-6-(piperidin-4-yl)pyridine,. LC-MS: RT = 1.68 min, [M+H]⁺ = 305.25.

### Step D: Synthesis of isopropyl 2-((S)-1-(4-(6-((2,5-difluorobenzyl)oxy)pyridin-2-yl)piperidin-1-yl)ethyl)-1-(((S)-oxetan-2-yl)methyl)-1H-benzo[d]imidazol-6-carboxylate

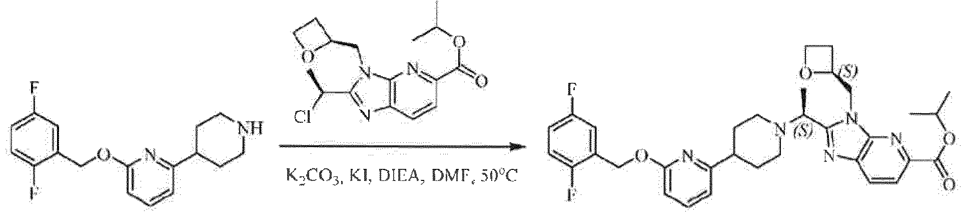

2-((2,5-difluorobenzyl)oxy)-6-(piperidin-4-yl)pyridine (60 mg, 0.20 mmol), DIEA (0.5 mL), potassium iodide (65 mg, 0.9 mmol), and potassium carbonate (54 mg, 0.39 mmol) were dissolved in *N,N*-dimethylformamide (5 mL) at room temperature, followed by the addition of isopropyl 2-((S)-1-chloroethyl)-1-(((S)-oxetan-2-yl)methyl)-3*H-*imidazo[4,5-*b*]pyridine-5-carboxylate (67 mg, 0.20 mmol), and the reaction was carried out at a temperature of 50°C.

After the completion of the reaction, the reaction was quenched with saturated sodium chloride solution, and the reaction solution was extracted with ethyl acetate (20 mL × 2 times). The organic phases were combined, dried with anhydrous sodium sulfate, filtered, and concentrated under reduced pressure to give a crude product, which was purified by silica gel column chromatography (eluent: ethyl acetate/n-hexane = 1/8) to obtain 65 mg of white solid isopropyl 2-((S)-1-(4-(6-((2,5-difluorobenzyl)oxy)pyridin-2-yl)piperidin-1-yl)ethyl)-1-(((S)-oxetan-2-yl)methyl)-1*H-*benzo[*d*]imidazol-6-carboxylate (yield: 32%). LC-MS: RT = 1.93 min, [M+H]⁺ = 606.38.

### Step E: Synthesis of 2-((S)-1-(4-(6-((2,5-difluorobenzyl)oxy)pyridin-2-yl)piperidin-1-yl)ethyl)-1-(((S)-oxetan-2-yl)methyl)-1H-benzo[d]imidazol-6-carboxylic acid

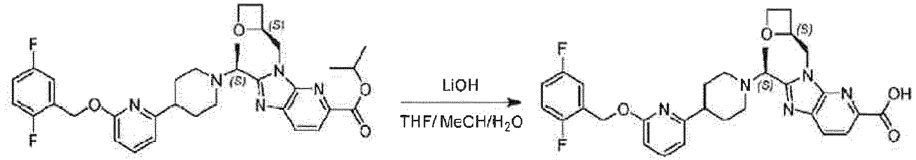

Isopropyl 2-((S)-1-(4-(6-((2,5-difluorobenzyl)oxy)pyridin-2-yl)piperidin-1-yl)ethyl)-1-(((S)-oxetan-2-yl)methyl)-1*H-*benzo[*d*]imidazol-6-carboxylate (65 mg, 0.11 mmol) was dissolved in a solvent mixture (6 mL, tetrahydrofuran/methanol/water = 2.5/2.5/1) at room temperature, followed by the addition of lithium hydroxide (16 mg, 0.44 mmol), and the reaction was carried out at room temperature.

After the completion of the reaction, the reaction was quenched with saturated ammonium chloride solution, and the reaction solution was sequentially extracted with ethyl acetate (10 mL × 2 times) and a solvent mixture (10 mL × 1 time, dichloromethane/methanol = 30/1). The organic phases were combined, dried with anhydrous sodium sulfate, filtered, and concentrated under reduced pressure to give a crude product, which was purified by silica gel column chromatography (eluent: dichloromethane/methanol=10/1) to obtain 30.75 mg of white solid 2-((S)-1-(4-(6-((2,5-difluorobenzyl)oxy)pyridin-2-yl)piperidin-1-yl)ethyl)-1-(((S)-oxetan-2-yl)methyl)-1*H-*benzo[*d*]imidazol-6-carboxylic acid (yield: 21%).
LC-MS: RT = 1.77 min, [M+H]⁻ = 564.31. ¹H NMR (400 MHz, DMSO-d₆) *δ* 8.15 (d, *J* = 8 2 Hz, 1H), 7.98 (d, *J=* 8.2 Hz, 1H), 7.64-7.58 (m, 1H), 7.39-7.14 (m, 4H), 6.83 (d, J= 7.3 Hz, 1H), 6 67 (dd, *J=* 8.1, 4.4 Hz, 1H), 5.32 (dd, *J* = 19.5, 6.1 Hz, 3H), 4.84 (dd, *J* = 15.0, 4.2 Hz, 1H), 4.72 (dd, *J*= 15.1, 3.6 Hz, 1H), 4.60 (d, *J* = 6.8 Hz, 1H), 439 (dd, *J* = 13 6, 7.7 Hz, 1H), 4 07 (dd, *J* = 15.0, 6.0 Hz, 1H), 2.98 (d, *J* = 11.0 Hz, 1H), 2 57 (dd, *J* = 11.7, 8.4 Hz, 3H), 2 26 (dd, *J* = 22 2, 11 6 Hz, 2H), 1.84-1.63 (m, 4H), 1 54 (dd, *J=* 12 0, 3 7 Hz, 1H), 1 46 (t, *J* = 6.5 Hz, 3H).

### Example 15 2-((S)-1-(4-(6-((4-cyano-2-fluorobenzyl)oxy)pyridin-2-yl)piperidin-1-yl)ethyl)-3-(((S)-oxetan-2-yl)methyl)-3H-imidazo[4,5-b]pyridin-5-carboxylic acid

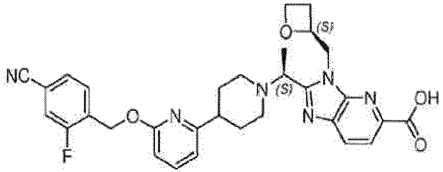

The specific synthetic route is as follows.

### Step A: Synthesis of tert-butyl 4-(6-((4-cyano-2-fluorobenzyl)oxy)pyridin-2-yl)piperidin-1-carboxylate

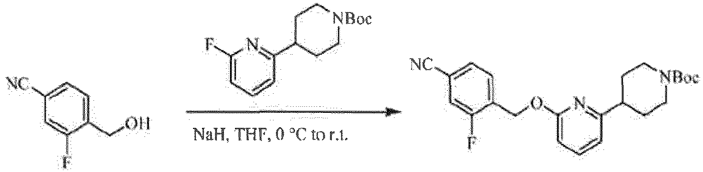

At 0°C, sodium hydride (357 mg, 60% dispersed in mineral oil, 8.94 mmol) was added to tetrahydrofuran (5.0 mL) containing 3-fluoro-4-(hydroxymethyl)benzonitrile (648 mg, 4.29 mmol), and stirred for 20 min, followed by adding tert-butyl 4-(6-fluoropyridin-2-yl)piperidin-1-formate (1.0 g, 3.57 mmol), and the reaction was carried out at room temperature for 4 hours.

After the reaction was completed, the reaction was quenched with water, and the solution was extracted with ethyl acetate (50 mL × 3 times), washed with saturated saline (50 mL), and dried with anhydrous sodium sulfate. The filtrate was concentrated under reduced pressure, and the residue was purified by silica gel column chromatography (eluent: ethyl acetate/n-hexane = 1/10). 850 mg of a white solid, tert-butyl 4-(6-((4-cyano-2-fluorobenzyl)oxy)pyridin-2-yl)piperidin-1-carboxylate, was obtained (yield: 57.9%). LC-MS: RT = 2.28 min, [M-H]⁻ = 410.32.

### Step B: Synthesis of 3-fluoro-4-(((6-(piperidin-4-yl)pyridin-2-yl)oxy)methyl)benzonitrile

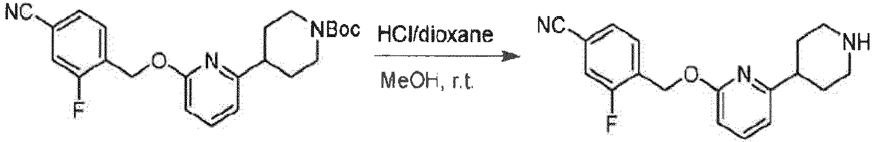

At 0°C, hydrochloric acid in 1,4-dioxane (2 mL, 4 M) was added to methanol (4 mL) containing tert-butyl 4-(6-((4-cyano-2-fluorobenzyl)oxy)pyridin-2-yl)piperidin-1-carboxylate (850 mg, 2.07 mmol), and the reaction was carried out at room temperature for 40 min.

After the reaction was completed, the reaction solution was directly concentrated to obtain 720 mg of a white solid, 3-fluoro-4-(((6-(piperidin-4-yl)pyridin-2-yl)oxy)methyl)benzonitrile, which was used directly in the next step. LC-MS: RT = 1.65 min, [M+H]⁺ = 312.26.

### Step C: Synthesis of isopropyl 2-((S)-1-(4-(6-((4-cyano-2-fluorobenzyl)oxy)pyridin-2-yl)piperidin-1-yl)ethyl)-3-(((S)-oxetan-2-yl)methyl)-3H-imidazo[4,5-b]pyridin-5-carboxylate

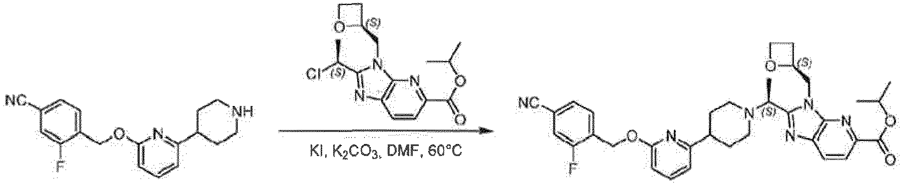

At room temperature, isopropyl 2-((S)-1-chloroethyl)-3-(((S)-oxetan-2-yl)methyl)-3*H-*imidazo[4,5-b]pyridin-5-carboxylate (98 mg, 0.29 mmol), potassium iodide (96 mg, 0.58 mmol) and potassium carbonate (80 mg, 0.58 mmol) were added to *N*,*N-*dimethylformamide (4.0 mL) containing 3-fluoro-4-(((6-(piperidin-4-yl)pyridin-2-yl)oxy)methyl)benzonitrile (120 mg, 0.35 mmol), and the reaction was carried out at 60°C for 4 hours.

After the reaction was completed, the reaction was quenched with water, extracted with ethyl acetate (50 mL × 3 times), washed with saturated saline (50 mL), dried with anhydrous sodium sulfate, filtered, and concentrated under reduced pressure. The resulting residue was purified by silica gel column chromatography (eluent: ethyl acetate/n-hexane = 1/5). 60 mg of a yellow solid compound, isopropyl 2-((S)-1-(4-(6-((4-cyano-2-fluorobenzyl)oxy)pyridin-2-yl)piperidin-1-yl)ethyl)-3-(((S)-oxetan-2-yl)methyl)-3*H-*imidazo[4,5-b]pyridin-5-carboxylate, was obtained (yield: 33.7%). LC-MS: RT = 1.84 min, [M+H]⁺ = 613.38.

### Step D: Synthesis of 2-((S)-1-(4-(6-((4-cyano-2-fluorobenzyl)oxy)pyridin-2-yl)piperidin-1-yl)ethyl)-3-(((S)-oxetan-2-yl)methyl)-3H-imidazo[4,5-b]pyridin-5-carboxylic acid

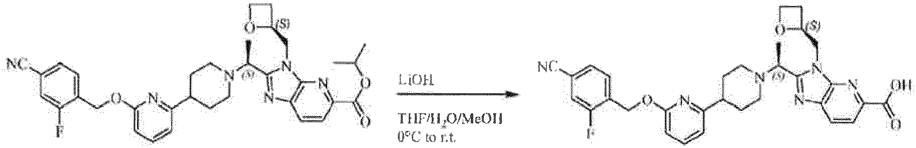

At 0°C, an aqueous solution (1 mL) of lithium hydroxide (17 mg, 0.40 mmol) was added dropwise to tetrahydrofuran (3 mL) and methanol (1 mL) containing isopropyl 2-((S)-1-(4-(6-((4-cyano-2-fluorobenzyl)oxy)pyridin-2-yl)piperidin-1-yl)ethyl)-3-(((S)-oxetan-2-yl)methyl)-3*H*-imidazo[4,5-b]pyridin-5-carboxylate (60 mg, 0.10 mmol), and the reaction was carried out at room temperature for 30 min.

After the reaction was completed, it was quenched with water, extracted with ethyl acetate (50 mL × 3 times), washed with saturated saline (50 mL), dried with anhydrous sodium sulfate, filtered, and concentrated under reduced pressure. The residue was purified by silica gel column chromatography (eluent: methanol/dichloromethane = 1/10). 43 mg of a white solid, 2-((S)-1-(4-(6-((4-cyano-2-fluorobenzyl)oxy)pyridin-2-yl)piperidin-1-yl)ethyl)-3-(((S)-oxetan-2-yl)methyl)-3*H-*imidazo[4,5-b]pyridin-5-carboxylic acid, was obtained (yield: 75.3%). LCMS: RT = 1.75 min, [M+H]⁺ = 571.36.

¹H NMR (400 MHz, DMSO) δ 8.22-8.08 (m,1H), 7.99 (dd, ./= 8.2, 5.2 Hz, 1H), 7.86 (d, *J* = 10.3 Hz, 1H), 7.68 (d, *J* = 3.9 Hz, 2H), 7.66-7.60 (m, 1H), 6.85 (t,*J*= 7.5 Hz, 1H), 6.70 (dt, *J*= 13.3, 6.6 Hz, 1H), 5 45 (s, 2H), 5.36-5.02 (m, 3H), 4.86-4.46 (m, 4H), 4.48-4.36 (m, 1H), 4.16-4.08 (m, lH), 4.07-3.98 (m, 1H), 3.13-2.91 (m, 2H), 2.67-2.54 (m, 2H), 2.31-2.14 (m, 2H), 2.02-1.90 (m, 1H), 1.23 (d, *J* = 3.7 Hz, 3H).

### Example 16 1-(((S)-oxetan-2-yl)methyl)-2-((S)-1-(4-(6-(quinolin-8-yl-methoxy)pyridin-2-yl)piperidin-1-yl)ethyl)-1H-benzo[d]imidazol-6-carboxylic acid

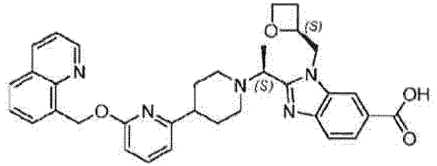

The specific synthetic route is as follows.

### Step A: Synthesis of methyl 1-(((S)-oxetan-2-yl)methyl)-2-((S)-1-(4-(6-(quinolin-8-yl-methoxy)pyridin-2-yl)piperidin-1-yl)ethyl)-1H-benzo[d]imidazol-6-carboxylate

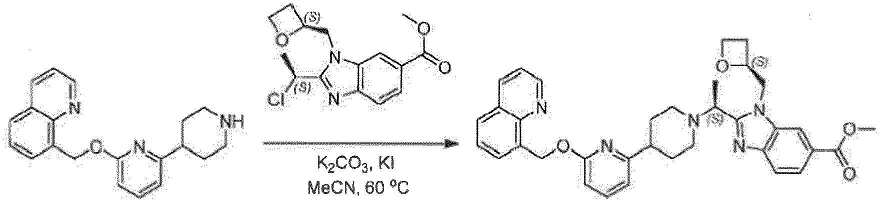

8-(((6-(Piperidin-4-yl)pyridin-2-yl)oxy)methyl)quinoline (60 mg, 0.169 mmol), potassium carbonate (47.0 mg, 0.338 mmol) and potassium iodide (40.5 mg, 0.253 mmol) were dissolved in 5 mL of anhydrous acetonitrile, followed by an addition of methyl 2-((S)-1-chloroethyl)-1-((S)-oxetan-2-yl)methyl)-1*H-*benzo[*d*]imidazol-6-carboxylate (52.1 mg, 0.169 mmol), and the reaction was carried out at 60°C for 2 hours. After the reaction was completed, the reaction solution was diluted with water, extracted with ethyl acetate (30 mL × 2). The organic phases were combined, washed with saturated saline (20 mL × 2), dried with anhydrous sodium sulfate, filtered, and concentrated under reduced pressure. The resulting crude product was purified by column chromatography to obtain 76 mg of a pale yellow solid, methyl 1-(((S)-oxetan-2-yl)methyl)-2-((S)-1-(4-(6-(quinolin-8-yl-methoxy)pyridin-2-yl)piperidin-1-yl)ethyl)-1*H-*benzo[*d*]imidazol-6-carboxylate. LC-MS: RT = 1.82 min, [M+H]⁺ = 592.30.

### Step B: Synthesis of 1-(((S)-oxetan-2-yl)methyl)-2-((S)-1-(4-(6-(quinolin-8-yl-methoxy)pyridin-2-yl)piperidin-1-yl)ethyl)-1H-benzo[d]imidazol-6-carboxylic acid

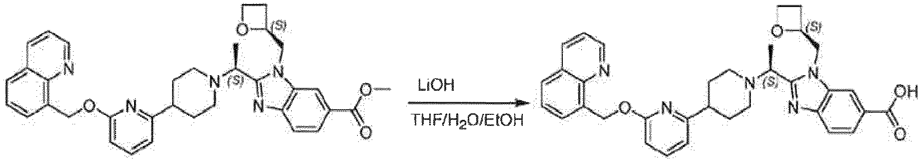

Methyl 1-(((S)-oxetan-2-yl)methyl)-2-((S)-1-(4-(6-(quinolin-8-yl-methoxy)pyridin-2-yl)piperidin-1-yl)ethyl)-1*H*-benzo[*d*]imidazol-6-carboxylate (76 mg, 0.128 mmol) was dissolved in 4 mL of tetrahydrofuran, followed by an addition of lithium hydroxide monohydrate (27 mg, 0.643 mmol), 1 mL of water and 1 mL of ethanol, and the reaction was carried out at room temperature for 50 min. After the reaction was completed, the reaction solution was neutralized with 20 mL of saturated ammonium chloride solution and extracted with ethyl acetate (30 mL × 2 times). The organic phases were combined, washed with saturated saline (20 mL × 2 times), dried with anhydrous sodium sulfate, filtered, and concentrated under reduced pressure. The resulting crude product was separated by column chromatography to obtain 24 mg of a pale yellow solid, 1-(((S)-oxetan-2-yl)methyl)-2-((S)-1-(4-(6-(quinolin-8-ylmethoxy)pyridin-2-yl)piperidin-1-yl)ethyl)-1*H-*benzo[*d*]imidazol-6-carboxylic acid.

LC-MS: RT =1.75 min, [M-H]⁻ =578.37. ¹H NMR (400 MHz, DMSO) δ 12.71 (s, 1H), 8.96 (dd, *J*= 4.2, 1.8 Hz, 1H), 8.41 (dd, *J* = 8 3, 1.7 Hz, 1H), 8.28 (s, 1H), 7.93- 7.96 (m, 1H), 7.79-7.86(m, 2H), 7.56-7.71 (m, 4H), 6.84 (d, *J* = 7.2 Hz, 1H), 6.73 (d, *J* = 8.3 Hz, 1H), 6.00 (s, 2H), 5.11-5.16 (m, 1H), 4.75-4.81 (m, 1H), 4.56-4.64 (m, 1H), 4.36-4.46 (m, 2H), 4.10-4.17 (m, 1H), 2.90-2.98 (m, 1H), 2.54-2.65 (m, 4H), 2.18-2.33 (m, 2H), 1.67-1.87 (m, 4H), 1.43-1.48 (m, 3H).

### Example 17 3-(((S)-oxetan-2-yl)methyl)-2-((S)-1-(4-(6-((1-(2,2,2-trifluoroethyl)-1H-indazol-6-yl)methoxy)pyridin-2-yl)piperidin-1-yl)ethyl)-3H-imidazo[4,5-b]pyridin-5-carbooylic acid

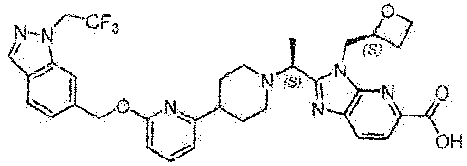

The specific synthetic route is as follows.

### Step A: Synthesis of isopropyl 3-(((S)-oxetan-2-yl)methyl)-2-((S)-1-(4-(6-((1-(2,2,2-trifluoroethyl)-1H-indazol-6-yl)methoxy)pyridin-2-yl)piperidin-1-yl)ethyl)-3H-imidazo[4,5-b]pyridin-5-carboxylate

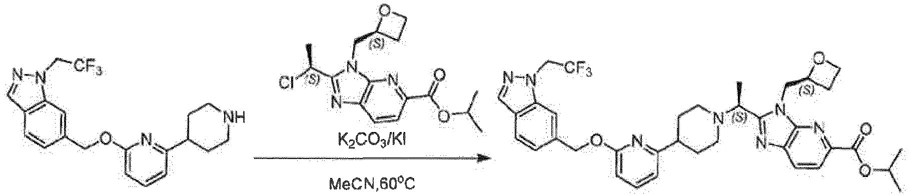

6-(((6-(Piperidin-4-yl)pyridin-2-yl)oxy)methyl)-1-(2,2,2-trifluoroethyl)-1*H-*indazole (78 mg, 0.183 mmol), potassium carbonate (50.5 mg, 0.366 mmol) and potassium iodide (43.8 mg, 0.274 mmol) were dissolved in 5 mL of anhydrous acetonitrile, followed by an addition of isopropyl 2-((S)-1-chloroethyl)-3-(((S)-oxetan-2-yl)methyl)-3*H-*imidazo[4,5-b]pyridin-5-carboxylate (61.6 mg, 0.183 mmol), and the reaction was carried out at 60°C for 2 hours. After the reaction was completed, the reaction solution was diluted with water and extracted with ethyl acetate (30 mL × 2 times), The organic phases were combined, washed with saturated saline (20 mL × 2 times), dried with anhydrous sodium sulfate, filtered, and concentrated under reduced pressure. The resulting crude product was separated by column chromatography to obtain 92 mg of a pale yellow solid, isopropyl 3-(((S)-oxetan-2-yl)methyl)-2-((S)-1-(4-(6-((1-(2,2.2-trifluoroethyl)-1*H-*indazol-6-yl)methoxy)pyridin-2-yl)piperidin-1-yl)ethyl)-3*H-*imidazo[4,5-b]pyridin-5-carboxylate. LC-MS: RT = 1.81 min, [M+H]⁺ = 692.27.

### Step B: Synthesis of 3-(((S)-oxetan-2-yl)methyl)-2-((S)-1-(4-(6-((1-(2,2,2-trifluoroethyl)-1H-indazol-6-yl)methoxy)pyridin-2-yl)piperidin-1-yl)ethyl)-3H-imidazo[4,5-b]pyridin-5-carboxylic acid

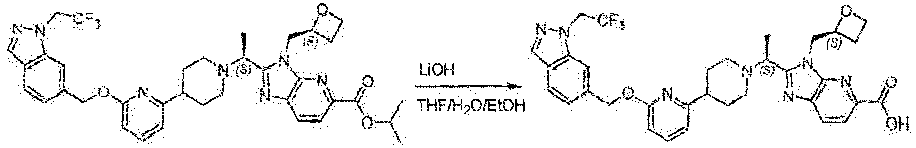

Isopropyl 3-(((S)-oxetan-2-yl)methyl)-2-((S)-1-(4-(6-((1-(2,2,2-trifluoroethyl)-1*H-*indazol-6-yl)methoxy)pyridin-2-yl)piperidin-1-yl)ethyl)-3*H-*imidazo[4,5-b]pyridin-5-carboxylate (92 mg, 0.133 mmol) was dissolved in 4 mL of tetrahydrofuran, followed by an addition of lithium hydroxide monohydrate (28 mg, 0.664 mmol), 1 mL of water and 1 mL of ethanol, and the reaction was carried out at room temperature for 50 min. After the reaction was completed, the reaction solution was neutralized with 20 mL of saturated ammonium chloride solution and extracted with ethyl acetate (30 mL × 2 times). The organic phases were combined, washed with saturated saline (20 mL × 2 times), dried with anhydrous sodium sulfate, filtered, and concentrated under reduced pressure. The resulting crude product was separated by column chromatography to obtain 22 mg of a pale yellow solid, 3-(((S)-oxetan-2-yl)methyl)-2-((S)-1-(4-(6-((1-(2,2,2-trifluoroethyl)-1*H-*indazol-6-yl)methoxy)pyridin-2-yl)piperidin-1-yl)ethyl)-3*H-*imidazo[4,5-b]pyridin-5-carboxylic acid.

LC-MS: RT =1.75 min, [M-H]⁺ =650.39. ¹H NMR (500 MHz, DMSO) δ 8.19 (s, 1H), 8.15 (d, *J* = 8.2 Hz, 1H), 7.99 (d, *J* = 8.3 Hz, 1H), 7.88 (s, 1H), 7 78 (d, *J* = 8.2 Hz, 1H), 7.62 (t, *J* = 7.8 Hz, 1H), 7.31 (d, *J* = 8.2 Hz, 1H), 6.84 (d, *J* = 7.4 Hz, 1H), 6.69 (d, *J* = 8.1 Hz, 1H), 5.48 (d, *J* = 1.7 Hz, 2H), 5.42 (dd, *J* = 18.6, 9.4 Hz, 2H), 5.30 5.26(m, 1H), 4.84 4.71 (m, 2H), 4.41-4.37 (m, 1H), 4.08-4.03 (m, 2H), 3.03-2.99 (m, 1H), 2.68-2.52 (m, 4H), 2.22-2.30 (m, 2H), 1.70-1.90 (m, 4H), 1.49 (d, *J* = 6.7 Hz, 3H).

### Example 18 2-((S)-1-(4-(6-((7-fluoroquinolin-4-yl)methoxy)pyridin-2-yl)piperidin-1-yl)ethyl)-3-(((S)-oxetan-2-yl)methyl)-3H-imidazo[4,5-b]pyridine-5-carboxylic acid

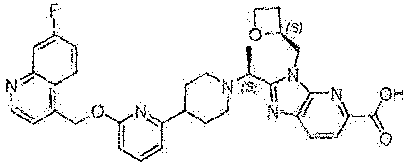

The specific synthetic route is as follows.

### Step A: Synthesis of 7-fluoro-4-methylquinoline

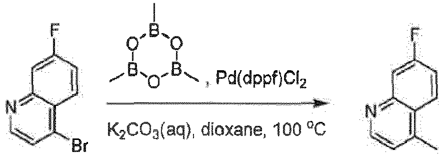

4-Bromo-7-fluoroquinoline (1.00 g, 4.42 mmol) was dissolved in 1,4-dioxane (15.0 mL), followed by an addition of trimethylboroxine (1.3 mL, 4.42 mmol), [1,1'-bis(diphenylphosphino)ferrocene]dichloropalladium (323.4 mg, 0.44 mmol) and aqueous potassium carbonate solution (3.3 mL, 6.63 mmol). Under nitrogen protection, the reaction was carried out at 100°C for 4 hours under stirring. The solid was removed by filtration, and the filtrate was spin-dried and purified by column chromatography (ethyl acetate/n-hexane = 1/5) to obtain 530.0 mg of a colorless liquid, 7-fluoro-4-methylquinoline (yield: 74.3%). LC-MS: RT = 0.84 min, [M+H]⁺ = 162.14.

### Step B: Synthesis of 4-(bromomethyl)-7-fluoroquinoline

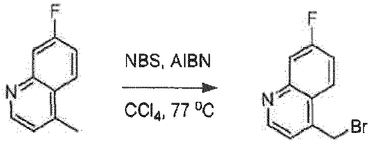

7-Fluoro-4-methylquinoline (530.0 mg, 3.29 mmol) was dissolved in tetrachloromethane (10.0 mL), followed by an addition of *N*-bromosuccinimide (703.0 mg, 3.95 mmol) and azodiisobutyronitrile (54.2 mg, 0.33 mmol). The reaction solution was heated to 77°C, and the reaction was carried out for 2 hours with stirring. The solid was removed by filtration, and the filtrate was spin-dried to obtain 780.0 mg of a beige solid 4-(bromomethyl)-7-fluoroquinoline (yield: 98.8%). LC-MS: RT = 1.88 min, [M+H]⁺ = 240.08.

### Step C: Synthesis of methyl (7-fluoroquinolin-4-yl)acetate

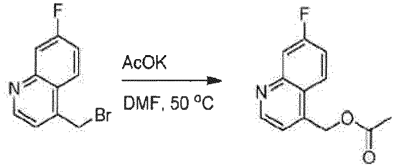

4-(Bromomethyl)-7-fluoroquinoline (775.0 mg, 3.23 mmol) was dissolved in *N*,*N-*dimethylformamide (6.0 mL), followed by an addition of potassium acetate (634.0 mg, 6.46 mmol). The reaction solution was heated to 50°C, and the reaction was carried out for 1 hour under stirring. The reaction solution was diluted with ethyl acetate (50 mL), washed with saturated saline, dry over anhydrous sodium sulfate, spin-dried to remove the solvent, and separated by column chromatography (ethyl acetate: n-hexane = 1:2) to obtain 400.0 mg of a beige solid, (7-fluoroquinolin-4-yl)methyl acetate (yield: 56.5%). LC-MS: RT = 1.73 min, [M+H]⁺ = 220.15.

### Step D: Synthesis of (7-fluoroquinolin-4-yl)methanol

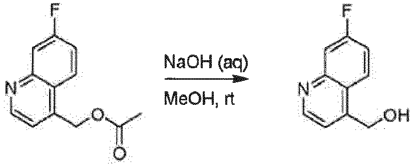

Methyl (7-fluoroquinolin-4-yl)acetate (400.0 mg, 1.82 mmol) was dissolved in methanol (3.0 mL), followed by an addition of aqueous sodium hydroxide solution (1.8 mL, 3.64 mmol), and the mixture was stirred for 1 hour at room temperature. The reaction solution was concentrated under reduced pressure to remove methanol, diluted with ethyl acetate (50 mL), extracted, washed with saturated saline, dried with anhydrous sodium sulfate, filtered, and concentrated under reduced pressure to remove the organic solvent. The product was concentrated and separated by column chromatography (ethyl acetate: n-hexane = 2:1) to obtain 120.0 mg of white solid (7-fluoroquinolin-4-yl)methanol (yield: 37.1%). LC-MS: RT = 0.60 min, [M+H]⁺ = 178.13.

### Step E: Synthesis of tert-butyl 4-(6-((7-fluoroquinolin-4-yl)methoxy)pyridin-2-yl)piperidin-1-carboxylate

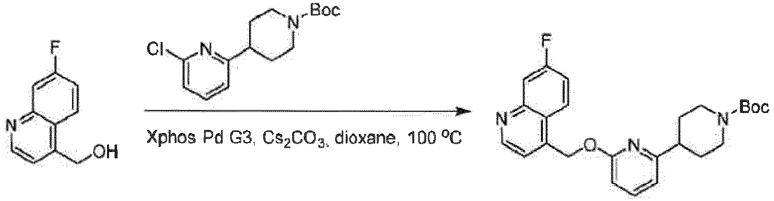

(7-Fluoroquinolin-4-yl)methanol (90.0 mg, 0.51 mmol) was dissolved in 1,4-dioxane (5 mL), followed by an addition of tert-butyl 4-(6-chloropyridin-2-yl)piperidin-1-carboxylate (151.4 mg, 0.51 mmol), methanesulfonato(2-dicyclohexylphosphino-2',4',6'-triisopropyl-1,1'-biphenyl)(2'-amino-1,1'-biphenyl-2-yl)palladium(II) (42.3 mg, 0.05 mmol) and cesium carbonate (249.3 mg, 0.77 mmol). Under nitrogen protection, the reaction solution was heated to 100°C and the reaction was carried out overnight with stirring. The solid was removed by filtration, and the filtrate was concentrated to dryness, then the product was separated by column chromatography (ethyl acetate: n-hexane = 1:2) to obtain 190.0 mg of a beige solid, tert-butyl 4-(6-((7-fluoroquinolin-4-yl)methoxy)pyridin-2-yl)piperidin-1-carboxylate (yield: 85.5%). LC-MS: RT = 2.25 min, [M+H]⁺ = 438.32.

### Step F: Synthesis of 7-fluoro-4-((6-(piperidin-4-yl)pyridin-2-yl)oxy)methyl)quinoline

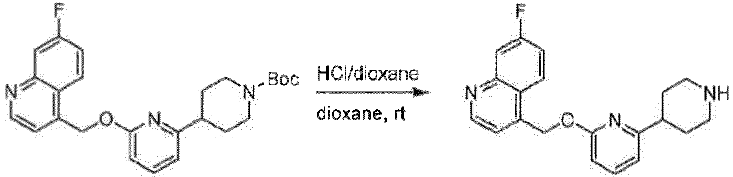

Tert-butyl 4-(6-((7-fluoroquinolin-4-yl)methoxy)pyridin-2-yl)piperidin-1-carboxylate (190.0 mg, 0.43 mmol) was dissolved in 1,4-dioxane (1.0 mL), followed by an addition of hydrochloric acid in 1,4-dioxane (1.0 mL, 4.0 mL), and the mixture was stirred at room temperature for 1 hour. The solvent was removed by concentration to obtain 149.0 mg of a white solid, 7-fluoro-4-((6-(piperidin-4-yl)pyridin-2-yl)oxy)methyl)quinoline (yield: 91.8%). LC-MS: RT = 1.63 min, [M+H]⁺ = 338.28.

### Step G: Synthesis of isopropyl 2-((S)-1-(4-(6-((7-fluoroquinolin-4-yl)methoxy)pyridin-2-yl)piperidin-1-yl)ethyl)-3-(((S)-oxetan-2-yl)methyl)-3H-imidazo[4,5-b]pyridin-5-carboxylate

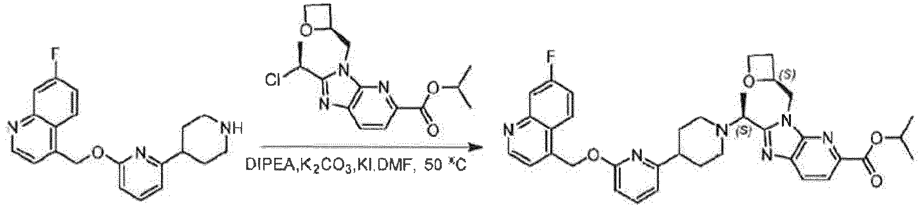

7-Fluoro-4-((6-(piperidin-4-yl)pyridin-2-yl)oxy)methyl)quinoline (70.0 mg, 0.19 mmol) was dissolved in *N*,*N*-dimethylformamide (3.0 mL), followed by an addition of *N*,*N-*diisopropylethylamine (132 µL, 0.80 mmol), which was dissolved under stirring. Then propyl 2-((S)-1-chloroethyl)-1-(((S)-oxetan-2-yl)methyl)-1*H-*benzo[*d*]imidazol-6-carboxylate (64 mg, 0.19 mmol), potassium carbonate (39 mg, 0.28 mmol), and potassium iodide (46 mg, 0.28 mmol) were added sequentially. The reaction solution was heated to 50°C and the reaction was carried out for 2 hours with stirring. It was diluted with ethyl acetate (50 mL), extracted, washed sequentially with water and saturated saline, dried with anhydrous sodium sulfate, filtered, and concentrated to remove the solvent. The product was separated by column chromatography (ethyl acetate: n-hexane = 5:1) to obtain 80.0 mg of a beige solid, isopropyl 2-((S)-1-(4-(6-((7-fluoroquinolin-4-yl)methoxy)pyridin-2-yl)piperidin-1-yl)ethyl)-3-(((S)-oxetan-2-yl)methyl)-3*H-*imidazo[4,5-b]pyridin-5-carboxylate (yield: 66.9%). LC-MS: RT = 1.81 min, [M+H]⁺ = 639.43.

### Step H: Synthesis of 2-((S)-1-(4-(6-((7-fluoroquinolin-4-yl)methoxy)pyridin-2-yl)piperidin-1-yl)ethyl)-3-(((S)-oxetan-2-yl)methyl)-3H-imidazo[4,5-b]pyridin-5-carboxylic acid

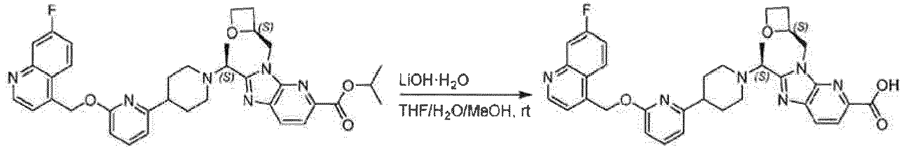

Isopropyl 2-((S)-1-(4-(6-((7-fluoroquinolin-4-yl)methoxy)pyridin-2-yl)piperidin-1-yl)ethyl)-3-(((S)-oxetan-2-yl)methyl)-3*H-*imidazo[4,5-b]pyridin-5-carboxylate (80.0 mg, 0.13 mmol) was dissolved in a mixed solvent of tetrahydrofuran (0.3 mL), methanol ( 0.1 mL) and water (0.1 mL), followed by an addition of lithium hydroxide monohydrate (26.3 mg, 0.63 mmol). The mixture was stirred at room temperature for 4 hours. The solvent was removed by concentration and the product was separated by column chromatography (methanol: dichloromethane = 7:93) to obtain 51.3 mg of a white solid 2-((S)-1-(4-(6-((7-fluoroquinolin-4-yl)methoxy)pyridin-2-yl)piperidin-1-yl)ethyl)-3-(((S)-oxetan-2-yl)methyl)-3*H*-imidazo[4,5-b]pyridin-5-carboxylic acid (yield: 68.8%).

LC-MS: RT = 1.70 min, [M+H]⁺ = 597.34. ¹H NMR (400 MHz, DMSO-d6) δ (ppm): 8.86 (d, *J* = 4.3 Hz, 1H), 8.25 (dd, *J* = 9.1, 6.2 Hz, 1H), 8.13 (d, *J* = 8.2 Hz, 1H), 7.97 (d, *J* = 8.2 Hz, 1H), 7.78 (dd, *J* = 10.3, 2.4 Hz, 1H), 7.68-7.56 (m, 2H), 7.53 (d, *J* = 4.3 Hz, 1H), 6.84 (d, J=7.4 Hz, 1H), 6.74 (d, *J* = 8.2 Hz, 1H), 5.87 (s, 2H), 5.31-5.18 (m, 1H), 4.83-4.73 (m, 1H), 4.71-4.63 (m, 1H), 4.61-4.52 (m, 1H), 4.41-4.32 (m, 1H), 4.09--4.00 (m, 1H), 2.98-2.89 (m, 1H), 2.63-2.51 (m, 3H), 2.38-2.10 (m, 3H), 1.84-1.57 (m, 4H), 1.44 (d, *J* = 6.6 Hz, 3H).

### Example 19 2-((S)-1-(4-(6-((3-fluoroquinolin-8-yl)methoxy)pyridin-2-yl)piperidin-1-yl)ethyl)-3-(((S)-oxetan-2-yl)methyl)-3H-imidazo[4,5-b]pyridin-5-carboxylic acid

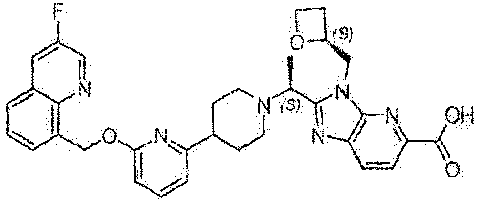

### Step A: Synthesis of 3-fluoro-8-methylquinoline

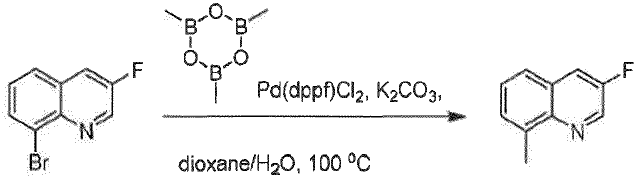

At room temperature, trimethylboroxine (0.25 mL, 0.89 mmol), [1,1'-bis(diphenylphosphino)ferrocene]dichloropalladium (32 mg, 0.044 mmol) and potassium carbonate (244 mg. 1.77 mmol) were added sequentially to a 1,4-dioxane/water solution (3.4 mL/1.0 mL) containing 8-bromo-3-fluoroquinoline (200 mg, 0.89 mmol). Then the reaction system was purged with nitrogen, and the reaction was carried out at 100°C for 3 hours. After the reaction was completed, the solution was extracted with ethyl acetate (30 mL × 3 times). The organic phases were combined, washed with saturated saline (30 mL × 3 times), dried with anhydrous sodium sulfate, filtered, and concentrated under reduced pressure. The resulting residue was purified by silica gel column chromatography (eluent: ethyl acetate/n-hexane = 1/10). 128 mg of a white solid, 3-fluoro-8-methylquinoline, was obtained (yield: 89.8%). LC-MS: RT = 2.01 min, [M+H]⁺ = 162.14.

### Step B: Synthesis of 8-(bromomethyl)-3-fluoroquinoline

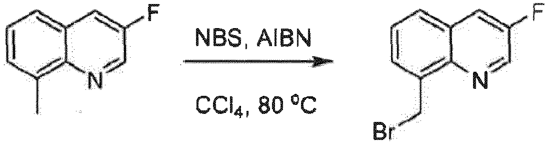

At room temperature, *N*-bromosuccinimide (156 mg, 0.88 mmol) and azodiisobutyronitrile (5 mg, 0.032 mmol) were added sequentially to tetrachloromethane (4.0 mL) containing 3-fluoro-8-methylquinoline (128 mg, 0.80 mmol), then the reaction solution was heated to 80°C and the reaction was carried out for 1 hour. After the reaction was completed, the reaction solution was diluted with dichloromethane (100 mL), extracted, washed sequentially with saturated sodium bicarbonate solution (50 mL) and saline (50 mL), dried with anhydrous sodium sulfate, filtered, and concentrated under reduced pressure. The resulting residue was purified by silica gel column chromatography (eluent: ethyl acetate/n-hexane = 1/15). 90 mg of a white solid, 8-(bromomethyl)-3-fluoroquinoline, was obtained (yield: 46.9%). LC-MS: RT = 2.05 min, [M+H]⁺ = 242.05.

### Step C: Synthesis of (3-fluoroquinolin-8-yl)methanol

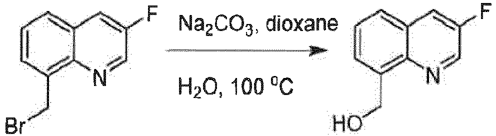

At room temperature, sodium carbonate (119 mg, 1.13 mmol) was added to a 1,4-dioxane/water solution (1.5 mL/1.5 mL) containing 8-(bromomethyl)-3-fluoroquinoline (90 mg, 0.38 mmol), and the reaction solution was heated to 100°C and the reaction was carried out for 3 hours.

After the reaction was completed, the reaction solution was cooled to room temperature, extracted with ethyl acetate (50 mL × 3 times), washed with saturated saline (50 mL), dried with anhydrous sodium sulfate, filtered, and concentrated under reduced pressure. Then the resulting residue was purified by silica gel column chromatography (eluent: ethyl acetate/n-hexane = 1/8). 40 mg of a white solid, (3-fluoroquinolin-8-yl)methanol, was obtained (yield: 59.5%). LC-MS: RT= 1.72 min, [M+H]⁺ = 178.15.

### Step D: Synthesis of tert-butyl 4-(6-((3-fluoroquinolin-8-yl)methoxy)pyridin-2-yl)piperidin-1-formate

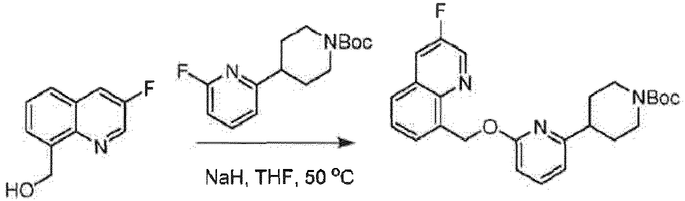

Under an ice-water bath and under nitrogen protection, sodium hydride (180 mg, 60% dispersed in mineral oil, 4.5 mmol) was added to a tetrahydrofuran solution (5.0 mL) containing (3-fluoroquinolin-8-yl)methanol (266 mg, 1.50 mmol). After the addition, the ice water bath was removed and the reaction solution was stirred at room temperature for 30 min. A solution of tert-butyl 4-(6-fluoropyridin-2-yl)piperidin-1-formate in tetrahydrofuran (2.0 mL) was added, and the reaction solution was heated to 50°C and the reaction was carried out for 3 hours.

After the reaction was completed, it was quenched with saturated ammonium chloride solution. The reaction solution was extracted with ethyl acetate (30 mL × 3 times), and the organic phases were combined, washed with saturated saline (30 mL × 3 times), dried with anhydrous sodium sulfate, filtered, and concentrated under reduced pressure. The resulting residue was purified by silica gel column chromatography (eluent: ethyl acetate/n-hexane = 1/8). 450 mg of a pale yellow oily product, tert-butyl 4-(6-((3-fluoroquinolin-8-yl)methoxy)pyridin-2-yl)piperidin-1-formate, was obtained (yield: 68.5%). LC-MS: RT = 2.37 min, [M+H]⁺ = 438.24.

### Step E: Synthesis of 3-fluoro-8-(((6-(piperidin-4-yl)pyridin-2-yl)oxy)methyl)quinoline

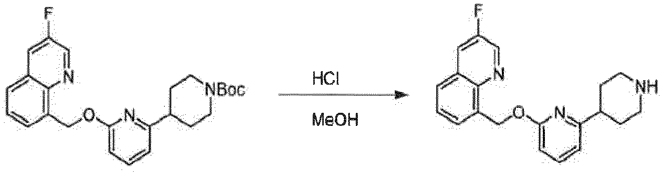

At room temperature, hydrochloric acid in 1,4-dioxane (1.3 mL, 4.0 mol/L, 5.14 mmol) was added to a solution of tert-butyl 4-(6-((3-fluoroquinolin-8-yl)methoxy)pyridin-2-yl)piperidin-1-formate (450 mg, 1.03 mmol) in methanol (4.0 mL). After the addition, it was stirred at room temperature for 2 hours. After the reaction was completed, the solvent was evaporated to dryness under reduced pressure to obtain 420 mg of a white solid, 3-fluoro-8-(((6-(piperidin-4-yl)pyridin-2-yl)oxy)methyl)quinoline. LC-MS: RT = 1.73 min, [M+H]⁺ = 338.27.

### Step F: Synthesis of isopropyl 2-((S)-1-(4-(6-((3-fluoroquinolin-8-yl)methoxy)pyridin-2-yl)piperidin-1-yl)ethyl)-3-(((S)-oxetan-2-yl)methyl)-3H-imidazo[4,5-b]pyridin-5-carboxylate

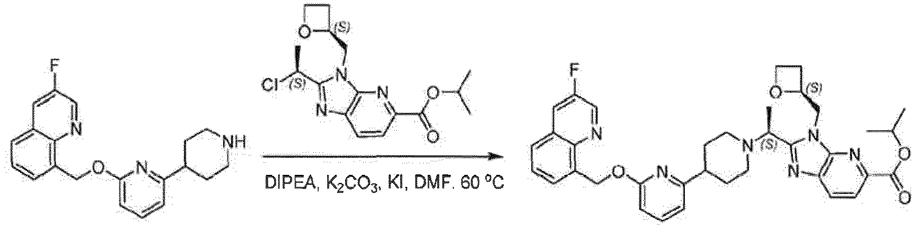

At room temperature, *N*,*N-*diisopropylethylamine (132 µL, 0.80 mmol) was added to *N,N-*dimethylformamide (2.0 mL) containing 3-fluoro-8-(((6-(piperidin-4-yl)pyridin-2-yl)oxy)methyl)quinoline (153 mg, 0.342 mmol), and was dissolved under stirring. Isopropyl 2-((S)-1-chloroethyl)-3-(((S)-oxetan-2-yl)methyl)-3*H-*imidazo[4,5-b]pyridin-5-carboxylate (90 mg, 0.27 mmol), potassium carbonate (74 mg, 0.53 mmol) and potassium iodide (89 mg, 0.53 mmol) were added sequentially. After the addition, the reaction solution was heated to 60°C and stirred for 1 hour.

After the reaction was completed, it was quenched with saturated ammonium chloride solution. The reaction solution was extracted with ethyl acetate (30 mL × 3 times), and the organic phases were combined, washed with saturated saline (30 mL × 3 times), dried with anhydrous sodium sulfate, filtered, and concentrated under reduced pressure. The resulting residue was purified by silica gel column chromatography (eluent: methanol/dichloromethane = 8/92). 90 mg of a colorless oily product, isopropyl 2-((S)-1-(4-(6-((3-fluoroquinolin-8-yl)methoxy)pyridin-2-yl)piperidin-1-yl)ethyl)-3-(((S)-oxetan-2-yl)methyl)-3*H*-imidazo[4,5-b]pyridin-5-carboxylate, was obtained (yield: 52.8%). LC-MS: RT = 1.89 min, [M+H]⁺ = 639.37.

### Step G: Synthesis of 2-((S)-1-(4-(6-((3-fluoroquinolin-8-yl)methoxy)pyridin-2-yl)piperidin-1-yl)ethyl)-3-(((S)-oxetan-2-yl)methyl)-3H-imidazo[4,5-b]pyridin-5-carboxylic acid

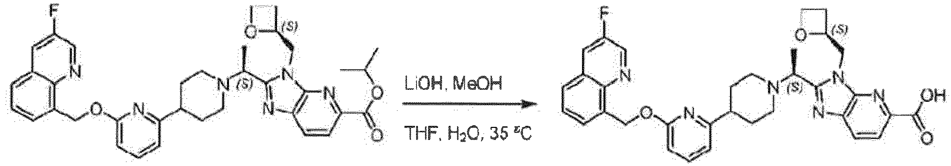

At room temperature, lithium hydroxide monohydrate (45 mg, 1.07 mmol) was added to a tetrahydrofuran/methanol/water solution (2.5 mL/2.5 mL/1.0 mL) containing isopropyl 2-((S)-1-(4-(6-((3-fluoroquinolin-8-yl)methoxy)pyridin-2-yl)piperidin-1-yl)ethyl)-3-((S)-oxetan-2-yl)methyl)-3*H-*imidazo[4,5-b]pyridin-5-carboxylate (90 mg, 0.14 mmol), and the reaction solution was heated to 35°C and the reaction was carried out for 1 hour. After the reaction was completed, the reaction solution was extracted with ethyl acetate (30 mL × 3 times), and the organic phases were combined, washed with saturated saline (30 mL × 3 times), dried with anhydrous sodium sulfate, filtered, and concentrated under reduced pressure. The resulting residue was purified by silica gel column chromatography (eluent: methanol/dichloromethane = 8/92). 34 mg of a white solid, 2-((S)-1-(4-(6-((3-fluoroquinolin-8-yl)methoxy)pyridin-2-yl)piperidin-1-yl)ethyl)-3-(((S)-oxetan-2-yl)methyl)-3*H-*imidazo[4,5-b]pyridin-5-carboxylic acid, was obtained (yield: 40.4%). LC-MS: RT = 1.80 min, [M+H]⁺ = 597.40.

¹H NMR (400 MHz, DMSO) δ 9.04-8.96 (m, 1H), 8.32 (dd, *J*= 9.6, 2.9 Hz, 1H), 8.09 (d, *J* = 8.2 Hz, 1H), 7.96 (d, *J*= 8.2 Hz, 2H), 7.82 (d, *J* = 6.7 Hz, 1H), 7.63 (dd, *J* = 15.5, 7 6 Hz, 2H), 6 83 (d, *J* = 7.2 Hz, 1H), 6.71 (d, *J* = 8.1 Hz, 1H), 5.97 (s, 2H), 5.29-5.19 (m, 1H), 4.72 (ddd, *J* = 38.7, 14.9, 4.0 Hz, 2H), 4.55 (q, *J* = 6.6 Hz, 1H), 4.41-4.32 (m, 1H), 4.05 (dt, *J* = 9.2, 5.9 Hz, 1H), 2.95 (d, *J* = 10.9 Hz, 1H), 2.63 -2.52 (m, 4H), 2.35-2.17 (m, 2H), 1.88-1.62 (m, 3H), 1.57-1.48 (m, 1H), 1.46 (d, *J* = 6.7 Hz, 3H).

### Example 20 2-((S)-1-(4-(6-((4-cyano-2-fluorobenzyl)oxy)pyridin-2-yl)piperazin-1-yl)ethyl)-1-(((S)-oxetan-2-yl)methyl)-1H-benzo[d]imidazol-6-carboxylic acid (20A) and 2-((R)-1-(4-(6-((4-cyano-2-fluorobenzyl)oxy)pyridine-2-yl)piperazin-1-yl)ethyl)-1-(((S)-oxetan-2-yl)methyl)-1H-benzo[d]imidazol-6-carboxylic acid (20B)

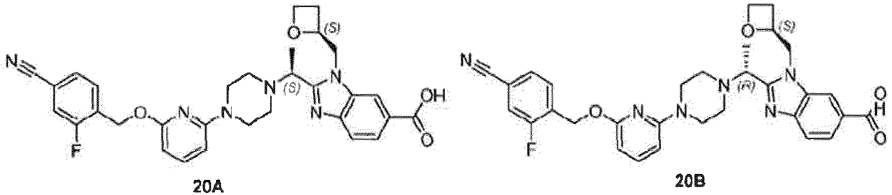

The specific synthetic route is as follows.

### Step A: Synthesis of tert-butyl 4-(6-chloropyridin-2-yl)piperazin-1-carboxylate

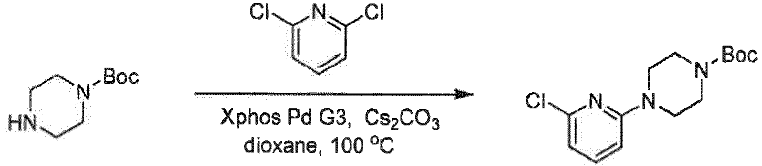

Tert-butyl piperazin-1-carboxylate (300.0 mg, 1.61 mmol) was dissolved in 1,4-dioxane (12.0 mL), followed by an addition of 2,6-dichloropyridine (262.0 mg, 1.77 mmol), methanesulfonato(2-dicyclohexylphosphino-2',4',6'-triisopropyl-1,1'-biphenyl)(2'-amino-1,1'-biphenyl-2-yl)palladium(II) (135.4 mg, 0.16 mmol) and cesium carbonate (788.5 mg, 2.42 mmol). Under nitrogen protection, the reaction solution was heated to 100°C and the reaction was carried out for 5 hours with stirring. The solvent was then spin-dried and the product was separated by column chromatography (ethyl acetate: n-hexane = 1:10) to obtain 150.0 mg of a beige solid, tert-butyl 4-(6-chloropyridin-2-yl)piperazin-1-carboxylate (yield: 31.3%). LC-MS: RT = 1.82 min, [M-^{t}Bu+H]⁺ = 242.36.

### Step B: Synthesis of tert-butyl 4-(6-((4-cyano-2-fluorobenzyl)oxy)pyridin-2-yl)piperazin-1-carboxylate

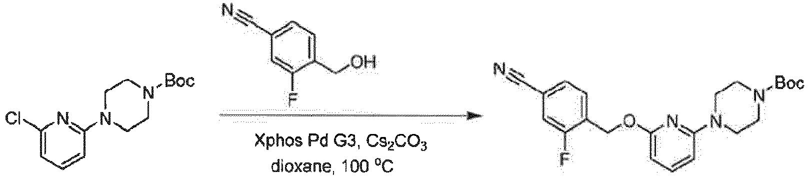

Tert-butyl 4-(6-chloropyridin-2-yl)piperazin-1-carboxylate (150.0 mg, 0.51 mmol) was dissolved in 1,4-dioxane (5.0 mL), followed by an addition of 3-fluoro-4-(hydroxymethyl)benzonitrile (76.3 mg, 0.51 mmol), methanesulfonato(2-dicyclohexylphosphino-2',4',6'-triisopropyl-1,1'-biphenyl)(2'-amino-1,1'-biphenyl-2-yl)palladium(II) (43.2 mg, 0.05 mmol) and cesium carbonate (246.8 mg, 0.76 mmol). Under nitrogen protection, the reaction solution was heated to 100°C and the reaction was carried out for 15 hours with stirring. The solvent was spin-dried and the product was separated by column chromatography (ethyl acetate: n-hexane = 1:10) to obtain 102.0 mg of a beige solid, tert-butyl 4-(6-((4-cyano-2-fluorobenzyl)oxy)pyridin-2-yl)piperazin-1-carboxylate (yield: 49.1%). LC-MS: RT = 2.23 min, [M-^{t}Bu+H]⁺ = 357.23.

### Step C: Synthesis of 3-fluoro-4-(((6-(piperazin-1-yl)pyridin-2-yl)oxy)methyl)benzonitrile

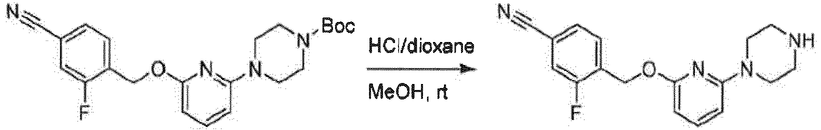

Tert-butyl 4-(6-((4-cyano-2-fluorobenzyl)oxy)pyridin-2-yl)piperazin-1-carboxylate (102.0 mg, 0.25 mmol) was dissolved in methanol (2.0 mL), followed by an addition of hydrochloric acid in 1,4-dioxane (0.5 mL, 2.0 mmol). At room temperature, the reaction was carried out for 3 hours with stirring. The solvent was spin-dried to obtain 73.7 mg of a white solid, 3-fluoro-4-(((6-(piperazin-1-yl)pyridin-2-yl)oxy)methyl)benzonitrile (yield: 73.5%). LC-MS: RT = 1.68 min, [M+H]⁺ = 313.25.

### Step D: Synthesis of methyl 2-(1-(4-(6-((4-cyano-2-fluorobenzyl)oxy)pyridin-2-yl)piperazin-1-yl)ethyl)-1H-benzo[d]imidazol-6-carboxylate

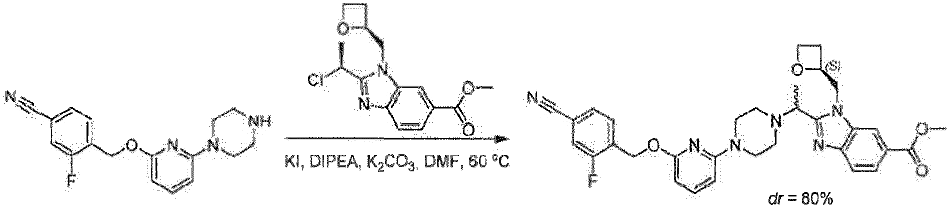

3-Fluoro-4-(((6-(piperazin-1-yl)pyridin-2-yl)oxy)methyl)benzonitrile (73.7 mg, 0.21 mmol) was dissolved in *N*,*N-*dimethylformamide (3.0 mL), followed by an addition of *N*,*N-*diisopropylethylamine (54.7 mg, 0.42 mmol), potassium carbonate (58.5 mg, 0.42 mmol), potassium iodide (52.8 mg, 0.32 mmol) and methyl 2-((S)-1-chloroethyl)-1-(((S)-oxetan-2-yl)methyl)-1*H-*benzo[*d*]imidazol-6-carboxylate (71.7 mg, 0.23 mmol). The reaction solution was heated to 60°C and the reaction was carried out for 4 hours with stirring. After the reaction was completed, the reaction solution was diluted with ethyl acetate (50 mL), extracted, washed with saturated saline, and dried with anhydrous sodium sulfate. Then the solvent was spin-dried and the product was separated by column chromatography (ethyl acetate: n-hexane = 4:1) to obtain 102.0 mg of a beige solid, methyl 2-(1-(4-(6-((4-cyano-2-fluorobenzyl)oxy)pyridin-2-yl)piperazin-1-yl)ethyl)-1-(((S)-oxetan-2-yl)methyl)-1*H-*benzo[*d*]imidazol-6-carboxylate (yield: 82.6%, *dr* = 80%). LC-MS: RT = 1.86 min, [M+H]⁺ = 585.42.

### Step E: Synthesis of 2-((S)-1-(4-(6-((4-cyano-2-fluorobenzyl)oxy)pyridin-2-yl)piperazin-1-yl)ethyl)-1-(((S)-oxetan-2-yl)methyl)-1H-benzo[d]imidazol-6-carboxylic acid (20A) and 2-((R)-1-(4-(6-((4-cyano-2-fluorobenzyl)oxy)pyridin-2-yl)piperazin-1-yl)ethyl)-1-(((S)-oxetan-2-yl)methyl)-1H-benzo[d]imidazol-6-carboxylic acid (20B)

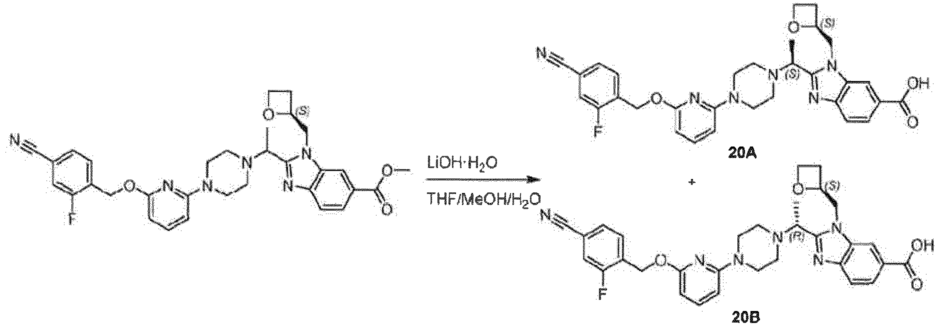

Methyl 2-(1-(4-(6-((4-cyano-2-fluorobenzyl)oxy)pyridin-2-yl)piperazin-1-yl)ethyl)-1-(((S)-oxetan-2-yl)methyl)-1*H-*benzo[*d*]imidazol-6-carboxylate (102.0 mg, 0.17 mmol) was dissolved in a mixed solvent of tetrahydrofuran (3.0 mL), methanol (3.0 mL) and water (1.0 mL), followed by an addition of lithium hydroxide monohydrate (36.6 mg, 0.87 mmol). At room temperature, the reaction was carried out for 4 hours with stirring. The solvent was spin-dried and the product was separated by preparative liquid chromatography to obtain 21.0 mg of white solid 2-((S)-1-(4-(6-((4-cyano-2-fluorobenzyl)oxy)pyridin-2-yl)piperazin-1-yl)ethyl)-1-(((S)-oxetan-2-yl)methyl)-1*H-*benzo[*d*]imidazol-6-carboxylic acid (20A) (yield: 20.6%), and 2.2 mg of white solid 2-((R)-1-(4-(6-((4-cyano-2-fluorobenzyl)oxy)pyridin-2-yl)piperazin-1-yl)ethyl)-1-(((S)-oxetan-2-yl)methyl)-1*H-*benzo[*d*]imidazol-6-carboxylic acid (20B) (yield 2.3%). The crude product was purified by preparative high performance liquid chromatography. The separation conditions are as follows: column: Agilent 5 Prep-C18 100 mm×30 mm 5 µM; mobile phase: water (containing 0.1% ammonia) and acetonitrile; flow rate: 20 mL/min; gradient: 20A and 20B were eluted by 23% acetonitrile at 7.67 min and 8.54 min, respectively; detection wavelength: 254 nm.

**Compound 20A:** HPLC: RT = 7.67 min. LC-MS: RT = 1.79 min, [M+H]⁺ = 571.39. ¹H NMR (500 MHz, DMSO-d₆) δ (ppm): 8 06 (s, 1H), 7.87 (dd, *J=* 9.9, 1.2 Hz, 1H), 7 78 (d, *J* = 8.3 Hz, 1H), 7.69 (dd, *J* = 7.9, 1.4 Hz, 1H), 7.64 (d, *J=* 7.4 Hz, 1H), 7.46 (s, 1H), 7.44 (s, 1H), 6.29 (d, *J* = 8.2 Hz, 1H), 6.09 (d, *J* = 7 8 Hz, 1H), 5.37 (s, 2H), 5.20-5.13 (m, 1H), 4.75 (dd, *J* = 15.5, 3.6 Hz, 1H), 4.55-4.53 (m, 1H), 4.48-4.42 (m, 2H), 4.18-4.12 (m, 1H), 3.40-3.30 (m, 4H), 2.65-2.63 (m, 1H), 2.58-2.50 (m, 4), 2.35-2.29 (m, 1H), 1 42 (d, J= 6.7 Hz, 3H).

**Compound 20B:** HPLC: RT = 8.54 min. LC-MS: RT = 1.78 min, [M+H]⁺ = 571.33 . ¹H NMR (500 MHz, DMSO-d₆) δ (ppm):12.74 (s, 1H), 8.27 (d, *J*= 1.2 Hz, 1H), 7.87 (dd, *J* = 9.9, 1.4 Hz, 1H), 7.79 (dd, *J* = 8.4, 1.5 Hz, 1H), 7.69 (dd, *J* = 7.9, 1.5 Hz, 1H), 7.68-7.61 (m, 2H), 7.46 (t, *J=* 8.0 Hz, 1H), 6.31 (d, *J* = 8.2 Hz, 1H), 6.11 (d, *J* = 7.8 Hz, 1H), 5.39 (s, 2H), 5.08-5.03 (m, 1H), 4.93-4.87 (m, 1H), 4.59-4.56 (m, 1H), 4.51-4.47 (m, 3H), 3.42-3.39 (m, 4H), 2.78-2.73 (m, 1H), 2.70-2.58 (m, 2H), 2.54-2.45 (m, 3H), 1.41 (d, *J* = 6.7 Hz, 3H).

### Example 21 2-((S)-1-((S)-4-(6-((4-cyano-2-fluorobenzyl)ouy)pyridin-2-yl)-2-methylpiperazin-1-yl)ethyl)-1-(((S)-oxetan-2-yl)methyl)-1H-benzo[d]imidazol-6-carboxylic acid

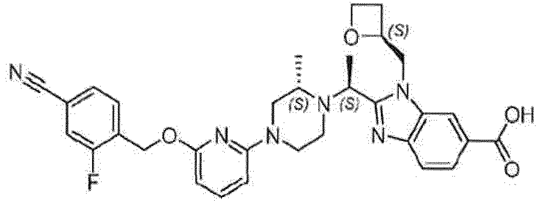

The specific synthetic route is as follows.

### Step A: Synthesis of 4-((6-chloropyridin-2-yl)oxy)methyl)-3-fluorobenzonitrile

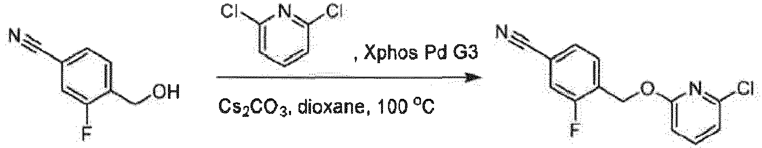

3-Fluoro-4-(hydroxymethyl)benzonitrile (1.00 g, 6.62 mmol) was dissolved in 1,4-dioxane (25.0 mL), followed by an addition of 2,6-dichloropyridine (1.18 g, 7.94 mmol), methanesulfonato(2-dicyclohexylphosphino-2',4',6'-triisopropyl-1,1'-biphenyl)(2'-amino-1,1'-biphenyl-2-yl)palladium(II) (558.7 mg, 0.66 mmol) and cesium carbonate (3.24 g, 9.93 mmol). Under nitrogen protection, the reaction solution was heated to 100°C and the reaction was carried out overnight with stirring. The solid was removed by filtration, the filtrate was spin-dried, and the product was purified by column chromatography (ethyl acetate: n-hexane = 1:10) to obtain 330.0 mg of a white solid, 4-((6-chloropyridin-2-yl)oxy)methyl)-3-fluorobenzonitrile (yield: 19.0%). LC-MS: RT = 2.13 min, [M+H]⁺ = 263.09.

### Step B: Synthesis of tert-butyl (S)-4-(6-((4-cyano-2-fluorobenzyl)oxy)pyridin-2-yl)-2-methylpiperazin-1-carboacylate

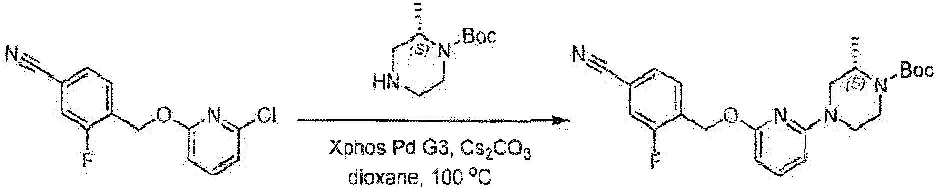

4-((6-Chloropyridin-2-yl)oxy)methyl)-3-fluorobenzonitrile (330.0 mg, 1.26 mmol) was dissolved in 1,4-dioxane (15.0 mL), followed by an addition of tert-butyl (S)-2-methylpiperazin-1-carboxylate (302.4 mg, 1.51 mmol), methanesulfonato(2-dicyclohexylphosphino-2',4',6'-triisopropyl-1,1'-biphenyl)(2'-amino-1,1'-biphenyl-2-yl)palladium(II) (110.0 mg, 0.13 mmol) and cesium carbonate (615.8 mg, 1.89 mmol). Under nitrogen protection, the reaction solution was heated to 100°C and the reaction was carried out for 14 hours with stirring. The solid was removed by filtration, the filtrate was spin-dried and the product was separated by column chromatography (ethyl acetate: n-hexane = 1:5) to obtain 260.3 mg of a white solid, tert-butyl (S)-4-(6-((4-cyano-2-fluorobenzyl)oxy)pyridin-2-yl)-2-methylpiperazin-1-carboxylate (yield: 48.6%). LC-MS: RT = 2.27 min, [M-^{t}Bu+H]⁺ = 371.21.

### Step C: Synthesis of (S)-3-fluoro-4-((6-(3-methylpiperazin-1-yl)pyridin-2-yl)oxy)methyl)benzonitrile

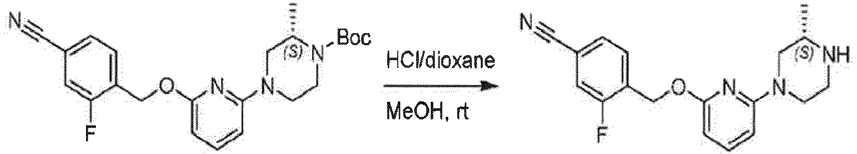

Tert-butyl (S)-4-(6-((4-cyano-2-fluorobenzyl)oxy)pyridin-2-yl)-2-methylpiperazin-1-carboxylate (260.3 mg, 0.61 mmol) was dissolved in methanol (3.0 mL), followed by an addition of hydrochloric acid in 1,4-dioxane (1 mL, 4.0 mmol). At room temperature, the reaction solution was reacted for 2 hours with stirring. The solvent was spin-dried to obtain 220.1 mg of a white solid, (S)-3-fluoro-4-((6-(3-methylpiperazin-1-yl)pyridin-2-yl)oxy)methyl)benzonitrile (yield: 99.4%). LC-MS: RT = 1.68 min, [M+H]⁺ = 327.25.

### Step D: Synthesis of methyl 2-((S)-1-((S)-4-(6-((4-cyano-2-fluorobenzyl)oxy)pyridin-2-yl)-2-methylpiperazin-1-yl)ethyl)-1-(((S)-oxetan-2-yl)methyl)-1H-benzo[d]imidazol-6-carboxylate

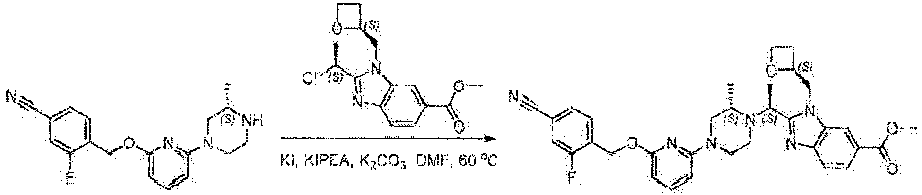

(S)-3-Fluoro-4-((6-(3-methylpiperazin-1-yl)pyridin-2-yl)oxy)methyl)benzonitrile (110.5 mg, 0.30 mmol) was dissolved in *N*,*N-*dimethylformamide (5.0 mL), followed by an addition of *N*,*N-*diisopropylethylamine (78.4 mg, 0.61 mmol), potassium carbonate (83.9 mg, 0.61 mmol), potassium iodide (75.7 mg, 0.46 mmol) and methyl 2-((S)-1-chloroethyl)-1-(((S)-oxetan-2-yl)methyl)-1*H-*benzo[*d*]imidazol-6-carboxylate (102.9 mg, 0.33 mmol). The reaction solution was heated to 60°C and the reaction was carried out overnight with stirring. The reaction solution was diluted with ethyl acetate (50 mL), extracted, washed with saturated saline, and dried with anhydrous sodium sulfate. Then the solvent was spin-dried and the product was purified by column chromatography (ethyl acetate: n-hexane = 4:1) to obtain 140.0 mg of a beige solid, methyl 2-((S)-1-((S)-4-(6-((4-cyano-2-fluorobenzyl)oxy)pyridin-2-yl)-2-methylpiperazin-1-yl)ethyl)-1-(((S)-oxetan-2-yl)methyl)-1*H-*benzo[*d*]imidazol-6-carboxylate (yield: 76.8%). LC-MS: RT = 1.93 min, [M+H]⁺ = 599.35.

### Step E: Synthesis of 2-((S)-1-((S)-4-(6-((4-cyano-2-fluorobenzyl)oxy)pyridin-2-yl)-2-methylpiperazin-1-yl)ethyl)-1-(((S)-oxetan-2-yl)methyl)-1H-benzo[d]imidazol-6-carboarylic acid

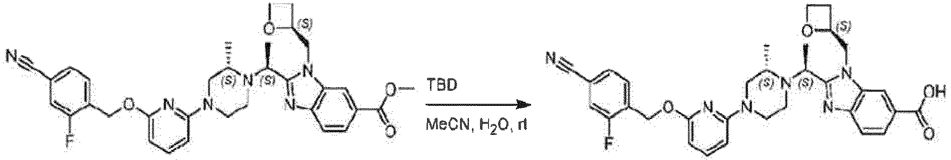

Methyl 2-((S)-1-((S)-4-(6-((4-cyano-2-fluorobenzyl)oxy)pyridin-2-yl)-2-methylpiperazin-1-yl)ethyl)-1-((S)-oxetan-2-yl)methyl)-1*H*-benzo[*d*]imidazol-6-carboxylate (140.0 mg, 0.23 mmol) was dissolved in a mixed solvent of acetonitrile (5.0 mL) and water (1.0 mL), followed by an addition of 1,5,7-triazabicyclo[4.4.0]dec-5-ene (65.1 mg, 0.47 mmol). At room temperature, the reaction was carried out overnight with stirring. The pH of the system was adjusted to 6 by addition of aqueous citric acid solution (2 mol/L), and the resultant was extracted with dichloromethane (30 mL × 3 times). Then the organic phases were combined, dried with anhydrous sodium sulfate, and spin-dried to remove the solvent. The product was separated by column chromatography (methanol: dichloromethane= 8:92) to obtain 45.0 mg of a white solid, 2-((S)-1-((S)-4-(6-((4-cyano-2-fluorobenzyl)oxy)pyridin-2-yl)-2-methylpiperazin-1-yl)ethyl)-1-(((S)-oxetan-2-yl)methyl)-1*H-*benzo[*d*]imidazol-6-carboxylic acid (yield: 32.1%).

LC-MS: RT = 1.84 min, [M+H]⁺ = 585.39. ¹H NMR (400 MHz, DMSO-d₆) δ (ppm): 8.26 (d, *J* = 10 Hz, 1H), 7.87 (dd, *J* = 10.0, 1.4 Hz, 1H), 7.81 (dd, *J* = 8.4, 1.6 Hz, 1H), 7.71 7.66 (m, 2H), 7 61 (t, *J* = 7.6 Hz, 1H), 7.44 (t, *J* = 8.0 Hz, 1H), 6.28 (d, J = 8.2 Hz, 1H), 6.10 (d, *J* = 7.8 Hz, 1H), 5.37 (s, 2H), 5.25-5.17 (m, 1H), 5.03 (dd, *J* = 15.6, 3.0 Hz, 1H), 4.95 (q, *J* = 6.6 Hz, 1H), 4.62 (dd, *J* = 15 4, 5.0 Hz, 1H), 4.49-4.39 (m, 1H), 4.19-4.11 (m, 1H), 4.07-4.00 (m, 1H), 3.88 (d, J = 12.6 Hz, 1H), 2.67-2.54 (m, 2H), 2.40-2.28 (m, 2H), 2.27-2.18 (m, 1H), 1.66-1.56 (m, 2H), 1.36 (d, *J* = 6 6 Hz, 3H), 1.17 (d,*J* = 6.2 Hz, 3H).

### Example 22 2-((S)-1-(4-(6-((4-cyano-2-fluorobenzyl)oxy)pyridin-2-yl)-2-oxopiperazin-1-yl)ethyl)-1-(((S)-oxetan-2-yl)methyl)-1H-benzo[d]imidazol-6-carboxylic acid

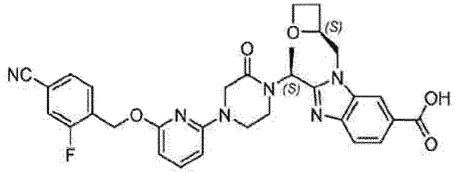

The specific synthetic route is as follows.

### Step A: Synthesis of methyl 2-((S)-1-(4-(tert-butoxycarbonyl)-2-oxopiperazin-1-yl)ethyl)-1-(((S)-oxetan-2-yl)methyl)-1H-benzo[d]imidazol-6-carboxylate

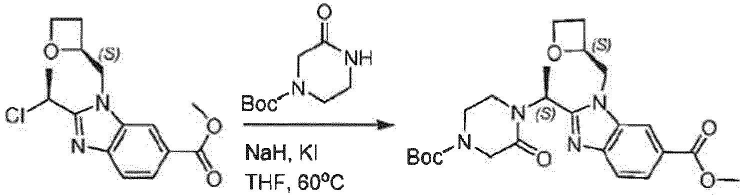

Tert-butyl 3-oxopiperazin-1-carboxylate (380 mg, 1.90 mmol), sodium hydride (380 mg, 9.5 mmol) and potassium iodide (473 mg, 2.85 mmol) were dissolved in 15 mL anhydrous tetrahydrofuran, followed by an addition of methyl 2-((S)-1-chloroethyl)-1-(((S)-oxetan-2-yl)methyl)-1*H*-benzo[*d*]imidazol-6-carboxylate (600 mg, 1.94 mmol), and the reaction was carried out at 60°C for 20 min. After the reaction was completed, the reaction solution was poured into 200 mL of ammonium chloride solution, and the reaction solution was extracted with ethyl acetate (50 mL × 3 times). Then the organic phases were combined, washed with saturated saline (60 mL × 2 times), dried with anhydrous sodium sulfate, filtered, and concentrated under reduced pressure. The resulting crude product was separated by column chromatography to obtain 110 mg of a pale yellow solid, methyl 2-((S)-1-(4-(tert-butoxycarbonyl)-2-oxopiperazin-1-yl)ethyl)-1-(((S)-oxetan-2-yl)methyl)-1*H-*benzo[*d*]imidazol-6-carboxylate. LC-MS: RT = 2.12 min, [M+H]⁺ = 473.27.

### Step B: Synthesis of methyl 1-(((S)-oxetan-2-yl)methyl)-2-((S)-1-(2-oxopiperazin-1-yl)ethyl)-1H-benzo[d]imidazol-6-carboxylate

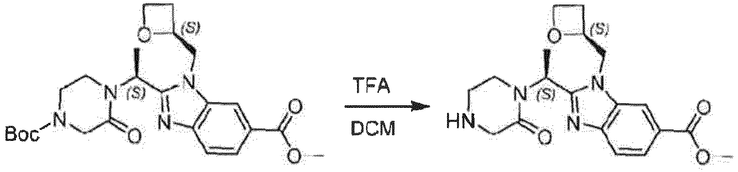

Methyl 2-((S)-1-(4-(tert-butoxycarbonyl)-2-oxopiperazin-1-yl)ethyl)-1-(((S)-oxetan-2-yl)methyl)-1*H-*benzo[*d*]imidazol-6-carboxylate (110 mg, 0.233 mmol) was dissolved in 4 mL of anhydrous dichloromethane, followed by an addition of 2 mL trifluoroacetic acid. The reaction was carried out at room temperature for 40 min. After the reaction was completed, the reaction solution was poured into 100 mL of sodium bicarbonate solution, and was extracted with ethyl acetate (30 mL × 3 times). Then the organic phases were combined, washed with saturated saline (20 mL × 2 times), dried with anhydrous sodium sulfate, filtered and concentrated under reduced pressure to obtain 90 mg of a crude product, methyl 1-(((S)-oxetan-2-yl)methyl)-2-((S)-1-(2-oxopiperazin-1-yl)ethyl)-1*H-*benzo[*d*]imidazol-6-carboxylate. LC-MS: RT= 1.96 min, [M+H]⁺ = 373.27.

### Step C: Synthesis of methyl 2-((S)-1-(4-(6-((4-cyano-2-fluorobenzyl)oxy)pyridin-2-yl)-2-oxopiperazin-1-yl)ethyl)-1-(((S)-oxetan-2-yl)methyl)-1H-benzo[d]imidazol-6-carboxylate

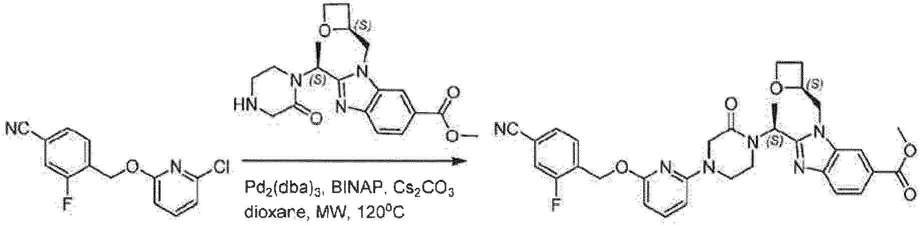

Methyl 1-(((S)-oxetan-2-yl)methyl)-2-((S)-1-(2-oxopiperazin-1-yl)ethyl)-1*H*-benzo[*d*]imidazol-6-carboxylate (90 mg, 0.241 mmol), 4-(((6-chloropyridin-2-yl)oxy)methyl)-3-fluorobenzonitrile (63.2 mg, 0.241 mmol), cesium carbonate ( 157 mg, 0.482 mmol) and 1,1'-binaphthyl-2,2'-bis-diphenylphosphine (15.2 mg, 0.0241 mmol) were dissolved in 5 mL of anhydrous 1,4-dioxane, followed by an addition of tris(dibenzylideneacetone)dipalladium (22.1 mg, 0.0241 mmol). The system was purged with nitrogen for three times, and the reaction solution was performed at 120°C under microwave for 2 hours. After the reaction was completed, the reaction solution was diluted with water and extracted with ethyl acetate (30 mL × 2 times). Then the organic phases were combined, washed with saturated saline (20 mL × 2 times), dried with anhydrous sodium sulfate, filtered, and concentrated under reduced pressure. The resulting crude product was separated by column chromatography to obtain 72 mg of a pale yellow solid, methyl 2-((S)-1-(4-(6-((4-cyano-2-fluorobenzyl)oxy)pyridin-2-yl)-2-oxopiperazin-1-yl)ethyl)-1-(((S)-oxetan-2-yl)methyl)-1*H-*benzo[*d*]imidazol-6-carboxylaye. LC-MS: RT = 2.08 min, [M+H]⁺ = 599.27.

### Step D: Synthesis of 2-((S)-1-(4-(6-((4-cyano-2-fluorobenzyl)oxy)pyridin-2-yl)-2-oxopiperazin-1-yl)ethyl)-1-(((S)-oxetan-2-yl)methyl)-1H-benzo[d]imidazol-6-carboxylic acid

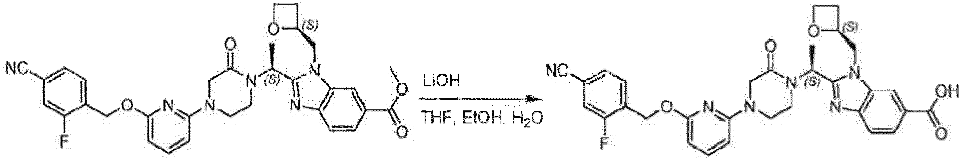

Methyl 2-((S)-1-(4-(6-((4-cyano-2-fluorobenzyl)oxy)pyridin-2-yl)-2-oxopiperazin-1-yl)ethyl)-1-((S)-oxetan-2-yl)methyl)-1*H-*benzo[*d*]imidazol-6-carboxylate (72 mg, 0.121 mmol) was dissolved in 4 mLof tetrahydrofuran, followed by an addition of lithium hydroxide monohydrate (26 mg, 0.602 mmol), 1 mL of water and 1 mL of ethanol, and the reaction was carried out at room temperature for 50 min. After the reaction was completed, the reaction solution was neutralized by adding 20 mL of saturated ammonium chloride solution, and was extracted with ethyl acetate (30 mL × 2 times). Then the organic phases were combined, washed with saturated saline (20 mL × 2 times), dried with anhydrous sodium sulfate, filtered, and concentrated under reduced pressure. The resulting crude product was separated by column chromatography to obtain 37 mg of a white solid, 2-((S)-1-(4-(6-((4-cyano-2-fluorobenzyl)oxy)pyridin-2-yl)-2-oxopiperazin-1-yl)ethyl)-1-(((S)-oxetan-2-yl)methyl)-1*H-*benzo[*d*]imidazol-6-carboxylic acid.

LC-MS: RT =1.95 min, [M+H]⁺ =585.38. ¹H NMR (500 MHz, DMSO) δ 12.67 (s, 1H), 8.30 (d, *J* = 1.0 Hz, 1H), 7.89 (dd, *J* = 9.7, 0.9 Hz, 1H), 7.82 (dd, *J* = 8.4, 1.6 Hz, IH), 7.75 (t, *J* = 8.0 Hz, 1H), 7.72-7.70 (m, 3H), 7.54-7.51 (m, 1H), 6.73-(m, 1H), 5.43 (s, 2H), 5.15-5.09 (m, 1H), 4.75-4.70 (m, 2H), 4.59-4.55 (m, 1H), 4.45-4.41 (m, 1H), 4.18-4.14 (m, 1H), 3.80-3.72 (m, 2H), 3.43 (d, *J* = 11.6 Hz, 2H), 2.94-2.88 (m, 2H), 2.65-2.57 (m, 1H), 2.34-2.24 (m, 1H), 1.55 (d, *J* = 6.8 Hz, 3H).

### Example 23 2-((S)-1-(4-(6-((4-cyano-2-fluorobenzyl)oxy)pyridin-2-yl)-3-oxopiperazin-1-yl)ethyl)-1-(((S)-oxetan-2-yl)methyl)-1H-benzo[d]imidazol-6-carboxylic acid

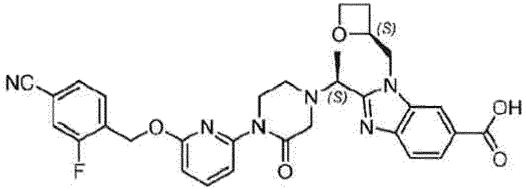

The specific synthetic route is as follows.

### Step A: Synthesis of tert-butyl 4-(6-((4-cyano-2-fluorobenzyl)oxy)pyridin-2-yl)-3-oxopiperazin-1-formate

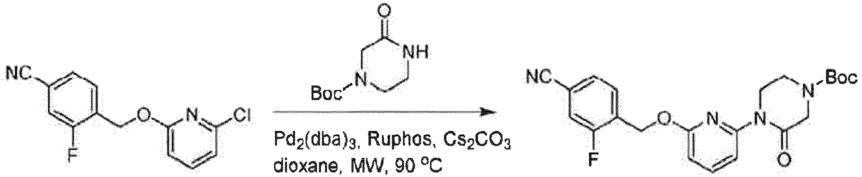

4-(((6-Chloropyridin-2-yl)oxy)methyl)-3-fluorobenzonitrile (130 mg, 0.496 mmol), tert-butyl 3-oxopiperazin-1-carboxylate (99.2 mg, 0.496 mmol), cesium carbonate (323 mg, 0.992 mmol) and 2-dicyclohexylphosphonium-2',6'-diisopropoxy-1,1'-biphenyl (22.5 mg, 0.0496 mmol) were dissolved in 5 mL of anhydrous 1,4-dioxane, followed by an addition of tris(dibenzylideneacetone)dipalladium (45.3 mg, 0.0496 mmol). The system was purged by nitrogen for three times, and the reaction solution was performed at 90°C under microwave for 2 hours. After the reaction was completed, the reaction solution was diluted with water, extracted with ethyl acetate (30 mL × 2 times), and then the organic phases were combined, washed with saturated saline (20 mL × 2 times), dried with anhydrous sodium sulfate, filtered, and concentrated under reduced pressure. Then the resulting crude product was separated by column chromatography to obtain 151 mg of a pale yellow solid, tert-butyl 4-(6-((4-cyano-2-fluorobenzyl)oxy)pyridin-2-yl)-3-oxopiperazin-1-formate. LC-MS: RT = 2.08 min, [M+H]⁺ = 427.21.

### Step B: Synthesis of 3-fluoro-4-(((6-(2-oxopiperazin-1-yl)pyridin-2-yl)oxy)methyl)benzonitrile

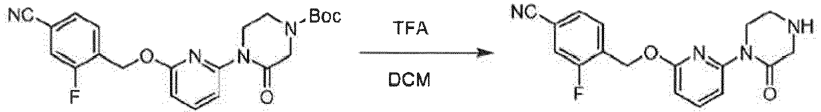

Tert-butyl 4-(6-(((4-cyano-2-fluorobenzyl)oxy)pyridin-2-yl)-3-oxopiperazin-1-formate (151 mg, 0.354 mmol) was dissolved in 4 mL of anhydrous dichloromethane, followed by an addition of 2 mL of trifluoroacetic acid, and the reaction was carried out at room temperature for 40 min. After the reaction was completed, the reaction solution was poured into 60 mL of sodium bicarbonate solution, and the reaction solution was extracted with ethyl acetate (30 mL × 3 times). Then the organic phases were combined, washed with saturated saline (20 mL × 2 times), dried with anhydrous sodium sulfate, and finally concentrated under reduced pressure to obtain 120 mg of a crude product, 3-fluoro-4-(((6-(2-oxopiperazin-1-yl)pyridin-2-yl)oxy)methyl)benzonitrile. LC-MS: RT = 1.89 min, [M+H]⁺ = 327.27.

### Step C: Synthesis of methyl 2-((S)-1-(4-(6-((4-cyano-2-fluorobenzyl)oxy)pyridin-2-yl)-3-oxopiperazin-1-yl)ethyl)-1-(((S)-oxetan-2-yl)methyl)-1H-benzo[d]imidazol-6-carboxylate

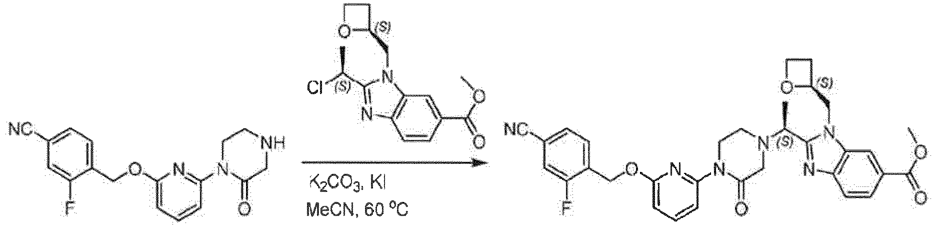

3-Fluoro-4-(((6-(2-oxopiperazin-1-yl)pyridin-2-yl)oxy)methyl)benzonitrile (120 mg, 0.367 mmol), potassium carbonate (101 mg, 0.733 mmol) and potassium iodide (91.4 mg, 0.551 mmol) were dissolved in 5 mL of anhydrous acetonitrile, followed by an addition of methyl 2-((S)-1-chloroethyl)-1-(((S)-oxetan-2-yl)methyl)-1*H-*benzo[*d*]imidazol-6-carboxylate (113 mg, 0.367 mmol), and the reaction was carried out at 60°C for 2 hours. After the reaction was completed, the reaction solution was diluted with water and extracted with ethyl acetate (30 mL × 2 times). Then the organic phases were combined, washed with saturated saline (20 mL × 2 times), dried with anhydrous sodium sulfate, and concentrated under reduced pressure. Finally, the resulting crude product was separated by column chromatography to obtain 146 mg of a pale yellow solid, methyl 2-((S)-1-(4-(6-((4-cyano-2-fluorobenzyl)oxy)pyridin-2-yl)-3-oxopiperazin-1-yl)ethyl)-1-(((S)-oxetan-2-yl)methyl)-1*H-*benzo[*d*]imidazol-6-carboxylate. LC-MS: RT = 1.93 min, [M+H]⁺ = 599.27.

### Step D: Synthesis of 2-((S)-1-(4-(6-((4-cyano-2-fluorobenzyl)oxy)pyridin-2-yl)-3-oxopiperazin-1-yl)ethyl)-1-(((S)-oxetan-2-yl)methyl)-1H-benzo[d]imidazol-6-carboxylic acid

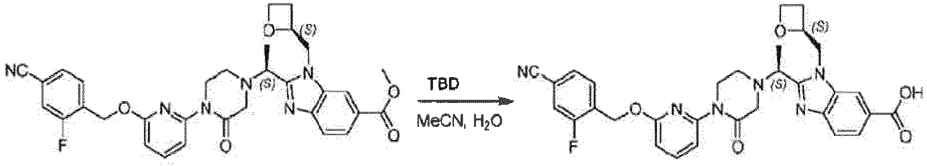

Methyl 2-((S)-1-(4-(6-((4-cyano-2-fluorobenzyl)oxy)pyridin-2-yl)-3-oxopiperazin-1-yl)ethyl)-1-((S)-oxetan-2-yl)methyl)-1*H-*benzo[*d*]imidazol-6-carboxylate (146 mg, 0.243 mmol) was dissolved in 4 mL of acetonitrile, followed by an addition of 1,5,7-triazabicyclo[4.4.0]dec-5-ene (71 mg, 0.510 mmol) and 1 mL of water, and the reaction was carried out at room temperature for 7 hours. After the reaction was completed, the reaction solution was neutralized by adding 20 mL of saturated ammonium chloride solution, and was extracted with dichloromethane (30 mL × 2 times). Then the organic phases were combined, washed with saturated saline (20 mL × 2 times), dried with anhydrous sodium sulfate, and concentrated under reduced pressure. Finally, the resulting crude product was separated by column chromatography to obtain 38 mg of a white solid, 2-((S)-1-(4-(6-((4-cyano-2-fluorobenzyl)oxy)pyridin-2-yl)-3-oxopiperazin-1-yl)ethyl)-1-(((S)-oxetan-2-yl)methyl)-1*H*-benzo[*d*]imidazol-6-carboxylic acid.

GC-MS: RT =1.87 min, [M+H]⁺ =585.33. ¹H NMR (500 MHz, DMSO) δ 12.69 (s, 1H), 8.30 (d, *J* = 1.0 Hz, 1H), 7.89 (dd, *J* = 9.7, 0.9 Hz, 1H), 7.82 (dd, *J=* 8.4, 1 6 Hz, 1H), 7.75 (t, *J* = 8.0 Hz, 1H), 7.72-7 70 (m ,3H), 7.54-7 .51 (m, 1H), 6.73-6.70 (m, 1H), 5.43 (s, 2H), 5.15-5.09 (m, 1H), 4.75-4.70 (m, 2H), 4.59-4.55 (m, 1H), 4.45-4.41 (m, 1H), 4.17 (dt, *J* = 9.0, 5.8 Hz, 1H), 3.78-3.72 (m, 2H), 3 43 (d, *J* = 116 Hz, 2H), 2.94-2.88 (m, 2H), 2.65-2.57 (m, 1H), 2 34-2.24 (m, 1H), 1.51 (d, *J* = 6 8 Hz, 3H).

### Example 24 2-((S)-1-(4-(6-((4-chloro-2-fluorobenzyl)oxy)pyridin-2-yl)piperidin-1-yl)propyl)-1-(((S)-oxetan-2-yl)methyl)-1H-benzo[d]imidazol-6-formic acid (24A) and 2-((R)-1-(4-(6-((4-chloro-2-fluorobenzyl)oxy)pyridin-2-yl)piperidin-1-yl)propyl)-1-(((S)-oxetan-2-yl)methyl)-1H-benzo[d]imidazol-6-formic acid (24B)

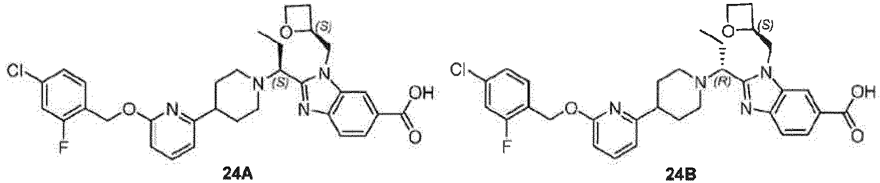

The specific synthetic route for compound **24A** is as follows:

### Step A: Synthesis of methyl 4-((R)-2-bromobutyramido)-3-((((S)-oxetan-2-yl)methyl)amino)benzoate

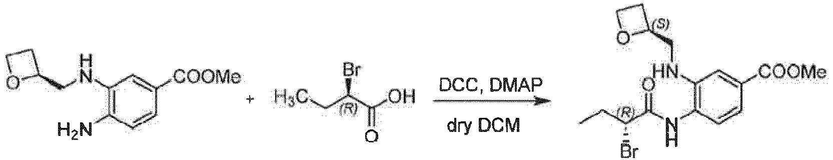

At room temperature, methyl 4-amino-3-((((S)-oxetan-2-yl)methyl)amino)benzoate (190.2 mg, 0.8 mmol) and (R)-2-bromobutyric acid (200.0 mg, 1.2 mmol) were dissolved in dry dichloromethane (5 mL). 4-Dimethylaminopyridine (9.7 mg, 80.0 µmol) and dicyclohexylcarbodiimide (494.4 mg, 2.4 mmol) were added to the above solution sequentially. The reaction was carried out at room temperature overnight with stirring, and after the reaction was completed, the reaction solution was filtered under reduced pressure with diatomite and concentrated under reduced pressure. The resulting residue was purified by silica gel column chromatography (eluent: petroleum ether/ethyl acetate = 1/1) to obtain 300.3 mg of a pale yellow oily product, methyl 4-((R)-2-bromobutyramido)-3-((((S)-oxetan-2-yl)methyl)amino)benzoate (yield : 97.1%). LC-MS: RT = 2.45 min, [M+H]⁺ = 384.76, 387.02.

### Step B: Synthesis of methyl 2-((R)-1-bromopropyl)-1-(((S)-oxetan-2-yl)methyl)-1H-benzo[d]imidazol-6-formate

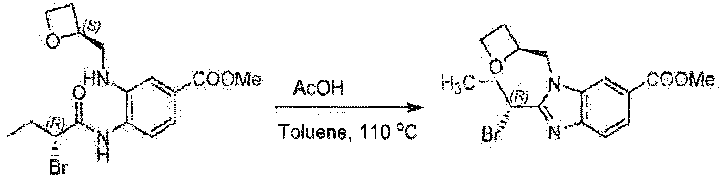

At room temperature, methyl 4-((R)-2-bromobutyramido)-3-((((S)-oxetan-2-yl)methyl)amino)benzoate (300.3 mg, 0.8 mmol) was dissolved in toluene (3 mL), followed by an addition of acetic acid (200 µL). The reaction was carried out at 110°C for 3 hours under reflux, and after the reaction was completed, the reaction solution was concentrated under reduced pressure. The resulting residue was purified by silica gel column chromatography (eluent: petroleum ether/ethyl acetate = 3/1) to obtain 195.2 mg of a yellow oily product, methyl 2-((R)-1-bromopropyl)-1-(((S)-oxetan-2-yl)methyl)-1*H*-benzo[*d*]imidazol-6-formate (yield: 68.3%). LC-MS: RT = 2.34 min, [M+H]⁺ = 366.93, 369.12.

### Step C: Synthesis of methyl 2-((S)-1-(4-(6-((4-chloro-2-fluorobenzyl)oxy)pyridin-2-yl)piperidin-1-yl)propyl)-1-(((S)-oxetan-2-yl)methyl)-1H-benzo[d]imidazol-6-formate

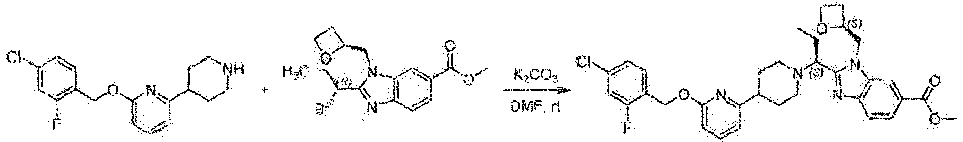

At room temperature, methyl 2-((R)-1-bromopropyl)-1-(((S)-oxetan-2-yl)methyl)-1*H*-benzo[*d*]imidazol-6-formate (195.2 mg, 0.5 mmol) and 2-((4-chloro-2-fluorobenzyl)oxy)-6-(piperidin-4-yl)pyridine (210.3 mg, 0.6 mmol) were dissolved in *N,N-*dimethylformamide (3 mL). Anhydrous potassium carbonate (208.5 mg, 1.5 mmol) was added to the above solution and the reaction was carried out at room temperature overnight with stirring. After the reaction was complete, the reaction solution was diluted with water (20 mL), extracted with ethyl acetate (30 mL × 3 times), and the organic phases were combined, washed with saturated saline (50 mL × 3 times), dried with anhydrous sodium sulfate, and finally concentrated under reduced pressure. The resulting residue was purified by silica gel column chromatography (eluent: petroleum ether/ethyl acetate = 1/1 to 15/1) to obtain 230.2 mg of a white solid compound, methyl 2-((S)-1-(4-(6-((4-chloro-2-fluorobenzyl)oxy)pyridin-2-yl)piperidin-1-yl)propyl)-1-(((S)-oxetan-2-yl)methyl)-1*H*-benzo[d]imidazol-6-formate (yield: 71.4%). LC-MS: RT = 3.43 min, [M+H]⁺ = 607.07.

### Step D: Synthesis of 2-((S)-1-(4-(6-((4-chloro-2-fluorobenzyl)oxy)pyridin-2-yl)piperidin-1-yl)propyl)-1-(((S)-oxetan-2-yl)methyl)-1H-benzo[d]imidazol-6-formic acid

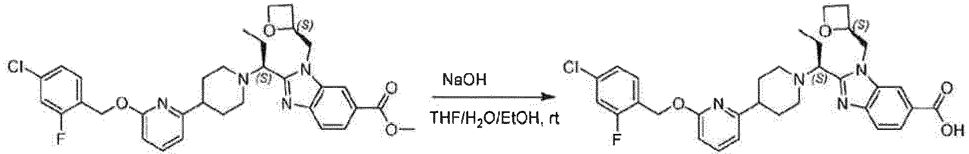

At room temperature, methyl 2-((S)-1-(4-(6-((4-chloro-2-fluorobenzyl)oxy)pyridin-2-yl)piperidin-1-yl)propyl)-1-(((S)-oxetan-2-yl)methyl)-1*H-*benzo[*d*]imidazol-6-formate (230.2 mg, 0.4 mmol) was dissolved in a mixture of tetrahydrofuran (6 mL), water (2 mL) and ethanol (2 mL), followed by an addition of sodium hydroxide (48.0 mg, 1.2 mmol), and the reaction was carried out at room temperature overnight with stirring.

After the reaction was complete, 1 mole per liter of aqueous citric acid solution was added dropwise until the pH of the solution was adjusted to 5. The reaction solution was diluted with water (20 mL), extracted with ethyl acetate (30 mL × 3 times), and the organic phases were combined, washed with saturated saline (25 mL × 3 times), dried with anhydrous sodium sulfate, and finally concentrated under reduced pressure. The resulting residue was purified by semi-preparative HPLC (mobile phase: water containing 0.1% ammonia/acetonitrile = 90/10 to 40/60) to obtain 68.8 mg of 2-((S)-1-(4-(6-((4-chloro-2-fluorobenzyl)oxy)pyridin-2-yl)piperidin-1-yl)propyl)-1-(((S)-oxetan-2-yl)methyl)-1*H-*benzo[d]imidazol-6-formic acid (yield: 30.7%).

LC-MS: RT = 2.68 min, [M+H]⁺ = 593.21. ¹H NMR (400 MHz, CD₃OD) δ 8 40 (s, 1H), 8 09 (dd, *J* = 8.6, 1.5 Hz, 1H), 7.86 (d, *J* = 8 5 Hz, 1H), 7.67 (t, *J* = 7 8 Hz, 1H), 7.51 (t, *J* = 8.0 Hz, 1H), 7.25 (ddd, *J* = 10 7, 9.0, 2.0 Hz, 2H), 6.91 (d, *J* = 7.2 Hz, 1H), 6 75 (d, *J* = 8.3 Hz, 1H), 5.43 (s, 2H), 5.22 (q, *J* = 7.9 Hz, 1H), 5.09 (dd,*J* = 11.U, 3.9 Hz, 1H), 4.92 (s, 1H), 4.80 (d, *J* = 15.3 Hz, 3H), 4.04 (s, 1H), 3.64 (s, 2H), 3.03 (s, 1H), 2.93 (p, *J* = 7.4 Hz, 1H), 2.70 (p, *J* = 8.3 Hz, 1H), 2.47 (d, *J* = 36.2 Hz, 2H), 220 (t, *J* = 12.0 Hz, 3H), 2.15-1.96 (m, 2H), 0.98 (t, *J* = 7.3 Hz, 3H).

The specific synthetic route for compound **24B** is as follows:

### Step A: Synthesis of methyl 4-((S)-2-bromobutyramido)-3-((((S)-oxetan-2-yl)methyl)amino)benzoate

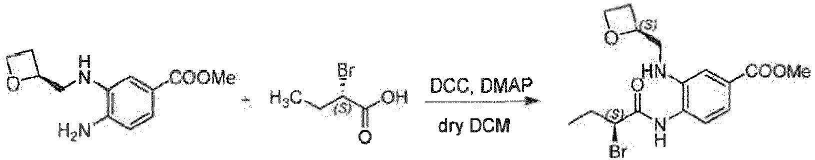

At room temperature, methyl 4-amino-3-((((S)-oxetan-2-yl)methyl)amino)benzoate (190.2 mg, 0.8 mmol) and (S)-2-bromobutyric acid (200.0 mg, 1.2 mmol) were dissolved in dry dichloromethane (5 mL). 4-Dimethylaminopyridine (9.7 mg, 80.0 micromolar) and dicyclohexylcarbodiimide (494.4 mg, 2.4 mmol) were added to the above solution sequentially. The reaction was carried out at room temperature overnight with stirring, and after the reaction was complete, the reaction solution was filtered under reduced pressure with diatomite and concentrated under reduced pressure. The resulting residue was purified by silica gel column chromatography (eluent: petroleum ether/ethyl acetate = 1/1) to obtain 300.3 mg of a pale yellow oily product, methyl 4-((S)-2-bromobutyramido)-3-((((S)-oxetan-2-yl)methyl)amino)benzoate (yield : 97.1%). LC-MS: RT = 2.45 min, [M+H]⁺ = 384.76, 387.02.

### Step B: Synthesis of methyl 2-((S)-1-bromopropyl)-1-(((S)-oxetan-2-yl)methyl)-1H-benzo[d]imidazol-6-formate

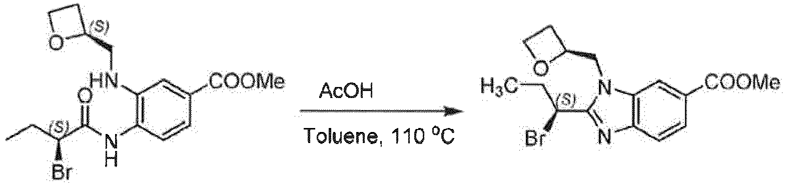

At room temperature, methyl 4-((S)-2-bromobutyramido)-3-((((S)-oxetan-2-yl)methyl)amino)benzoate (300.3 mg, 0.8 mmol) was dissolved in toluene (3 mL), followed by an addition of acetic acid (200 µL). The reaction was carried out at 1 10°C for 3 hours under reflux, and after the reaction was complete, the reaction solution was concentrated under reduced pressure. Then the resulting residue was purified by silica gel column chromatography (eluent: petroleum ether/ethyl acetate = 3/1) to obtain 195.2 mg of a yellow oily product, methyl 2-((S)-1-bromopropyl)-1-(((S)-oxetan-2-yl)methyl)-1*H-*benzo[*d*]imidazol-6-formate (yield: 68.3%). LC-MS: RT = 2.34 min, [M+H]⁺ = 366.93, 369.12.

### Step C: Synthesis of methyl 2-((R)-1-(4-(6-((4-chloro-2-fluorobenzyl)oxy)pyridin-2-yl)piperidin-1-yl)propyl)-1-(((S)-oxetan-2-yl)methyl)-1H-benzo[d]imidazol-6-formate

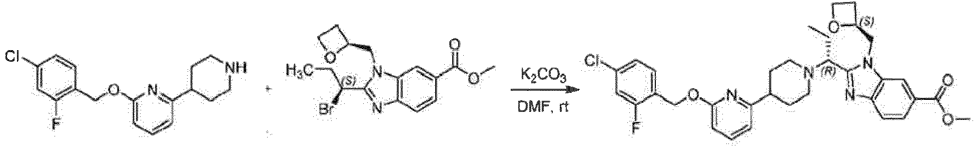

At room temperature, methyl 2-((S)-1-bromopropyl)-1-(((S)-oxetan-2-yl)methyl)-1*H*-benzo[*d*]imidazol-6-formate (195.2 mg, 0.5 mmol) and 2-((4-chloro-2-fluorobenzyl)oxy)-6-(piperidin-4-yl)pyridine (210.3 mg, 0.6 mmol) were dissolved in *N,N-*dimethylformamide (3 mL). Anhydrous potassium carbonate (208.5 mg, 1.5 mmol) was added to the above solution and the reaction was carried out at room temperature overnight with stirring. After the reaction was complete, the reaction solution was diluted with water (20 mL) and extracted with ethyl acetate (30 mL × 3 times). Then the organic phases were combined, washed with saturated saline (50 mL × 3 times), dried with anhydrous sodium sulfate, filtered, and concentrated under reduced pressure. The resulting residue was purified by silica gel column chromatography (eluent: petroleum ether/ethyl acetate = 1/1 to 15/1) to obtain 230.2 mg of a white solid compound, methyl 2-((R)-1-(4-(6-((4-chloro-2-fluorobenzyl)oxy)pyridin-2-yl)piperidin-1-yl)propyl)-1-((S)-oxetan-2-yl)methyl)-1*H*-benzo[*d*]imidazol-6-formate (yield: 71.4%). LC-MS: RT = 3.43 min, [M+H]⁺ = 607.07.

### Step D: Synthesis of 2-((R)-1-(4-(6-((4-chloro-2-fluorobenzyl)oxy)pyridin-2-yl)piperidin-1-yl)propyl)-1-(((S)-oxetan-2-yl)methyl)-1H-benzo[d]imidazol-6-formic acid

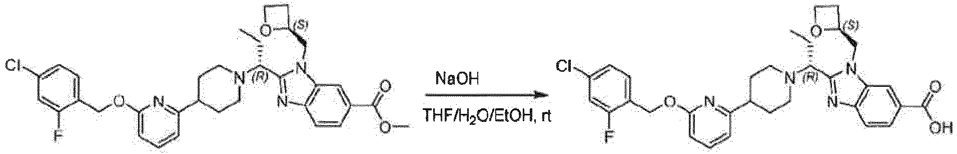

At room temperature, methyl 2-((R)-1-(4-(6-((4-chloro-2-fluorobenzyl)oxy)pyridin-2-yl)piperidin-1-yl)propyl)-1-(((S)-oxetan-2-yl)methyl)-1*H*-benzo[*d*]imidazo-6-formate (230.2 mg, 0.4 mmol) was dissolved in a mixture of tetrahydrofuran (6 mL), water (2 mL) and ethanol (2 mL), followed by an addition of sodium hydroxide (48.0 mg, 1.2 mmol), and the reaction was carried out at room temperature overnight with stirring.

After the reaction was complete, 1 mole per liter of aqueous citric acid solution was added dropwise until the pH of the solution was adjusted to 5. The reaction solution was diluted with water (20 mL), extracted with ethyl acetate (30 mL × 3 times), and the organic phases were combined, washed with saturated saline (25 mL × 3 times), dried with anhydrous sodium sulfate, and finally concentrated under reduced pressure. The resulting residue was purified by semi-preparative HPLC (mobile phase: water containing 0.1% ammonia/acetonitrile = 90/10 to 40/60) to obtain 68.8 mg of 2-((R)-1-(4-(6-((4-chloro-2-fluorobenzyl)oxy)pyridin-2-yl)piperidin-1-yl)propyl)-1-(((S)-oxetan-2-yl)methyl)-1*H-*benzo[d]imidazol-6-formic acid (yield: 30.7%).

LC-MS: RT = 2.68 min, [M+H]⁺ = 593.21. ¹H NMR (400 MHz, CD₃OD) δ 8 34 (d, *J* = 1.5 Hz, 1H), 8.03 (dd, *J* = 8.5, 1 5 Hz, 1H), 7.77 (d, *J* = 8.5 Hz, 1H), 7.60 (dd, *J* = 8 2, 7.3 Hz, 1H), 7.50 (t, *J* = 8.3 Hz, 1H), 7.25-7.14 (m, 2H), 6.83 (d, *J* = 7.3 Hz, 1H), 6.66 (d, *J* = 8.1 Hz, 1H), 5.41 (s, 2H), 5.32 (qd, *J* = 7.2, 2.5 Hz, 1H), 4.85 (dd, *J* = 15.8, 2.7 Hz, 1H), 4.75 (dd, *J* = 15.8, 5.9 Hz, 1H), 4.66 (td, *J* = 8.0, 5.9 Hz, 1H), 4.51 (dd, *J* = 10.6, 4.1 Hz, 1H), 4.40 (dt, *J* = 9.3, 5.8 Hz, 1H), 3.22 (t, *J* = 10.9 Hz, 2H), 2.92 (td, *J* = 10 8, 4.8 Hz, 1H), 2.80 (ddt, *J* = 13.8, 8.1, 4.0 Hz, 1H), 2.68 (dq, *J* = 10 9, 5.5, 4.8 Hz, 1H), 2.60 (td, *J* = 12.1, 3.4 Hz, 1H), 2.56-2.46 (m, 1H), 2.35 (ddd, *J* = 13.1, 10.6, 7.1 Hz, 1H), 2.19 (ddt, *J* = 11.6, 7.3, 4.3 Hz, 1H), 1.96 (td, *J* = 10.5, 9.5, 3.4 Hz, 2H), 1.91-1.72 (m, 2H), 0.99 (t, *J* = 7.3 Hz, 3H).

### Example 25 2-((4-(6-((4-chloro-2-fluorobenzyl)oxy)pyridin-2-yl)piperidin-1-yl)(cyclopropyl)methyl)-1-(((S)-oxetan-2-yl)methyl)-1H-benzo[d]imidazol-6-carboxylic acid

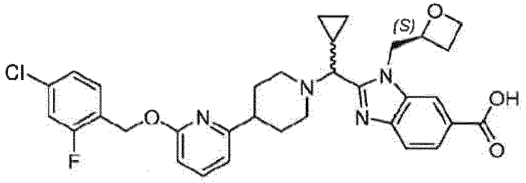

The specific synthetic route is as follows.

### Step A: Synthesis of ethyl 2-(4-(6-((4-chloro-2-fluorobenzyl)oxy)pyridin-2-yl)piperidin-1-yl)-2-cyclopropylacetate

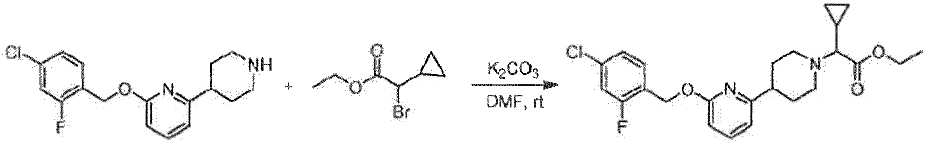

At room temperature, 4-(6-((4-chloro-2-fluorobenzyl)oxy)pyridin-2-yl)piperidine (155.0 mg, 483.2 µmol), ethyl 2-bromo-2-cyclopropylacetate (200.0 mg, 966.4 µmol) and potassium carbonate (267.3 mg, 1.9 mmol) were dissolved in *N,N-*dimethylformamide (5 mL), and the reaction was carried out at room temperature for 16 hours. After the reaction was complete, the reaction solution was diluted with water (80 mL), extracted with ethyl acetate (50 mL × 3 times), and the organic phases were combined, washed with saturated saline (50 mL × 3 times), dried with anhydrous sodium sulfate, and finally concentrated under reduced pressure. The resulting residue was purified by silica gel column chromatography (eluent: petroleum ether/ethyl acetate = 5/1) to obtain 177.2 mg of a pale yellow oily product, ethyl 2-(4-(6-((4-chloro-2-fluorobenzyl)oxy)pyridin-2-yl)piperidin-1-yl)-2-cyclopropylacetate (a mixture of S- and R-configurations) (yield: 82.5%). LC-MS: RT = 3.00 min, [M+H]⁺ = 446.97.

### Step B: Synthesis of 2-(4-(6-((4-chloro-2-fluorobenzyl)oxy)pyridin-2-yl)piperidin-1-yl)-2-cyclopropylacetic acid

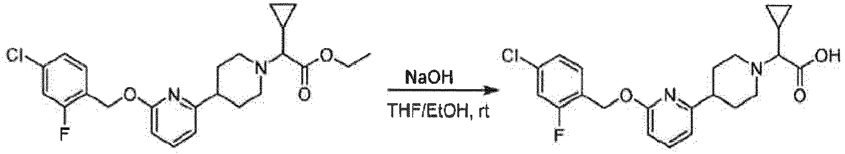

At room temperature, ethyl 2-(4-(6-((4-chloro-2-fluorobenzyl)oxy)pyridin-2-yl)piperidin-1-yl)-2-cyclopropylacetate (177.0 mg, 396.7 µmol) was dissolved in a mixture of tetrahydrofuran (10 mL), ethanol (3 mL) and water (3 mL), followed by an addition of sodium hydroxide (160.0 mg, 4.0 mmol). The reaction was carried out at room temperature for 48 hours. After the reaction was complete, 1 mole per liter of aqueous citric acid solution was added dropwise until the pH of the solution was adjusted to 5. The resultant was diluted with water (50 mL), extracted with ethyl acetate (25 mL × 3 times), washed with saturated saline (25 mL × 3 times), dried with anhydrous sodium sulfate, and finally concentrated under reduced pressure. The resulting residue was purified by silica gel column chromatography (eluent: petroleum ether/ethyl acetate = 1/1 to 10/1) to obtain 132.0 mg of a yellow oily product, 2-(4-(6-((4-chloro-2-fluorobenzyl)oxy)pyridin-2-yl)piperidin-1-yl)-2-cyclopropylacetic acid (a mixture of S- and R-configurations) (yield: 76.9%). LC-MS: RT = 2.45 min, [M+H]⁺ = 419.05.

### Step C: Synthesis of methyl 4-(2-(4-(6-((4-chloro-2-fluorobenzyl)oxy)pyridin-2-yl)piperidin-1-yl)-2-cyclopropylacetamino)-3-(((S)-oxetan-2-yl)methyl)benzoate

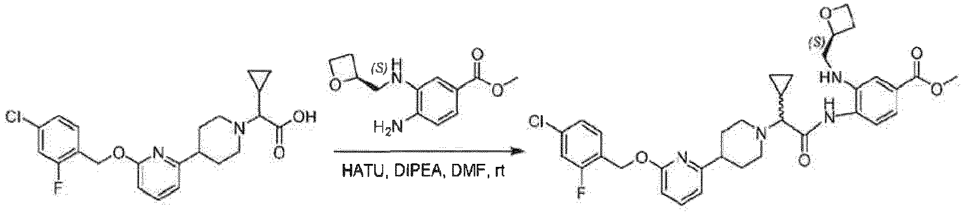

At room temperature, 2-(4-(6-((4-chloro-2-fluorobenzyl)oxy)pyridin-2-yl)piperidin-1-yl)-2-cyclopropylacetic acid (132.0 mg, 315.1 µmol), 2-(7-azabenzotriazole)-*N,N,N',N*'-tetramethyluronium hexafluorophosphate (120.0 mg, 315.1 µmol) and *N,N-*diisopropyl ethylamine (122.5 mg, 945.2 µmol) were dissolved in *N,N*-dimethylformamide (5 mL). The reaction was carried out at room temperature for half an hour, followed by adding methyl (S)-4-amino-3-((((S)-oxetan-2-yl)methyl)amino)benzoate (74.3 mg, 315.1 µmol), and the reaction was continued for 24 hours.

After the reaction was complete, the reaction solution was diluted with ethyl acetate (50 mL), extracted, washed sequentially with water (30 mL × 3 times), saturated saline (30 mL × 3 times), dried with anhydrous sodium sulfate, and finally concentrated under reduced pressure. The resulting residue was purified by silica gel column chromatography (eluent: petroleum ether/ethyl acetate = 5/1 to 1/1) to obtain 85.0 mg of a yellow oily product, methyl 4-(2-(4-(6-((4-chloro-2-fluorobenzyl)oxy)pyridin-2-yl)piperidin-1-yl)-2-cyclopropylacetamino)-3-(((S)-oxetan-2-yl)methyl)benzoate (a mixture of S- and R-configurations) (yield: 42.2%). LC-MS: RT = 3.38 min, [M+H]⁺ = 637.14.

### Step D: Synthesis of methyl 2-((4-(6-((4-chloro-2-fluorobenzyl)oxy)pyridin-2-yl)piperidin-1-yl)(cyclopropyl)methyl)-1-(((S)-oxetan-2-yl)methyl)-1H-benzo[d|imidazol-6-carboxylate

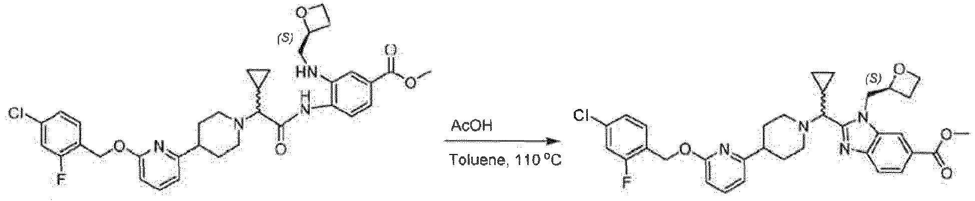

At room temperature, methyl 4-(2-(4-(6-((4-chloro-2-fluorobenzyl)oxy)pyridin-2-yl)piperidin-1-yl)-2-cyclopropylacetamino)-3-(((S)-oxetan-2-yl)methyl)benzoate (85.0 mg, 137.2 µmol) was added to toluene (5 mL), followed by an addition of acetic acid (200 µL). The reaction solution was heated to 110°C and the reaction was carried out for 4 hours. After the reaction was complete, the reaction solution was cooled to room temperature, diluted with water (30 mL), and extracted with ethyl acetate (15 mL × 3 times). Then the organic phases were combined, washed sequentially with saturated aqueous sodium carbonate solution (15 mL × 3 times) and saturated saline (15 mL × 3 times), dried with anhydrous sodium sulfate, and finally concentrated under reduced pressure. The resulting residue was purified by silica gel column chromatography (eluent: petroleum ether/ethyl acetate = 1/1 to 10/1) to obtain 51.0 mg of a yellow oily product, methyl 2-((4-(6-((4-chloro-2-fluorobenzyl)oxy)pyridin-2-yl)piperidin-1-yl)(cyclopropyl)methyl)-1-(((S)-oxetan-2-yl)methyl)-1*H*-benzo[*d*]imidazol-6-carboxylate (a mixture of S- and R- configurations) (yield: 60.1%). LC-MS: RT = 3.48min, [M+H]⁺ = 619.29.

### Step E: Synthesis of 2-((4-(6-((4-chloro-2-fluorobenzyl)oxy)pyridin-2-yl)piperidin-1-yl)(cyclopropyl)methyl)-1-(((S)-oxetan-2-yl)methyl)-1H-benzo[d]imidazol-6-carboxylic acid

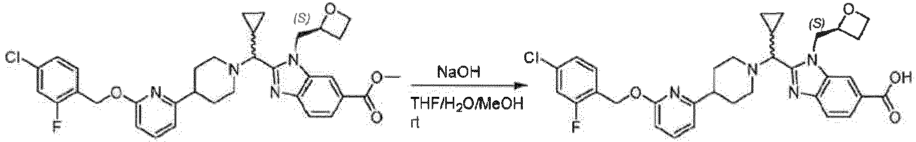

At room temperature, methyl 2-((4-(6-((4-chloro-2-fluorobenzyl)oxy)pyridin-2-yl)piperidin-1-yl)(cyclopropyl)methyl)-1-(((S)-oxetan-2-yl)methyl)-1*H*-benzo[*d*]imidazol-6-carboxylate (51.0 mg, 82.4 µmol) was dissolved in a solution of tetrahydrofuran (6 mL), methanol (2 mL) and water (2 mL), followed by an addition of sodium hydroxide (32.9 mg, 823.7 µmol), and the reaction was carried out at room temperature for 24 hours. After the reaction was complete, 1 mole per liter of aqueous citric acid was solution added dropwise until the pH of the solution was adjusted to 5. The resultant was diluted with water (50 mL) and extracted with ethyl acetate (25 mL × 3 times), and the organic phases were combined, washed with saturated saline (25 mL × 3 times), dried with anhydrous sodium sulfate, and finally concentrated under reduced pressure. The resulting residue was purified by reversed-phase HPLC (mobile phase: water containing 0.1% ammonia/acetonitrile = 90/10 to 40/60) and lyophilized to obtain 9.2 mg of a white solid, 2-((4-(6-((4-chloro-2-fluorobenzyl)oxy)pyridin-2-yl)piperidin-1-yl)(cyclopropyl)methyl)-1-(((S)-oxetan-2-yl)methyl)-1*H*-benzo[*d*]imidazol-6-carboxylic acid (a mixture of S- and R- configurations) (yield: 18.4%). LC-MS: RT = 2.84 min, [M+H]⁺ = 605.22.

### Example 26 2-(1-(4-(6-((4-chloro-2-fluorobenzyl)oxy)pyridin-2-yl)piperidin-1-yl)-2-methoxyethyl)-1-(((S)-oxetan-2-yl)methyl)-1H-benzo[d]imidazol-6-carboxylic acid

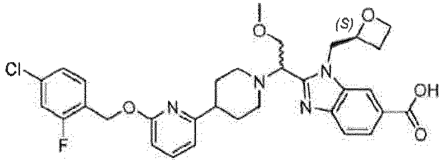

The specific synthetic route is as follows.

### Step A: Synthesis of methyl 2-(4-(6-((4-chloro-2-fluorobenzyl)oxy)pyridin-2-yl)piperidin-1-yl)-3-methoxypropionate

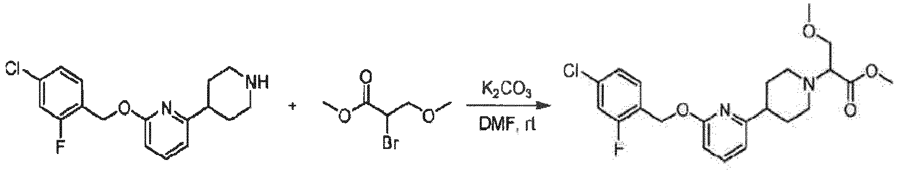

At room temperature, 4-(6-((4-chloro-2-fluorobenzyl)oxy)pyridin-2-yl)piperidine (155.0 mg, 483.2 µmol), methyl 2-bromo-3-methoxypropionate (200.0 mg, 966.4 µmol) and potassium carbonate (267.3 mg, 1.9 mmol) were dissolved in *N,N-*dimethylformamide (5 mL), and the reaction was carried out at room temperature for 16 hours.

After the reaction was complete, the reaction solution was diluted with water (80 mL), extracted with ethyl acetate (50 mL × 3 times), washed with saturated saline (50 mL × 3 times), dried with anhydrous sodium sulfate, and finally concentrated under reduced pressure. The resulting residue was purified by silica gel column chromatography (eluent: petroleum ether/ethyl acetate = 5/1) to obtain 177.2 mg of a pale yellow oily product, methyl 2-(4-(6-((4-chloro-2-fluorobenzyl)oxy)pyridin-2-yl)piperidin-1-yl)-3-methoxypropionate (a mixture of S- and R- configurations) (yield: 82.5%). LC-MS: RT = 2.63 min, [M+H]⁺ = 436.96.

### Step B: Synthesis of 2-(4-(6-((4-chloro-2-fluorobenzyl)oxy)pyridin-2-yl)piperidin-1-yl)-3-methoxypropionic acid

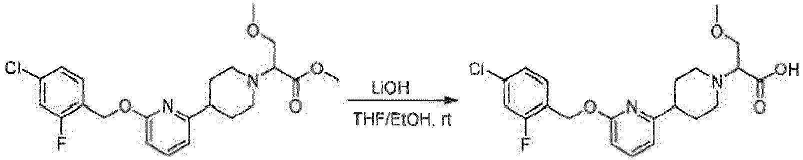

At room temperature, methyl 2-(4-(6-((4-chloro-2-fluorobenzyl)oxy)pyridin-2-yl)piperidin-1-yl)-3-methoxypropionate (177.0 mg, 396.7 µmol) was dissolved in a solution of tetrahydrofuran (10 mL), ethanol (3 mL) and water (3 mL), followed by an addition of lithium hydroxide (96.0 mg, 4.0 mmol), and the reaction was carried out at room temperature for 6 hours. After the reaction was complete, 1 mole per liter of aqueous citric acid solution was added dropwise until the pH of the solution was adjusted to 5. The reaction solution was diluted with water (50 mL), extracted with ethyl acetate (25 mL × 3 times), washed with saturated saline (25 mL × 3 times), dried with anhydrous sodium sulfate, and finally concentrated under reduced pressure. The resulting residue was purified by silica gel column chromatography (eluent: petroleum ether/ethyl acetate = 1/1 to 10/1) to obtain 139.0 mg of a yellow oily product, 2-(4-(6-((4-chloro-2-fluorobenzyl)oxy)pyridin-2-yl)piperidin-1-yl)-3-methoxypropionic acid (a mixture of S- and R- configurations) (yield: 69.2%). LC-MS: RT = 2.37 min, [M+H]⁺ = 423.03.

### Step C: Synthesis of methyl 4-(2-(4-(6-((4-chloro-2-fluorobenzyl)oxy)pyridin-2-yl)piperidin-1-yl)-3-methoxypropionamido)-3-(((S)-oxetan-2-yl)methyl)benzoate

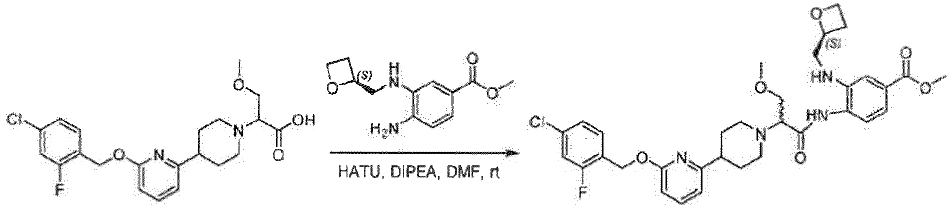

At room temperature, 2-(4-(6-((4-chloro-2-fluorobenzyl)oxy)pyridin-2-yl)piperidin-1-yl)-3-methoxyacetic acid (195.0 mg, 460.1 µmol), 2-(7-azabenzotriazol)-*N,N,N',N'-*tetramethyluronium hexafluorophosphate (174.8 mg, 460.1 µmol) and *N,N-*diisopropyl ethylamine (178.3 mg, 1.4 mmol) were dissolved in *N,N-*dimethylformamide (5 mL), and the reaction was carried out at room temperature for 0.5 hour. Then methyl 4-amino-3-((((S)-oxetan-2-yl)methyl)amino)benzoate (109.0 mg, 460.1 µmol) was added, and the reaction was continued for 24 hours.

After the reaction was complete, the reaction solution was diluted with ethyl acetate (50 mL), extracted, washed sequentially with water (30 mL × 3 times) and saturated saline (30 mL × 3 times), dried with anhydrous sodium sulfate, and finally concentrated under reduced pressure. The resulting residue was purified by silica gel column chromatography (eluent: petroleum ether/ethyl acetate = 2/1 to 1/1) to obtain 145.0 mg of a yellow oily product, methyl 4-(2-(4-(6-((4-chloro-2-fluorobenzyl)oxy)pyridin-2-yl)piperidin-1-yl)-3-methoxypropionamido)-3-(((S)-oxetan-2-yl)methyl)benzoate (a mixture of S- and R- configurations) (yield: 49.0%). LC-MS: RT = 3.12 min, [M+H]⁺ = 641.05.

### Step D: Synthesis of methyl 2-(1-(4-(6-((4-chloro-2-fluorobenzyl)oxy)pyridin-2-yl)piperidin-1-yl)-2-methoxyethyl)-1-(((S)-oxetan-2-yl)methyl)-1H-benzo[d]imidazol-6-carboxylate

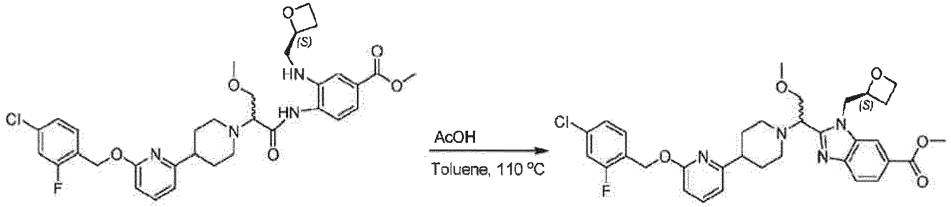

At room temperature, methyl 4-(2-(4-(6-((4-chloro-2-fluorobenzyl)oxy)pyridin-2-yl)piperidin-1-yl)-3-methoxypropionamido)-3-(((S)-oxetan-2-yl)methyl)benzoate (145.0 mg, 227.8 µmol) was added to toluene (5 mL), followed by an addition of acetic acid (100 µL). The reaction solution was heated to 110°C and the reaction was carried out for 4 hours.

After the reaction was complete, the reaction solution was cooled to room temperature, diluted with water (30 mL), and extracted with ethyl acetate (15 mL × 3 times). Then the organic phases were combined, washed sequentially with saturated aqueous sodium carbonate solution (15 mL × 3 times) and saturated saline (15 mL × 3 times), dried with anhydrous sodium sulfate, and finally concentrated under reduced pressure. The resulting residue was purified by silica gel column chromatography (eluent: petroleum ether/ethyl acetate = 1/1 to 10/1) to obtain 98.5 mg of a yellow oily product, methyl 2-(1-(4-(6-((4-chloro-2-fluorobenzyl)oxy)pyridin-2-yl)piperidin-1-yl)-2-methoxyethyl)-1-(((S)-oxetan-2-yl)methyl)-1*H-*benzo[*d*]imidazol-6-carboxylate (a mixture of S- and R- configurations) (yield: 68.0%). LC-MS: RT = 3.48 min, [M+H]⁺ = 623.48.

### Step E: Synthesis of 2-(1-(4-(6-((4-chloro-2-fluorobenzyl)oxy)pyridin-2-yl)piperidin-1-yl)-2-methoxyethyl)-1-(((S)-oxetan-2-yl)methyl)-1H-benzo[d]imidazol-6-carboxylic acid

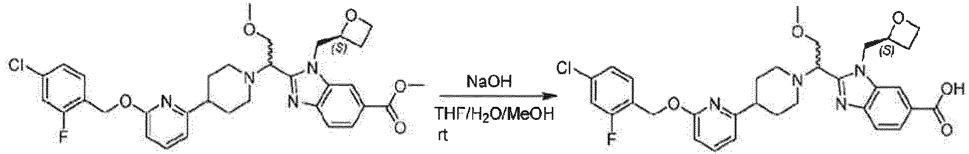

At room temperature, methyl 2-(1-(4-(6-((4-chloro-2-fluorobenzyl)oxy)pyridin-2-yl)piperidin-1-yl)-2-methoxyethyl)-1-(((S)-oxetan-2-yl)methyl)-1*H*-benzo[*d*]imidazol-6-carboxylate (98.5 mg, 158.1 µmol) was dissolved in a solution of tetrahydrofuran (6 mL), methanol (2 mL) and water (2 mL), followed by an addition of sodium hydroxide (101.8 mg, 2.5 mmol), and the reaction was carried out at room temperature for 24 hours. After the reaction was complete, 1 mole per liter of aqueous citric acid solution was added dropwise until the pH of the solution was adjusted to 5. The reaction solution was diluted with water (50 mL), extracted with ethyl acetate (25 mL × 3 times), and the organic phases were combined, washed with saturated saline (25 mL × 3 times), dried with anhydrous sodium sulfate, and finally concentrated under reduced pressure. The resulting residue was further purified by reversed-phase HPLC (mobile phase: water containing 0.1% ammonia/acetonitrile = 90/10 to 40/60) and lyophilized to obtain 18.7 mg of a white solid, 2-(1-(4-(6-((4-chloro-2-fluorobenzyl)oxy)pyridin-2-yl)piperidin-1-yl)-2-methoxyethyl)-1-(((S)-oxetan-2-yl)methyl)-1*H*benzo[*d*]imidazol-6-carboxylic acid (a mixture of S- and R- configurations) (yield: 20.5%). LC-MS: RT = 2.82 min, [M+H]⁺ = 609.27.

### Example 27 2-((S)-1-(4-(6-((4-cyano-2-fluorobenzyl)oxy)pyridin-2-yl)-4-hydroxypiperidin-1-yl)ethyl)-1-(((S)-oxetan-2-yl)methyl)-1H-benzo[d]imidazol-6-carboxylic acid

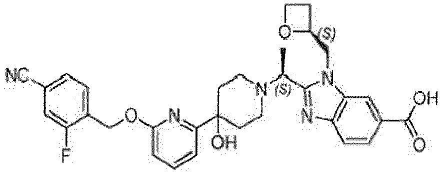

The specific synthetic route is as follows.

### Step A: Synthesis of tert-butyl 4-(6-bromopyridin-2-yl)-4-hydroxypiperidin-1-formate

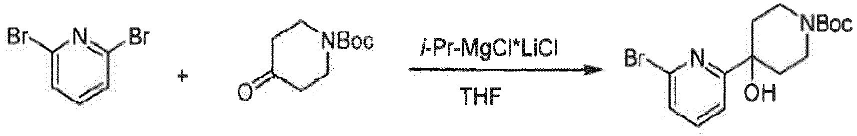

At room temperature, 2,6-dibromopyridine (5 g, 21.18 mmol) was dissolved in tetrahydrofuran (30 mL), followed by an addition of isopropyl magnesium chloride-lithium chloride (16.3 mL) under nitrogen protection. The mixture was stirred at room temperature for 2 hours, followed by an addition of tert-butyl 4-oxopiperidin-1-carboxylate (4.23 g, 21.18 mmol), and the reaction was carried out at room temperature overnight.

After the reaction was completed, the reaction solution was quenched with saturated ammonium chloride solution and extracted with ethyl acetate (30 mL × 3 times). Then the organic phases were combined, dried with anhydrous sodium sulfate, filtered, and concentrated. The resulting crude product was purified by silica gel column chromatography (eluent: ethyl acetate/n-hexane = 5/1) to obtain 2 g of a pale yellow oily liquid, tert-butyl 4-(6-bromopyridin-2-yl)-4-hydroxypiperidin-1-formate (yield: 27%). LC-MS: RT = 2.04 min, [M-55]⁺ = 301.11.

### Step B: Synthesis of tert-butyl 4-(6-((4-cyano-2-fluorobenzyl)oxy)pyridin-2-yl)-4-hydroxypiperidin-1- carboxylate

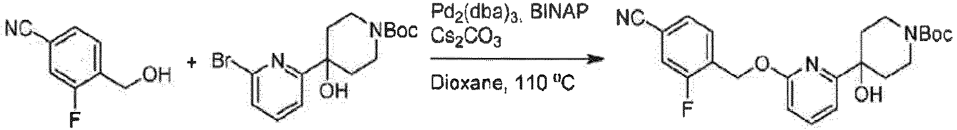

3-Fluoro-4-(hydroxymethyl)benzonitrile (425 mg, 2.81 mmol), tert-butyl 4-(6-bromopyridin-2-yl)-4-hydroxypiperidin-1-formate (1 g, 2.81 mmol), palladium catalyst (128 mg), BINAP (175 mg, 0.28 mmol) and cesium carbonate (1.83 g, 5.62 mmol) were dissolved in 1,4-dioxane (50 mL), and the reaction solution was heated to 110°C and the reaction solution was stirred for 8 hours.

After the reaction was completed, the reaction solution was filtrated with diatomaceous earth and eluted with dichloromethane. The filtrate was concentrated, and the resulting crude product was purified by column chromatography (eluent: ethyl acetate/n-hexane = 10/1) to obtain 820 mg of a pale yellow oily liquid, tert-butyl 4-(6-((4-cyano-2-fluorobenzyl)oxy)pyridin-2-yl)-4-hydroxypiperidin-1-carboxylate (yield: 68%). LC-MS: RT = 2.12 min, [M-55]⁺ = 372.33.

### Step C: Synthesis of 3-fluoro-4-(((6-(4-hydroxypiperidin-4-yl)pyridin-2-yl)oxy)methyl)benzonitrile

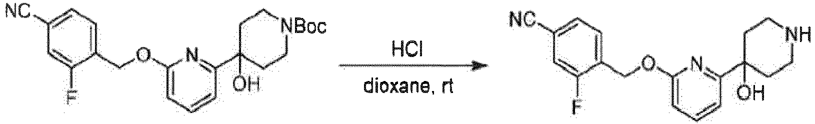

Tert-butyl 4-(6-((4-cyano-2-fluorobenzyl)oxy)pyridin-2-yl)-4-hydroxypiperidin-1-carboxylate (820 mg, 1.92 mmol) was dissolved in hydrochloric acid in 1,4-dioxane (15 mL), and the mixture was stirred at room temperature.

After the reaction was complete, the reaction solution was spin-dried under reduced pressure to obtain 410 mg of pale yellow solid 3-fluoro-4-(((6-(4-hydroxypiperidin-4-yl)pyridin-2-yl)oxy)methyl)benzonitrile (yield: 69%). LC-MS: RT = 1.63 min, [M+H]⁺ = 328.22.

### Step D: Synthesis of methyl 2-((S)-1-(4-(6-((4-cyano-2-fluorobenzyl)oxy)pyridin-2-yl)-4-hydroacypiperidin-1-yl)ethyl)-1-(((S)-oxetan-2-yl)methyl)-1H-benzo[d]imidazol-6-carboxylate

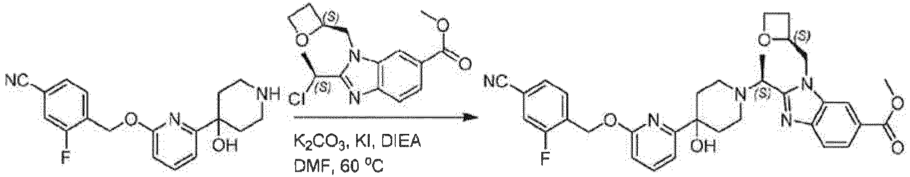

At room temperature, 3-fluoro-4-(((6-(4-hydroxypiperidin-4-yl)pyridin-2-yl)oxy)methyl)benzonitrile (150 mg, 0.46 mmol), potassium iodide (153 mg, 0.92 mmol) and potassium carbonate (127 mg, 0.92 mmol) were dissolved in *N,N-*dimethylformamide (10 mL), followed by an addition of methyl 2-((S)-1-chloroethyl)-1-(((S)-oxetan-2-yl)methyl)-1*H*-benzo[*d*]imidazol-6-carboxylate (141 mg, 0.46 mmol). The reaction solution was heated to 50°C for reaction.

After the reaction was completed, the reaction solution was quenched with saturated sodium chloride solution and extracted with ethyl acetate (30 mL × 3 times). Then the organic phases were combined, dried with anhydrous sodium sulfate, filtered, and concentrated. The resulting crude product was purified by silica gel column chromatography (eluent: ethyl acetate/n-hexane = 10/1) to obtain 100 mg of a white solid, methyl 2-((S)-1-(4-(6-((4-cyano-2-fluorobenzyl)oxy)pyridin-2-yl)-4-hydroxypiperidin-1-yl)ethyl)-1-(((S)-oxetan-2-yl)methyl)-1*H*-benzo[*d*]imidazol-6-carboxylate (yield: 36%). LC-MS: RT = 1.73 min, [M+H]⁺ = 600.34.

### Step E: Synthesis of 2-((S)-1-(4-(6-((4-cyano-2-fluorobenzyl)oxy)pyridin-2-yl)-4-hydroarypiperidin-l-yl)ethyl)-1-(((S)-oxetan-2-yl)methyl)-1H-benzo[d]imidazol-6-carboxylic acid

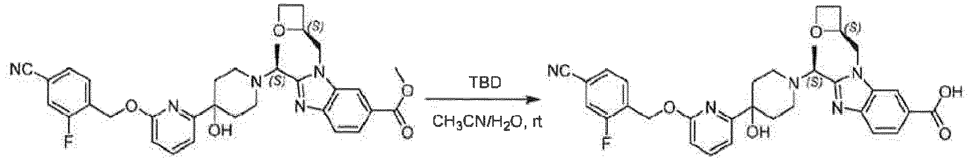

At room temperature, methyl 2-((S)-1-(4-(6-((4-cyano-2-fluorobenzyl)oxy)pyridin-2-yl)-4-hydroxypiperidin-1-yl)ethyl)-1-(((S)-oxetan-2-yl)methyl)-1*H*-benzo[*d*]imidazol-6-carboxylate (100 mg, 0.17 mmol) was dissolved in a mixed solvent (6 mL, acetonitrile/water = 5/1), followed by an addition of 1,5,7-triazabicyclo(4,4,0)dec-5-ene (47 mg, 0.34 mmol). The reaction was carried out at room temperature.

After the reaction was completed, it was quenched with citric acid and extracted with ethyl acetate (10 mL × 3 times). The organic phases were combined, dried with anhydrous sodium sulfate, filtered, and concentrated. Then the crude product obtained from the concentration was purified by silica gel column chromatography (eluent: dichloromethane/methanol = 10/1) to obtain 58.26 mg of a white solid, 2-((S)-1-(4-(6-((4-cyano-2-fluorobenzyl)oxy)pyridin-2-yl)-4-hydroxypiperidin-1-yl)ethyl)-1-(((S)-oxetan-2-yl)methyl)-1*H*-benzo[*d*]imidazol-6-carboxylic acid (yield: 58%).

LC-MS: RT = 1.72 min, [M+H]⁺ = 585.36. ¹H NMR (400 MHz, DMSO-d₆) δ 12.86 (s, 1H), 10.63 (s, 1H), 8.38 (s, 1H), 7.94-7.67 (m, 6H), 7 24 (d, *J=* 7.4 Hz, 1H), 6.78 (d, *J=* 8.0 Hz, 1H), 5.70-5.40 (m, 3H), 5 28-5.06 (m, 2H), 4.90-4.72 (m, 2H), 4.46 (d, *J* = 6.2 Hz, 1H), 4.18 (dt, *J* = 9.2, 5.9 Hz, 1H), 3.86 3.70 (m, 1H), 3.60 3.40 (m, 2H), 3.24 -3.10 (m, 2H), 2.69 2.54 (m, 2H), 2.30-2.22 (m, 1H), 1.82-1.64 (m, 4H).

### Example 28 Synthesis of 2-(1-(4-(6-((4-cyano-2-fluorobenzyl)oxy)pyridin-2-yl)piperidin-1-yl)-2-fluoroethyl)-1-(((S)-oxetan-2-yl)methyl)-1H-benzo[d]imidazol-6-carboxylic acid

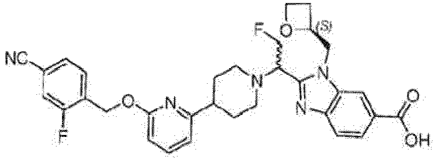

The specific synthetic route is as follows.

### Step A: Synthesis of methyl 2-bromo-3-hydroxypropionate

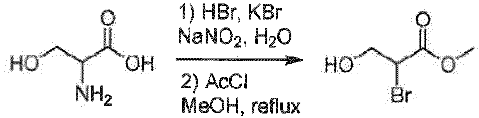

Under an ice-salt bath, hydrobromic acid (2.8 mL, 48% aqueous solution), potassium bromide (4.6 g, 38.8 mmol) and potassium carbonate (244 mg, 1.77 mmol) were added sequentially to an aqueous solution (9.3 mL) containing DL-serine (1.2 g, 11.4 mmol). The reaction solution was stirred at this temperature for 10 min, and the reaction was carried out at room temperature for 3 hours. After the reaction was completed, the solution was extracted with ethyl acetate (30 mL × 3 times), and the organic phases were combined, washed with saturated saline (30 mL × 3 times), dried with anhydrous sodium sulfate, filtered, and concentrated under reduced pressure.

The resulting residue was dissolved in methanol (8.8 mL), followed by an addition of acetyl chloride (924 µL, 13.0 mmol), and then it was heated under reflux and the reaction was carried out for 3 hours. After the reaction was completed, the solvent was removed by evaporation under reduced pressure, and the resulting residue was diluted with ethyl acetate (100 mL), washed sequentially with saturated sodium bicarbonate solution (50 mL) and saturated saline (30 mL × 3 times), dried with anhydrous sodium sulfate, filtered, and concentrated under reduced pressure. The resulting 1.5 g of crude product was used directly in the next reaction step.

### Step B: Synthesis of methyl 2-(4-(6-((4-cyano-2-fluorobenzyl)oxy))pyridin-2-yl)piperidin-1-yl)-3-hydroxypropionate

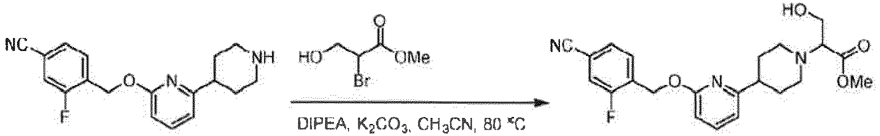

At room temperature, 3-fluoro-4-((((6-(piperidin-4-yl)pyridin-2-yl)oxy)methyl)benzonitrile (950 mg, 2.73 mmol), *N,N-*diisopropylethylamine (1.0 mL, 6.06 mmol) and potassium carbonate (1.0 g, 7.25 mmol) were added sequentially to acetonitrile (4.0 mL) containing methyl 2-bromo-3-hydroxypropionate (1.5 g, 8.2 mmol). The reaction solution was heated to 80°C, and the reaction was carried out for 2 hours. After the reaction was completed, the reaction solution was extracted with ethyl acetate (30 mL × 3 times), and the organic phases were combined, washed with saturated saline (50 mL), dried with anhydrous sodium sulfate, filtered, and concentrated under reduced pressure. The resulting residue was purified by silica gel column chromatography (eluent: methanol/dichloromethane = 5/95) to obtain 800 mg of a yellow oily product, methyl 2-(4-(6-((4-cyano-2-fluorobenzyl)oxy)pyridin-2-)piperidin-1-yl)-3-hydroxypropionate (a mixture of S- and R- configurations) (yield: 71.3%). LC-MS: RT = 1.70 min, [M+H]⁺ = 414.26.

### Step C: Synthesis of methyl 2-(4-(6-((4-cyano-2-fluorobenzyl)oxy)pyridin-2-yl)piperidin-1-yl)-3-fluoropropionate

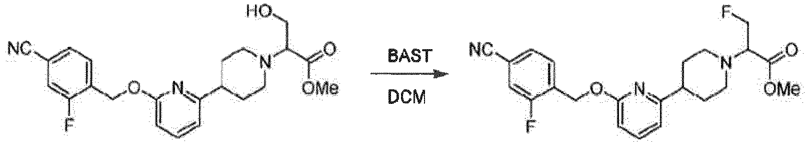

Under an ice-water bath, bis(2-methoxyethyl)aminotrifluorosulfide (179 µL, 0.969 mmol) was added to a solution of methyl 2-(4-(6-((4-cyano-2-fluorobenzyl)oxy)pyridin-2-yl)piperidin-1-yl)-3-hydroxypropionate (200 mg, 0.484 mmol) in dichloromethane (5.0 mL), and the temperature was naturally warmed to room temperature and the reaction was carried out for 2 hours.

After the reaction was completed, the resultant was cooled under an ice-water bath, quenched with water, and extracted with dichloromethane (80 mL). The organic phase was washed with saturated sodium bicarbonate solution (50 mL) and saturated saline (50 mL) sequentially, dried with anhydrous sodium sulfate, filtered, and concentrated under reduced pressure. Then the resulting residue was purified by silica gel column chromatography (eluent: methanol/dichloromethane = 3/97) to obtain 160 mg of a pale yellow oily product, methyl 2-(4-(6-((4-cyano-2-fluorobenzyl)oxy)pyridin-2-yl)piperidin-1-yl)-3-fluoropropionate (a mixture of S- and R- configurations) (yield: 79.7%). LC-MS: RT = 1.73 min, [M+H]⁺ = 416.26.

### Step D: Synthesis of methyl 4-(2-(4-(6-((4-cyano-2-fluorobenzyl)oxy)pyridin-2-yl)piperidin-1-yl)-3-fluoropropionamido)-3-((((S)-oxetan-2-yl)methyl)amino)benzoate

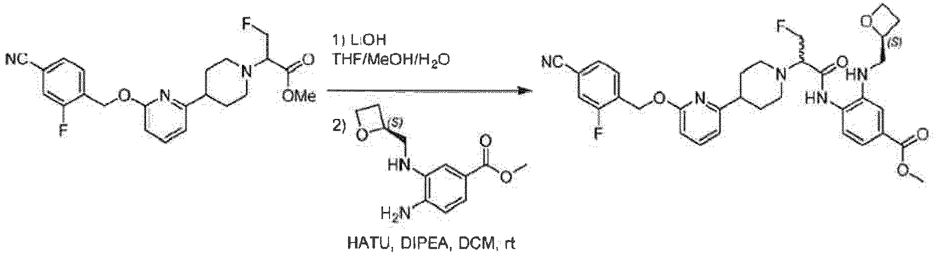

At room temperature, lithium hydroxide monohydrate (71 mg, 1.54 mmol) was added to a tetrahydrofuran/methanol/water solution (3.0 mL/0.5 mL/0.5 mL) containing methyl 2-(4-(6-((4-cyano-2-fluorobenzyl)oxy)pyridin-2-yl)piperidin-1-yl)-3-fluoropropionate (160 mg, 0.386 mmol), and the reaction was carried out at room temperature for 0.5 hour. After the reaction was completed, the reaction solution was quenched with saturated ammonium chloride solution and extracted with ethyl acetate (30 mL × 3 times). Then the organic phases were combined, washed with saturated saline (30 mL × 3 times), dried with anhydrous sodium sulfate, filtered, and concentrated under reduced pressure. The resulting crude product was used directly in the next reaction step. LC-MS: RT = 1.72 min, [M+H]⁺ = 402.26.

The above crude product was dissolved in dichloromethane (4.0 mL), then 2-(7-azabenzotriazole)-*N,N,N',N'*-tetramethyluronium hexafluorophosphate (293 mg, 0.77 mmol), *N,N-*diisopropylethylamine (127 µL, 0.77 mmol) and methyl (S)-4-amino-3-(((oxetan-2-yl)methyl)amino)benzoate (90 mg, 0.386 mmol) were added sequentially. Then the reaction was carried out at room temperature for 40 min. After the reaction was completed, the reaction solution was diluted with dichloromethane (100 mL), and the organic phase was washed sequentially with water (50 mL) and saturated saline (50 mL), dried with anhydrous sodium sulfate, filtered, and concentrated under reduced pressure. The resulting residue was purified by silica gel column chromatography (eluent: methanol/dichloromethane = 5/95). 200 mg of pale yellow oily product methyl 4-(2-(4-(6-((4-cyano-2-fluorobenzyl)oxy)pyridin-2-yl)piperidin-1-yl)-3-fluoropropionamido)-3-((((S)-oxetan-2-yl)methyl)amino)benzoate (a mixture of S- and R-configurations) was obtained (yield: 83.6%). LC-MS: RT = 1.79 min, [M+H]⁺ = 620.36.

### Step E: Synthesis of methyl 2-(1-(4-(6-((4-cyano-2-fluorobenzyl)oxy)pyridin-2-yl)piperidin-1-yl)-2-fluoroethyl)-1-(((S)-oxetan-2-yl)methyl)-1H-benzo[d]imidazol-6-carboxylate

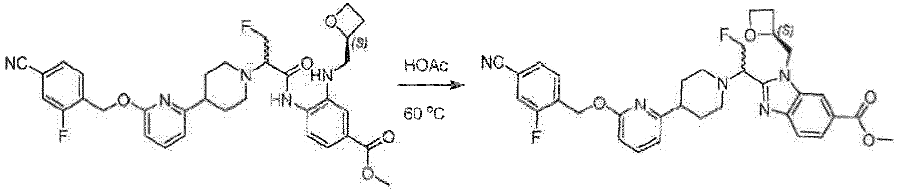

Methyl 4-(2-(4-(6-((4-cyano-2-fluorobenzyl)oxy)pyridin-2-yl)piperidin-1-yl)-3-fluoropropionamido)-3-((((S)-oxetan-2-yl)methyl)amino)benzoate (200 mg, 0.323 mmol) was dissolved in acetic acid (2.0 mL), and the reaction was carried out at 60°C for 2 hours. After the reaction was completed, ethyl acetate (100 mL) was added, and saturated sodium bicarbonate solution was added with stirring until no bubbles were produced. Then the organic phases was separated, washed sequentially with water (50 mL) and saturated saline (50 mL), dried with anhydrous sodium sulfate, filtered, and concentrated under reduced pressure. The resulting residue was purified by silica gel column chromatography (eluent: methanol/dichloromethane = 5/95). 130 mg of a pale yellow oil, methyl 2-(1-(4-(6-((4-cyano-2-fluorobenzyl)oxy)pyridin-2-yl)piperidin-l-yl)-2-fluoroethyl)-1-(((S)-oxetan-2-yl)methyl)-1*H-*benzo[*d*]imidazol-6-carboxylate (a mixture of S- and R- configurations), was obtained (yield: 67.0%). LC-MS: RT = 1.81 min, [M+H]⁺ = 602.30.

### Step F: Synthesis of 2-(1-(4-(6-((4-cyano-2-fluorobenzyl)oxy)pyridin-2-yl)piperidin-1-yl)-2-fluoroethyl)-1-(((S)-oxetan-2-yl)methyl)-1H-benzo[d]imidazol-6-carboxylic acid

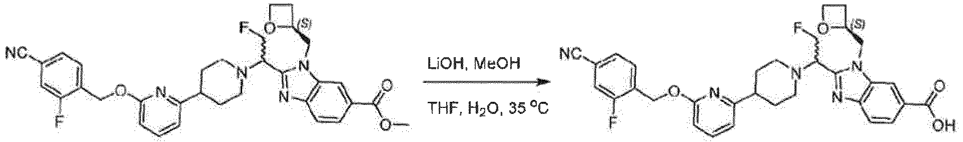

At room temperature, lithium hydroxide monohydrate (36 mg, 0.86 mmol) was added to a solution of methyl 2-(1 -(4-(6-((4-cyano-2-fluorobenzyl)oxy)pyridin-2-yl)piperidin-1-yl)-2-fluoroethyl)-1-(((S)-oxetan-2-yl)methyl)-1*H*-benzo[*d*]imidazol-6-carboxylate (130 mg, 0.216 mmol) in tetrahydrofuran/methanol/water (2.5 mL/2.5 mL/1.0 mL), and the reaction solution was heated to 35°C and the reaction was carried out for 1 hour. After the reaction was completed, the reaction solution was quenched with saturated ammonium chloride solution and extracted with ethyl acetate (30 mL × 3 times). Then the organic phases were combined, washed with saturated saline (30 mL × 3 times), dried with anhydrous sodium sulfate, filtered, and concentrated under reduced pressure. The resulting residue was purified by silica gel column chromatography (eluent: methanol/dichloromethane = 6/94). 19 mg of a white solid, 2-(1-(4-(6-((4-cyano-2-fluorobenzyl)oxy)pyridin-2-yl)piperidin-1-yl)-2-fluoroethyl)-1-(((S)-oxetan-2-yl)methyl)-1*H-*benzo[*d*]imidazol-6-carboxylic acid (a mixture of S- and R-configurations), was obtained (yield: 15.0%). LC-MS: RT = 1.77 min, [M+H]⁺ = 588.23.

### Example 29 2-((S)-1-((R)-4-(6-((4-cyano-2-fluorobenzyl)oxy)pyridin-2-yl)-2-(methoxymethyl)piperazin-1-yl)ethyl)-1-(((S)-oxetan-2-yl)methyl)-1H-benzo[d]imidazol-6-carboxylic acid

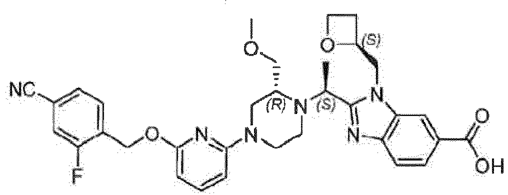

The specific synthetic route is as follows.

### Step A: Synthesis of 4-(((6-bromopyridin-2-yl)oxy)methyl)-3-fluorobenzonitrile

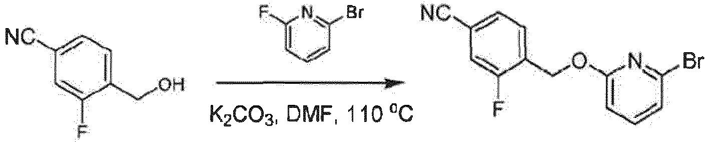

3-Fluoro-4-(hydroxymethyl)benzonitrile (5.00 g, 28.41 mmol) was dissolved in *N,N-*dimethylformamide (30 mL), followed by an addition of potassium carbonate (7.84 g, 56.82 mmol) and 2-bromo-6-fluoropyridine (4.30 mg, 28.41 mmol). Then the reaction solution was heated to 110°C and the reaction was carried out.

After the reaction was completed, the reaction was quenched with saturated sodium chloride solution, and the reaction solution was extracted with ethyl acetate (30 mL × 3 times). Then the organic phases were combined, dried with anhydrous sodium sulfate, filtered, and concentrated. The resulting crude product was purified by silica gel column chromatography (eluent: ethyl acetate/n-hexane = 1/10) to obtain 4.50 g of a white solid, 4-(((6-bromopyridin-2-yl)oxy)methyl)-3-fluorobenzonitrile (yield: 52%). LC-MS: RT = 2.14 min, [M+H]⁺ = 307.03.

### Step B: Synthesis of tert-butyl (R)-4-(6-((4-cyano-2-fluorobenzyl)oxy)pyridin-2-yl)-2-(hydroxymethyl)piperazin-1-formate

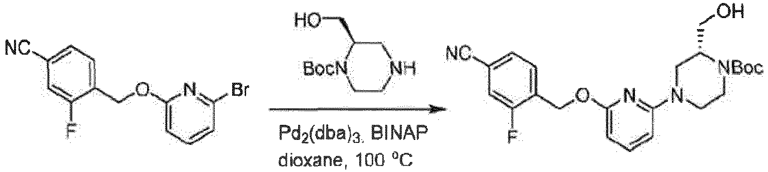

4-(((6-Bromopyridin-2-yl)oxy)methyl)-3-fluorobenzonitrile (1.00 g, 3.26 mmol), tert-butyl (R)-2-(hydroxymethyl)piperazin-1-carboxylate (0.70 g, 3.26 mmol), palladium catalyst (302 mg), BINAP (411 mg, 0.66 mmol) and cesium carbonate (2 g, 6.52 mmol) were dissolved in 1,4-dioxane (20 mL), and then the reaction solution was heated to 100°C and the reaction solution was stirred for 8 hours.

After the reaction was completed, the reaction solution was filtrated by suction through diatomite and eluted with dichloromethane. The filtrate was concentrated, and the resulting crude product was purified by column chromatography (eluent: ethyl acetate/n-hexane = 1/1) to obtain 1.00 g of a pale yellow oily liquid, tert-butyl (R)-4-(6-((4-cyano-2-fluorobenzyl)oxy)pyridin-2-yl)-2-(hydroxymethyl)piperazin-1-formate (yield: 69%). LC-MS: RT = 2.09 min, [M-55]⁺ = 443.22.

### Step C: Synthesis of tert-butyl (R)-4-(6-((4-cyano-2-fluorobenzyl)oxy)pyridin-2-yl)-2-(methoxymethyl)piperazin-1-formate

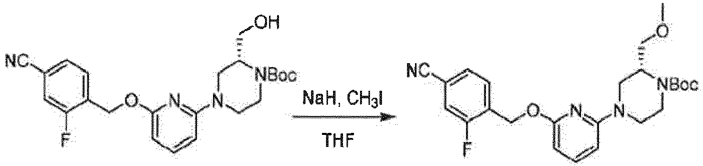

Tert-butyl (R)-4-(6-((4-cyano-2-fluorobenzyl)oxy)pyridin-2-yl)-2-(hydroxymethyl)piperazin-1-formate (100 mg, 0.23 mmol) was dissolved in tetrahydrofuran (5 mL). The solution was cooled to 0°C, followed by an addition of sodium hydride (11 mg, 0.46 mmol). The mixture was stirred at room temperature for 0.5 hour, and then iodomethane (65 mg, 0.46 mmol) was added, and the reaction was carried out overnight at room temperature.

After the reaction was completed, the reaction was quenched with saturated sodium chloride solution, and the reaction solution was extracted with ethyl acetate (30 mL) and washed twice with water. Then the organic phases were combined, dried with anhydrous sodium sulfate, filtered, and concentrated. The resulting crude product was purified by silica gel column chromatography (eluent: ethyl acetate/n-hexane = 1/2) to obtain 280 mg of a pale yellow solid, tert-butyl (R)-4-(6-((4-cyano-2-fluorobenzyl)oxy)pyridin-2-yl)-2-(methoxymethyl)piperazin-1-formate (yield: 48%). LC-MS: RT = 2.25 min, [M-55]⁺ = 457.27.

### Step D: Synthesis of (R)-3-fluoro-4-(((6-(3-(methoxymethyl)piperazin-1-yl)pyridin-2-yl)oxy)methyl)benzonitrile

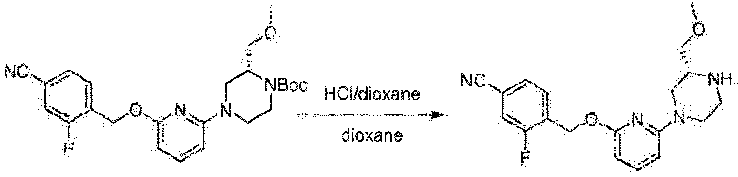

Tert-butyl (R)-4-(6-((4-cyano-2-fluorobenzyl)oxy)pyridin-2-yl)-2-(methoxymethyl)piperazin-1-formate (50 mg, 0.11 mmol) was dissolved in hydrochloric acid in 1,4-dioxane (5 mL) and stirred at room temperature.

After the reaction was complete, the solution was spin-dried under reduced pressure to obtain 61 mg of a crude product, (R)-3-fluoro-4-(((6-(3-(methoxymethyl)piperazin-1-yl)pyridin-2-yl)oxy)methyl)benzonitrile. LC-MS: RT = 1.70 min, [M+H]⁺ = 357.18.

### Step E: Synthesis of methyl 2-((S)-1-((R)-4-(6-((4-cyano-2-fluorobenzyl)oxy)pyridin-2-yl)-2-(methoxymethyl)piperazin-1-yl)ethyl)-1-(((S)-oxetan-2-yl)methyl)-1H-benzo[d]imidazol-6-carboxylate

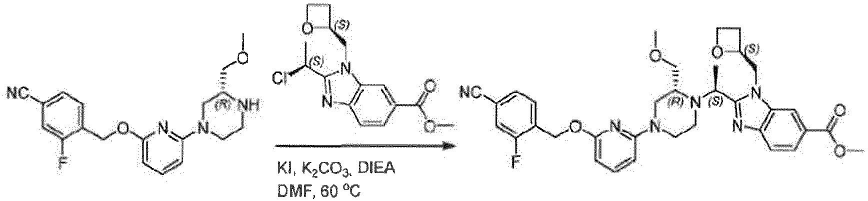

At room temperature, (R)-3-fluoro-4-(((6-(3-(methoxymethyl)piperazin-1-yl)pyridin-2-yl)oxy)methyl)benzonitrile (60 mg, 0.17 mmol), DIEA (1 mL), potassium iodide (56 mg, 0.34 mmol) and potassium carbonate (47 g, 0.34 mmol) were dissolved in *N,N-*dimethylformamide (5 mL), followed by an addition of methyl 2-((S)-1-chloroethyl)-1-(((S)-oxetan-2-yl)methyl)-1*H-*benzo[*d*]imidazol-6-carboxylate (52 mg, 0.17 mmol), and the reaction was carried out with the temperature raised to 60°C.

After the reaction was completed, the reaction was quenched with saturated sodium chloride solution, and the reaction solution was extracted with ethyl acetate (20 mL × 2 times). Then the organic phases were combined, dried with anhydrous sodium sulfate, filtered, and concentrated. The resulting crude product was purified by silica gel column chromatography (eluent: ethyl acetate/n-hexane = 10/1) to obtain 20 mg of a pale yellow solid, methyl 2-((S)-1-((R)-4-(6-((4-cyano-2-fluorobenzyl)oxy)pyridin-2-yl)-2-(methoxymethyl)piperazin-1-yl)ethyl)-1-(((S)-oxetan-2-yl)methyl)-1*H*-benzo[*d*]imidazol-6-carboxylate (yield: 19%). LC-MS: RT = 1.97 min, [M+H]⁺ = 629.33.

### Step F: Synthesis of 2-((S)-1-((R)-4-(6-((4-cyano-2-fluorobenzyl)oxy)pyridin-2-yl)-2-(methoxymethyl)piperazin-1-yl)ethyl)-1-(((S)-oxetan-2-yl)methyl)-1H-benzo[d]imidazol-6-carboxylic acid

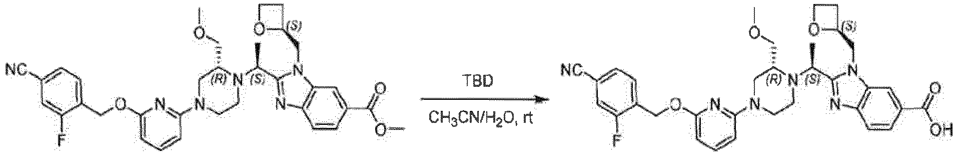

At room temperature, methyl 2-((S)-1-((R)-4-(6-((4-cyano-2-fluorobenzyl)oxy)pyridin-2-yl)-2-(methoxymethyl)piperazin-1-yl)ethy1)-1-(((S)-oxetan-2-yl)methyl)-1*H*-benzo[*d*]imidazol-6-carboxylate (20 mg, 0.03 mmol) was dissolved in a mixed solvent ( 3.5 mL, acetonitrile/water = 3/0.5), followed by an addition of 1,5,7-triazabicyclo(4,4,0)dec-5-ene (9 mg, 0.06 mmol), and the reaction was carried out at room temperature.

After the reaction was completed, the reaction was quenched with citric acid, and the reaction solution was extracted with ethyl acetate (10 mL × 2 times). Then the organic phases were combined, dried with anhydrous sodium sulfate, filtered and concentrated. The resulting crude product was purified by silica gel column chromatography (eluent: dichloromethane/methanol = 10/1) to obtain 12 mg of a white solid, 2-((S)-1-((R)-4-(6-((4-cyano-2-fluorobenzyl)oxy)pyridin-2-yl)-2-(methoxymethyl)piperazin-1-yl)ethyl)-1-(((S)-oxetan-2-yl)methyl)-1*H*-benzo[*d*]imidazol-6-carboxylic acid (yield: 61%).

LC-MS: RT = 1.86 min, [M+H]⁺ = 615.31. ¹H NMR (400 MHz, DMSO-d₆) δ 12.71 (s, 1H), 8.27 (s, 1H), 7.86 (dd, *J=* 10.0, 1.3 Hz, 1H), 7.79 (dd, *J* = 84, 1.5 Hz, 1H), 7.69-7.64 (m, 2H), 7.61 (t, *J* = 7.6 Hz, 1H), 7.44 (t, *J* = 8.0 Hz, 1H), 6.26 (d, *J* = 8.2 Hz, 1H), 6.10 (d, *J* = 7.8 Hz, 1H), 5.37 (s, 2H), 5.13 (d, *J* = 13.0 Hz, 2H), 4.87 (d, *J* = 6.6 Hz, 1H), 4.61 (dd, J = 15.8, 6.5 Hz, 1H), 4.44 (dd, *J* = 13.4, 8.0 Hz, 1H), 4.20 (dt, *J* = 9.0, 5.9 Hz, 1H), 4.08-3.96 (m, 1H), 3.82 (d, *J* = 11.8 Hz, 1H), 3.65 (dd, *J* = 10.5, 3.4 Hz, 1H), 3.53-3.41 (m, 1H), 3.33 (s, 3H), 2.81 (t, *J* = 11.3 Hz, 1H), 2.71-2.60 (m, 2H), 2.35 (d, *J=* 9.1 Hz, 2H), 2.27-2.13 (m, 2H), 1.39 (d, *J=* 6.6 Hz, 3H).

### Example 30 2-((S)-1-(4-(6-((4-cyano-2-fluorobenzyl)oxy)-3-fluoropyridin-2-yl)piperazin-1-yl)ethyl)-1-(((S)-oxetan-2-yl)methyl)-1H-benzo[d]imidazol-6-carboxylic acid

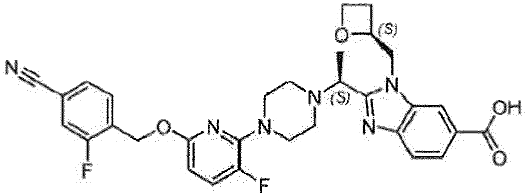

The specific synthetic route is as follows.

### Step A: Synthesis of tert-butyl 4-(6-bromo-3-fluoropyridin-2-yl)piperazin-1-formate

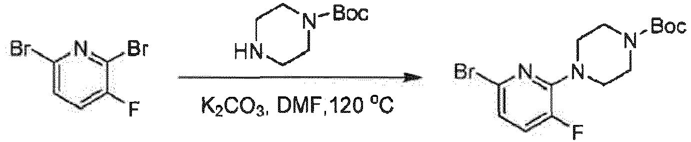

2,6-Dibromo-3-fluoropyridine (1.0 g, 3.93 mmol), potassium carbonate (1.08 g, 7.86 mmol) and tert-butyl piperazin-1-carboxylate (731 mg, 3.93 mmol) were dissolved in 10 mL of anhydrous *N,N-*dimethylformamide, and the reaction was carried out at 120°C for 2 hours. After the reaction was completed, the reaction solution was diluted with water and extracted with ethyl acetate (50 mL × 2 times). Then the organic phases were combined, washed with saturated saline (30 mL × 2 times), dried with anhydrous sodium sulfate, filtered, and concentrated under reduced pressure. The resulting crude product was separated by column chromatography to obtain 1.18 g of a white solid, tert-butyl 4-(6-bromo-3-fluoropyridin-2-yl)piperazin-1-formate. LC-MS: RT = 1.98 min, [M+H]⁺ = 360.10.

### Step B: Synthesis of tert-butyl 4-(6-((4-cyano-2-fluorobenzyl)oxy)-3-fluoropyridin-2-yl)piperazin-1-formate

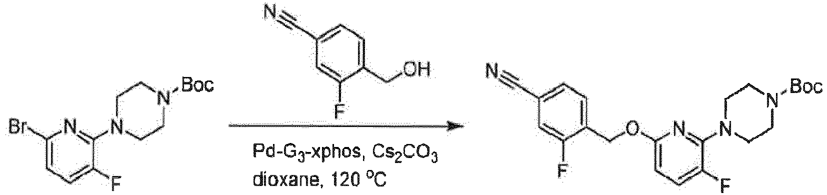

Tert-butyl 4-(6-bromo-3-fluoropyridin-2-yl)piperazin-1-formate (400 mg, 1.11 mmol) and 3-fluoro-4-(hydroxymethyl)benzonitrile (168 mg, 1.11 mmol) were dissolved in 5 mL of anhydrous 1,4-dioxane, followed by an addition of cesium carbonate (723 mg, 2.22 mmol) and methanesulfonato(2-dicyclohexylphosphino-2',4',6'-triisopropyl-1,1'-biphenyl)(2'-amino-1,1'-biphenyl-2-yl)palladium (94 mg, 0.111 mmol), and the system was purged with nitrogen for three times. The reaction was carried out at 120°C for 5 hours. After the reaction was completed, the reaction solution was diluted with water and extracted with ethyl acetate (50 mL × 2 times). Then the organic phases were combined, washed with saturated saline (30 mL × 2 times), dried with anhydrous sodium sulfate, filtered, and concentrated under reduced pressure. The resulting crude product was separated by column chromatography to obtain 367 mg of a pale yellow solid, tert-butyl 4-(6-((4-cyano-2-fluorobenzyl)oxy)-3-fluoropyridin-2-yl)piperazin-1-formate. LC-MS: RT = 1.95 min, [M-H]⁻ = 431.22.

### Step C: Synthesis of 3-fluoro-4-(((5-fluoro-6-(piperazin-1-yl)pyridin-2-yl)oxy)methyl)benzonitrile

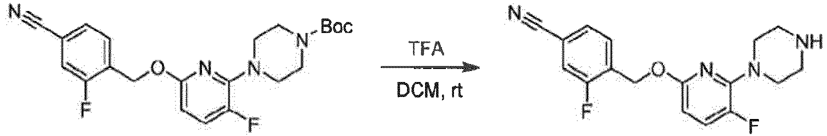

Tert-butyl 4-(6-((4-cyano-2-fluorobenzyl)oxy)-3-fluoropyridin-2-yl)piperazin-1-formate (367 mg, 0.85 mmol) was dissolved in 4 mL of anhydrous dichloromethane, followed by an addition of 2 mL of trifluoroacetic acid, and the reaction was carried out at room temperature for 40 min. After the reaction was completed, the reaction solution was poured into 60 mL of sodium bicarbonate solution, and was extracted with ethyl acetate (30 mL × 3 times). Then the organic phases were combined, washed with saturated saline (20 mL × 2 times), dried with anhydrous sodium sulfate, filtered, and concentrated under reduced pressure to obtain 260 mg of 3-fluoro-4-(((5-fluoro-6-(piperazin-1-yl)pyridin-2-yl)oxy)methyl)benzonitrile. LC-MS: RT = 1.81 min, [M+H]⁺ = 331.27.

### Step D: Synthesis of methyl 2-((S)-1-(4-(6-((4-cyano-2-fluorobenzyl)oxy)-3-fluoropyridin-2-yl)piperazin-1-yl)ethyl)-1-(((S)-oxetan-2-yl)methyl)-1H-benzo[d]imidazol-6-carboxylate

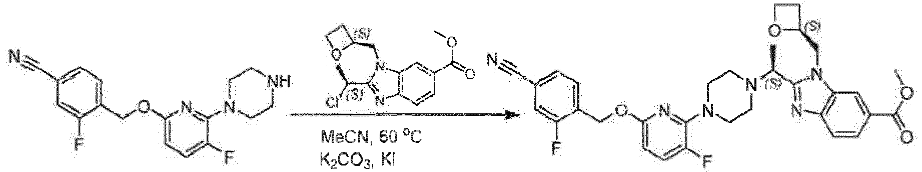

3-Fluoro-4-(((5-fluoro-6-(piperazin-1-yl)pyridin-2-yl)oxy)methyl)benzonitrile (260 mg, 0.785 mmol), potassium carbonate (216 mg, 1.57 mmol) and potassium iodide (195 mg, 1.17 mmol) were dissolved in 5 mL of anhydrous acetonitrile, followed by an addition of methyl 2-((S)-1-chloroethyl)-1-(((S)-oxetan-2-yl)methyl)-1*H*-benzo[*d*]imidazol-6-carboxylate (241 mg, 0.785 mmol), and the reaction was carried out at 60°C for 2 hours. After the reaction was completed, the reaction solution was diluted with water and extracted with ethyl acetate (40 mL × 2 times). Then the organic phases were combined, washed with saturated saline (30 mL × 2 times), dried with anhydrous sodium sulfate, filtered, and concentrated under reduced pressure. The resulting crude product was separated by column chromatography to obtain 310 mg of a pale yellow solid, methyl 2-((S)-1-(4-(6-((4-cyano-2-fluorobenzyl)oxy)-3-fluoropyridin-2-yl)piperazin-1-yl)ethyl)-1-(((S)-oxetan-2-yl)methyl)-1*H*-benzo[*d*]imidazol-6-carboxylate. LC-MS: RT = 1.91 min, [M+H]⁺ = 603.27.

### Step E: Synthesis of 2-((S)-1-(4-(6-((4-cyano-2-fluorobenzyl)oxy)-3-fluoropyridin-2-yl)piperazin-1-yl)ethyl)-1-(((S)-oxetan-2-yl)methyl)-1H-benzo[d]imidazol-6-carboxylic acid

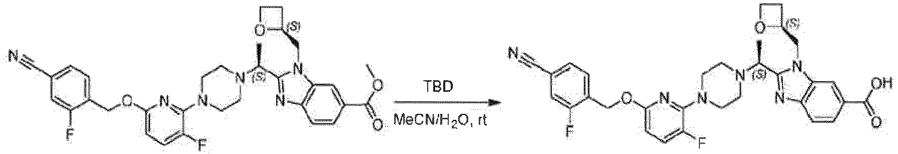

Methyl 2-((S)-1-(4-(6-((4-cyano-2-fluorobenzyl)oxy)-3-fluoropyridin-2-yl)piperazin-1-yl)ethyl)-1-(((S)-oxetan-2-yl)methyl)-1*H*-benzo[*d*]imidazol-6-carboxylate (100 mg, 0.166 mmol) was dissolved in 4 mL of acetonitrile, followed by an addition of 1,5,7-triazabicyclo[4.4.0]dec-5-ene (48.4 mg, 0.348 mmol) and 1 mL of water, and the reaction was carried out at room temperature for 7 hours. After the reaction was completed, the reaction solution was neutralized with 20 mL of saturated ammonium chloride solution, and was extracted with dichloromethane (30 mL × 2 times). Then the organic phases were combined, washed with saturated saline (20 mL × 2 times), dried with anhydrous sodium sulfate, filtered, and concentrated under reduced pressure. The resulting crude product was separated by column chromatography to obtain 38 mg of a white solid, 2-((S)-1-(4-(6-((4-cyano-2-fluorobenzyl)oxy)-3-fluoropyridin-2-yl)piperazin-1-yl)ethyl)-1-(((S)-oxetan-2-yl)methyl)-1*H-*benzo[*d*]imidazol-6-carboxylic acid. LC-MS: RT = 1.79 min,

[M+H]⁺ =589.27. ¹H NMR (400 MHz, DMSO) δ 12.78 (s, 1H), 8.29 (d, *J* = 1.0 Hz, 1H), 7 88 (dd, *J* = 10.0, 1.2 Hz, 1H), 7.81 (dd, *J* = 8.4, 1.5 Hz, 1H), 7.72-7.64 (m, 3H), 7.49-7.44 (m, 1H), 6.26 (dd, *J* = 8.4, 1.6 Hz, 1H), 5.37 (s, 2H), 5.19-5.11 (m, 1H), 4.83-4.78 (m, 1H), 4.69 (dd, *J* = 15.6, 5.6 Hz, 1H), 4.49-4.43 (m, 2H), 4.21-4.16 (m, 1H), 2.66-2.62 (m, 2H), 2.60-2.55 (m, 6H), 2 37-2.30 (m, 2H), 1.44 (d, *J* = 6.7 Hz, 3H).

### Example 31 2-((15)-1-((3,4-trans)-4-(6-((4-chloro-2-fluorobenzyl)oxy)pyridin-2-yl)-3-hydroxypiperidin-1-yl)ethyl)-1-(((S)-oxetan-2-yl)methyl)-1H-benzo[d]imidazol-6-carbouylic acid

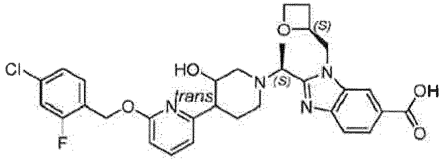

The specific synthetic route is as follows.

### Step A: Synthesis of (3,4-trans)-4-(6-((4-chloro-2-fluorobenzyl)oxy)pyridin-2-yl)piperidin-3-ol

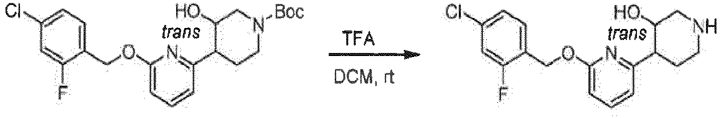

Tert-butyl (3,4-*trans*)-4-(6-((4-chloro-2-fluorobenzyl)oxy)pyridin-2-yl)-3-hydroxypiperidin-1-formate (350 mg, 0.802 mmol) was dissolved in 5 mL of anhydrous dichloromethane, followed by an addition of 2 mL of trifluoroacetic acid, and the reaction was carried out at room temperature for 40 min. After the reaction was completed, the reaction solution was poured into 60 mL of sodium bicarbonate solution, and the reaction solution was extracted with ethyl acetate (50 mL × 2 times). Then the organic phases were combined, washed with saturated saline (30 mL × 2 times), dried with anhydrous sodium sulfate, filtered, and concentrated under reduced pressure to obtain 270 mg of (3,4-*trans*)-4-(6-((4-chloro-2-fluorobenzyl)oxy)pyridin-2-yl)piperidin-3-ol. LC-MS: RT = 1.81 min, [M+H]⁺ = 337.17.

### Step B: Synthesis of methyl 2-((1S)-1-((3,4-trans)-4-(6-((4-chloro-2-fluorobenzyl)oxy)pyridin-2-yl)-3-hydroxypiperidin-1-yl)ethyl)-1-(((S)-oxetan-2-yl)methyl)-1H-benzo[d]imidazol-6-carboxylate

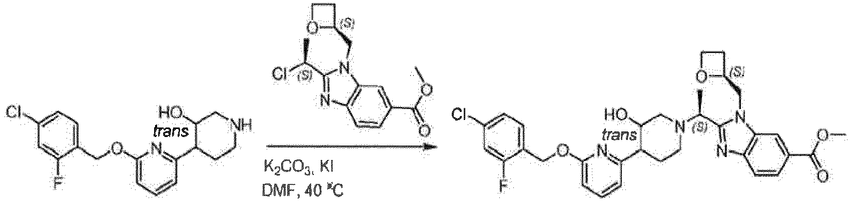

(3,4-*Trans*)-4-(6-((4-chloro-2-fluorobenzyl)oxy)pyridin-2-yl)piperidin-3-ol (270 mg, 0.801 mmol), potassium carbonate (222 mg, 1.61 mmol) and potassium iodide (199 mg, 1.21 mmol) were dissolved in 5 mL of anhydrous acetonitrile, followed by an addition of methyl 2-((S)-1-chloroethyl)-1-(((S)-oxetan-2-yl)methyl)-1*H*-benzo[*d*]imidazol-6-carboxylate (246 mg, 0.801 mmol), and the reaction was carried out at 60°C for 2 hours. After the reaction was completed, the reaction solution was diluted with water and extracted with ethyl acetate (40 mL × 2 times). Then the organic phases were combined, washed with saturated saline (30 mL × 2 times), dried with anhydrous sodium sulfate, filtered, and concentrated under reduced pressure. The resulting crude product was separated by column chromatography to obtain 320 mg of a pale yellow solid, methyl 2-((1S)-1-((3,4-*trans*)-4-(6-((4-chloro-2-fluorobenzyl)oxy)pyridin-2-yl)-3-hydroxypiperidin-1-yl)ethyl)-1-(((S)-oxetan-2-yl)methyl)-1*H-*benzo[*d*]imidazol-6-carboxylate. LC-MS: RT = 1.86 min, [M+H]⁺ = 609.27.

### Step C: Synthesis of 2-((1S)-1-((3,4-trans)-4-(6-((4-chloro-2-fluorobenzyl)oxy)pyridin-2-yl)-3-hydroxypiperidin-1-yl)ethyl)-1-(((S)-oxetan-2-yl)methyl)-1H-benzo[d]imidazol-6-carboxylic acid

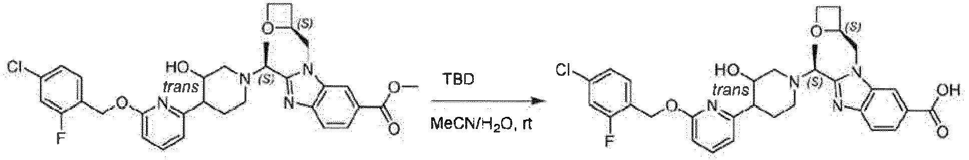

Methyl 2-((1S)-1-((3,4-*trans*)-4-(6-((4-chloro-2-fluorobenzyl)oxy)pyridin-2-yl)-3-hydroxypiperidin-1-yl)ethyl)-1-(((S)-oxetan-2-yl)methyl)-1*H-*benzo[*d*]imidazol-6-carboxylate (150 mg, 0.246 mmol) was dissolved in 4 mL of acetonitrile, followed by an addition of 1,5,7-triazabicyclo[4.4.0]dec-5-ene (71.8 mg, 0.517 mmol) and 1 mL of water, and the reaction was carried out at room temperature for 7 hours. After the reaction was completed, the reaction solution was neutralized with 20 mL of saturated ammonium chloride solution and extracted with dichloromethane (30 mL × 2 times). Then the organic phases were combined, washed with saturated saline (20 mL × 2 times), dried with anhydrous sodium sulfate, filtered, and concentrated under reduced pressure. The resulting crude product was separated by column chromatography to obtain 41 mg of a white solid, 2-((1S)-1-((3,4-*trans*)-4-(6-((4-chloro-2-fluorobenzyl)oxy)pyridin-2-yl)-3-hydroxypiperidin-1-yl)ethyl)-1-(((S)-oxetan-2-yl)methyl)-1H-benzo[*d*]imidazol-6-carboxylic acid (yield: 8.6% for three steps).

LC-MS: RT=1.78 min, [M+H]⁺ =595.25. ¹H NMR (400 MHz, DMSO) δ 12.78 (s, 1H), 8.30 (dd, *J* = 5.1, 1.0 Hz, 1H), 7.83-7.80 (m, 1H), 7.74-7.71 (m, 1H), 7.70-7.68 (m, 1H), 7.61-7.54 (m, 2H), 7.48-7.42 (m, 1H), 7.28-7.24 (m, 1H), 6.84-6.81 (m, 1H), 6.65 (d, *J* = 8.2 Hz, 1H), 5.42-5.32 (m, 2H), 5.24-5.14 (m, 1H), 4.85-4.80 (m, 1H), 4.72-4.66 (m, 1H), 4.55-4.41 (m, 3H), 4.22-4.16 (m, 1H), 3.20-3.17 (m, 2H), 2.70-2.60 (m, 2H), 2.40-2.32 (m, 3H), 1.88 (t, *J* = 5.9 Hz, 2H), 1.48 (dd, *J* = 6.7, 2.7 Hz, 3H).

### Example 32 2-((S)-1-(4-(6-((4-cyano-2-fluorobenzyl)oxy)-3,5-difluoropyridin-2-yl)piperazin-1-yl)ethyl)-1-(((S)-oxetan-2-yl)methyl)-1H-benzo[d]imidazol-6-carboxylic acid

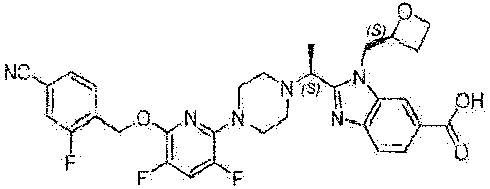

The specific synthetic route is as follows.

### Step A: Synthesis of 3-fluoro-4-(((3,5,6-trifluoropyridin-2-yl)oxy)methyl)benzonitrile

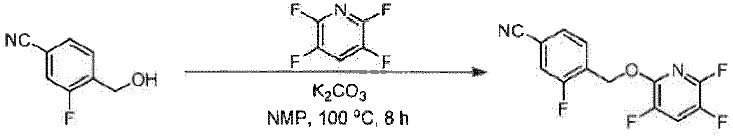

3-Fluoro-4-(hydroxymethyl)benzonitrile (151 mg, 1 mmol) and 2,3,5,6-tetrafluoropyridine (151 mg, 1 mmol) were dissolved in 2 mL of *N*-methylpyrrolidone, followed by an addition of potassium carbonate (414 mg, 3 mmol). The system was purged with nitrogen for 3 times, and the reaction was carried out at 100°C for 8 hours.

After the reaction was completed, the reaction solution was diluted with water and extracted with ethyl acetate (50 mL × 3 times), and the organic phases were combined, washed with saturated saline (20 mL × 3 times), dried with anhydrous sodium sulfate, filtered, and concentrated under reduced pressure. The resulting crude product was separated by column chromatography (n-hexane/ethyl acetate = 8/1) to obtain 212 mg of a pale yellow solid, 3-fluoro-4-(((3,5,6-trifluoropyridin-2-yl)oxy)methyl)benzonitrile (yield: 76.3%). LC-MS: RT=2.09 min, [M-H]⁺=279.13.

### Step B: Synthesis of tert-butyl 4-(6-((4-cyano-2-fluorobenzyl)oxy)-3,5-difluoropyridin-2-yl)piperazin-1-formate

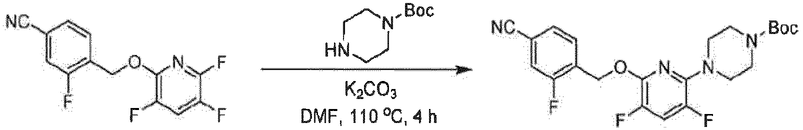

3-Fluoro-4-(((3,5,6-trifluoropyridin-2-yl)oxy)methyl)benzonitrile (212 mg, 0.76 mmol) and tert-butyl piperazin-1-carboxylate (157 mg, 0.84 mmol) were dissolved in 2 mL of *N,N-*dimethylformamide, followed by an addition of potassium carbonate (210 mg, 1.52 mmol). The system was purged with nitrogen for 3 times, and the reaction was carried out at 110°C for 8 hours.

After the reaction was completed, the reaction solution was diluted with water and extracted with ethyl acetate (50 mL × 3 times), and then the organic phases were combined, washed with saturated saline (20 mL × 3 times), dried with anhydrous sodium sulfate, filtered, and concentrated under reduced pressure. The resulting crude product was separated by column chromatography (n-hexane/ethyl acetate = 8/1) to obtain 198 mg of a pale yellow solid, tert-butyl 4-(6-((4-cyano-2-fluorobenzyl)oxy)-3,5-difluoropyridin-2-yl)piperazin-1-formate, was obtained (yield: 58.0%).

### Step C: Synthesis of 4-(6-((4-cyano-2-fluorobenzyl)oxy)-3,5-difluoropyridin-2-yl)piperazine

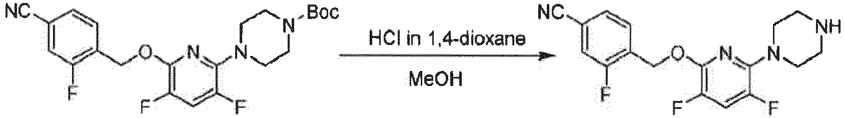

Tert-butyl 4-(6-((4-cyano-2-fluorobenzyl)oxy)-3,5-difluoropyridin-2-yl)piperazin-1-formate (198 mg, 0.44 mmol) was dissolved in 3 mL of methanol, and at 0°C, 4 M hydrochloric acid in 1,4-dioxane (3 mL) was added and the reaction was warmed to room temperature and carried out for 40 min.

After the reaction was completed, the reaction solution was spin-dried directly to give 215 mg of a white solid, 4-(6-((4-cyano-2-fluorobenzyl)oxy)-3,5-difluoropyridin-2-yl)piperazine, which was used directly in the next reaction step. LC-MS: RT = 1.68 min, [M+H]⁺ = 349.16.

### Step D: Synthesis of methyl 2-((S)-1-(4-(6-((4-cyano-2-fluorobenzyl)oxy)-3,5-difluoropyridin-2-yl)piperazin-1-yl)ethyl)-1-(((S)-oxetan-2-yl)methyl)-1H-benzo[d]imidazol-6-carboxylate

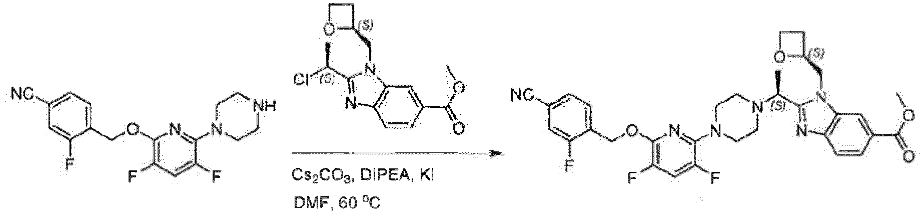

At room temperature, 4-(6-((4-cyano-2-fluorobenzyl)oxy)-3,5-difluoropyridin-2-yl)piperazine (215 mg, 0.44 mmol), methyl 2-((S)-1-chloroethyl)-1-(((S)-oxetan-2-yl)methyl)-1*H*-benzo[*d*]imidazol-6-carboxylate (135 mg, 0.44 mmol), potassium iodide (73 mg, 0.44 mmol) and cesium carbonate (287 mg, 0.88 mmol) were added to *N,N-*dimethylformamide (10.0 mL), followed by an addition of *N,N-*diisopropylethylamine (1.0 mL). The reaction was carried out at 60°C for 3.0 hours under nitrogen protection.

After the reaction was completed, the reaction was quenched with water, and the solution was extracted with ethyl acetate (50 mL × 3 times). The organic phases were combined, washed with saturated saline (30 mL × 3 times), dried with anhydrous sodium sulfate, filtered, and concentrated under reduced pressure. The resultant was purified by silica gel column chromatography (eluent: dichloromethane/methanol = 20/1) to obtain 205 mg of a pale yellow solid, methyl 2-((S)-1-(4-(6-((4-cyano-2-fluorobenzyl)oxy)-3,5-difluoropyridin-2-yl)piperazin-1-yl)ethyl)-1-(((S)-oxetan-2-yl)methyl)-1*H*-benzo[*d*]imidazol-6-carboxylate (yield: 75.1%). LC-MS: RT= 1.89min, [M+H]⁺ = 621.34.

### Step E: Synthesis of 2-((S)-1-(4-(6-((4-cyano-2-fluorobenzyl)oxy)-3,5-difluoropyridin-2-yl)piperazin-1-yl)ethyl)-1-(((S)-oxetan-2-yl)methyl)-1H-benzo[d]imidazol-6-carboxylic acid

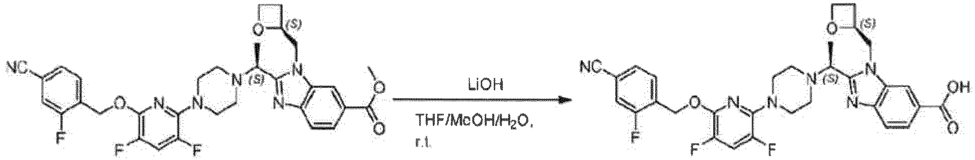

At room temperature, lithium hydroxide (16 mg, 0.66 mmol) in water (1.0 mL) was added dropwise to tetrahydrofuran (3.0 mL) containing methyl 2-((S)-1-(4-(6-((4-cyano-2-fluorobenzyl)oxy)-3,5-difluoropyridin-2-yl)piperazin-1-yl)ethyl)-1-(((S)-oxetan-2-yl)methyl)-1*H*-benzo[*d*]imidazol-6-carboxylate (205 mg, 0.33 mmol), followed by an addition of 1.0 mL of methanol. The reaction was carried out at 25°C for 3.0 hours.

After the reaction was completed, the reaction was quenched with water, and the pH was adjusted to 6 with aqueous ammonium chloride solution (0.5 mol/L), and the resultant was extracted with dichloromethane (30 mL × 3 times). Then the organic phases were combined, washed with aqueous ammonium chloride solution (30 mL × 3 times), dried with anhydrous sodium sulfate, filtered, and concentrated under reduced pressure. The resulting crude product was separated and purified by column chromatography (eluent: dichloromethane/methanol = 15/1) to obtain 132 mg of a pale yellow solid, 2-((S)-1-(4-(6-((4-cyano-2-fluorobenzyl)oxy)-3,5-difluoropyridin-2-yl)piperazin-1-yl)ethyl)-1-(((S)-oxetan-2-yl)methyl)-1*H-*benzo[*d*]imidazol-6-carboxylic acid (yield: 51.1%).

LC-MS: RT = 1.79 min, [M+H]⁺ = 607.29. ¹H NMR (400 MHz, DMSO-d₆) δ 12.74 (s, 1H), 827 (s, 1H), 7.92-7.86 (m, 1H), 7.83--75 (m, 2H), 7.72-7.62 (m, 3H), 5.45 (d, *J* = 13.7 Hz, 2H), 5.13 (d, *J* = 2.9 Hz, 1H), 4.83 4.74 (m, 1H), 4.67 (dd, *J* = 15.4, 5.5 Hz, 1H), 4.49-4.40 (m, 2H), 4.17 (dt, *J* = 9.1, 5.8 Hz, 1H), 3.29-3.18 (m, 3H), 2.67-2.52 (m, 5H), 2.29 (dd, *J* = 18.2, 7.3 Hz, 1H), 1.42 (t, *J* = 8.0 Hz, 3H).

### Example 33 2-((1S)-1-((3,4-cis)-4-(6-((4-chloro-2-fluorobenzyl)oay)pyridin-2-yl)-3-fluoropiperidin-1-yl)ethyl)-1-(((S)-oxetan-2-yl)methyl)-1H-benzo[d]imidazol-6-carboxylic acid

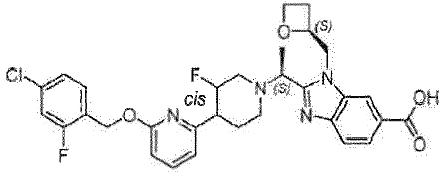

The specific synthetic route is as follows.

### Step A: Synthesis of tert-butyl (3,4-cis)-4-(6-((4-chloro-2-fluorobenzyl)oxy)pyridin-2-yl)-3-fluoropiperidin-1-formate

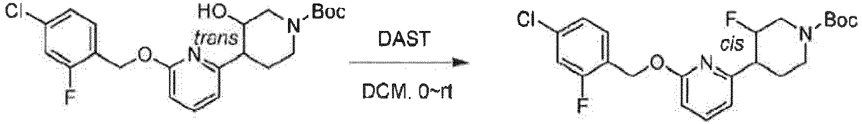

Tert-butyl (3,4-*trans*)-4-(6-((4-chloro-2-fluorobenzyl)oxy)pyridin-2-yl)-3-hydroxypiperidin-1-formate (500 mg, 1.14 mmol) was dissolved in 5 mL of anhydrous dichloromethane, and at 0°C, diethylaminosulfur trifluoride (220 mg, 1.37 mmol) was added, and the reaction was carried out at 0°C for 30 min. After the reaction was completed, the reaction solution was poured into 60 mL of ice water and extracted with ethyl acetate (40 mL × 2 times). The organic phases were combined, washed with saturated saline (30 mL × 2 times), dried with anhydrous sodium sulfate, filtered, and concentrated under reduced pressure to obtain 485 mg of tert-butyl (3,4-*cis*)-4-(6-((4-chloro-2-fluorobenzyl)oxy)pyridin-2-yl)-3-fluoropiperidin-1-formate. LC-MS: RT = 1.97min, [M+H]⁺ = 439.22.

### Step B: Synthesis of 2-((4-chloro-2-fluorobenzyl)oxy)-6-((3,4-cis)-3-fluoropiperidin-4-yl)pyridine

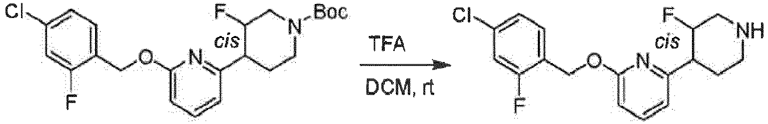

Tert-butyl (3,4-*cis*)-4-(6-((4-chloro-2-fluorobenzyl)oxy)pyridin-2-yl)-3-fluoropiperidin-1-formate (485 mg, 1.10 mmol) was dissolved in 5 mL of anhydrous dichloromethane, followed by an addition of 2 mL of trifluoroacetic acid, and the reaction was carried out at room temperature for 40 min. After the reaction was completed, the reaction solution was poured into 60 mL of sodium bicarbonate solution and extracted with ethyl acetate (50 mL × 2 times). Then the organic phases were combined, washed with saturated saline (30 mL × 2 times), dried with anhydrous sodium sulfate, and finally concentrated under reduced pressure to obtain 330 mg of 2-((4-chloro-2-fluorobenzyl)oxy)-6-((3,4-c*is*)-3-fluoropiperidin-4-yl)pyridine. LC-MS: RT = 1.92 min, [M+H]⁺ = 339.17.

### Step C: Synthesis of methyl 2-((1S)-1-((3,4-cis)-4-(6-((4-chloro-2-fluorobenzyl)oxy)pyridin-2-yl)-3-fluoropiperidin-1-yl)ethyl)-1-(((S)-oxetan-2-yl)methyl)-1H-benzo[d]imidazol-6-carboxylate

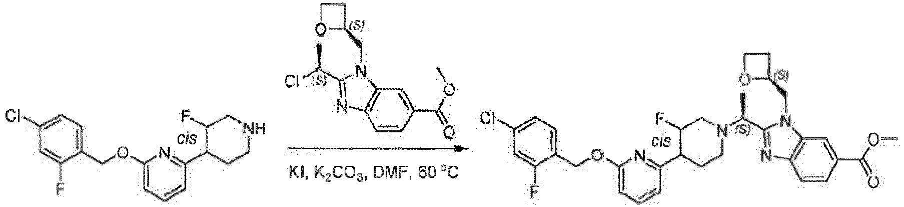

2-((4-Chloro-2-fluorobenzyl)oxy)-6-((3,4-cis)-3-fluoropiperidin-4-yl)pyridine (330 mg, 0.973 mmol), potassium carbonate (268 mg, 1.94 mmol) and potassium iodide (242 mg, 1.46 mmol) were dissolved in 5 mL of anhydrous acetonitrile, followed by an addition of methyl 2-((S)-1-chloroethyl)-1-(((S)-oxetan-2-yl)methyl)-1*H*-benzo[*d*]imidazol-6-carboxylate (299 mg, 0.973 mmol), and the reaction carried out at 60°C for 2 hours. After the reaction was completed, the reaction solution was diluted with water and extracted with ethyl acetate (40 mL × 2 times). Then the organic phases were combined, washed with saturated saline (30 mL × 2 times), dried with anhydrous sodium sulfate, and finally concentrated under reduced pressure. The resulting crude product was separated by column chromatography to obtain 510 mg of a pale yellow solid, methyl 2-((1S)-1-((3,4-cis)-4-(6-((4-chloro-2-fluorobenzyl)oxy)pyridin-2-yl)-3-fluoropiperidin-1-yl)cthyl)-1-(((S)-oxetan-2-yl)methyl)-1*H*-benzo[*d*]imidazol-6-carboxylate. LC-MS: RT = 2.08 min, [M+H]⁺ = 611.23.

### Step D: Synthesis of 2-((1S)-1-((3,4-cis)-4-(6-((4-chloro-2-fluorobenzyl)oxy)pyridin-2-yl)-3-fluoropiperidin-1-yl)ethyl)-1-(((S)-oxetan-2-yl)methyl)-1H-benzo[d]imidazol-6-carboxylic acid

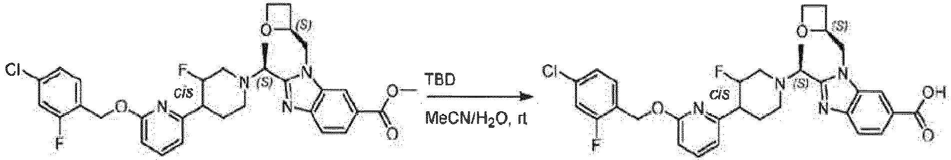

Methyl 2-((1S)-1-((3,4-*cis*)-4-(6-((4-chloro-2-fluorobenzyl)oxy)pyridin-2-yl)-3-fluoropiperidin-1-yl)ethyl)-1-(((S)-oxetan-2-yl)methyl)-1*H*-benzo[*d*]imidazol-6-carboxylate (200 mg, 0.327 mmol) was dissolved in 4 mL of acetonitrile, then 1,5,7-triazabicyclo[4.4.0]dec-5-ene (95.5 mg, 0.687 mmol) and 1 mL of water were added, and the reaction was carried out at room temperature for 7 hours. After the reaction was completed, the reaction solution was neutralized with 20 mL of saturated ammonium chloride solution and extracted with dichloromethane (40 mL × 2 times). Then the organic phases were combined, washed with saturated saline (20 mL × 2 times), dried with anhydrous sodium sulfate, filtered, and concentrated under reduced pressure. The resulting crude product was separated by column chromatography to obtain 47 mg of a white solid, 2-((1S)-1-((3,4-cis)-4-(6-((4-chloro-2-fluorobenzyl)oxy)pyridin-2-yl)-3-fluoropiperidin-1-yl)ethyl)-1-(((S)-oxetan-2-yl)methyl)-1*H* benzo[d]imidazol-6-carboxylic acid, was obtained (yield: 6.9% for four steps).

LC-MS: RT =1.96 min, [M+H]⁺ =597.23. ¹H NMR (400 MHz, DMSO) δ 12.77 (s, 1H), 8.31 (s, 1H), 7.84-7.80 (m, 1H), 7.73-7.69 (m, 1H), 7.66-7.60 (m, 1H), 7.57-7.53 (m, 1H), 7.42 (ddd,J= 10.0, 4.8, 2.0 Hz, 1H), 7.28 7.23 (m, 1H), 6.90 (d, *J* = 7.4 Hz, 1H), 6.72 (d, *J* = 8.2 Hz, 1H), 5.43-5.34 (m, 2H), 5.20-5.14 (m, 1H), 5.02-4.82 (m, 1H), 4.78-4.68 (m, 2H), 4.62-4.53 (m, 2H), 4.43 (dd, *J* = 13.5, 7.8 Hz, 1H), 4.21-4.16 (m, 1H), 3.03-2.91 (m, 1H), 2.77-2.57 (m, 3H), 2.34-2.18 (m, 211), 1.85-1.61 (m, 2H), 1.50 (dd, *J* = 6.7, 2.8 Hz, 3H).

### Example 34 2-((S)-1-((R)-4-(6-((4-cyano-2-fluorobenzyl)oxy)pyridin-2-yl)-2-(fluoromethyl)piperazin-1-yl)ethyl)-1-(((S)-oxetan-2-yl)methyl)-1H-benzo[d]imidazol-6-carboxylic acid

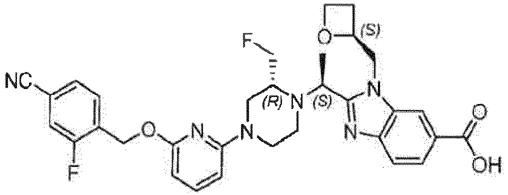

The specific synthetic route is as follows.

### Step A: Synthesis of methyl 2-((S)-1-((R)-4-(6-((4-cyano-2-fluorobenzyl)oxy)pyridin-2-yl)-2-(hydroxymethyl)piperazin-1-yl)ethyl)-1-(((S)-oxetan-2-yl)methyl)-1H-benzo[d]imidazol-6-carboxylate

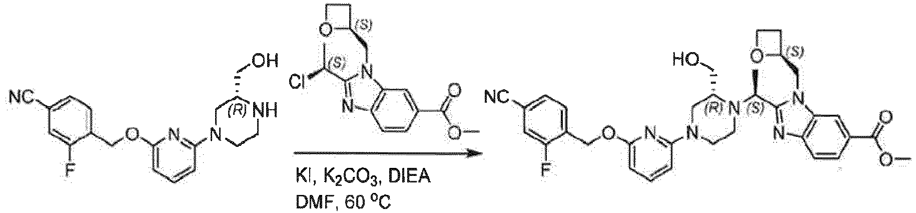

At room temperature, (R)-3-fluoro-4-(((6-(3-(hydroxymethyl)piperazin-1-yl)pyridin-2-yl)oxy)methyl)benzonitrile (160 mg, 0.47 mmol), DIEA (1 mL), potassium iodide (155 mg, 0.93 mmol) and potassium carbonate (129 g, 0.93 mmol) were dissolved in *N,N-*dimethylformamide (5 mL), then methyl 2-((S)-1-chloroethyl)-1-(((S)-oxetan-2-yl)methyl)-1*H*-benzo[*d*]imidazol-6-carboxylate (144 mg, 0.47 mmol) was added, and the reaction solution was heated to 50°C and reacted.

After the reaction was completed, the reaction solution was quenched with saturated sodium chloride solution and extracted with ethyl acetate (20 mL × 2 times). Then the organic phases were combined, dried with anhydrous sodium sulfate, filtered, and concentrated. The resulting crude product was purified by silica gel column chromatography (eluent: ethyl acetate/n-hexane = 10/1) to obtain 94 mg of a pale yellow solid, methyl 2-((S)-1-((R)-4-(6-((4-cyano-2-fluorobenzyl)oxy)pyridin-2-yl)-2-(hydroxymethyl)piperazin-1-yl)ethyl)-1-(((S)-oxetan-2-yl)methyl)-1*H*-benzo[*d*]imidazol-6-carboxylate (yield: 33%). LC-MS: RT= 1.81 min, [M+H]⁺ = 615.30.

### Step B: Synthesis of methyl 2-((S)-1-((R)-4-(6-((4-cyano-2-fluorobenzyl)oxy)pyridin-2-yl)-2-(fluoromethyl)piperazin-1-yl)ethyl)-1-(((S)-oxetan-2-yl)methyl)-1H-benzo[d]imidazol-6-carboxylate

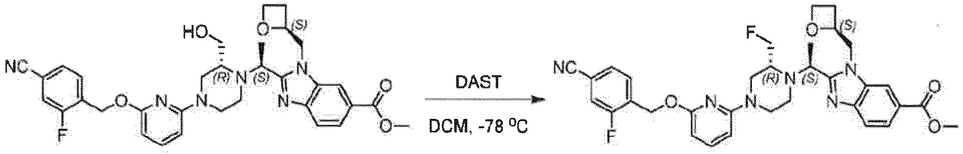

Methyl 2-((S)-1-((R)-4-(6-((4-cyano-2-fluorobenzyl)oxy)pyridin-2-yl)-2-(hydroxymethyl)piperazin-1-yl)ethyl)-1-(((S)-oxetan-2-yl)methyl)-1*H*-benzo[*d*]imidazol-6-carboxylate (90 mg, 0.15 mmol) was dissolved in dichloromethane (2 mL). Then the solution was cooled to -78°C, followed by an addition of DAST (63 mg, 0.29 mmol), and was stirred at low temperature.

After the reaction was completed, the reaction was quenched with saturated sodium chloride, and the reaction solution was extracted with ethyl acetate (10 mL × 2 times). The organic phases were combined, dried with anhydrous sodium sulfate, filtered and concentrated. The resulting crude product was purified by silica gel column chromatography (eluent: ethyl acetate/n-hexane = 10/1), and 80 mg of a pale yellow solid, methyl 2-((S)-1-((R)-4-(6-((4-cyano-2-fluorobenzyl)oxy)pyridin-2-yl)-2-(fluoromethyl)piperazin-1-yl)ethyl)-1-(((S)-oxetan-2-yl)methyl)-1*H*-benzo[*d*]imidazol-6-carboxylate, was obtained (yield: 33%). LC-MS: RT = 2.09 min, [M+H]⁺ = 617.27.

### Step C: Synthesis of 2-((S)-1-((R)-4-(6-((4-cyano-2-fluorobenzyl)oxy)pyridin-2-yl)-2-(fluoromethyl)piperazin-1-yl)ethyl)-1-(((S)-oxetan-2-yl)methyl)-1H-benzo[d]imidazol-6-carboxylic acid

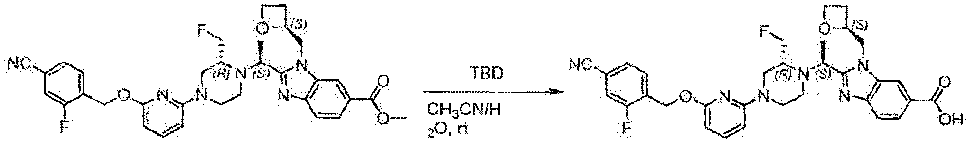

Methyl 2-((S)-1-((R)-4-(6-((4-cyano-2-fluorobenzyl)oxy)pyridin-2-yl)-2-(fluoromethyl)piperazin-1-yl)ethyl)-1-(((S)-oxetan-2-yl)methyl)-1*H*-benzo[*d*]imidazol-6-carboxylate (80 mg, 0.13 mmol) was dissolved in a mixed solvent (6 mL, acetonitrile/water=5/1), then 1,5,7-triazabicyclo(4,4,0)dec-5-ene (36 mg, 0.26 mmol) was added and the reaction was carried out at room temperature.

After the reaction was completed, the reaction was quenched with citric acid, and the reaction solution was extracted with ethyl acetate (10 mL × 2 times). Then the organic phases were combined, dried with anhydrous sodium sulfate, filtered and concentrated, and the crude product obtained from the concentration was processed by high performance liquid phase chromatography to obtain 9 mg of a white solid, 2-((S)-1-((R)-4-(6-((4-cyano-2-fluorobenzyl)oxy)pyridin-2-yl)-2-(fluoromethyl)piperazin-1-**yl)ethyl)-1-(((S)-oxetan-2-yl)methyl)-1*H-*benzo[*d*]imidazol-6-carboxylic** acid (yield: 12%). LC-MS: RT = 1.97 min, [M+H]⁺ = 603.31.

### Example 35 2-((1S)-1-((3,4-cis)-4-(6-((4-chloro-2-fluorobenzyl)oaey)pyridin-2-yl)-3-hydroxypiperidin-l-yl)ethyl)-1-(((S)-oxetan-2-yl)methyl)-1H-benzo[d]imidazol-6-carboxylic acid

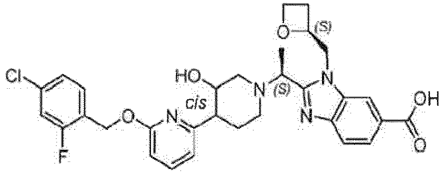

The specific synthetic route is as follows.

### Step A: Synthesis of tert-butyl 6-((4-chloro-2-fluorobenzyl)oxy)-3',6'-dihydro-[2,4'-dipyridyl]-1'(2'H)-carboxylate

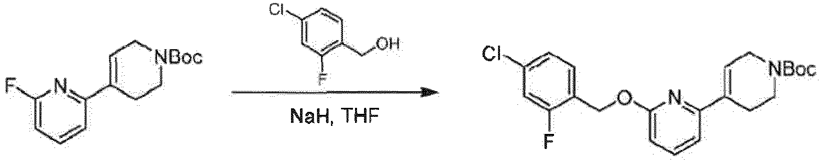

(4-Chloro-2-fluorophenyl)methanol (17 g, 106.5 mmol) was dissolved in tetrahydrofuran (300 mL), then it was cooled to 0°C, followed by an addition of sodium hydride (5.6 g, 142 mmol). The mixture was stirred at room temperature for 0.5 hour, and then tert-butyl 6-fluoro-3',6'-dihydro-[2,4'-dipyridyl]-1'(2'H)-carboxylate (20 g, 71 mmol) was added, and was stirred at room temperature overnight.

After the reaction was completed, the reaction was quenched with saturated sodium chloride solution, and the reaction solution was extracted with ethyl acetate (30 mL), washed twice with water, dried with anhydrous sodium sulfate, filtered, and concentrated. The resulting crude product was purified by silica gel column chromatography (eluent: ethyl acetate/n-hexane = 1/10) to obtain 10 g of a white solid, tert-butyl 6-((4-chloro-2-fluorobenzyl)oxy)-3',6'-dihydro-[2,4'-dipyridyl]-1'(2'H)-carboxylate (yield: 34%). LC-MS: RT = 2.15 min, [M+H]⁺ = 419.23.

### Step B: Synthesis of tert-butyl (3,4-trans)-4-(6-((4-chloro-2-fluorobenzyl)oxy)pyridin-2-yl)-3-hydroxypiperidin-1-formate

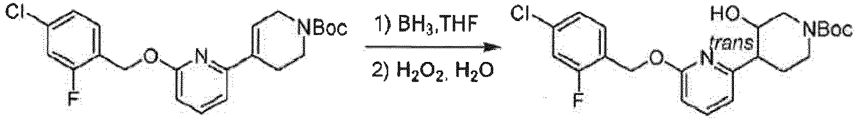

Tert-butyl 6-((4-chloro-2-fluorobenzyl)oxy)-3',6'-dihydro-[2,4'-dipyridin]-1'(2'H)-carboxylate (10 g, 24 mmol) was dissolved in tetrahydrofuran (150 mL), and the solution was cooled to 0°C, then borane in tetrahydrofuran (1 M, 26 mL, 26 mmol) was added slowly dropwise. The mixture was stirred at room temperature for 0.5 hour, followed by an addition of sodium hydroxide (1 M, 2.4 g) and hydrogen peroxide (30%, 10 mL), and was stirred at room temperature overnight.

After the reaction was completed, the reaction was quenched with saturated sodium bisulfite solution and saturated sodium bicarbonate solution, and the reaction solution was extracted with ethyl acetate (30 mL × 3 times). Then the organic phases were combined, dried with anhydrous sodium sulfate, filtered and concentrated, and the resulting crude product was purified by silica gel column chromatography (eluent: ethyl acetate/n-hexane = 1/5) to obtain 9 g of a white solid, tert-butyl *(3,4-trans)-4-(6-((4-*chloro-2-fluorobenzyl)oxy)pyridin-2-yl)-3-hydroxypiperidin-1-formate (yield: 90%). LC-MS: RT = 1.90 min, [M+H]⁺ = 437.37.

### Step C: Synthesis of tert-butyl (3,4-cis)-4-(6-(((4-chloro-2-fluorobenzyl)oxy)pyridin-2-yl)-3-((4-nitrobenzoyl)oxy)piperidin-1-formate

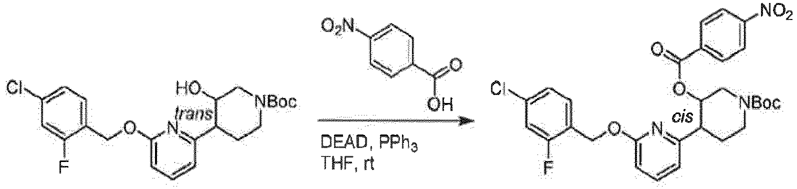

Triphenylphosphine was dissolved in tetrahydrofuran, and diethyl azodicarboxylate (800 mg, 4.6 mmol) was added under ice bath. The mixture was stirred for 0.5 hour, followed by an addition of tert-butyl (3,4-*trans*)-4-(6-((4-chloro-2-fluorobenzyl)oxy)pyridin-2-yl)-3-hydroxypiperidin-1-formate (1 g, 2.3 mmol), and was stirred for 15 min. Then p-nitrobenzoic acid (768 mg, 4.6 mmol) was added, and the mixture was stirred at room temperature for 16 hours.

After the reaction was completed, the reaction was quenched with saturated sodium chloride solution and extracted with ethyl acetate (100 mL × 3 times). Then the organic phases were combined, washed twice with water, dried with anhydrous sodium sulfate, filtered, and concentrated. The resulting crude product was purified by silica gel column chromatography (eluent: ethyl acetate/n-hexane = 1/10) to obtain 800 mg of a white solid, tert-butyl (3,4-*cis*)-4-(6-(((4-chloro-2-fluorobenzyl)oxy)pyridin-2-yl)-3-((4-nitrobenzoyl)oxy)piperidin-1-formate (yield: 45%). LC-MS: RT = 2.13 min, [M+H]⁺ = 586.19.

### Step D: Synthesis of tert-butyl (3,4-cis)-4-(6-((4-chloro-2-fluorobenzyl)oxy)pyridin-2-yl)-3-hydroxypiperidin-1-formate

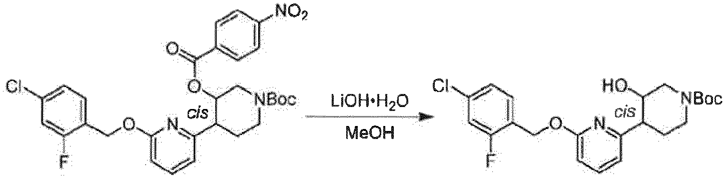

Tert-butyl (3,4-cis)-4-(6-(((4-chloro-2-fluorobenzyl)oxy)pyridin-2-yl)-3-((4-nitrobenzoyl)oxy)piperidin-1-formate (500 mg, 0.85 mmol) was dissolved in methanol (10 mL) and water (2 mL), followed by an addition of lithium hydroxide monohydrate (107 mg, 2.55 mmol), and the reaction was carried out at room temperature for 1 hour.

After the reaction was completed, the reaction solution was quenched and diluted with water, and was extracted with ethyl acetate (20 mL × 3 times). Then the organic phases were combined, washed twice with saturated sodium bicarbonate solution, dried with anhydrous sodium sulfate, filtered and concentrated to obtain 400 mg of a yellow solid, tert-butyl (3,4-*cis*)-4-(6-((4-chloro-2-fluorobenzyl)oxy)pyridin-2-yl)-3-hydroxypiperidin-1-formate (yield: 98%). LC-MS: RT = 1.90 min, [M+H]⁺ = 437.25.

### Step E: Synthesis of (3,4-cis)-4-(6-((4-chloro-2-fluorobenzyl)oxy)pyridin-2-yl)piperidin-3-ol

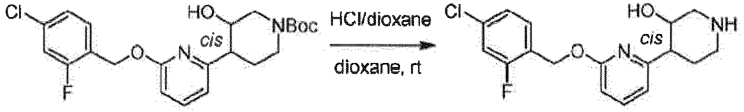

At room temperature, tert-butyl (3,4-*cis*)-4-(6-((4-chloro-2-fluorobenzyl)oxy)pyridin-2-yl)-3-hydroxypiperidin-1-formate (150 mg, 0.34 mmol) was dissolved in 1,4-dioxane (2 mL), and hydrochloric acid in 1,4-dioxane (2 mL) was added under an ice water bath. The mixture was stirred at room temperature for 0.5 hour.

After the reaction was completed, 120 mg of a white solid, (3,4-*cis*)-4-(6-((4-chloro-2-fluorobenzyl)oxy)pyridin-2-yl)piperidin-3-ol, was obtained by concentration. LC-MS: RT = 1.65 min, [M+H]⁺ = 337.25.

### Step F: Synthesis of methyl 2-((1S)-1-((3,4-cis)-4-(6-((4-chloro-2-fluorobenzyl)oxy)pyridin-2-yl)-3-hydroxypiperidin-1-yl)ethyl)-1-(((S)-oxetan-2-yl)methyl)-1H-benzo[d]imidazol-6-carboxylate

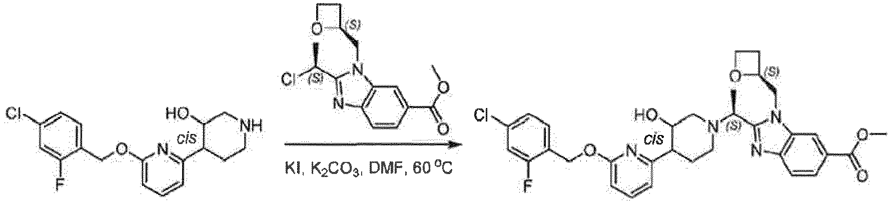

At room temperature, (3,4-cis)-4-(6-((4-chloro-2-fluorobenzyl)oxy)pyridin-2-yl)piperidin-3-ol (120 mg, 0.34 mmol) was dissolved in *N,N-*dimethylformamide (2 mL), followed by an addition of methyl 2-((S)-(1-chloroethyl)-1-(((S)-oxetan-2-yl)methyl)-1*H*-benzo[*d*]imidazol-6-carboxylate (157 mg, 0.51 mmol), potassium iodide (113 mg, 0.68 mmol) and potassium carbonate (88 mg, 0.68 mmol), and then the mixture was stirred at 60°C for 12 hours.

After the reaction was completed, the reaction was quenched with saturated sodium chloride solution, and the reaction solution was extracted with ethyl acetate (10 mL × 2 times). The organic phases were combined, dried with anhydrous sodium sulfate, filtered and concentrated, and the resulting crude product was purified by silica gel column chromatography (eluent: ethyl acetate/n-hexane = 1/1) to obtain 60 mg of a colorless oily liquid, methyl 2-((1S)-1-((3,4-cis)-4-(6-((4-chloro-2-fluorobenzyl)oxy)pyridin-2-yl)-3-hydroxypiperidin-1-yl)ethyl)-1-(((S)-oxetan-2-yl)methyl)-1*H-*benzo[*d*]imidazol-6-carboxylate (yield: 42%). LC-MS: RT = 1.97 min, [M+H]⁺ = 609.35.

### Step G: Synthesis of 2-((1S)-1-((3,4-cis)-4-(6-((4-chloro-2-fluorobenzyl)oxy)pyridin-2-yl)-3-hydroxypiperidin-1-yl)ethyl)-1-(((S)-oxetan-2-yl)methyl)-1H-benzo[d]imidazol-6-carboarylic acid

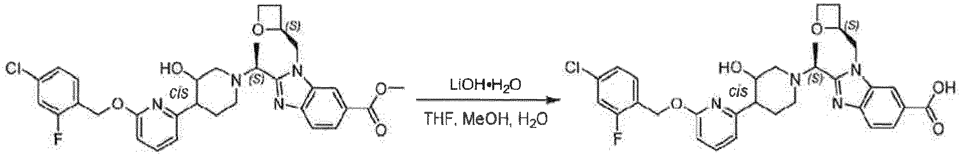

Methyl 2-((1S)-1-((3,4-*cis*)-4-(6-((4-chloro-2-fluorobenzyl)oxy)pyridin-2-yl)-3-hydroxypiperidin-1-yl)ethyl)-1-(((S)-oxetan-2-yl)methyl)-1*H*-benzo[*d*]imidazol-6-carboxylate (60 mg, 0.1 mmol) was dissolved in a mixed solvent of tetrahydrofuran (3 mL), methanol (1 mL) and water (1 mL), then lithium hydroxide monohydrate (12 mg, 0.3 mmol) was added at room temperature, and the reaction was carried out for 4 hours.

After the reaction was completed, the reaction solution was concentrated, and the resulting crude product was purified by high performance liquid phase chromatography to obtain 18.22 mg of a white solid, 2-((1S)-1-((3,4-*cis*)-4-(6-((4-chloro-2-fluorobenzyl)oxy)pyridin-2-yl)-3-hydroxypiperidin-1-**yl)ethyl)-1-(((S)-oxetan-2-yl)methyl)-1*H-*benzo[*d*]imidazol-6-carboxylic** acid (yield: 30%). LC-MS: RT = 1.81 min, [M+H]⁺ = 595.27.

### Example 36 2-((1S)-1-((3,4-trans)-4-(6-((4-chloro-2-fluorobenzyl)oxy)pyridin-2-yl)-3-fluoropiperidin-1-yl)ethyl)-1-(((S)-oxetan-2-yl)methyl)-1H-benzo[d]imidazol-6-carboxylic acid

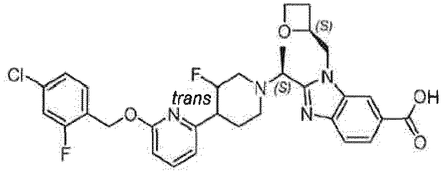

The specific synthesis route is as follows.

### Step A: Synthesis of tert-butyl (3,4-trans)-4-(6-((4-chloro-2-fluorobenzyl)oaη)pyridin-2-yl)-3-fluoropiperidin-1-formate

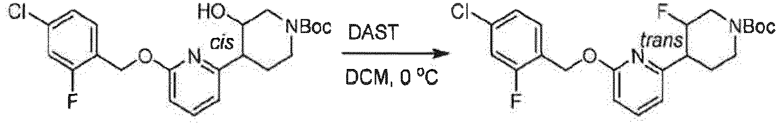

Tert-butyl (3,4-*cis*)-4-(6-((4-chloro-2-fluorobenzyl)oxy)pyridin-2-yl)-3-hydroxypiperidin-1-formate (200 mg, 0.45 mmol) was dissolved in tetrahydrofuran, and diethylaminosulfur trifluoride (147 mg, 0.9 mmol) was added under an ice bath, and the mixed solution was stirred for 0.5 h.

After the reaction was completed, the reaction solution was quenched by adding saturated sodium chloride solution, extracted with ethyl acetate (50 mL × 3 times), washed twice with water, dried with anhydrous sodium sulfate, filtered, and concentrated. Then the crude product was purified by silica gel column chromatography (eluent: ethyl acetate/n-hexane = 1/10) to obtain 150 mg of yellow solid tert-butyl (3,4-*trans*)-4-(6-((4-chloro-2-fluorobenzyl)oxy)pyridin-2-yl)-3-fluoropiperidin-1 -formate (yield: 76%). LC-MS: RT = 2.03 min, [M+H]⁺ = 439.19.

### Step B: Synthesis of 2-((4-chloro-2-fluorobenzyl)oxy)-6-((3,4-trans)-3-fluoropiperidin-4-yl)-pyridine

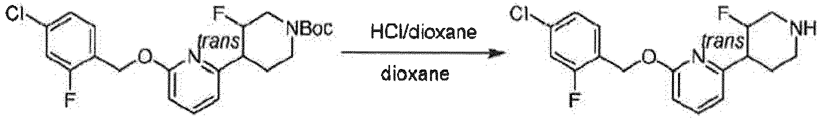

Tert-butyl (3,4-*trans*)-4-(6-((4-chloro-2-fluorobenzyl)oxy)pyridin-2-yl)-3-fluoropiperidin-1-formate (150 mg, 0.34 mmol) was dissolved in 1,4-dioxane (2 mL) at room temperature, and hydrochloric acid in 1,4-dioxane (2 mL) was added under an ice bath. The mixed solution was stirred at room temperature for 0.5 h.

After the reaction was completed, the reaction solution was concentrated to obtain 120 mg of white solid 2-((4-chloro-2-fluorobenzyl)oxy)-6-((3,4-trans)-3-fluoropiperidin-4-yl)-pyridine. LC-MS: RT = 1.68 min, [M+H]⁺ = 339.29.

### Step C: Synthesis of methyl 2-((1S)-1-((3,4-trans)-4-(6-((4-chloro-2-fluorobenzyl)oxy)pyridin-2-yl)-3-fluoropiperidin-1-yl)ethyl)-1-(((S)-oxetan-2-yl)methyl)-1H-benzo[d]imidazol-6-carbooylate

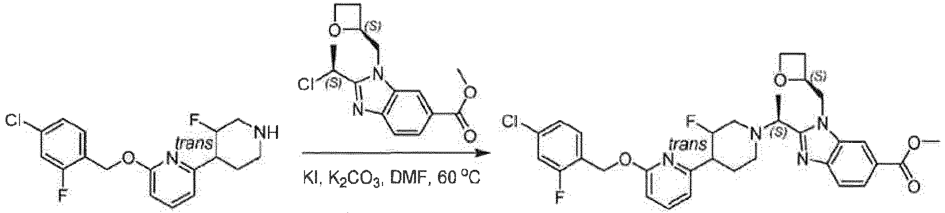

2-((4-Chloro-2-fluorobenzyl)oxy)-6-((3,4-trans)-3-fluoropiperidin-4-yl)-pyridine (120 mg, 0.34 mmol) was dissolved in N,N-dimethylformamide (2 mL) at room temperature, and methyl 2-((S)-1-chloroethyl)-1-(((S)-oxetan-2-yl)methyl)-1*H-*benzo[*d*]imidazol-6-carboxylate (157 mg, 0.51 mmol), potassium iodide (113 mg, 0.68 mmol) and potassium carbonate (88 mg, 0.68 mmol) were added, and the mixed solution was stirred at 60°C for 12 h.

After the reaction was completed, the reaction solution was quenched by adding saturated sodium chloride solution, and was extracted with ethyl acetate (10 mL × 2 times). The organic phases were combined, dried with anhydrous sodium sulfate, filtered and concentrated, and the resulting crude product was purified by silica gel column chromatography (eluent: ethyl acetate/n-hexane = 1/1) to obtain 60 mg of colorless oily liquid methyl 2-((1S)-1-((3,4*-trans*)-4-(6-((4-chloro-2-fluorobenzyl)oxy)pyridin-2-yl)-3-fluoropiperidin-1-yl)ethyl)-1-(((S)-oxetan-2-yl)methyl)-1*H-*benzo[*d*]imidazol-6-carboxylate (yield: 42%). LC-MS: RT= 1.99 min, [M+H]⁺ = 611.38.

### Step D: Synthesis of 2-((1S)-1-((3,4-trans)-4-(6-((4-chloro-2-fluorobenzyl)oxy)pyridin-2-yl)-3-fluoropiperidin-1-yl)ethyl)-1-(((S)-oxetan-2-yl)methyl)-1H-benzo[d]imidazol-6-carboxylic acid

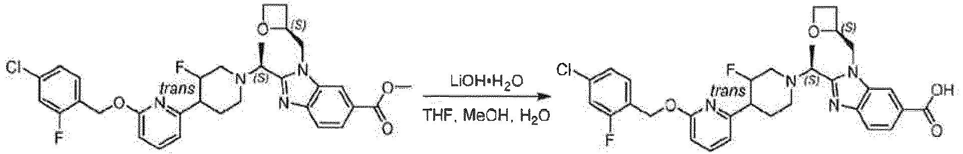

Methyl 2-((1S)-1-((3,4-*trans*)-4-(6-((4-chloro-2-fluorobenzyl)oxy)pyridin-2-yl)-3-fluoropiperidin-1-yl)ethyl)-1-(((S)-oxetan-2-yl)methyl)-1*H*-benzo[*d*]imidazol-6-carboxylate (60 mg, 0.1 mmol) was dissolved in a solvent mixture of tetrahydrofuran (3 mL), methanol (1 mL) and water (1 mL), and lithium hydroxide monohydrate (12 mg, 0.3 mmol) was added at room temperature, and the reaction was carried out for 4 h.

After the reaction was completed, the reaction solution was concentrated, and the crude product was purified by high performance liquid chromatography to obtain 18.22 mg of white solid 2-((1S)-1-((3,4-*trans*)-4-(6-((4-chloro-2-fluorobenzyl)oxy)pyridin-2-yl)-3-fluoropiperidin-1-yl)ethyl)-1-(((S)-oxetan-2-yl)methyl)-1*H*-benzo[*d*]imidazol-6-carboxylic acid (yield: 30%). LC-MS: RT = 1.88 min, [M+H]⁺ = 597.23.

### Example 37 2-((S)-1-(4-(6-((3-fluoroquinolin-8-yl)methooy)pyridin-2-yl)piperidin-1-yl)ethyl)-1-(((S)-oxetan-2-yl)methyl)-1H-benzo[d]imidazol-6-carboxylic acid

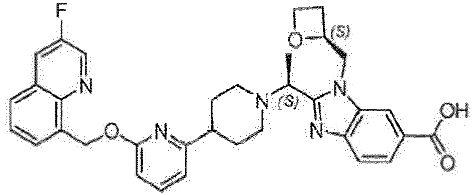

The specific synthesis route is as follows.

### Step A: Synthesis of methyl 2-((S)-1-(4-(6-((3-fluoroquinolin-8-yl)methoxy)pyridin-2-yl)piperidin-1-yl)ethyl)-1-(((S)-oxetan-2-yl)methyl)-1H-benzo[d]imidazol-6-carboxylate

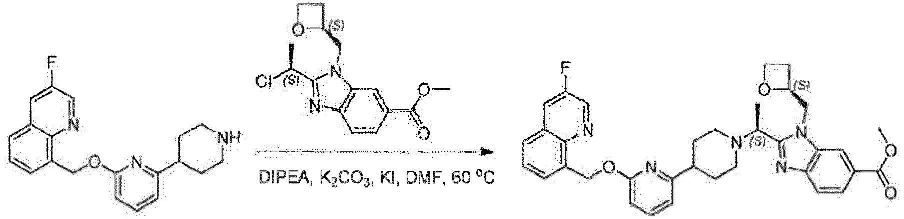

N,N-diisopropylethylamine (161 µL, 0.975 mmol) was added into a solution of N,N-dimethylmethaneamide (2.0 mL) containing 3-fluoro-8-(((6-(piperidin-4-yl)pyridin-2-yl)oxy)methyl)quinoline (132 mg, 0.325 mmol) at room temperature and was dissolved under stirring. Methyl 2-((S)-1-chloroethyl)-1-(((S)-oxetan-2-yl)methyl)-1*H*-benzo[*d*]imidazol-6-carboxylate (100 mg, 0.325 mmol), potassium carbonate (90 mg, 0.65 mmol) and potassium iodide (108 mg, 0.65 mmol) were added sequentially. After the addition, the mixed solution was heated to 60°C and stirred for 1 h.

After the reaction was completed, the reaction solution was quenched by adding saturated ammonium chloride solution and extracted with ethyl acetate (30 mL × 3 times). The organic phases were combined, then washed with saturated saline (30 mL × 3 times), dried with anhydrous sodium sulfate, filtered and concentrated under reduced pressure. The residue was purified by silica gel column chromatography (eluent: methanol/dichloromethane = 6/94). 111 mg of colorless oily product, methyl 2-((S)-1-(4-(6-((3-fluoroquinolin-8-yl)methoxy)pyridin-2-yl)piperidin-l-yl)ethyl)-1-(((S)-oxetan-2-yl)methyl)-1*H-*benzo[*d*]imidazol-6-carboxylate, was obtained (yield: 56.0%). LC-MS: RT = 1.87 min, [M+H]⁺ = 610.33.

### Step B: Synthesis of 2-((S)-1-(4-(6-((3-fluoroquinolin-8-yl)methoxy)pyridin-2-yl)piperidin-1-yl)ethyl)-1-(((S)-oxetan-2-yl)methyl)-1H-benzo[d]imidazol-6-carboxylic acid

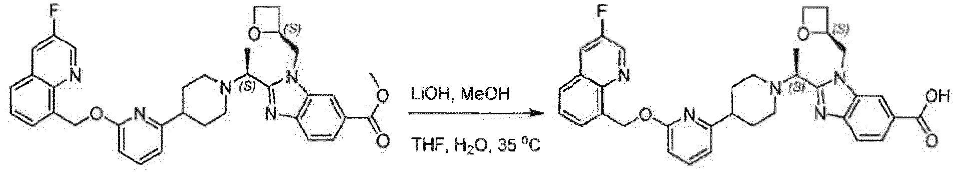

Lithium hydroxide monohydrate (31 mg, 0.73 mmol) was added into tetrahydrofuran/methanol/water solution (2.5 mL /2.5 mL /1.0 mL) containing methyl 2-((S)-1-(4-(6-((3-fluoroquinolin-8-yl)methoxy)pyridin-2-yl)piperidin-1-yl)ethyl)-1-(((S)-oxetan-2-yl)methyl)-1*H*-benzo[*d*]imidazol-6-carboxylate (111 mg, 0.18 mmol) at room temperature, and the mixed solution was heated to 35°C and the reaction was carried out for 1 hour. After the reaction was completed, the reaction solution was quenched by adding saturated ammonium chloride solution and extracted with ethyl acetate (30 mL × 3 times). The organic phases were combined, then washed with saturated saline (30 mL × 3 times), dried with anhydrous sodium sulfate, filtered and concentrated under reduced pressure. The residue was purified by silica gel column chromatography (eluent: methanol/dichloromethane = 8/92). 27 mg of white solid, 2-((S)-1-(4-(6-((3-fluoroquinolin-8-yl)methoxy)pyridin-2-yl)piperidin-1-yl)ethyl)-1-(((S)-oxetan-2-yl)methyl)-1*H-*benzo[*d*]imidazol-6-carboxylic acid, was obtained (yield: 24.9%). LC-MS: RT = 1.82 min, [M+H]⁺ = 596.32. ¹H NMR (400 MHz, DMSO) δ 12.75 (s, 1H), 9.00 (d, *J* = 2.9 Hz, 1H), 8.30 (dd, *J* = 9.6, 2.9 Hz, 1H), 8.27 (d, *J* = 0.8 Hz, 1H), 7.95 (d, *J* = 8.1 Hz, 1H), 7.85 - 7.77 (m, 2H), 7.70 - 7.58 (m, 3H), 6.83 (d, *J* = 7.3 Hz, 1H), 6.71 (d, *J* = 8.1 Hz, 1H), 5.97 (s, 2H), 5.18 - 5.10 (m, 1H), 4.77 (dd, *J* = 15.5, 2.7 Hz, 1H), 4.62 (dd, *J* = 15.5, 5.4 Hz, 1H), 4.46 - 4.35 (m, 2H), 4.14 (dt, *J* = 9.1, 5.8 Hz, 1H), 2.94 (d, *J=* 10.6 Hz, 1H), 2.66 - 2.51 (m, 4H), 2.34 - 2.17 (m, 2H), 1.85 - 1.62 (m, 3H), 1.55 - 1.47 (m, 1H), 1.45 (d, *J* = 6.7 Hz, 3H).

### Example 38 2-((S)-1-(4-(6-((4-cyano-2-fluorobenzyl)amino)pyridin-2-yl)piperidin-1-yl)ethyl)-1-(((S)-oxetanyl-2-yl)methyl)-1-H-benzo[d]imidazol-6-carboaylic acid

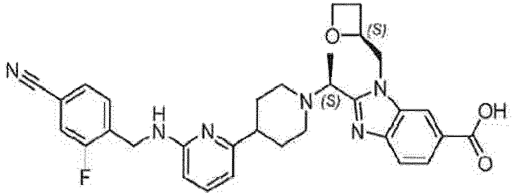

The specific synthesis route is as follows.

### Step A: Synthesis of tert-butyl 6-amino-3'6'-dihydro-[2,4'-bipyridin]-1'(2'H)-carboxylate

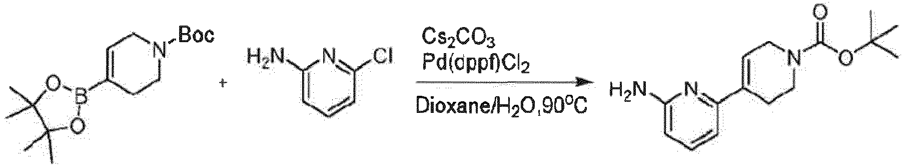

Tert-butyl 4-(4,4,5,5-tetramethyl-1,3,2-dioxaborolan-2-yl)-3,6-dihydropyridin-1(2H)-carboxylate (500.4 mg, 1.6 mmol), 2-amino-6-chloropyridine (420.3 mg, 3.2 mmol), [1,1'-bis(diphenylphosphino)ferrocene]dichloropalladium (146.3 mg, 0.2 mmol) and cesium carbonate (1.2 g, 3.5 mmol) were added into a solvent mixture of 1,4-dioxane (30 mL) and water (6 mL) at room temperature. Under nitrogen protection, the mixed solution was heated to 90°C and refluxed for 24 h.

After the reaction was completed, the reaction solution was filtered through diatomite, and the filtrate was concentrated under reduced pressure. It was diluted by adding water (100 mL), extracted with ethyl acetate (50 mL × 3 times), then washed with saturated saline (50 mL × 3 times), dried with anhydrous sodium sulfate, filtered and concentrated under reduced pressure. The residue was purified by silica gel column chromatography (eluent: petroleum ether/ethyl acetate = 6/1) to obtain 310.7 mg of yellow oily product, tert-butyl 6-amino-3'6'-dihydro-[2,4'-bipyridin]-1'(2'H)-carboxylate. (yield: 69.6%).

### Step B: Synthesis of tert-butyl 4-(6-aminopyridin-2-yl)piperidin-1-carboxylate

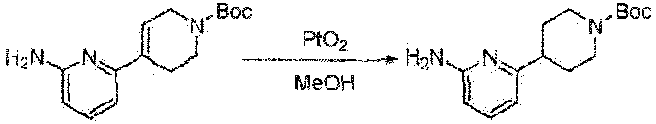

Tert-butyl 6-amino-3'6'-dihydro-[2,4'-bipyridin]-1'(2'H)-carboxylate (310.2 mg, 1.1 mmol) was dissolved in methanol (10 mL) at room temperature, and platinum dioxide was added (12.5 mg, 55.0 mmol). Hydrogen replacement was performed for three times, and the reaction was carried out for 24 h under hydrogen protection. After the reaction was completed, the reaction solution was filtered through diatomite and the filtrate was concentrated under reduced pressure. The residue was purified by silica gel column chromatography (eluent: petroleum ether/ethyl acetate = 1/1) to obtain 103.5 mg of white oily product tert-butyl 4-(6-aminopyridin-2-yl)piperidin-1-carboxylate (yield: 33.0%). LC-MS: RT = 3.25 min, [M+H]⁺ = 278.21.

### Step C: Synthesis of tert-butyl 4-(6-((4-cyano-2-fluorobenzyl)amino)pyridin-2-yl)piperidin-1-carboxylate

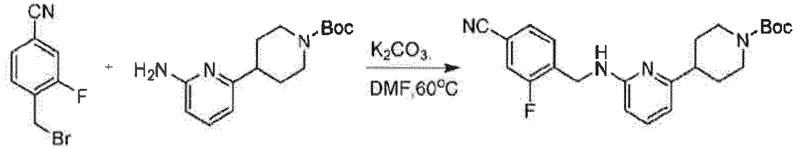

Tert-butyl 4-(6-chloropyridin-2-yl)piperidin-1-carboxylate (103.2 mg, 371.4 mmol), 4-(bromomethyl)-3-fluorobenzonitrile (159.02 mg, 742.8 µmol) and potassium carbonate (151.8 mg, 1.1 mmol) were dissolved in *N,N-*dimethylformamide (3 mL) at room temperature. The mixed solution was heated to 60°C and the reaction was carried out for 6 h.

After the reaction was completed, the reaction solution was diluted by adding water (20 mL), extracted with ethyl acetate (10 mL × 3 times), then washed with saturated saline (15 mL × 3 times), dried with anhydrous sodium sulfate, filtered and concentrated under reduced pressure. The residue was purified by silica gel column chromatography (eluent: petroleum ether/ethyl acetate = 5/1) to obtain 101.2 mg of yellow oily product tert-butyl 4-(6-((4-cyano-2-fluorobenzyl)amino)pyridin-2-yl)piperidin-1-carboxylate (yield: 66.4%). LC-MS: RT = 4.07 min, [M+H]⁺ = 433.25.

### Step D: Synthesis of 3-fluoro-4-(((6-(piperidin-4-yl)pyridin-2-yl)amino)methyl)benzonitrile

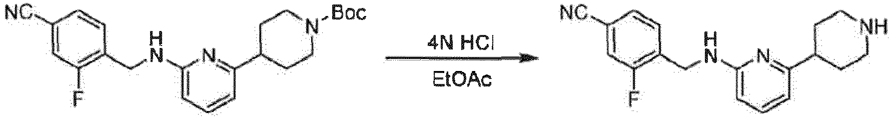

Tert-butyl 4-(6-((4-cyano-2-fluorobenzyl)amino)pyridin-2-yl)piperidin-1-carboxylate (101.3 mg, 246.0 µmol) was dissolved in ethyl acetate hydrochloride solution (5 mL) with a concentration of 4 mol/L under room temperature, and the mixed solution was stirred for 1h to gradually precipitate a white solid. After the reaction was completed, the reaction solution was concentrated under reduced pressure, diluted by adding water (20 mL), added with saturated sodium carbonate aqueous solution dropwise to adjust the mixed solution to be alkaline. The water layer was extracted with ethyl acetate (10 mL × 3 times), then washed with saturated saline (15 mL × 3 times), dried with anhydrous sodium sulfate, filtered and concentrated under reduced pressure. 76.4 mg of white powder, 3-fluoro-4-(((6-(piperidin-4-yl)pyridin-2-yl)amino)methyl)benzonitrile, was obtained, which was directly used in the next reaction without purification. LC-MS: RT = 2.69 min, [M+H]⁺ = 311.04.

### Step E: Synthesis of methyl 2-((S)-1-(4-(6-((4-cyano-2-fluorobenzyl)amino)pyridin-2-yl)piperidin-1-yl)ethyl)-1-(((S)-oxetan-2-yl)methyl)-1H-benzo[d]imidazol-6-carboacylate

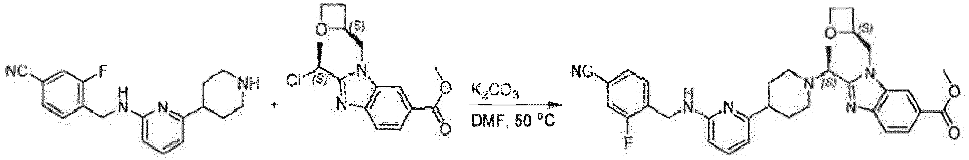

3-Fluoro-4-(((6-(piperidin-4-yl)pyridin-2-yl)amino)methyl)benzonitrile (76.1 mg, 244.7 µmol), methyl 2-((S)-1-chloroethyl)-1-(((S)-oxetan-2-yl)methyl)-1H-benzo[d]imidazol-6-formate (83.2 mg, 269.2 µmol), and potassium carbonate (170.3 mg, 1.2 mmol) were dissolved in *N,N-*dimethylformamide (10 mL) at room temperature, and the reaction was carried out at 50°C for 16 h.

After the reaction was completed, the reaction solution was diluted by adding water (20 mL), extracted with ethyl acetate (50 mL × 3 times), then washed with saturated saline (20 mL × 3 times), dried with anhydrous sodium sulfate, filtered and concentrated under reduced pressure. The residue was purified by silica gel column chromatography (eluent: petroleum ether/ethyl acetate = 1/ 1 to 0/1) to obtain 102.9 mg of methyl 2-((S)-1-(4-(6-((4-cyano-2-fluorobenzyl)amino)pyridin-2-yl)piperidin-1-yl)ethyl)-1-(((S)-oxetan-2-yl)methyl)-1H-benzo[*d*]imidazol-6-carboxylate (yield: 72.1%). LC-MS: RT = 1.97 min, [M+H]⁺ = 583.73.

### Step F: Synthesis of 2-((S)-1-(4-(6-((4-cyano-2-fluorobenzyl)amino)pyridin-2-yl)piperidin-1-yl)ethyl)-1-(((S)-oxetan-2-yl)methyl)-1H-benzo[d]imidazol-6-carboxylic acid

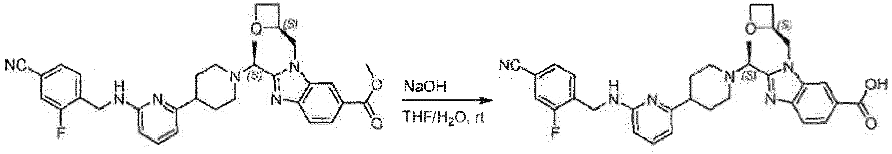

Methyl 2-((S)-1 -(4-(6-((4-cyano-2-fluorobenzyl)amino)pyridin-2-yl)piperidin-1-yl)ethyl)-1-(((S)-oxetan-2-yl)methyl)-1-*H*-benzo[*d*]imidazol-6-carboxylate (102.9 mg, 171.8 µmol) was dissolved in a mixture of tetrahydrofuran (10 mL), ethanol (3 mL) and water (3 mL) at room temperature. Then sodium hydroxide (76.0 mg, 1.9 mmol) was added, and the reaction was carried out at room temperature for 16 h.

After the reaction was completed, aqueous citric acid solution with a concentration of 1 mol/L was added dropwise until the pH was 5. The reaction solution was diluted by adding water (20 mL), extracted with ethyl acetate (25 mL × 3 times), washed with saturated saline (25 mL × 3 times), dried with anhydrous sodium sulfate, filtered, and concentrated under reduced pressure. The resulting residue was purified by semi-preparative HPLC (mobile phase: water containing 0.1% ammonia/acetonitrile = 90/10 to 40/60) to obtain 20.5 mg of white solid 2-((S)-1-(4-(6-((4-cyano-2-fluorobenzyl)amino)pyridin-2-yl)piperidin-1-yl)ethyl)-1-(((S)-oxetan-2-yl)methyl)-1-*H-*benzo[*d*]imidazol-6-carboxylic acid (yield: 20.4%). LC-MS: RT = 1.08 min, [M+H]⁺ = 569.60.

### Example 39 2-((S)-1-(4-(6-((4-chloro-2-fluorobenzyl)oxy)pyridin-2-yl)piperazin-1-yl)ethyl-1-(((S)-oxetan-2-yl)methyl)-1H-benzo[d]imidazol-6-carboxylic acid (39A) and 2-((R)-1-(4-(6-((4-chloro-2-fluorobenzyl)oxy)pyridin-2-yl)piperazin-1-yl)ethyl-1-(((S)-oxetan-2-yl)methyl)-1H-benzo[d]imidazol-6-carboxylic acid (39B)

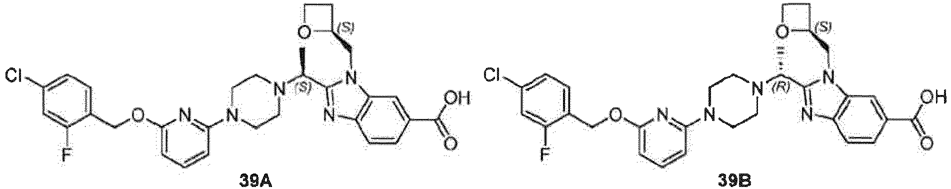

The specific synthesis route is as follows.

### Step A: Synthesis of 2-chloro-6-((4-chloro-2-fluorobenzyl)oxy)pyridine

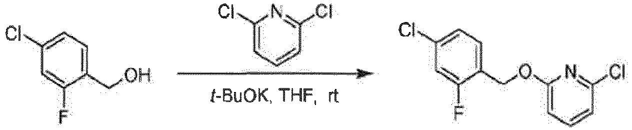

Potassium tert butoxide (4.88 g, 43.60 mmol) was added into a solution of tetrahydrofuran (50.0 mL) containing (4-chloro-2-fluorophenyl)methanol (5.0 g, 31.14 mmol) at 0°C, and the mixed solution was stirred for 30 min. Then 2,6-dichloropyridine (4.2 g, 28.38 mmol) was added, and the reaction was carried out overnight at 25°C.

After the reaction was completed, the reaction solution was quenched by adding water (50 mL) and extracted with ethyl acetate (50 mL × 3 times). The organic phases were combined, then washed with saturated saline (50 mL × 3 times), dried with anhydrous sodium sulfate, and concentrated under reduced pressure. The residue was purified by silica gel column chromatography (eluent: ethyl acetate/n-hexane = 1/2). 7.53 g of yellow oily product 2-chloro-6-((4-chloro-2-fluorobenzyl)oxy)pyridine was obtained (yield: 88.9%). LC-MS: RT = 2.27 min, [M+H]⁺ = 272.05.

### Step B: Synthesis of tert-butyl 4-(6-((4-chloro-2-fluorobenzyl)oxy)pyridin-2-yl)piperazin-1-carboxylate

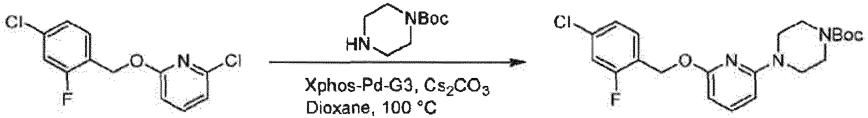

Tert-butyl piperazin-1-carboxylate (5.15 g, 27.68 mmol), cesium carbonate (18.05 g, 55.36 mmol) and methanesulfonato(2-dicyclohexylphosphino-2',4',6'-triisopropyl-1,1 '-biphenyl)(2'-amino-1,1'-biphenyl-2-yl)palladium (II) (702.5 mg, 0.83 mmol) were added into a solution of 1,4-dioxane containing 2-chloro-6-((4-chloro-2-fluorobenzyl)oxy)pyridine (7.53 g, 27.68 mmol) at 25°C, and the reaction was carried out at 100°C for 3 h.

After the reaction was completed, the reaction solution was quenched by adding water and extracted with ethyl acetate (50 mL × 3 times). The organic phases were combined, then washed with saturated saline (50 mL × 2 times), dried with anhydrous sodium sulfate, and concentrated under reduced pressure. The residue was purified by silica gel column chromatography (eluent: ethyl acetate/n-hexane = 1/2). 4.7 g of white solid, tert-butyl 4-(6-((4-chloro-2-fluorobenzyl)oxy)pyridin-2-yl)piperazin-1-carboxylate, was obtained (yield: 40.2%). LC-MS: RT = 2.39 min, [M+H]⁺ = 422.22.

### Step C: Synthesis of 4-(6-((4-chloro-2-fluorobenzyl)oxy)pyridin-2-yl)piperazine

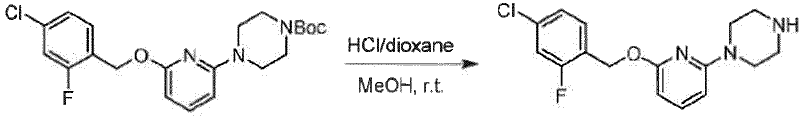

Hydrochloric acid in 1,4-dioxane (20.0 mL, 4 mol/L) was added into a solution of methanol (30 mL) containing tert-butyl 4-(6-((4-chloro-2-fluorobenzyl)oxy)pyridin-2-yl)piperazin-1-carboxylate (4.34 g, 10.28 mmol), and the reaction was carried out at room temperature for 3 h.

After the reaction was completed, the reaction solution was directly spin-dried to obtain 3.68 g of white solid 4-(6-((4-chloro-2-fluorobenzyl)oxy)pyridin-2-yl)piperazine, which was directly used in the next step. LC-MS: RT = 1.65 min, [M+H]⁺ = 312.26.

### Step D: Synthesis of methyl 2-(1-(4-(6-((4-chloro-2-fluorobenzyl)oxy)pyridin-2-yl)piperazin-1-yl)ethyl)-1-(((S)-oxetan-2-yl)methyl)-1H-benzo[d]imidazol-6-carboxylate

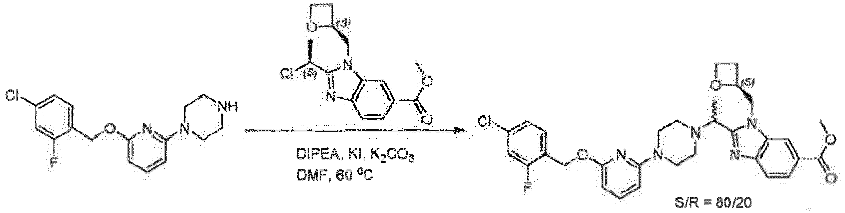

*N,N-*diisopropylethylamine (1.0 mL), potassium iodide (420 mg, 3.13 mmol), potassium carbonate (577 mg, 4.18 mmol), *N,N-*dimethylformamide (10.0 mL) were added into a solution of acetonitrile (8.0 mL) containing 4-(6-((4-chloro-2-fluorobenzyl)oxy)pyridin-2-yl)piperazine (750 mg, 2.09 mmol). Then methyl 2-((S)-1-chloroethyl)-1-(((S)-oxetan-2-yl)methyl)-1*H*-benzo[*d*]imidazol-6-carboxylate (650 mg, 2.09 mmol) was added, and the reaction was carried out at 60°C for 3 h under N₂ protection.

After the reaction was completed, the reaction solution was quenched by adding water and extracted with ethyl acetate (30 mL × 2 times). The organic phases were combined, then washed with saturated saline (30 mL), dried with anhydrous sodium sulfate, and concentrated under reduced pressure. The residue was purified by silica gel column chromatography (eluent: ethyl acetate/n-hexane = 2/1). 770 mg of yellow solid, methyl 2-(1-(4-(6-((4-chloro-2-fluorobenzyl)oxy)pyridin-2-yl)piperazin-1-yl)ethyl)-1-(((S)-oxetan-2-yl)methyl)-1*H*-benzo[*d*]imidazol-6-carboxylate, was obtained (yield: 62.1%, *dr* = 60%). LC-MS: RT = 1.92 min, [M+H]⁺ = 594.26.

### Step E: Synthesis of 2-((S)-1-(4-(6-((4-chloro-2-fluorobenzyl)oxy)pyridin-2-yl)piperazin-1-yl)ethyl-1-(((S)-oxetan-2-yl)methyl)-1H-benzo[d]imidazol-6-carboxylic acid (39A) and 2-((R)-1-(4-(6-((4-chloro-2-fluorobenzyl)oxy)pyridin-2-yl)piperazin-1-yl)ethyl-1-(((S)-oxetan-2-yl)methyl)-1H-benzo[dlimidazol-6-carboxylic acid (39B)

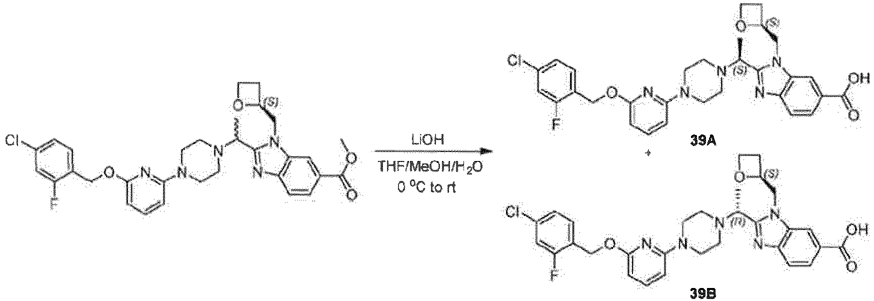

Lithium hydroxide (307 mg, 7.30 mmol) in water (1 mL) was added dropwise into a mixed solution of tetrahydrofuran (3 mL) and methanol (1 mL) containing methyl 2-(1-(4-(6-((4-chloro-2-fluorobenzyl)oxy)pyridin-2-yl)piperidin-1-yl)ethyl)-1-(((S)-oxetan-2-yl)methyl)-1*H*-benzo[*d*]imidazol-6-carboxylate (770 mg, 1.30 mmol) at 0°C, and the reaction was carried out at 25°C for 30 min.

After the reaction was completed, the reaction solution was quenched by adding water and extracted with ethyl acetate (30 mL × 2 times). The organic phases were combined, then washed with saturated saline (20 mL), dried with anhydrous sodium sulfate, and concentrated under reduced pressure. The residue was purified by silica gel column chromatography (eluent: methanol/dichloromethane = 1/10). 313 mg of white solid 2-((S)-1-(4-(6-((4-chloro-2-fluorobenzyl)oxy)pyridin-2-yl)piperazin-1-yl)ethyl-1-(((S)-oxetan-2-yl)methyl)-1*H*-benzo[d*]*imidazol-6-carboxylic acid (**39A**) (yield: 41.6%) and 78 mg of white solid 2-((R)-1-(4-(6-((4-chloro-2-fluorobenzyl)oxy)pyridin-2-yl)piperazin-1-yl)ethyl-1-(((S)-oxetan-2-yl)methyl)-1*H-*benzo[*d*]imidazol-6-carboxylic acid (**39B**) (yield: 10.4%) were obtained. The crude products were purified by preparative high performance liquid chromatography. The separation conditions are as follows: chromatographic column: Agilent 5 Prep-C18 100 mm×30 mm 5 µM; mobile phase: water (containing 0.1% ammonia) and acetonitrile elution; flow rate: 20 mL/min; gradient: 25% acetonitrile, 39A and 39B were eluted at 15.23 min and 17.06 min, respectively; detection wavelength: 254 nm.

Compound 39A: HPLC: RT = 15.23 min. LC-MS: RT = 1.88 min, [M+H]⁺ = 580.26. ¹H NMR (400 MHz, DMSO-d₆) δ 8.26 (s, 1H), 7.80 (dd, *J* = 8.4, 1.5 Hz, 1H), 7.66 (d, *J* = 8.4 Hz, 1H), 7.49 (t, *J* = 8.2 Hz, 1H), 7.44 (dt, *J* = 16.0, 4.9 Hz, 2H), 7.28 (dd, *J=* 8.2, 1.8 Hz, 1H), 6.28 (d, *J* = 8.1 Hz, 1H), 6.06 (d, *J* = 7.8 Hz, 1H), 5.29 (d, *J* = 8.8 Hz, 2H), 5.14 (d, *J=* 3.0 Hz, 1H), 4.80 (dd, *J* = 15.5, 2.9 Hz, 1H), 4.68 (dd, *J* = 15.5, 5.5 Hz, 1H), 4.52-4.40 (m, 2H), 4.18 (dt, *J* = 9.1, 5.7 Hz, 1H), 3.40 (d, .1 = 5.3 Hz, 4H), 2.68-2.59 (m, 1H), 2.54 (d, *J=* 16.1 Hz, 4H), 2.38-2.26 (m, 1H), 1.45 (d, *J=* 6.7 Hz, 3H).

The single crystal structure of compound 39A is shown in figure 1, and the specific crystal parameters are as follows:

| **Crystal system** | **orthorhombic** |
|---|---|
| **Space group** | P2₁2₁2₁ |
| **a/Å** | 6.8179(3) |
| **b/Å** | 20.0543(12) |
| **c/Å** | 20.3816(9) |
| **α/°** | 90 |
| **β/°** | 90 |
| **γ/°** | 90 |
| **Volume/Å³** | 2786.7(2) |
| **Z** | 4 |
| **ρ_{calc}g/cm³** | 1.3825 |
| **µ/mm⁻¹** | 0.190 |
| **F(000)** | 1217.2 |
| **Crystal size/mm³** | 0_{.}205 × 0.182 × 0.097 |
| **Radiation** | Mo Kα (λ = 0.71073) |
| **2Θ range for data collection/°** | 4.06 to 62 |
| **Index ranges** | -9 < h ≤8. -28 < k ≤ 29, -29 < 1 < 22 |
| **Reflections collected** | 25423 |
| **Independent reflections** | 8847 [Rᵢₙₜ = 0.0434, R_{sigma} = 0.0632] |
| **Data/restraints/parameters** | 8847/0/355 |
| **Goodness-of-fit on F²** | 1.227 |
| **Final R indexes [I>=2σ (I)]** | R₁ = 0.0578, wR₂ = 0.1306 |
| **Final R indexes [all data]** | R₁ = 0.1173, wR₂ = 0.1546 |
| **Largest diff. peak/hole / e Å⁻³** | 0.60/-0.62 |
| **Flack parameter** | -0.11(10) |

Compound 39B: HPLC: RT = 17.06 min. LC-MS: RT = 1.88 min, [M+H]⁺ = 580.26. ¹H NMR (400 MHz, DMSO-d₆) δ12.74 (s, 1H), 8.27-8.26 (m, 1H), 7.79 (dd, *J=* 9.9, 1.4 Hz, 1H), 7.66 (dd, *J* = 8.4, 1.5 Hz, 1H), 7.50 (t, J= 8.0 Hz, 1H), 7.46-7.42 (m, 2H), 7.29-7.27 (m, 1H), 6.30 (d, *J=* 8.2 Hz, 1H), 6.07 (d, *J=* 7.8 Hz, 1H), 5.29 (s, 2H), 4.93-4.87 (m, 1H), 4.60-4.55 (m, 1H), 4.52-4.47 (m, 3H), 3.47-3.38 (m, 5H), 2.79-2.72 (m, 1H), 2.64-2.61 (m, 2H), 2.56-2.47 (m, 2H), 1.42 (d, *J=* 6.7 Hz, 3H).

### Example 40 2-(1-(1-(6-((4-cyano-2-fluorobenzyl)oxy)pyridin-2-yl)piperidin-4-yl)ethyl)-1-(((S)-oxetan-2-yl)methyl)-1H-benzo[d]imidazol-6-carboxylic acid

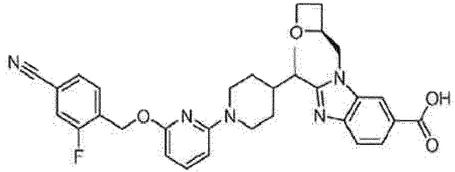

The specific synthesis route is as follows.

### Step A: Synthesis of ethyl 2-(piperidin-4-yl)propionate

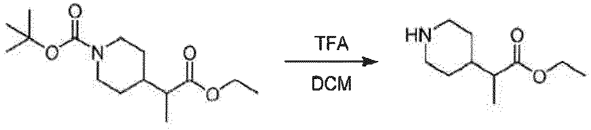

Tert butyl 4-(1-ethoxy-1-oxypropan-2-yl)piperidin-1-formate (520 mg, 1.82 mmol) was dissolved in 4 mL of anhydrous dichloromethane, and was added with 2 mL of trifluoroacetic acid. The reaction was carried out at room temperature for 40 min. After the reaction was completed, the reaction solution was poured into 50 mL of sodium bicarbonate solution, and the mixed solution was extracted with ethyl acetate (30 mL × 2 times). The organic phases were combined, then washed with saturated saline (20 mL × 2 times), dried with anhydrous sodium sulfate, and concentrated under reduced pressure to obtain 310 mg of ethyl 2-(piperidin-4-yl)propionate (yield: 92.1%).

### Step B: Synthesis of ethyl 2-(1-(6-chloropyridin-2-yl)piperidin-4-yl)propionate

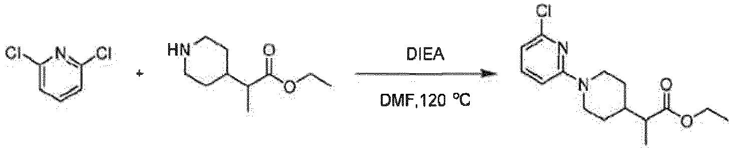

*N,N-*Diisopropylethylamine (432 mg, 3.35 mmol) was added into a solution of *N,N-*dimethylformamide (10 mL) containing ethyl 2-(piperidin-4-yl)propionate (310 mL, 1.67 mmol), and the mixed solution was stirred for 2 min. Then 2,6-dichloropyridine (245 mg, 1.67 mmol) was added and the reaction was carried out at 120°C for 3 h.

After the reaction was completed, the reaction solution was quenched by adding water (50 mL) and extracted with ethyl acetate (50 mL × 3 times). The organic phases were combined, then washed with saturated saline (50 mL), dried with anhydrous sodium sulfate, and concentrated under reduced pressure. The residue was purified by silica gel column chromatography (eluent: ethyl acetate/n-hexane = 1/2). 422 mg of pale yellow solid, ethyl 2-(1-(6-chloropyridin-2-yl)piperidin-4-yl)propionate, was obtained (yield: 85.1%). LC-MS: RT = 2.21 min, [M+H]⁺= 297.23.

### Step C: Synthesis of 2-(1-(6-chloropyridin-2-yl)piperidin-4-yl)propionic acid

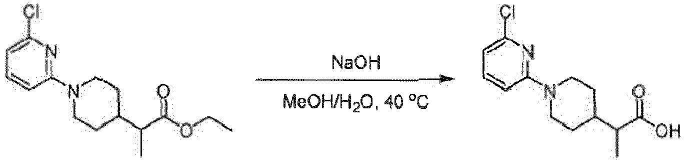

Ethyl 2-(1-(6-chloropyridin-2-yl)piperidin-4-yl)propionate (422 mg, 1.42 mmol) was dissolved in 10 mL solvent mixture of methanol and water, and solid sodium hydroxide (142 mg, 3.55 mmol) was added. Then the reaction was carried out at 40°C for 3 h.

After the reaction was completed, the reaction solution was neutralized with diluted hydrochloric acid until the pH is about 6 and extracted with ethyl acetate (50 mL × 3 times). The organic phases were combined, then washed with saturated saline (50 mL), dried with anhydrous sodium sulfate, and concentrated under reduced pressure to obtain 264 mg of 2-(1-(6-chloropyridin-2-yl)piperidin-4-yl)propionic acid (yield: 69.1%). LC-MS: RT= 1.94 min, [M+H]⁺ = 269.11.

### Step D: Synthesis of methyl 4-(2-(1-(6-chloropyridin-2-yl)piperidin-4-yl)propionamido)-3-((((S)-oxetan-2-yl)methyl)amino)benzoate

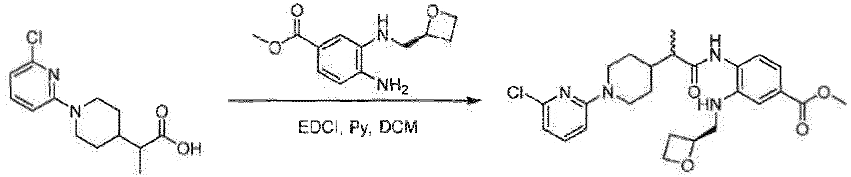

Pyridine (141 mg, 1.78 mmol) was added into a solution of dichloromethane (10.0 mL) containing 2-(1-(6-chloropyridin-2-yl)piperidin-4-yl)propionic acid (240 mg, 0.892 mmol) and 1-(3-dimethylaminopropyl)-3-ethylcarbodiimide (255 mg, 1.34 mmol), and the mixed solution was stirred for 10 min. Then methyl (S)-4-amino-3-(((oxetan-2-yl)methyl)amino)benzoate (211 mg, 0.892 mmol) was added and the reaction was carried out at room temperature for 3 h.

After the reaction was completed, the reaction solution was quenched by adding water (50 mL) and extracted with dichloromethane (50 mL × 3 times). The organic phases were combined, dried with anhydrous sodium sulfate, and concentrated under reduced pressure. The residue was purified by silica gel column chromatography (eluent: ethyl acetate/n-hexane = 1/1). 166 mg of pale yellow solid methyl 4-(2-(1-(6-chloropyridin-2-yl)piperidin-4-yl)propionamido)-3-((((S)-oxetan-2-yl)methyl)amino)benzoate was obtained (yield: 38.2%). LC-MS: RT = 1.98 min, [M+H]⁺= 487.23.

### Step E: Synthesis of methyl 2-(1-(1-(6--chloropyridin-2-yl)piperidin-4-yl)ethyl)-1-(((S)-oxetan-2-yl)methyl)-1H-benzo[d]imidazol-6-carboxylate

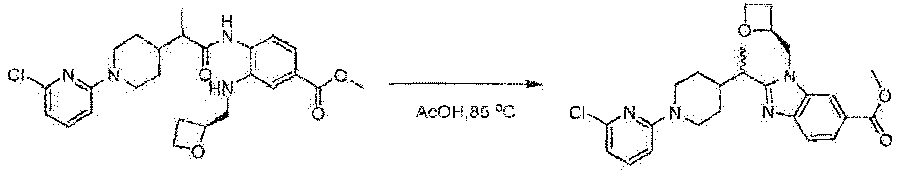

Methyl 4-(2-(1-(6-chloropyridin-2-yl)piperidin-4-yl)propionam ido)-3-((((S)-oxetan-2-yl)methyl)amino)benzoate (166 mg, 0.341 mmol) was dissolved in 5 mL of acetic acid, and the reaction was carried out at 85°C for 12 h.

After the reaction was completed, the reaction solution was neutralized with saturated sodium bicarbonate solution until the pH was about 7 and extracted with ethyl acetate (50 mL × 3 times). The organic phases were combined, then washed with saturated saline (50 mL), dried with anhydrous sodium sulfate, and concentrated under reduced pressure to obtain 86 mg of methyl 2-(1-(1-(6-chloropyridin-2-yl)piperidin-4-yl)ethyl)-1-(((S)-oxetan-2-yl)methyl)-1*H*-benzo[*d*]imidazol-6-carboxylate (yield: 53.7%). LC-MS: RT = 2.24 min, [M+H]⁺= 469.23.

### Step F: Synthesis of methyl 2-(1-(1-(6-((4-cyano-2-fluorobenzyl)oxy)pyridin-2-yl)piperidin-4-yl)ethyl)-1-(((S)-oxetan-2-yl)methyl)-1H-benzo[d]imidazol-6-carboxylate

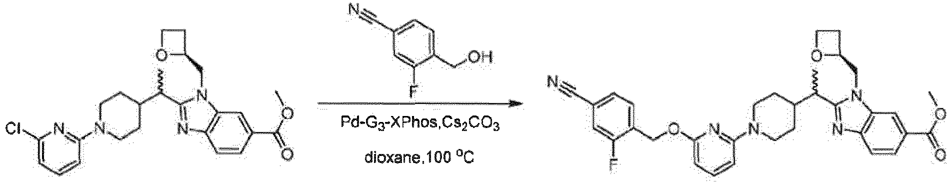

Cesium carbonate (119 mg, 0.366 mmol) and methanesulfonato(2-dicyclohexylphosphino-2',4',6'-triisopropyl-1,1'-biphenyl)(2'-amino-1,1'-biphenyl-2-yl)palladium (II) (15.5 mg, 0.0183 mmol) were added into a solution of 1,4-dioxane (5 mL) containing methyl 2-(1-(1-(6-chloropyridin-2-yl)piperidin-4-yl)ethyl)-1-(((S)-oxetan-2-yl)methyl)-1*H*-benzo[*d*]imidazol-6-carboxylate (86.0 mg, 0.183 mmol) and 3-fluoro-4-(hydroxymethyl)benzonitrile (33.2 mg, 0.221 mmol) at room temperature, and the reaction was carried out at 100°C for 3 h.

After the reaction was completed, the reaction solution was quenched by adding water and extracted with ethyl acetate (20 mL × 3 times). The organic phases were combined, washed with saturated saline (30 mL × 2 times), dried with anhydrous sodium sulfate, and concentrated under reduced pressure. The residue was purified by silica gel column chromatography (eluent: ethyl acetate/n-hexane = 5/1). 52 mg of pale yellow solid, methyl 2-(1-(1-(6-((4-cyano-2-fluorobenzyl)oxy)pyridin-2-yl)piperidin-4-yl)ethyl)-1-(((S)-oxetan-2-yl)methyl)-1*H*-benzo[*d*]imidazol-6-carboxylate, was obtained (yield: 48.6%). LC-MS: RT = 2.12 min, [M+H]⁺= 584.27.

### Step G: Synthesis of 2-(1-(1-(6-((4-cyano-2-fluorobenzyl)oxy)pyridin-2-yl)piperidin-4-yl)ethyl)-1-(((S)-oxetan-2-yl)methyl)-1H-benzo[d]imidazol-6-carboxylic acid

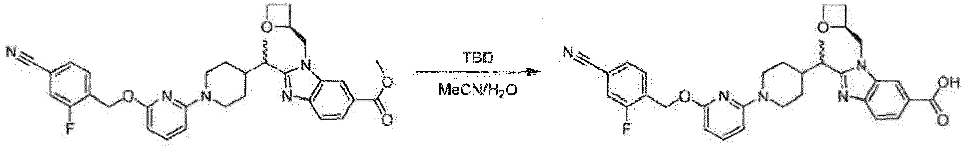

Methyl 2-(1-(1-(6-((4-cyano-2-fluorobenzyl)oxy)pyridin-2-yl)piperidin-4-yl)ethyl)-1-(((S)-oxetan-2-yl)methyl)-1*H*-benzo[*d*]imidazol-6-carboxylate (52.0 mg, 0.089 mmol) was dissolved in 4 mL of acetonitrile, then 1,5,7-triazabicyclo[4.4.0]dec-5-ene (25 mg, 0.187 mmol) and 1 mL water were added, and the reaction was carried out at room temperature for 7 h.

After the reaction was completed, the reaction solution was neutralized by adding 20 mL of saturated ammonium chloride solution and extracted with dichloromethane (30 mL × 2 times). The organic phases were combined, then washed with saturated saline (20 mL × 2 times), dried with anhydrous sodium sulfate, and concentrated under reduced pressure. The obtained crude product was separated by prep-HPLC (ammonia) to obtain 22 mg of white solid 2-(1-(1-(6-((4-cyano-2-fluorobenzyl)oxy)pyridin-2-yl)piperidin-4-yl)ethyl)-1-(((S)-oxetan-2-yl)methyl)-1*H*-benzo[*d*]imidazol-6-carboxylic acid (yield: 43.4%). ¹H NMR (400 MHz, DMSO) δ 12.71 (s, 1H), 8.20 (dd, *J* = 6.1, 1.3 Hz, 1H), 7.87-7.90 (m, 1H), 7.79 (dd, *J* = 8.4, 1.6 Hz, 1H), 7.68-7.71 (m, 1H), 7.59-7.66 (m, 2H), 7.41-7.45 (m, 1H), 6.30 (dd, *J* = 8.2, 4.8 Hz, 1H), 6.05-6.08 4(m, 1H), 5.38 (s, 2H), 4.95-5.05 (m, 1H), 4.44-4.55 (m, 2H), 4.25-4.28 (m, 1H), 4.06-4.13 (m, 1H), 3.06-3.15 (m, 2H), 2.58-2.77 (m, 5H), 2.32-2.38 (m, 1H),1.96-2.10 (m, 2H), 1.80-1.95 (m, 2H), 1.28 (t, *J=* 6.7 Hz, 3H). LC-MS: RT = 1.97 min, [M+H]⁺ = 570.29.

### Example 41 2-((S)-1-(4-(6-((4-cyano-2-fluorobenzyl)ooy)-4-(methoxymethyl)pyridin-2-yl)piperazin-1-yl)ethyl)-1-(((S)-oxetan-2-yl)methyl)-1H-benzo[d]imidazol-6-carboxylic acid

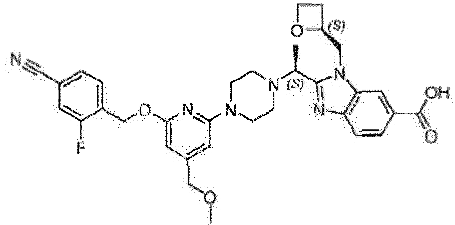

The specific synthesis route is as follows.

### Step A: Synthesis of 2,6-dichloro-4-(methoxymethyl)pyridine

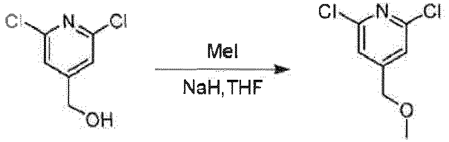

60% Sodium hydride (272 mg, 6.81 mmol) was added into a solution of tetrahydrofuran (10.0 mL) containing (2,6-dichloropyridin-4-yl)methanol (1.00 g, 5.68 mmol) at 0°C, and the mixed solution was stirred for 5 min. Then iodomethane (873 mg, 6.24 mmol) was added and the reaction was carried out at room temperature for 2 h.

After the reaction was completed, the reaction solution was quenched by adding water (50 mL) and extracted with ethyl acetate (50 mL × 3 times). The organic phases were combined, then washed with saturated saline (50 mL), dried with anhydrous sodium sulfate, and concentrated under reduced pressure. The residue was purified by silica gel column chromatography (eluent: ethyl acetate/n-hexane = 1/5). 670 mg of white solid, 2,6-dichloro-4-(methoxymethyl)pyridine, was obtained (yield: 61.4%). LC-MS: RT = 2.24 min, [M+H]⁺= 192.23.

### Step B: Synthesis of tert-butyl 4-(6-chloro-4-(methoxymethyl)pyridin-2-yl)piperazin-1-formate

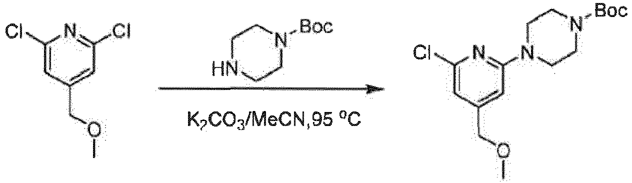

Potassium carbonate (960 mg, 6.96 mmol) was added into a solution of acetonitrile (10.0 mL) containing 2,6-dichloro-4-(methoxymethyl)pyridine (670 mg, 3.48 mmol) and tert-butyl piperazin-1-carboxylate (647 mg, 3.48 mmol), and the reaction was carried out at 95°C for 3 h.

After the reaction was completed, the reaction solution was quenched by adding water (50 mL) and extracted with ethyl acetate (50 mL × 3 times). The organic phases were combined, then washed with saturated saline (50 mL), dried with anhydrous sodium sulfate, and concentrated under reduced pressure. The residue was purified by silica gel column chromatography (eluent: ethyl acetate/n-hexane = 1/3). 746 mg of pale yellow solid, tert-butyl 4-(6-chloro-4-(methoxymethyl)pyridin-2-yl)piperazin-1-formate, was obtained (yield: 62.6%). LC-MS: RT = 2.18 min, [M+H]⁺ = 342.17.

### Step C: Synthesis of tert-butyl 4-(6-((4-cyano-2-fluorobenzyl)oxy)-4-(methoxymethyl)pyridin-2-yl)piperidin-1-formate

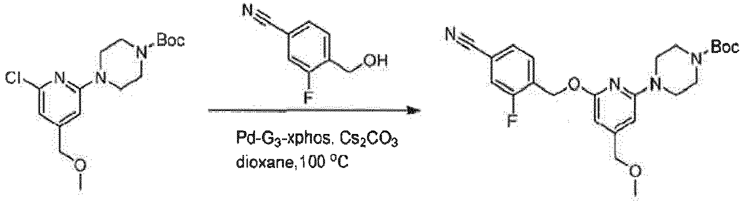

Cesium carbonate (477 mg, 1.46 mmol) and methanesulfonato(2-dicyclohexylphosphino-2',4',6'-triisopropyl-1,1'-biphenyl)(2'-amino-1,1'-biphenyl-2-yl)palladium (II) (62.1 mg, 0.0733 mmol) were added into a solution of 1,4-dioxane (5 mL) containing tert-butyl 4-(6-chloro-4-(methoxymethyl)pyridin-2-yl)piperazin-1-formate (250 mg, 0.733 mmol) and 3-fluoro-4-(hydroxymethyl)benzonitrile (122 mg, 0.806 mmol) at room temperature, and the reaction was carried out at 100°C for 3 h.

After the reaction was completed, the reaction solution was quenched by adding water and extracted with ethyl acetate (50 mL × 3 times). The organic phases were combined, then washed with saturated saline (50 mL × 2 times), dried with anhydrous sodium sulfate, and concentrated under reduced pressure. The residue was purified by silica gel column chromatography (eluent: ethyl acetate/n-hexane = 1/2). 320 mg of pale yellow solid, tert-butyl 4-(6-((4-cyano-2-fluorobenzyl)oxy)-4-(methoxymethyl)pyridin-2-yl)piperidin-1-formate, was obtained (yield: 69.5%). LC-MS: RT= 1.98 min, [M+H]⁺= 456.27.

### Step D: Synthesis of 3-fluoro-4-(((4-(methoxymethyl)-6-piperazin-1-yl)pyridin-2-yl)oxy)methyl)benzonitrile

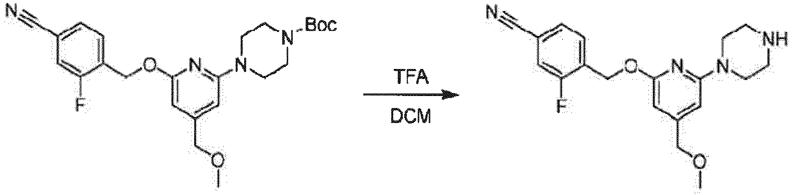

Tert butyl 4-(6-((4-cyano-2-fluorobenzyl)oxy)-4-(methoxymethyl)pyridin-2-yl)piperidin-1-formate (320 mg, 0.701 mmol) was dissolved in 4 mL of anhydrous dichloromethane, and 2 mL trifluoroacetic acid was added. The reaction was carried out at room temperature for 40 min.

After the reaction was completed, the reaction solution was poured into 50 mL of sodium bicarbonate solution and extracted with ethyl acetate (30 mL × 2 times). The organic phases were combined, then washed with saturated saline (20 mL × 2 times), dried with anhydrous sodium sulfate, and concentrated under reduced pressure to obtain 236 mg of 3-fluoro-4-(((4-(methoxymethyl)-6-piperazin-1-yl)pyridin-2-yl)oxy)methyl)benzonitrile (yield: 94.4%). LC-MS: RT= 1.83 min, [M+H]⁺= 356.27.

### Step E: Synthesis of methyl 2-((S)-1-(4-(6-((4-cyano-2-fluorobenzyl)oxy)-4-(methoxymethyl)pyridin-2-yl)piperazin-1-yl)ethyl)-1-(((S)-oxetan-2-yl)methyl)-1H benzo[d]imidazol-6-carboxylate

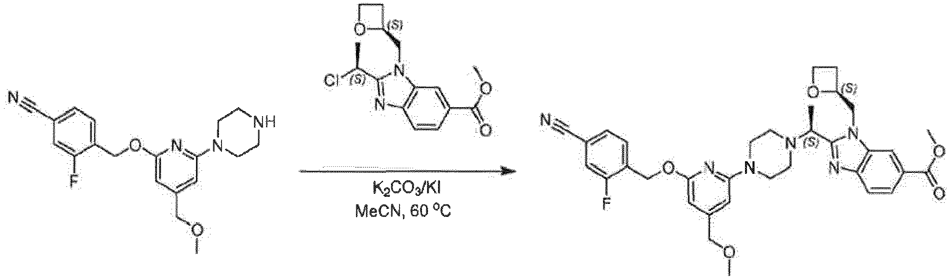

Methyl 2-((S)-1-chloroethyl)-1-(((S)-oxetan-2-yl)methyl)-1*H*-benzo[*d*]imidazol-6-carboxylate (204 mg, 0.662 mmol), potassium iodide (165 mg, 0.993 mmol), and potassium carbonate (183 mg, 1.32 mmol) were added into a solution of acetonitrile (8.0 mL) containing 3-fluoro-4-(((4-(methoxymethyl)-6-piperazin-1-yl)pyridin-2-yl)oxy)methyl)benzonitrile (236 mg, 0.662 mmol) at room temperature, and the reaction solution was carried out at 60°C for 5 h.

After the reaction was completed, the reaction solution was quenched by adding water and extracted with ethyl acetate (30 mL × 2 times). The organic phases were combined, then washed with saturated saline (30 mL), dried with anhydrous sodium sulfate, and concentrated under reduced pressure. The residue was purified by silica gel column chromatography (eluent: ethyl acetate/n-hexane = 5/1). 296 mg of pale yellow solid, methyl 2-((S)-1-(4-(6-((4-cyano-2-fluorobenzyl)oxy)-4-(methoxymethyl)pyridin-2-yl)piperazin-1-yl)ethyl)-1-(((S)-oxetan-2-yl)methyl)-1*H-*benzo[d]imidazol-6-carboxylate, was obtained (yield: 71.1%). LC-MS: RT= 1.89 min, [M+H]⁺= 629.27.

### Step F: Synthesis of 2-((S)-1-(4-(6-((4-cyano-2-fluorobenzyl)oxy)-4-(methoxymethyl)pyridin-2-yl)piperazin-1-yl)ethyl)-1-(((S)-oxetan-2-yl)methyl)-1H-benzo[d]imidazol-6-carboaylic acid

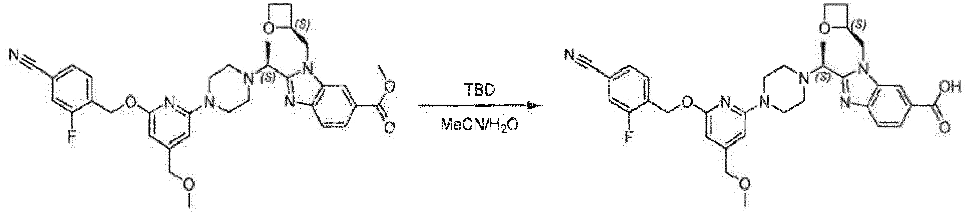

Methyl 2-((S)-1-(4-(6-((4-cyano-2-fluorobenzyl)oxy)-4-(methoxymethyl)pyridin-2-yl)piperazin-1 - yl)ethyl)-1-(((S)-oxetan-2-yl)methyl)-1*H*-benzo[*d*]imidazol-6-carboxylate (120 mg, 0.191 mmol) was dissolved in 4 mL of acetonitrile, then 1,5,7-triazabicyclo[4.4.0]dec-5-ene (55.7 mg, 0.401 mmol) and 1 mL water were added, and the reaction was carried out at room temperature for 7 h.

After the reaction was completed, the reaction solution was neutralized by adding 20 mL saturated ammonium chloride solution and extracted with dichloromethane (30 mL × 2 times). The organic phases were combined, then washed with saturated saline (20 mL × 2 times), dried with anhydrous sodium sulfate, and concentrated under reduced pressure. The obtained crude product was separated by column chromatography to obtain 36 mg of white solid 2-((S)-1-(4-(6-((4-cyano-2-fluorobenzyl)oxy)-4-(methoxymethy))pyridin-2-yl)piperazin-1-yl)ethyl)-1-(((S)-oxetan-2-yl)methyl)-1*H*-benzo[d]imidazol-6-carboxylic acid (yield: 30.7%). ¹H NMR (400 MHz, DMSO) δ 12.77 (s, 1H), 8.28 (d, *J* = 0.9 Hz, 1H), 7.85-7.91 (m, 1H), 7.81 (dd, *J* = 8.4, 1.5 Hz, 1H), 7.67-7.72 (m, 2H), 7.60-7.66 (m, 1H), 6.23 (s, 1H), 6.05 (s, 1H), 5.39 (s, 2H), 5.12-5.19 (m, 1H), 4.80 (dd, *J* = 16.2, 3.7 Hz, 1H), 4.65-4.73 (m, 1H), 4.42-4.52 (m, 2H), 4.29 (s, 2H), 4.14-4.23 (m, 1H), 3.27 (s, 3H), 3.14-3.23 (m, 2H), 2.60-2.68 (m, 2H), 2.52-2.56 (m, 3H), 2.30-2.36(m, 1H), 1.81-1.96 (m, 2H), 1.44 (d, *J* = 6.7 Hz, 3H). LC-MS: RT = 1.79 min, [M+H]⁺= 615.38.

### Example 42 2-((S)-1-(4-(4-((4-cyano-2-fluorobenzyl)oxy)-5-fluoropyrimidin-2-yl)piperazin-1-yl)ethyl)-1-(((S)-oxetan-2-yl)methyl)-1H-benzo[d]imidazol-6-carboxylic acid

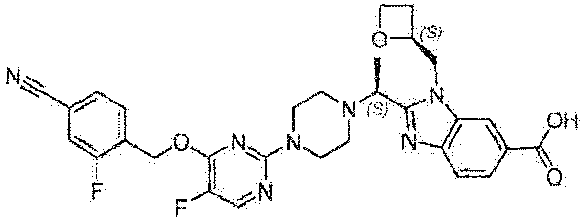

The specific synthesis route is as follows.

### Step A: Synthesis of 4-(((2-chloro-5-fluoropyrimidin-4-yl)oxy)methyl)-3-fluorobenzonitrile

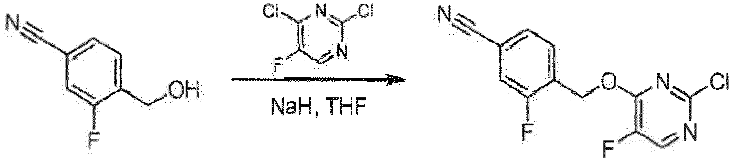

(4-Cyano-2-fluorophenyl)methanol (2 g, 13.2 mmol) was dissolved in tetrahydrofuran (100 mL). The mixed solution was cooled to 0°C, and then sodium hydride (0.8 g, 19.5 mmol) was added. The mixed solution was stirred at room temperature for 0.5 h, follow by the addition of 2,4-dichloro-5-fluoropyrimidine (3.3 g, 71 mmol). The reaction was carried out overnight at room temperature.

After the reaction was completed, the reaction solution was quenched by adding saturated sodium chloride solution, extracted with ethyl acetate (30 mL), washed twice with water, dried with anhydrous sodium sulfate, and concentrated under reduced pressure. The obtained crude product was purified by silica gel column chromatography (eluent: ethyl acetate/n-hexane = 1/10) to obtain 2 g of white solid 4-(((2-chloro-5-fluoropyrimidin-4-yl)oxy)methyl)-3-fluorobenzonitrile (yield: 53%). LC-MS: RT = 2.12 min, [M+H]⁺= 282.23.

### Step B: Synthesis of tert-butyl 4-(4-(((4-cyano-2-fluorobenzyl)oxy)-5-fluoropyrimidin-2-yl)piperazin)-1-formate

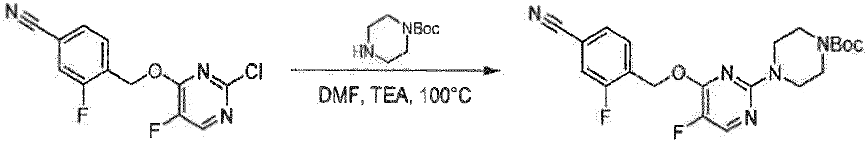

4-(((2-Cyano-5-fluoropyrimidin-4-yl)oxy)methyl)-3-fluorobenzonitrile (1 g, 3.5 mmol) was dissolved in tetrahydrofuran (50 mL), then tert-butyl piperazin-1-carboxylate (1 g, 5.3 mmol) was added, and the reaction was carried out overnight at 90°C.

After the reaction was completed, the reaction solution was quenched by adding saturated sodium chloride and extracted with ethyl acetate (30 mL × 2 times). The organic phases were combined, dried, and concentrated under reduced pressure. The obtained crude product was purified by silica gel column chromatography (eluent: ethyl acetate/n-hexane = 1/5) to obtain 1 g of white solid tert-butyl 4-(4-(((4-cyano-2-fluorobenzyl)oxy)-5-fluoropyrimidin-2-yl)piperazin)-1-formate (yield: 44%). LC-MS: RT = 1.89 min, [M+H]⁺= 432.37.

### Step C: Synthesis of 3-fluoro-4-(((5-fluoro-2-(piperazin-1-yl)pyrimidin-4-yl)oxy)methyl)benzonitrile

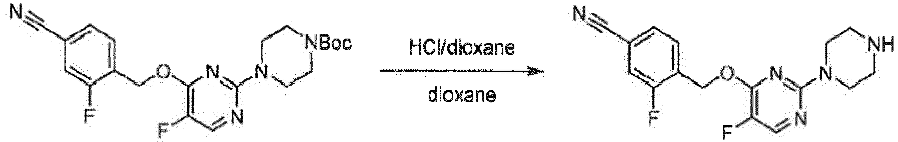

Tert-butyl 4-(4-(((4-cyano-2-fluorobenzyl)oxy)-5-fluoropyrimidin-2-yl)piperazin)-1-formate (1 g, 2.3 mmol) was dissolved in dioxane (10 mL) at room temperature, and hydrogen chloride in dioxane (10 mL) was added under an ice bath. The mixed solution was stirred at room temperature for 0.5 h.

After the reaction was completed, the reaction solution was concentrated to obtained 120 mg of white solid 3-fluoro-4-(((5-fluoro-2-(piperazin-1-yl)pyrimidin-4-yl)oxy)methyl)benzonitrile. LC-MS: RT = 1.62 min, [M+H]⁺= 332.25.

### Step D: Synthesis of methyl 2-((S)-1-(4-(4-((4-cyano-2-fluorobenzyl)oxy)-5-fluoropyrimidin-2-yl)piperazin-1-yl)ethyl)-1-(((S)-oxetan-2-yl)methyl)-1H-benzo[d]imidazol-6-carboxylate

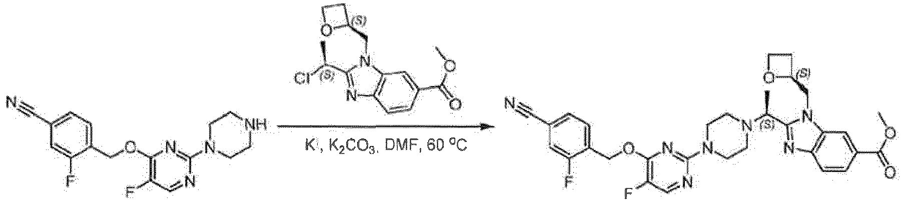

3-Fluoro-4-(((5-fluoro-2-(piperazin-1-yl)pyrimidin-4-yl)oxy)methyl)benzonitrile (200 mg, 0.6 mmol) was dissolved in *N,N*-dimethylformamide (10 mL) at room temperature, and then methyl (S)-2-(chloroethyl)-1-(((S)-oxetan-2-yl)methyl)-1*H*-benzo[*d*]imidazol-6-carboxylate (186 mg, 0.6 mmol), potassium iodide (200 mg, 1.2 mmol) and potassium carbonate (166 mg, 1.2 mmol) were added. The mixture was stirred at 60°C for 12 h.

After the reaction was completed, the reaction solution was quenched by adding saturated sodium chloride solution and extracted with ethyl acetate (10 mL × 2 times). The organic phases were combined, dried with anhydrous sodium sulfate, filtered and concentrated, and the crude product was purified by silica gel column chromatography (eluent: ethyl acetate/n-hexane = 1/1) to obtain 150 mg of colorless oily liquid methyl 2-((S)-1-(4-(4-((4-cyano-2-fluorobenzyl)oxy)-5-fluoropyrimidin-2-yl)piperazin-1-yl)ethyl)-1-(((S)-oxetan-2-yl)methyl)-1*H*-benzo[*d*]imidazol-6-carboxylate (yield: 41%). LC-MS: RT = 1.95 min, [M+H]⁺= 603.35.

### Step E: Synthesis of 2-((S)-1-(4-(4-((4-cyano-2-fluorobenzyl)oxy)-5-fluoropyrimidin-2-yl)piperazin-1-yl)ethyl)-1-(((S)-oxetan-2-yl)methyl)-1H-benzo[d]imidazol-6-carboxylic acid

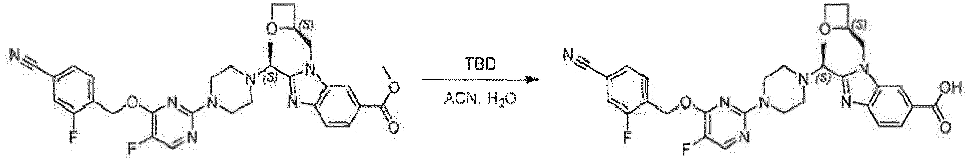

Methyl 2-((S)-1-(4-(4-((4-cyano-2-fluorobenzyl)oxy)-5-fluoropyrimidin-2-yl)piperazin-1-yl)ethyl)-1-(((S)-oxetan-2-yl)methyl)-1*H*-benzo[*d*]imidazol-6-carboxylate (150 mg, 0.25 mmol) was dissolved in acetonitrile (10 mL) and water (2 mL), then 1,3,4,6,7,8-hexahydro-2*H*-pyrimidin[1,2-a]pyrimidine (139 mg, 1.0 mmol) was added, and the reaction was carried out at room temperature for 4 h.

After the reaction was completed, the reaction solution was concentrated. The obtained crude product was purified by high performance liquid chromatography to obtain 55.11 mg of white solid 2-((S)-1-(4-(4-((4-cyano-2-fluorobenzyl)oxy)-5-fluoropyrimidin-2-yl)piperazin-1-yl)ethyl)-1-(((S)-oxetan-2-yl)methyl)-1*H*-benzo[*d*]imidazol-6-carboxylic acid (yield: 37%). LC-MS: RT = 1.80 min, [M+H]⁺ = 590.27.

### Example 43 2-((S)-1-(4-(6-((4-cyano-2-fluorobenzyl)oxy)-4-methoxypyridin-2-yl)piperazin-1-yl)ethyl)-1-(((S)-oxetan-2-yl)methyl)-1H-benzo[d]imidazol-6-carboarylic acid

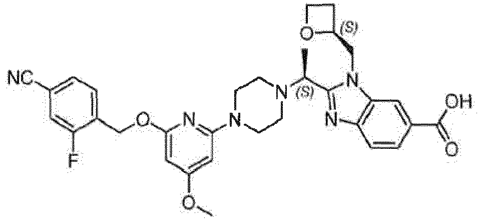

The specific synthesis route is as follows.

### Step A: Synthesis of 2-chloro-6-((4-cyano-2-fluorobenzyl)oxy)-4-methoxypyridine

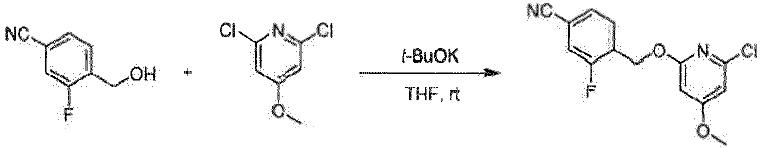

Potassium tert butoxide (0.98 g, 8.72 mmol) was added into a solution of tetrahydrofuran (10.0 mL) containing (4-cyano-2-fluorophenyl)methanol (1.0 g, 6.23 mmol) at 0°C, and the mixed solution was stirred for 30 min. Then 2,6-dichloro-4-methoxypyridine (1.1 g, 6.23 mmol) was added and the reaction was carried out overnight at 25°C.

After the reaction was completed, the reaction solution was quenched by adding water (50 mL) and extracted with ethyl acetate (50 mL × 3 times). The organic phases were combined, then washed with saturated saline (50 mL × 3 times), dried with anhydrous sodium sulfate, and concentrated under reduced pressure. The residue was purified by silica gel column chromatography (eluent: ethyl acetate/n-hexane = 1/2). 0.98 g of yellow oily product 2-chloro-6-((4-cyano-2-fluorobenzyl)oxy)-4-methoxypyridine was obtained (yield: 53.9%). LC-MS: RT = 2.15 min, [M+H]+ = 293.05.

### Step B: Synthesis of tert-butyl 4-(6-((4-cyano-2-fluorobenzyl)oxy)-4-methoxypyridin-2-yl)piperazin-1-carboaylate

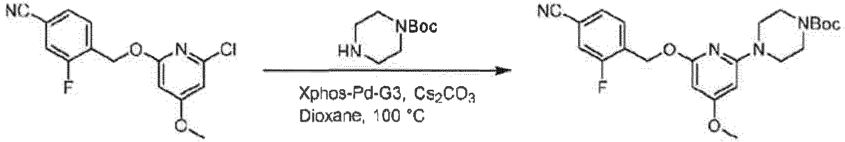

Tert-butyl piperazin-1-carboxylate (0.62 g, 3.36 mmol), cesium carbonate (2.2 g, 6.72 mmol) and methanesulfonato(2-dicyclohexylphosphino-2',4',6'-triisopropyl-1,1'-biphenyl)(2'-amino-1,1'-biphenyl-2-yl)palladium (II) (288 mg, 0.34 mmol) were added into a solution of 1,4-dioxane containing 2-chloro-6-((4-cyano-2-fluorobenzyl)oxy)-4-methoxypyridine (0.98 g, 3.36 mmol) at 25°C, and the reaction was carried out at 100°C for 3 h.

After the reaction was completed, the reaction solution was quenched by adding water and extracted with ethyl acetate (50 mL × 3 times). The organic phases were combined, then washed with saturated saline (50 mL × 2 times), dried with anhydrous sodium sulfate, and concentrated under reduced pressure. The residue was purified by silica gel column chromatography (eluent: ethyl acetate/n-hexane = 1/2). 1.2 g of white solid tert-butyl 4-(6-((4-cyano-2-fluorobenzyl)oxy)-4-methoxypyridin-2-yl)piperazin-1-carboxylate was obtained (yield: 80.8%). LC-MS: RT = 2.23 min, [M+H]⁺= 443.29.

### Step C: Synthesis of 4-(6-((4-cyano-2-fluorobenzyl)oxy)-4-methoxypyridin-2-yl)piperazine

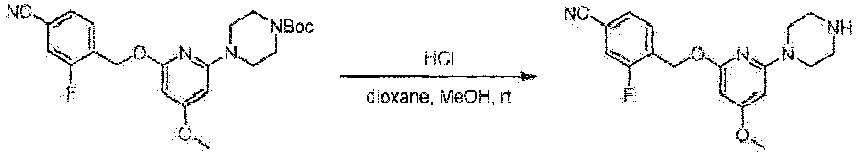

Hydrochloric acid in 1,4-dioxane (10.0 mL, 4 mol/L) was added into a solution of methanol (20 mL) containing tert-butyl 4-(6-((4-cyano-2-fluorobenzyl)oxy)-4-methoxypyridin-2-yl)piperazin-1-carboxylate (1.2 g, 2.71 mmol) at 25°C, and the reaction was carried out at 25°C for 3 h.

After the reaction was completed, the reaction solution was directly spin-dried to obtain 1.33 g of white solid 4-(6-((4-cyano-2-fluorobenzyl)oxy)-4-methoxypyridin-2-yl)piperazine, which was directly used in the next step. LC-MS: RT = 1.72 min, [M+H]⁺= 343.18.

### Step D: Synthesis of methyl 2-((S)-1-(4-(6-((4-cyano-2-fluorobenzyl)oxy)-4-methoxypyridin-2-yl)piperazin-1-yl)ethyl)-1-(((S)-oxetan-2-yl)methyl)-1H-benzo[d]imidazol-6-carboxylate

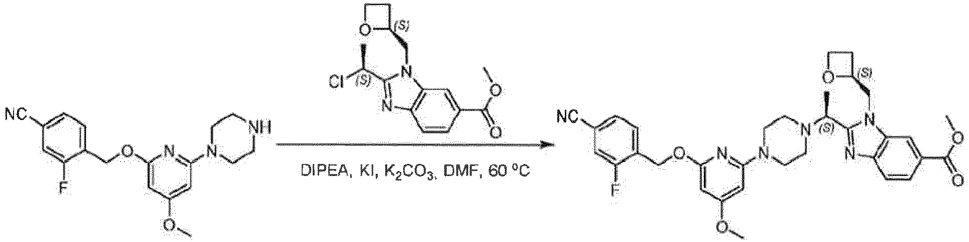

At 25°C, *N,N-*diisopropylethylamine (1.0 mL), potassium iodide (676 mg, 4.07 mmol), potassium carbonate (748 mg, 5.42 mmol), and *N,N*-dimethylformamide (10.0 mL) were added into a solution of acetonitrile (8.0 mL) containing 4-(6-((4-cyano-2-fluorobenzyl)oxy)-4-methoxypyridin-2-yl)piperazine hydrochloride (1.33 mg, 2.71 mmol), and then methyl 2-((S)-1-chloroethyl)-1-(((S)-oxetan-2-yl) methyl)-1*H*-benzo[*d*]imidazol-6-carboxylate (837 mg, 2.71 mmol) was added. The reaction was carried out at 60°C for 3 h under N₂ protection.

After the reaction was completed, the reaction solution was quenched by adding water and extracted with ethyl acetate (30 mL × 2 times). The organic phases were combined, then washed with saturated saline (30 mL), dried with anhydrous sodium sulfate, and concentrated under reduced pressure. The residue was purified by silica gel column chromatography (eluent: ethyl acetate/n-hexane = 2/1). 900 mg of yellow solid methyl 2-((S)-1-(4-(6-((4-cyano-2-fluorobenzyl)oxy)-4-methoxypyridin-2-yl)piperazin-1-yl)ethyl)-1-(((S)-oxetan-2-yl)methyl)-1*H*-benzo[*d*]imidazol-6-carboxylate was obtained (yield: 54.0%). LC-MS: RT = 1.92 min, [M+H]+ = 615.31.

### Step E: Synthesis of 2-((S)-1-(4-(6-((4-cyano-2-fluorobenzyl)oxy)-4-methoxypyridin-2-yl)piperazin-1-yl)ethyl)-1-(((S)-oxetan-2-yl)methyl)-1H-benzo[d]imidazol-6-carboxylic acid

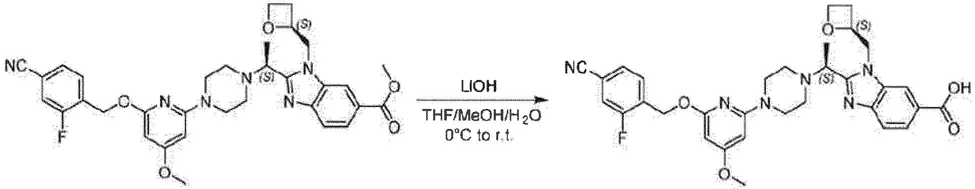

Lithium hydroxide (307 mg, 7.30 mmol) in water (1 mL) was added dropwise into a mixed solution of tetrahydrofuran (3 mL) and methanol (1 mL) containing methyl 2-((S)-1-(4-(6-((4-cyano-2-fluorobenzyl)oxy)-4-methoxypyridin-2-yl)piperazin-1 -yl)ethyl)-1 -(((S)-oxetan-2-yl)methyl)-1*H-*benzo[*d*]imidazol-6-carboxylate (900 mg, 1.46 mmol) at 0°C, and the reaction was carried out at 25°C for 30 min.

After the reaction was completed, the reaction solution was quenched by adding water and extracted with ethyl acetate (30 mL × 2 times). The organic phases were combined, then washed with saturated saline (20 mL), dried with anhydrous sodium sulfate, and concentrated under reduced pressure. The residue was purified by silica gel column chromatography (eluent: methanol/dichloromethane = 1/10). 430 mg of white solid, 2-((S)-1-(4-(6-((4-cyano-2-fluorobenzyl)oxy)-4-methoxypyridin-2-yl)piperazin-1-yl)ethyl)-1-(((S)-oxetan-2-yl)methyl)-1*H*-benzo[*d*]imidazol-6-carboxylic acid, was obtained (yield: 49.0%). LC-MS: RT = 1.79 min, [M+H]+ = 601.39. ¹H NMR (400 MHz, DMSO-*d₆*) δ 12.75 (s, 1H), 8.27 (t, *J* = 2.4 Hz, 1H), 7.86 (dd, *J* = 10.0, 1.3 Hz, 1H), 7.79 (ddd, *J* = 5.6, 4.1, 1.5 Hz, 1H), 7.71-7.65 (m, 2H), 7.62 (t, *J* = 7.6 Hz, 1H), 5.83 (t, *J* = 4.1 Hz, 1H), 5.76-5.71 (m, 1H), 5.35 (s, 2H), 5.12 (d, *J* = 3.0 Hz, 1H), 4.78 (dd, *J* = 15.5, 2.9 Hz, 1H), 4.67 (dd, *J* = 15.5, 5.5 Hz, 1H), 4.52-4.41 (m, 2H), 4.20-4.11 (m, 1H), 3.71 (d, *J* = 3.0 Hz, 3H), 3.33 (s, 4H), 2.67-2.59 (m, 1H), 2.50 (d, *J* = 1.2 Hz, 4H), 2.31 (dt, *J* = 18.4, 7.4 Hz, 1H), 1.48-1.39 (m, 3H).

### Example 44 2-((S)-1-(4-(6-((4-cyano-2-fluorobenzyl)oxy)-4-(trifluoromethyl)pyridin-2-yl)piperazin-1-yl)ethyl)-1-(((S)-oxetan-2-yl)methyl)-1H-benzo[d]imidazol-6-carboxylic acid

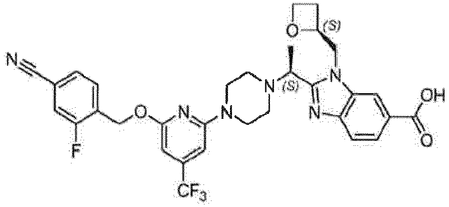

The specific synthesis route is as follows.

### Step A: Synthesis of tert-butyl 4-(6-chloro-4-(trifluoromethyl)pyridin-2-yl)piperazin-1-formate

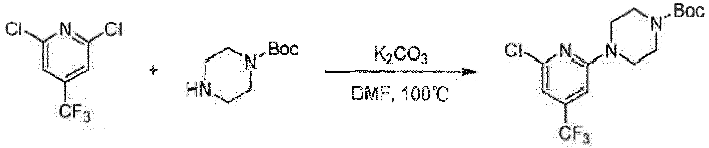

2,6-Dichloro-4-(trifluoromethyl)pyridine (500.0 mg, 2.32 mmol), tert-butyl piperazin-1-carboxylate (431.5 mg, 2.32 mmol) and potassium carbonate (641.3 mg, 4.64 mmol) were added to N,N-dimethylformamide (20.0 mL) at room temperature. The reaction was carried out at 100°C for 6 h under N₂ protection.

After the reaction was completed, the reaction solution was quenched by adding water and extracted with ethyl acetate (30 mL × 3 times). The organic phases were combined, then washed with saturated saline (20 mL × 2 times), dried with anhydrous sodium sulfate, and concentrated under reduced pressure. The residue was purified by silica gel column chromatography (eluent: ethyl acetate/n-hexane = 5/1) to obtain 677.0 mg of pale yellow solid tert-butyl 4-(6-chloro-4-(trifluoromethyl)pyridin-2-yl)piperazin-1-formate (yield: 79.6%). LC-MS: RT= 1.93 min, [M+H]⁺-56= 310.11.

### Step B: Synthesis of tert-butyl 4-(6-(((4-cyano-2-fluorobenzyl)oxy)-4-(trifluoromethyl)pyridin-2-yl)piperazin)-1-formate

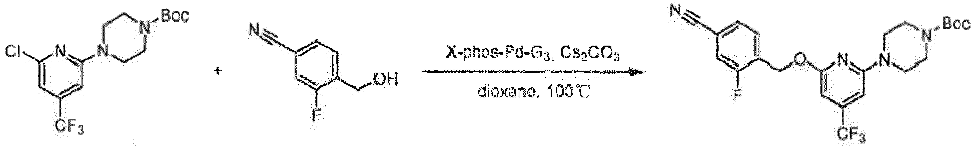

Tert-butyl 4-(6-chloro-4-(trifluoromethyl)pyridin-2-yl)piperazin-1-formate (300.0 mg, 0.82 mmol), 3-fluoro-4-(hydroxymethyl)benzonitrile (148.6 mg, 0.98 mmol) and cesium carbonate (534.6 mg, 1.64 mmol) were added to dioxane (15.0 mL) at room temperature, then methanesulfonato(2-dicyclohexylphosphino-2',4',6'-triisopropyl-1,1'-biphenyl)(2'-amino-1,1'-biphenyl-2-yl)palladium(II) (35.0 mg, 0.04 mmol) was added. The reaction was carried out at 100°C for 0.5 h under N₂ protection.

After the reaction was completed, the reaction solution was quenched by adding water and extracted with dichloromethane (50 mL × 3 times). The organic phases were combined, washed with saturated saline (30 mL × 3 times), then dried with anhydrous sodium sulfate, and finally purified by silica gel column chromatography (eluent: n-hexane: ethyl acetate = 10 : 1) to obtain 240.0 mg of pale yellow oily product tert-butyl 4-(6-(((4-cyano-2-fluorobenzyl)oxy)-4-(trifluoromethyl)pyridin-2-yl)piperazin)-1 - formate (yield: 60.1%). LC-MS: RT = 1.95 min, [M-56+H]⁺ = 425.26.

### Step C: Synthesis of 3-fluoro-4-((((6-(piperazin-1-yl)-4-(trifluoromethyl)pyridin-2-yl)oxy)methyl)benzonitrile

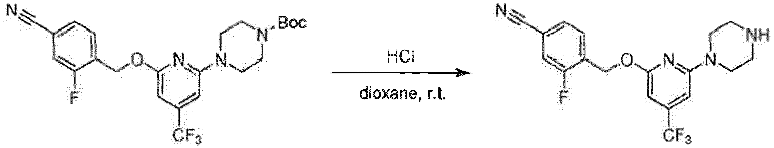

Tert-butyl 4-(6-(((4-cyano-2-fluorobenzyl)oxy)-4-(trifluoromethyl)pyridin-2-yl)piperazin)-1-formate (240.0 mg, 0.50 mmol) was added to hydrochloric acid in 1,4-dioxane (1.00 mmol/mL, 5 mL) at room temperature. The reaction was carried out at room temperature for 1 h under N₂ protection.

After the reaction was completed, the reaction solution was concentrated under reduced pressure. 195.0 mg of pale yellow solid 3-fluoro-4-((((6-(piperazin-1-yl)-4-(trifluoromethyl)pyridin-2-yl)oxy)methyl)benzonitrile (yield: 100.0%) was obtained, which was directly used in the next reaction. LC-MS: RT = 1.72 min, [M+H]⁺= 381.17.

### Step D: Synthesis of methyl 2-((S)-1-(4-(6-((4-cyano-2-fluorobenzyl)oary)-4-(trifluoromethyl)pyridin-2-yl)piperazin-1-yl)ethyl)-1-(((S)-oxetan-2-yl)methyl)-1H-benzo[d]imidazol-6-carboxylate

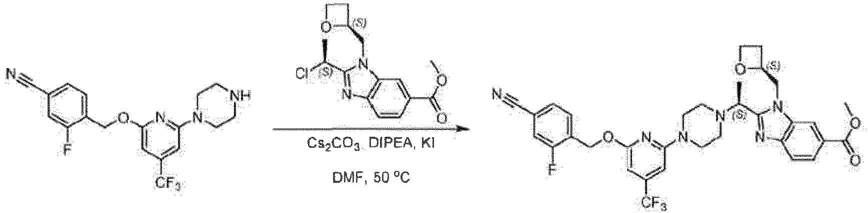

3-Fluoro-4-((((6-(piperazin-1-yl)-4-(trifluoromethyl)pyridin-2-yl)oxy)methyl)benzonitrile (50.0 mg, 0.13 mmol), methyl 2-((S)-1-chloroethyl)-1-(((S)-oxetan-2-yl)methyl)-1*H*-benzo[*d*]imidazol-6-carboxylate (40.0 mg, 0.13 mmol), cesium carbonate (40.4 mg, 0.13 mmol), and potassium iodide (22.0 mg, 0.13 mmol) were added to N,N-dimethylformamide (5.0 mL), follow by the addition of *N,N-*diisopropylethylamine (0.50 mL) at room temperature. The reaction was carried out at 50°C for 4.0 h under N₂ protection.

After the reaction was completed, the reaction solution was quenched by adding water and extracted with ethyl acetate (50 mL × 3 times). The organic phases were combined, washed with saturated saline (30 mL × 3 times), then dried with anhydrous sodium sulfate, and finally purified by silica gel column chromatography (eluent: dichloromethane/methanol = 20 : 1) to obtain 50.2 mg of white solid methyl 2-((S)-1--(4-(6-((4-cyano-2-fluorobenzyl)oxy)-4-(trifluoromethyl)pyridin-2-yl)piperazin-1 -yl)ethyl)-1 - (((S)-oxetan-2-yl)methyl)-1*H*-benzo[*d*]imidazol-6-carboxylate (yield: 59.1%). LC-MS: RT = 1.83 min, [M+H]⁺= 653.27.

### Step E: Synthesis of 2-((S)-1-(4-(6-((4-cyano-2-fluorobenzyl)oxy)-4-(trifluoromethyl)pyridin-2-yl)piperazin-1-yl)ethyl)-1-(((S)-oxetan-2-yl)methyl)-1H-benzo[d]imidazol-6-carboxylic acid

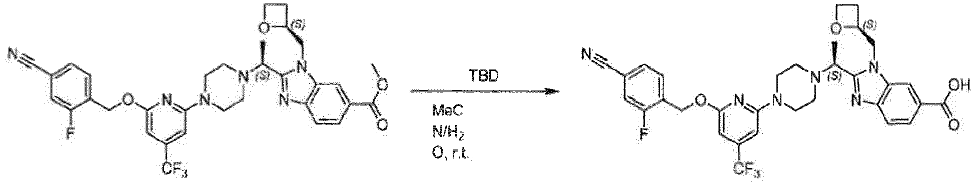

1,5,7-Triazabicyclo[4.4.0]dec-5-ene (33.4 mg, 0.24 mmol) in water (3.0 mL) was added dropwise into a solution of acetonitrile (12.0 mL) containing methyl 2-((S)-1-(4-(6-((4-cyano-2-fluorobenzyl)oxy)-4-(trifluoromethyl)pyridin-2-yl)piperazin-1-yl)ethyl)-1-(((S)-oxetan-2-yl)methyl)-1*H*-benzo[*d*]imidazol-6-carboxylate (50.2 mg, 0.08 mmol) at room temperature. The reaction was carried out at room temperature for 3 h.

After the reaction was completed, the reaction solution was quenched by adding water, and the pH was adjusted to 6 by aqueous citric acid solution (0.5 mol/L). The mixed solution was extracted with dichloromethane (30 mL × 3 times). The organic phases were combined, washed with aqueous citric acid solution (30 mL × 2 times), and dried with anhydrous sodium sulfate. The crude product was purified by preparative high performance liquid chromatography. The separation conditions are as follows: chromatographic column: Agilent 5 Prep-C18 100 mm×30 mm 5µM; mobile phase: water (containing 0.1% ammonia) and acetonitrile; flow rate: 20 mL/min; gradient: eluted by 32.3% acetonitrile at 8.12 min; detection wavelength: 254 nm. Upon purification and lyophilization, 22.0 mg of white solid, 2-((S)-1-(4-(6-((4-cyano-2-fluorobenzyl)oxy)-4-(trifluoromethyl)pyridin-2-yl)piperazin-1-yl)ethyl)-1-(((S)-oxetan-2-yl)methyl)-1*H*-benzo[dJimidazol-6-carboxylic acid, was obtained (yield: 44.1%). LC-MS: RT = 1.75 min, [M+H]⁺= 639.33. ¹H NMR (400 MHz, DMSO) δ 8.26 (d, *J* = 0.9 Hz, 1H), 7.88 (dd, *J* = 10.0, 1.2 Hz, 1H), 7.79 (dd, *J* = 8.4, 1.5 Hz, 1H), 7.68 (qd, *J* = 7.8, 3.6 Hz, 3H), 6.55 (s, 1H), 6.33 (s, 1H), 5.43 (s, 2H), 5.13 (dd, *J* = 13.9, 8.9 Hz, 1H), 4.70 (ddd, *J* = 25.6, 10.8, 4.3 Hz, 2H), 4.46 (dd, *J* = 17.0, 4.8 Hz, 2H), 4.20-4.12 (m, 1H), 3.48 (d, *J* = 5.4 Hz, 4H), 2.67-2.51 (m, 5H), 2.38-2.24 (m, 1H), 1.43 (d, *J* = 6.7 Hz, 3H).

### Example 45 2-((S)-1-(4-(4-cyano-6-((4-cyano-2-fluorobenzyl)oxo)pyridin-2-yl)piperazin-1-yl)ethyl)-1-(((S)-oxetan-2-yl)methyl)-1H-benzo[d]imidazol-6-carboxylic acid

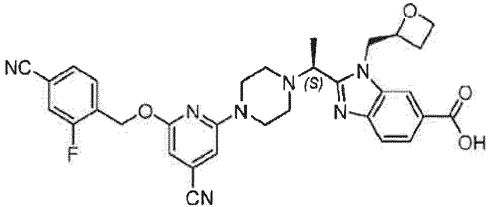

The specific synthesis route is as follows.

### Step A: Synthesis of tert-butyl 4-(6-chloro-4-cyanopyridin-2-yl)piperazin-1-carboxylate

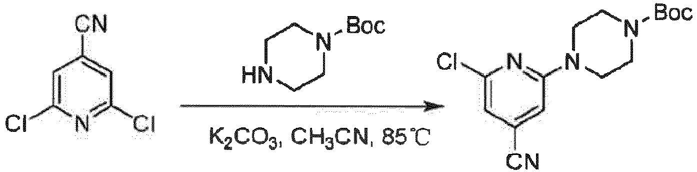

2,6-Dichloro-4-cyanopyridine (1.0 g, 5.78 mmol), N-Boc-piperazine (1.29 g, 6.94 mmol) and potassium carbonate (1.6 g, 11.56 mmol) were added to 10 mL of dry acetonitrile at room temperature, and the reaction was carried out at 85°C for 4 h.

The reaction solution was cooled to room temperature after the reaction was completed, and was poured into 40 mL ice water solution. The mixed solution was stirred for 30 min and filtered by suction. The filter cake was washed with water and dried to obtain 1.7 g of white solid tert-butyl 4-(6-chloro-4-cyanopyridin-2-yl)piperazin-1-carboxylate (yield: 91.4%). LC-MS: RT= 2.18 min, [M+H]⁺ = 223.10.

### Step B: Synthesis of tert-butyl 4-(4-cyano-6-((4-cyano-2-fluorobenzyl)oxy)pyridin-2-yl)piperazin-1-carboxylate

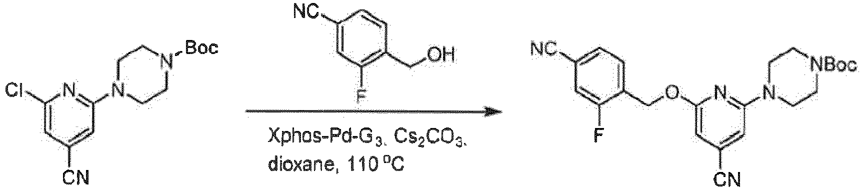

The intermediate tert-butyl 4-(6-chloro-4-cyanopyridin-2-yl)piperazin-1-carboxylate (500 mg, 1.55 mmol), 3-fluoro-4-hydroxymethyl benzonitrile (258 mg, 1.70 mmol), methanesulfonato(2-dicyclohexylphosphino-2',4',6'-triisopropyl-1,1'-biphenyl)(2'-amino-1,1'-biphenyl-2-yl)palladium(II) (65.7 mg, 0.08 mmol) and cesium carbonate (1012 mg, 3.1 mmol) were added to 10 mL of dried dioxane solution at room temperature. The reaction was carried out at 110°C for 2 h under N₂ protection.

After the reaction was completed, the reaction solution was evaporated, extracted with ethyl acetate (20 mL), dried with anhydrous sodium sulfate, and evaporated to obtain pale yellow solid, which was purified by silica gel column chromatography (n-hexane/ethyl acetate = 5/2) to obtain 383 mg of pale yellow solid tert-butyl 4-(4-cyano-6-((4-cyano-2-fluorobenzyl)oxy)pyridin-2-yl)piperazin-1-carboxylate (yield: 56.1%). LC-MS: RT = 2.21 min, [M-56]⁺= 379.25.

### Step C: Synthesis of 2-((4-cyano-2-fluorobenzyl)oxy)-6-(piperazin-l-yl)isonicotinonitrile

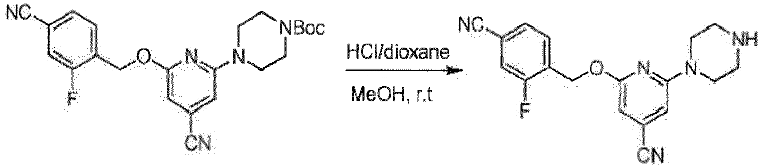

Tert-butyl 4-(4-cyano-6-((4-cyano-2-fluorobenzyl)oxy)pyridin-2-yl)piperazin-1-carboxylate (380 mg, 0.87 mmol) was dissolved in 10 mL of methanol at room temperature, and then 10 mL of hydrogen chloride in dioxane with a concentration of 4M/L was added under an ice-bath. The reaction was carried out at room temperature for 2 h.

After the reaction was completed, the reaction solution was evaporated to obtain 340 mg of white solid (2-((4-cyano-2-fluorobenzyl)oxy)-6-(piperazin-1-yl)isonicotinonitrile (yield: 116.0%). LC-MS: RT = 1.70 min, [M+H]⁺= 338.18.

### Step D: Synthesis of methyl 2-((S)-1-(4-(4-cyano-6-((4-cyano-2-fluorobenzyl)oxo)pyridin-2-yl)piperazin-1-yl)ethyl)-1-(((S)-oxetan-2-yl)methyl)-1H-benzo[dlimidazol-6-carboxylate

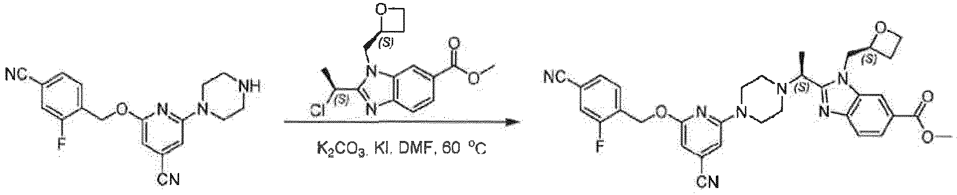

The intermediate 2-((4-cyano-2-fluorobenzyl)oxy)-6-(piperazin-1-yl)isonicotinonitrile (380 mg, 0.87 mmol), methyl 2-((S)-1-chloroethyl)-1-(((S)-oxetan-2-yl)methyl)-1*H*-benzo[*d*]imidazol-6-carboxylate (267 mg, 0.87 mmol), potassium carbonate (240 mg, 1.74 mmol) and potassium iodide (217 mg, 1.3 mmol) were added to 10 mL of DMF solution at room temperature, and the reaction was carried out at 60 °C for 10 h.

The reaction solution was poured into ice water solution after the reaction was completed, and a solid was precipitated. The mixture was then filtered by suction, and the filter cake was washed with water to obtain 300 mg of white product methyl 2-((S)-1-(4-(4-cyano-6-((4-cyano-2-fluorobenzyl)oxo)pyridin-2-yl)piperazin-1-yl)ethyl)-1-(((S)-oxetan-2-yl)methyl)-1*H*-benzo[*d*]imidazol-6-carboxylate (yield: 56.6%). LC-MS: RT = 1.93 min, [M+H]⁺= 610.34.

### Step E: Synthesis of 2-((S)-1-(4-(4-cyano-6-((4-cyano-2-fluorobenzyl)oxy)pyridin-2-yl)piperazin-1-yl)ethyl)-1-(((S)-oxetan-2-yl)methyl)-1H-benzo[d]imidazol-6-carboarylic acid

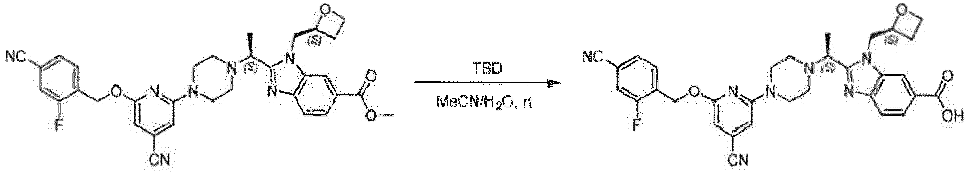

The intermediate methyl 2-((S)-1-(4-(4-cyano-6-((4-cyano-2-fluorobenzyl)oxy)pyridin-2-yl)piperazin-1-yl)ethyl)-1-(((S)-oxetan-2-yl)methyl)-1*H*-benzo[*d*]imidazol-6-carboxylate (100 mg, 0.16 mmol) was dissolved in a mixed solution of 5mL of acetonitrile/water (4:1) at room temperature, then 1,5,7-triazabicyclo[4.4.0]dec-5-ene (48 mg, 0.34 mmol) was added at room temperature, and the reaction was carried out at room temperature for 4 h.

After the reaction was completed, the reaction solution was poured into 15% aqueous citric acid solution, and the pH was adjusted to 3-4. The reaction solution was extracted with dichloromethane (20 mL), washed with water for 3 times, dried with anhydrous sodium sulfate, and evaporated to obtain a pale yellow solid. After purification by silica gel column chromatography (dichloromethane/methanol = 10/1), 60 mg of white solid, 2-((S)-1-(4-(4-cyano-6-((4-cyano-2-fluorobenzyl)oxy)pyridin-2-yl)piperazin-1-yl)ethyl)-1-(((S)-oxetan-2-yl)methyl)-1*H*-benzo[*d*]imidazol-6-carboxylic acid, was obtained (yield: 61.4%). LC-MS: RT = 1.82 min, [M+H]⁺= 596.29. ¹H NMR (400 MHz, DMSO) δ 8.25 (s, 1H), 7.88 (dd, *J* = 10.0, 1.3 Hz, 1H), 7.79 (dd, *J* = 8.4, 1.4 Hz, 1H), 7.71-7.60 (m, 3H), 6.76 (s, 1H), 6.46 (s, 1H), 5.42 (d, *J* = 13.5 Hz, 2H), 5.18 - 5.06 (m, 1H), 4.81-4.72 (m, 1H), 4.66 (dd, *J* = 15.4, 5.4 Hz, 1H), 4.52-4.36 (m, 2H), 4.16 (dt, *J* = 9.1, 5.8 Hz, 1H), 3.50-3.42 (m, 4H), 2.67-2.56 (m, 1H), 2.56-2.50 (m, 4H), 2.37-2.21 (m, 1H), 1.42 (d, *J* = 6.7 Hz, 3H).

### Example 46 2-((S)-1-(4-(4-((4-cyano-2-fluorobenzyl)oxy)pyrimidin-2-yl)piperidin-1-yl)ethyl)-1-(((S)-oxetan-2-yl)methyl)-1H-benzo[d]imidazol-6-carboxylic acid

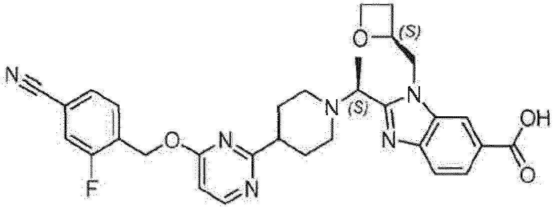

The specific synthesis route is as follows.

### Step A: Synthesis of 4-(((2-chloropyrimidin-4-yl)oxy)methyl)-3-fluorobenzonitrile

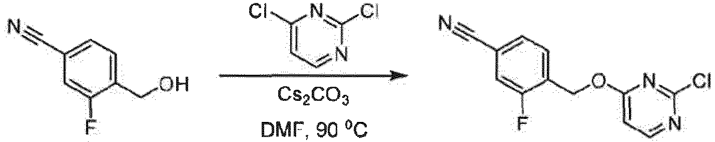

Cesium carbonate (860 mg, 2.64 mmol) was added into a solution of N,N-dimethylformamide (10 mL) containing (4-cyano-2-fluorophenyl)methanol (2.0 g, 1.32 mmol), and the mixed solution was stirred for 2 min. Then 2,4-dichloropyrimidine (1.97 g, 1.32 mmol) was added and the reaction was carried out at 90°C for 3 h.

After the reaction was completed, the reaction solution was quenched by adding water (50 mL) and extracted with ethyl acetate (50 mL × 3 times). The organic phases were combined, then washed with saturated saline (50 mL), dried with anhydrous sodium sulfate, and concentrated under reduced pressure. The residue was purified by silica gel column chromatography (eluent: ethyl acetate/n-hexane = 1/2). 2.3 g of yellow oily product, 4-(((2-fluoropyrimidin-4-yl)oxy)methyl)-3-fluorobenzonitrile, was obtained (yield: 67.6%). LC-MS: RT = 2.36 min, [M+H]⁺ = 264.17.

### Step B: Synthesis of tert-butyl 4-(4-((4-cyano-2-fluorobenzyl)oxy)pyrimidin-2-yl)-3,6-dihydropyridin-1(2H)-formate

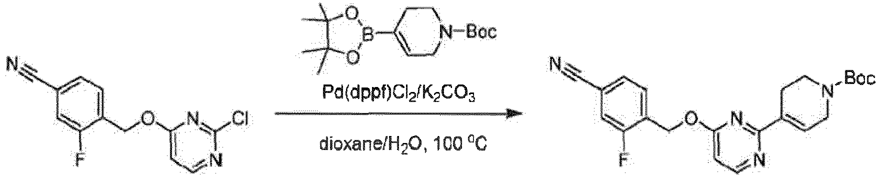

Potassium carbonate (731 mg, 5.30 mmol) and 1,1'-bis(diphenylphosphino)ferrocene dichloropalladium (II) (193 mg, 0.265 mmol) were added into a mixed solution of 1,4-dioxane (5 mL) and water containing 4-(((2-fluoropyrimidin-4-yl)oxy)methyl)-3-fluorobenzonitrile (700 mg, 2.65 mmol) and tert-butyl 4-(4,4,5,5-tetramethyl-1,3,2-dioxaborolan-2-yl)-3,6-dihydropyridin-1(2H)-carboxylate (820 mg, 2.65 mmol) at room temperature. The system was purged with nitrogen for three times, and the reaction was carried out at 100°C for 3 h.

After the reaction was completed, the reaction solution was quenched by adding water and extracted with ethyl acetate (50 mL × 3 times). The organic phases were combined, then washed with saturated saline (50 mL × 2 times), dried with anhydrous sodium sulfate, and concentrated under reduced pressure. The residue was purified by silica gel column chromatography (eluent: ethyl acetate/n-hexane = 1/2). 570 mg of pale yellow solid, tert-butyl 4-(4-((4-cyano-2-fluorobenzyl)oxy)pyrimidin-2-yl)-3,6-dihydropyridin-1(2H)-formate, was obtained (yield: 52.3%). LC-MS: RT= 2.22 min, [M+H]⁺= 411.22.

### Step C: Synthesis of tert-butyl 4-(4-((4-cyano-2-fluorobenzyl)oxy)pyrimidin-2-yl)piperidin-1-formate

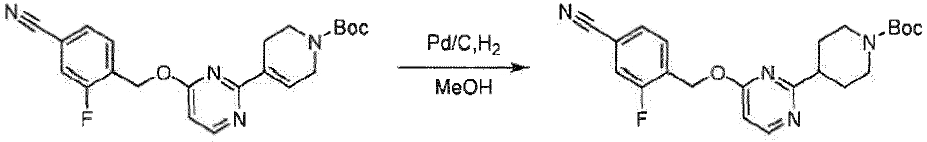

Tert-butyl 4-(4-((4-cyano-2-fluorobenzyl)oxy)pyrimidin-2-yl)-3,6-dihydropyridin-1(2H)-formate (300 mg, 0.729 mmol) was dissolved in 10 mL methanol, and 30 mg wet palladium/carbon (10%) was added. The reaction was carried out at room temperature for 3 h under hydrogen protection.

After the reaction was completed, the reaction solution was filtered, and then the filtrate was dried with anhydrous sodium sulfate, and concentrated under reduced pressure to obtain 186 mg of tert-butyl 4-(4-((4-cyano-2-fluorobenzyl)oxy)pyrimidin-2-yl)piperidin-1-formate (yield: 61.8%). LC-MS: RT = 2.03 min, [M+H]⁺= 413.23.

### Step D: Synthesis of 3-fluoro-4-(((2-(piperidin-4-yl)pyrimidin-4-yl)oxy)methyl)benzonitrile

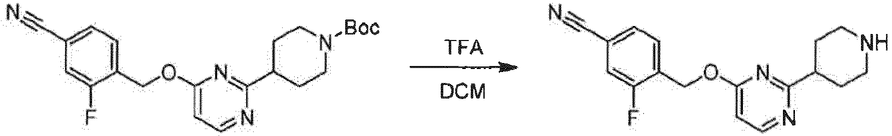

Tert butyl 4-(4-((4-cyano-2-fluorobenzyl)oxy)pyrimidin-2-yl)piperidin-1-formate (186 mg, 0.451 mmol) was dissolved in 4 mL of anhydrous dichloromethane, and 2 mL of trifluoroacetic acid was added. Then the reaction was carried out at room temperature for 40 min.

After the reaction was completed, the reaction solution was poured into 50 mL of sodium bicarbonate solution, and the mixed solution was extracted with ethyl acetate (30 mL × 2 times). The organic phases were combined, then washed with saturated saline (20 mL × 2 times), dried with anhydrous sodium sulfate, and concentrated under reduced pressure to obtain 110 mg of 3-fluoro-4-(((2-(piperidin-4-yl)pyrimidin-4-yl)oxy)methyl)benzonitrile (yield: 78.1%). LC-MS: RT = 1.86 min, [M+H]⁺= 313.27.

### Step E: Synthesis of methyl 2-((S)-1-(4-(4-((4-cyano-2-fluorobenzyl)oxy)pyrimidin-2-yl)piperidin-1-yl)ethyl)-1-(((S)-oxetan-2-yl)methyl)-1H-benzo[d]imidazol-6-carboxylate

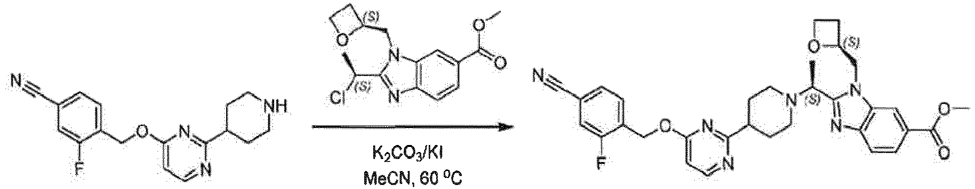

Methyl 2-((S)-1-chloroethyl)-1-(((S)-oxetan-2-yl)methyl)-1*H*-benzo[*d*]imidazol-6-carboxylate (109 mg, 0.352 mmol), potassium iodide (234 mg, 1.41 mmol) and potassium carbonate (242 mg, 1.76 mmol) were added into a solution of acetonitrile (8.0 mL) containing 3-fluoro-4-(((2-piperidin-4-yl)pyrimidin-4-yl)oxy)methyl)benzonitrile (110 mg, 0.352 mmol) at room temperature, and the reaction solution was carried out at 60°C for 5 h.

After the reaction was completed, the reaction solution was quenched by adding water and extracted with ethyl acetate (30 mL × 2 times). The organic phases were combined, then washed with saturated saline (30 mL), dried with anhydrous sodium sulfate, and concentrated under reduced pressure. The residue was purified by silica gel column chromatography (eluent: ethyl acetate/n-hexane = 5/1). 84 mg of yellow solid, methyl 2-((S)-1-(4-(4-((4-cyano-2-fluorobenzyl)oxy)pyrimidin-2-yl)piperidin-1-yl)ethyl)-1-(((S)-oxetan-2-yl)methyl)-1*H*-benzo[*d*]imidazol-6-carboxylate, was obtained (yield: 70.4%). LC-MS: RT = 1.97 min, [M+H]⁺= 585.31.

### Step F: Synthesis of 2-((S)-1-(4-(4-((4-cyano-2-fluorobenzyl)oxy)pyrimidin-2-yl)piperidin-1-yl)ethyl)-1-(((S)-oxetan-2-yl)methyl)-1H-benzo[d]imidazol-6-carboxylic acid

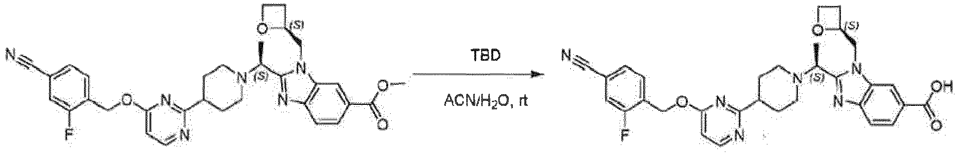

Methyl 2-((S)-1-(4-(4-((4-cyano-2-fluorobenzyl)oxy)pyrimidin-2-yl)piperidin-1-yl)ethyl)-1-(((S)-oxetan-2-yl)methyl)-1*H*-benzo[*d*]imidazol-6-carboxylate (82.0 mg, 0.140 mmol) was dissolved in 4 mL of acetonitrile, and then 1,5,7-triazabicyclo[4.4.0]dec-5-ene (41.1 mg, 0.294 mmol) and 1 mL water were added. The reaction was carried out at room temperature for 7 h.

After the reaction was completed, the reaction solution was neutralized by adding 20 mL of saturated ammonium chloride solution and extracted with dichloromethane (30 mL × 2 times). The organic phases were combined, then washed with saturated saline (20 mL × 2 times), dried with anhydrous sodium sulfate, and concentrated under reduced pressure. The obtained crude product was separated by column chromatography to obtain 26 mg of white solid 2-((S)-1-(4-(4-((4-cyano-2-fluorobenzyl)oxy)pyrimidin-2-yl)piperidin-1-yl)ethyl)-1-(((S)-oxetan-2-yl)methyl)-1*H*-benzo[*d*]imidazol-6-carboxylic acid (yield: 32.5%). ¹H NMR (400 MHz, DMSO) δ 12.75 (s, 1H), 8.49 (d, *J* = 5.1 Hz, 1H), 8.27 (d, *J* = 1.1 Hz, 1H), 7.91 (dd, *J* = 10.2, 1.1 Hz, 1H), 7.80 (dd, *J* = 8.4, 1.5 Hz, 1H), 7.74-7.76(m, 1H), 7.71-7.73 (m, 1H), 7.67 (d, *J* = 8.7 Hz, 1H), 7.07 (d, *J* = 5.1 Hz, 1H), 5.48 (s, 2H), 5.13-5.19(m, 1H), 4.83 (dd, *J* = 16.2, 3.7 Hz, 1H), 4.68 (dd, *J* = 15.5, 5.7 Hz, 1H), 4.41-4.50 (m, 2H), 4.16-4.21 (m, 1H), 2.96-2.98 (m, 1H), 2.54-2.67 (m, 4H), 2.21-2.35 (m, 2H), 1.83-1.86 (m, 2H), 1.67-1.76 (m, 2H), 1.47(d, *J* = 6.8Hz, 3H). LC-MS: RT = 1.70 min, [M+H]⁺= 571.31.

### Example 47 1-(((S)-oxetan-2-yl)methyl)-2-((S)-1-(4-(6-(pyrazolo[1,5-a]pyridin-2-methoxy)pyridin-2-yl)piperidin-1-yl)ethyl)-1H-benzo[d]imidazol-6-carboxylic acid

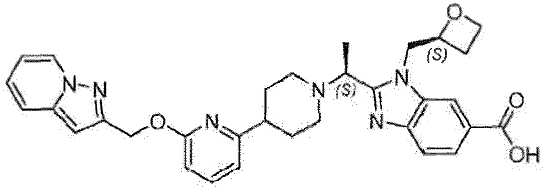

The specific synthesis route is as follows.

### Step A: Synthesis of 2-(((6-chloropyridin-2-yl)oxy)methyl)pyrazolo[1,5-a]pyridine

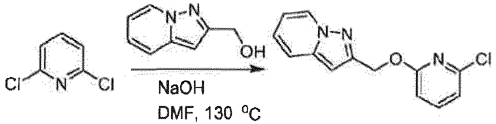

2,6-Dichloropyridine (375 mg, 2.55 mmol), pyrazolo[1,5-a]pyridin-2-methanol (453 mg, 3.06 mmol) and sodium hydroxide (510 mg, 12.75 mmol) were added to 10 mL of dried DMF, and the reaction was carried out at a temperature raised to 130°C for 2 h.

After the reaction was completed, the reaction solution was cooled to room temperature and poured into 20 mL of ice water solution. The mixed solution was stirred for 30 min and then filtered by suction. The filter cake was washed with water and dried to obtain 480 mg of white solid 2-(((6-chloropyridin-2-yl)oxy)methyl)pyrazolo[1,5-a]pyridine (yield: 72.7%). LC-MS: RT = 2.04 min, [M+H]⁺ = 260.10.

### Step B: Synthesis of tert-butyl 6-(pyrazolo[1,5-a]pyridin-2-methoxy)-3',6'-dihydro-[2,4'-bipyridin]1'(2'H)-carboxylate

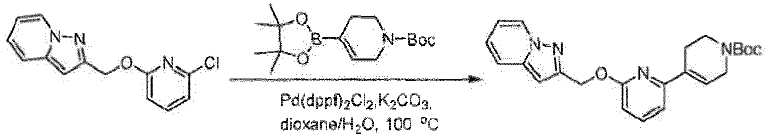

The intermediate 2-(((6-chloropyridin-2-yl)oxy)methyl)pyrazolo[1,5-a]pyridine (300 mg, 1.16 mmol), N-butyloxycarbonyl-1,2,5,6-tetrahydropyridin-4-borate pinanol ester (430 mg, 1.39 mmol), [1,1'-bis(diphenylphosphino)ferrocene]dichloropalladium (127.3 mg, 0.17 mmol), and potassium carbonate (320 mg, 2.32 mmol) were added into a mixed solution of 10 mL dioxane/water (4/1) at room temperature. The reaction was carried out at 100°C for 2 h under N₂ protection.

The reaction solution was evaporated after the reaction was completed, and then extracted with ethyl acetate, dried with anhydrous sodium sulfate, and evaporated to obtain a pale yellow solid. After purification by silica gel column chromatography (n-hexane/ethyl acetate = 5/1), 266 mg of tert-butyl 6-(pyrazolo[1,5-a]pyridin-2-methoxy)-3',6'-dihydro-[2,4'-bipyridin]1'(2'H)-carboxylate was obtained (yield: 56.5%). LC-MS: RT = 2.21 min, [M+H]⁺= 407.21.

### Step C: Synthesis of tert-butyl 4-(6-(pyrazolo[1,5-a]pyridin-2-methoxy)pyridin-2-yl)piperidin-1-carboxylate

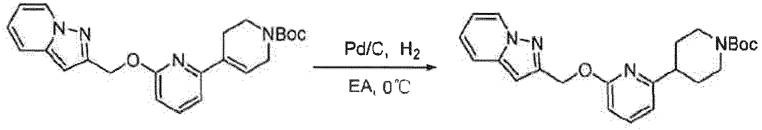

Tert-butyl 6-(pyrazolo[1,5-a]pyridin-2-methoxy)-3',6'-dihydro-[2,4'-bipyridin]1'(2'H)-carboxylate (240 mg, 0.59 mmol) was dissolved in 5 mL of methanol solvent under an ice-bath, and 10% palladium/carbon (24 mg, 0.0059 mmol) was added under an ice-bath. The reaction was carried out at 0°C for 12 h under H₂ protection.

After the reaction was completed, the reaction solution was filtered by suction, and the filtrate was evaporated to obtain 200 mg of white oily product tert-butyl 4-(6-(pyrazolo[1,5-a]pyridin-2-methoxy)pyridin-2-yl)piperidin-1-carboxylate (yield: 83.0%). LC-MS: RT = 2.21 min, [M+H]⁺ = 409.29.

### Step D: Synthesis of 2-(((6-(piperidin-4-yl)pyridin-2-yl)oxy)methyl)pyrazolo[1,5-a]pyridine

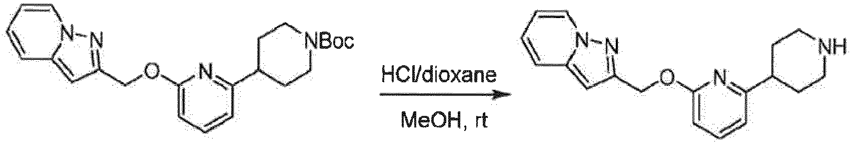

Tert-butyl 4-(6-(pyrazolo[1,5-a]pyridin-2-methoxy)pyridin-2-yl)pyridin-1-carboxylate (200 mg, 0.49 mmol) was dissolved in 10 mL of methanol solvent at room temperature, and 5 mL of hydrogen chloride in dioxane with a concentration of 4M was added under an ice-bath. The reaction was carried out at room temperature for 2 h.

After the reaction was completed, the reaction solution was evaporated to obtain 200 mg of white solid 2-(((6-(piperidin-4-yl)pyridin-2-yl)oxy)methyl)pyrazolo[1,5-a]pyridine (yield: 132.5%). LC-MS: RT = 1.65 min, [M+H]⁺= 309.22.

### Step E: Synthesis of methyl 1-(((S)-oxetan-2-yl)methyl)-2-((S)-1-(4-(6-(pyrazolo[1,5-a]pyridin-2-methoxy)pyridin-2-yl)piperidin-1-yl)ethyl)-1H-benzo[d]imidazol-6-carboxylate

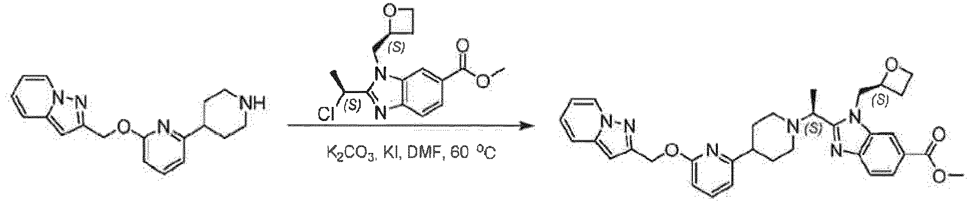

The intermediate 2-(((6-(piperidin-4-yl)pyridin-2-yl)oxy)methyl)pyrazolo[1,5-a]pyridine (200 mg, 0.49 mmol), methyl 2-((S)-1-chloroethyl)-1-(((S)-oxetan-2-yl)methyl)-1*H*-benzo[*d*]imidazol-6-carboxylate (150 mg, 0.49 mmol), potassium carbonate (136.2 mg, 0.98 mmol) and potassium iodide (122 mg, 0.98 mmol) were added to 10 mL of DMF solution at room temperature, and the reaction was carried out at a temperature of 60°C for 10 h.

After the reaction was completed, the reaction solution was poured into ice water, and a solid was precipitated. The mixture was filtered by suction, and the filter cake was washed with water to obtain 200 mg of white product methyl 1-(((S)-oxetan-2-yl)methyl)-2-((S)-1-(4-(6-(pyrazolo[1,5-a]pyridin-2-methoxy)pyridin-2-yl)piperidin-1-yl)ethyl)-1*H*-benzo[*d*]imidazol-6-carboxylate (yield: 70.4%). LC-MS: RT= 1.79 min, [M+H]⁺= 581.38.

### Step F: Synthesis of 1-(((S)-oxetan-2-yl)methyl)-2-((S)-1-(4-(6-(pyrazolo[1,5-a]pyridin-2-methoxy)pyridin-2-yl)piperidin-1-yl)ethyl)-1H-benzo[d]imidazol-6-carboxylic acid

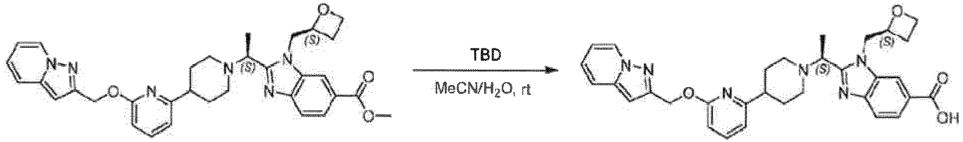

The intermediate methyl 1-(((S)-oxetan-2-yl)methyl)-2-((S)-1-(4-(6-(pyrazolo[1,5-a]pyridin-2-methoxy)pyridin-2-yl)piperidin-1-yl)ethyl)-1*H*-benzo[*d*]imidazol-6-carboxylate (200 mg, 0.34 mmol) was dissolved in 5mL of solvent mixture of acetonitrile/water (4:1) at room temperature, and then 1,5,7-triazabicyclo[4.4.0]dec-5-ene (101 mg, 0.71 mmol) was added at room temperature. The reaction was carried out at room temperature for 7 h.

The reaction solution was poured into 15% aqueous citric acid solution after the reaction was completed, and the pH adjusted was to 3-4. The reaction solution was extracted with dichloromethane (20 mL), washed with water for 3 times, dried with anhydrous sodium sulfate, and evaporated to obtain pale yellow solid. After purification by column chromatography (dichloromethane/methanol = 10/1), 130 mg of white solid, 1-(((S)-oxetan-2-yl)methyl)-2-((S)-1-(4-(6-(pyrazolo[1,5-a]pyridin-2-methoxy)pyridin-2-yl)piperidin-1-yl)ethyl)-1*H*-benzo[*d*]imidazol-6-carboxylic acid, was obtained (yield: 67.7%). LC-MS: RT = 1.73 min, [M+H]⁺= 567.35.

### Example 48 2-((S)-1-((R)-4-(6-(((4-cyano-2-fluorobenzyl)oxy)pyridin-2-yl)-3-methylpiperazin-1-yl)ethyl-1-(((S)-oxetan-2-yl)methyl)-1H-benzo[d]imidazol-6-carboxylic acid (48A) and 2-((S)-1-((S)-4-(6-(((4-cyano-2-fluorobenzyl)oxy)pyridin-2-yl)-3-methylpiperazin-1-yl)ethyl-1-(((S)-oxetan-2-yl)methyl)-1H-benzo[d]imidazol-6-carboxylic acid (48B).

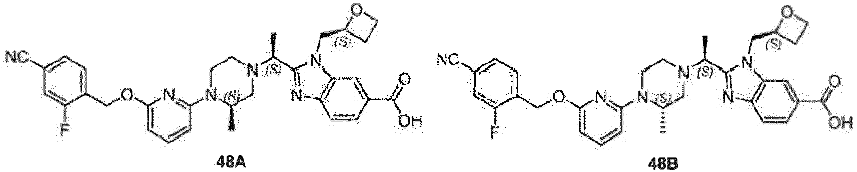

The specific synthesis route of compound **48A** is as follows.

### Step A: Synthesis of tert-butyl (R)-4-(6-chloropyridin-2-yl)-3-methylpiperazin-1-carboxylate

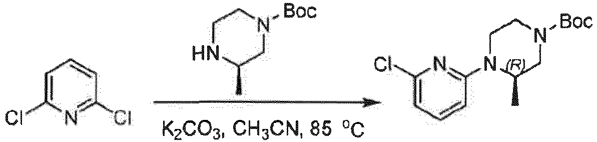

2,6-Dichloropyridine (1.5 g, 10 mmol), tert-butyl (R)-3-methylpiperazin-1-carboxylate (2.64 g, 13 mmol) and potassium carbonate (2.76 g, 20 mmol) were added to 20 mL of dried acetonitrile, and the reaction was carried out at a temperature of 85°C for 15 h.

The reaction solution was cooled to room temperature after the reaction was completed, and was poured into 40 mL of ice water solution. The mixed solution was stirred for 30 min then filtered by suction. The filter cake was washed with water, dried, and purified by column chromatography (n-hexane/ethyl acetate = 5/1). 350 mg of white solid, tert-butyl (*R*)-4-(6-chloropyridin-2-yl)-3-methylpiperazin-1-carboxylate, was obtained (yield: 11%). LC-MS: RT= 2.20 min, [M-56]⁺= 256.16.

### Step B: Synthesis of tert-butyl (R)-4-(6-((4-cyano-2-fluorobenzyl)oxy)pyridin-2-yl)-3-methylpiperazin-1-carboxylate

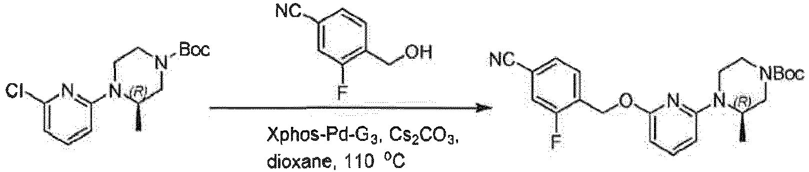

The intermediate tert-butyl (*R*)-4-(6-chloropyridin-2-yl)-3-methylpiperazin-1-carboxylate (150 mg, 0.48 mmol) and 3-fluoro-4-hydroxymethyl benzonitrile (80 mg, 0.53 mmol), methanesulfonato(2-dicyclohexylphosphino-2',4',6'-triisopropyl1,1'-biphenyl)(2'-amino-1,1'-biphenyl-2-yl)palladium (II) (20.4 mg, 0.024 mmol) and cesium carbonate (314 mg, 0.96 mmol) were added to 10 mL of dried dioxane solution at room temperature. The reaction was carried out at 110°C for 2 h under N₂ protection.

The reaction solution was evaporated after the reaction was completed, and then extracted with ethyl acetate (20 mL), dried with anhydrous sodium sulfate, and evaporated to obtain a pale yellow solid. After purification by column chromatography (n-hexane/ethyl acetate = 5/2), 150 mg of pale yellow solid, tert-butyl (R)-4-(6-((4-cyano-2-fluorobenzyl)oxy)pyridin-2-yl)-3-methylpiperazin-1-carboxylate, was obtained (yield: 73.5%). LC-MS: RT = 2.27 min, [M-56]⁺= 371.19.

### Step C: Synthesis of (R)-3-fluoro-4-(((6-(2-methylpiperazin-1-yl)pyridin-2-yl)oxy)methyl)benzonitrile

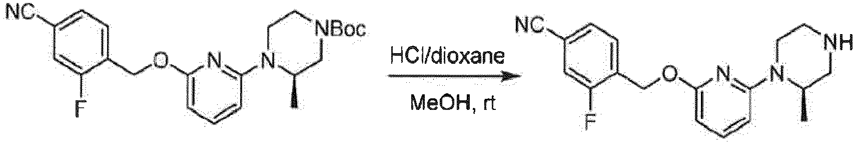

Tert-butyl (R)-4-(6-((4-cyano-2-fluorobenzyl)oxy)pyridin-2-yl)-3-methylpiperazin-1-carboxylate (150 mg, 0.35 mmol) was dissolved in 10 mL of methanol solvent at room temperature, and 5 mL of hydrogen chloride in dioxane with a concentration of 4 M was added under an ice-bath. The reaction was carried out at room temperature for 2 h.

After the reaction was completed, the reaction solution was evaporated to obtain 118 mg of white solid (*R*)-3-fluoro-4-(((6-(2-methylpiperazin-1-yl)pyridin-2-yl)oxy)methyl)benzonitrile (yield: 103.5%). LC-MS: RT= 1.70 min, [M+H]⁺= 327.21.

### Step D: Synthesis of methyl 2-((S)-1-((R)-4-(6-(((4-cyano-2-fluorobenzyl)oxy)pyridin-2-yl)-3-methylpiperazin-1-yl)ethyl)-1-(((S)-oxetan-2-yl)methyl)-1H-benzo[d]imidazol-6-carboarylate

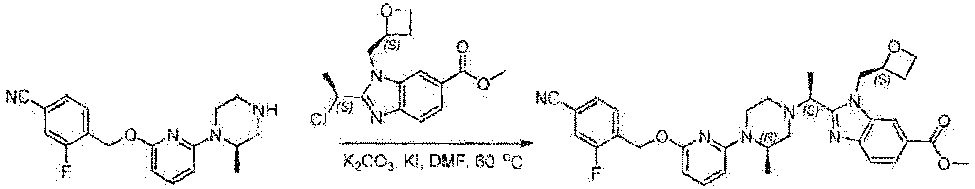

The intermediate (R)-3-fluoro-4-(((6-(2-methylpiperazin-1-yl)pyridin-2-yl)oxy)methyl)benzonitrile (150 mg, 0.35 mmol), methyl 2-((S)-1-chloroethyl)-1-(((S)-oxetan-2-yl)methyl)-1*H*-benzo[*d*]imidazol-6-carboxylate (108 mg, 0.35 mmol), potassium carbonate (97.2 mg, 0.70 mmol) and potassium iodide (87.7 mg, 0.53 mmol) were added to 10 mL of DMF solution at room temperature, and the reaction was carried out at a temperature of 60°C for 10 h.

The reaction solution was poured into ice water after the reaction was completed, and white solids were precipitated. Then the mixture was filtered by suction, and the filter cake was washed with water to obtain 140 mg of white product methyl 2-((S)-1-((R)-4-(6-(((4-cyano-2-fluorobenzyl)oxy)pyridin-2-yl)-3-methylpiperazin-1-yl)ethyl)-1-(((S)-oxetan-2-yl)methyl)-1*H*-benzo[*d*]imidazol-6-carboxylate (yield: 67.0%). LC-MS: RT = 1.97 min, [M+H]⁺= 599.34.

### Step E: Synthesis of 2-((S)-1-((R)-4-(6-(((4-cyano-2-fluorobenzyl)oxy)pyridin-2-yl)-3-methylpiperazin-1-yl)ethyl)-1-(((S)-oxetan-2-yl)methyl)-1H-benzo[d]imidazol-6-carboxylic acid (48A)

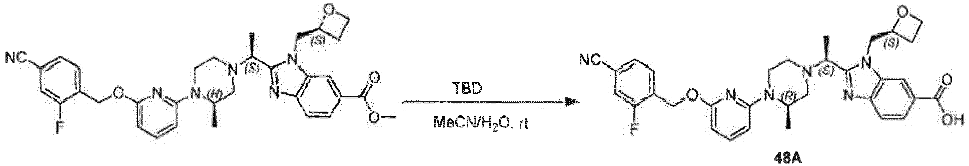

The intermediate methyl 2-((S)-1-((R)-4-(6-(((4-cyano-2-fluorobenzyl)oxy)pyridin-2-yl)-3-methylpiperazin-1-yl)ethyl)-1-(((S)-oxetan-2-yl)methyl)-1*H*-benzo[d]imidazol-6-carboxylate (100 mg, 0.17 mmol) was dissolved in 5 mL of mixed solution of acetonitrile/water (4:1) at room temperature, and then 1,5,7-triazabicyclo[4.4.0]dec-5-ene (58 mg, 0.42 mmol) was added at room temperature. The reaction was carried out at room temperature for 4 h.

The reaction solution was poured into 15% aqueous citric acid solution after the reaction was completed, and the pH was adjusted to 3-4. The reaction solution was extracted with dichloromethane (20 mL), washed with water for 3 times, dried with anhydrous sodium sulfate, and evaporated to obtain pale yellow solid. After purification by column chromatography (dichloromethane/methanol = 10/1), 38 mg of white solid, 2-((S)-1-((R)-4-(6-(((4-cyano-2-fluorobenzyl)oxy)pyridin-2-yl)-3-methylpiperazin-1-yl)ethyl)-1-(((S)-oxetan-2-yl)methyl)-1*H*-benzo[*d*]imidazol-6-carboxylic acid **(48A)**, was obtained (yield: 38.2%). LC-MS: RT = 1.86 min, [M+H]⁺= 585.31.

¹H NMR (400 MHz, DMSO) δ 8.26 (s, 1H), 7.87 (dd, *J* = 10.0, 1.3 Hz, 1H), 7.80 (dd, *J* = 8.4, 1.4 Hz, 1H), 7.67 (td, *J* = 5.8, 3.0 Hz, 2H), 7.60 (t, *J* = 7.6 Hz, 1H), 7.42 (t, *J* = 8.0 Hz, 1H), 6.20 (d, *J* = 8.2 Hz, 1H), 6.07 (d, *J* = 7.8 Hz, 1H), 5.41 (d, *J* = 13.7 Hz, 1H), 5.32 (d, *J* = 13.7 Hz, 1H), 5.20-5.07 (m, 1H), 4.86-4.76 (m, 1H), 4.71 (dd, *J* = 15.5, 5.6 Hz, 1H), 4.45 (dt, *J* = 13.4, 7.3 Hz, 3H), 4.17 (dt, *J* = 9.1,5.8 Hz, 1H), 3.73 (d, *J* = 11.7 Hz, 1H), 2.82-2.56 (m, 4H), 2 52 (d, *J* = 9 6 Hz, 1H), 2.32-2.22 (m, 1H), 2.17 (dd, *J* = 11.5, 8.3 Hz, 1H), 1 43 (d, *J* = 6.7 Hz, 3H), 1.01 (d, *J* = 6.5 Hz, 3H).

The specific synthesis route of compound **48B** was as follows.

### Step A: Synthesis of tert-butyl (S)-4-(6-chloropyridin-2-yl)-3-methylpiperazin-1-carboxylate

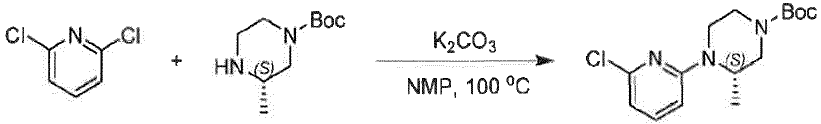

2,6-Dichloropyridine (1.5 g, 10.00 mmol), tert-butyl (S)-3-methylpiperazin-1-carboxylate (2.4 g, 12.00 mmol) and potassium carbonate (2.7 g, 20.00 mmol) were added to N,N-dimethylformamide (50.0 mL) at room temperature. The reaction was carried out at 100°C for 16.0 h under N₂ protection.

After the reaction was completed, the reaction solution was quenched by adding water and extracted with ethyl acetate (50 mL × 3 times). The organic phases were combined, then washed with saturated saline (50 mL × 2 times), dried with anhydrous sodium sulfate, and concentrated under reduced pressure. The residue was purified by silica gel column chromatography (eluent: ethyl acetate/n-hexane = 5/1) to obtain 108.0 mg of pale yellow solid tert-butyl (S)-4-(6-chloropyridin-2-yl)-3-methylpiperazin-1-carboxylate (yield: 3.5%). LC-MS: RT= 2.21 min, [M-56+H]⁺= 256.11.

### Step B: Synthesis of tert-butyl (S)-4-(6-((4-cyano-2-fluorobenzyl)oxy)pyridin-2-yl)-3-methylpiperazin-1-carboxylate

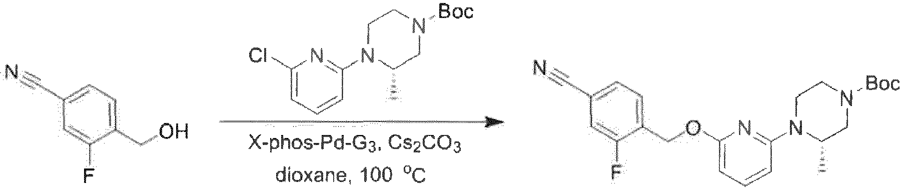

Tert-butyl (S)-4-(6-chloropyridin-2-yl)-3-methylpiperazin-1-carboxylate (108.0 mg, 0.35 mmol), 3-fluoro-4-(hydroxymethyl)benzonitrile (63.4 mg, 0.42 mmol) and cesium carbonate (228.2 mg, 0.70 mmol) were added to dioxane (10.0 mL) at room temperature, then methanesulfonato(2-dicyclohexylphosphino-2',4',6'-triisopropyl-1,1 '-biphenyl)(2'-amino-1, 1'-biphenyl-2-yl)palladium(II) (12.0 mg, 0.01 mmol) was added. The reaction was carried out at 100°C for 5.0 h under N₂ protection.

After the reaction was completed, the reaction solution was quenched by adding water and extracted with dichloromethane (30 mL × 3 times). The organic phases were combined, washed with saturated saline (30 mL × 3 times), then dried with anhydrous sodium sulfate, and finally purified by silica gel column chromatography (eluent: n-hexane/ethyl acetate = 10 : 1) to obtain 76.5 mg of brown oily solid tert-butyl (S)-4-(6-((4-cyano-2-fluorobenzyl)oxy)pyridin-2-yl)-3-methylpiperazin-1-carboxylate (yield: 51.3%). LC-MS: RT = 2.28 min, [M+ H-56]⁺= 371.26.

### Step C: Synthesis of (S)-3-fluoro-4-(((6-(2-methylpiperazin-1-yl)pyridin-2-yl)oxy)methyl)benzonitrile

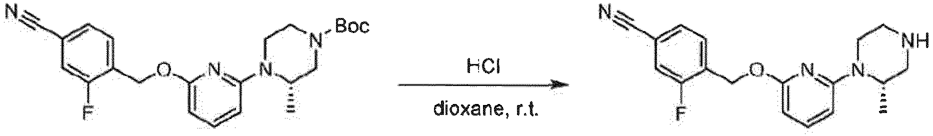

Tert-butyl (S)-4-(6-((4-cyano-2-fluorobenzyl)oxy)pyridin-2-yl)-3-methylpiperazin-1-carboxylate (76.5 mg, 0.18 mmol) was added to hydrochloric acid in 1,4-dioxane (1.00 mmol/mL, 5 mL) at room temperature. The reaction was carried out at room temperature for 1 h under N₂ protection.

After the reaction was completed, the reaction solution was concentrated under reduced pressure. 60.0 mg of pale yellow solid, (S)-3-fluoro-4-(((6-(2-methylpiperazin-1-yl)pyridin-2-yl)oxy)methyl)benzonitrile, was obtained (yield: 100.0%), which was directly used in the next reaction. LC-MS: RT= 1.74 min, [M+H]⁺= 327.14.

### Step D: Synthesis of methyl 2-((S)-1-((S)-4-(6-(((4-cyano-2-fluorobenzyl)oxy)pyridin-2-yl)-3-methylpiperazin-1-yl)ethyl)-1-(((S)-oxetan-2-yl)methyl)-1H-benzo[d]imidazol-6-carboxylate

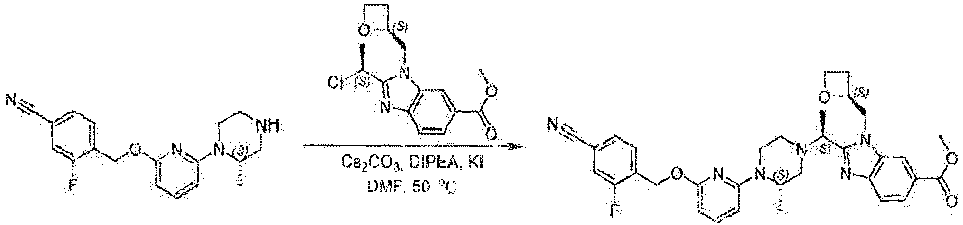

(S)-3-fluoro-4-(((6-(2-methylpiperazin-1-yl)pyridin-2-yl)oxy)methyl)benzonitrile (60.0 mg, 0.18 mmol), methyl 2-((S)-1-chloroethyl)-1-(((S)-oxetan-2-yl)methyl)-1*H*-benzo[*d*]imidazol-6-carboxylate (55.4 mg, 0.18 mmol), cesium carbonate (117.4 mg, 0.36 mmol), and potassium iodide (30.0 mg, 0.18 mmol) were added to *N,N*-dimethylformamide (5.0 mL) at room temperature, and finally *N,N-*diisopropylethylamine (0.50 mL) was added. The reaction was carried out at 50°C for 4.0 h under N₂ protection.

After the reaction was completed, the reaction solution was quenched by adding water and extracted with ethyl acetate (50 mL × 3 times). The organic phases were combined, washed with saturated saline (30 mL × 3 times), then dried with anhydrous sodium sulfate, and finally purified by silica gel column chromatography (eluent: dichloromethane/methanol = 20 : 1) to obtain 43.0 mg of white solid methyl 2-((S)-1-((S)-4-(6-(((4-cyano-2-fluorobenzyl)oxy)pyridin-2-yl)-3-methylpiperazin-1-yl)ethyl)-1-(((S)-oxetan-2-yl)methyl)-1*H-*benzo[d]imidazol-6-carboxylate (yield: 40.1%). LC-MS: RT = 2.00 min, [M+H]⁺= 599.37.

### Step E: Synthesis of 2-((S)-1-((S)-4-(6-(((4-cyano-2-fluorobenzyl)oxy)pyridin-2-yl)-3-methylpiperazin-1-yl)ethyl)-1-(((S)-oxetan-2-yl)methyl)-1H-benzo[d]imidazol-6-carboarylic acid (48B)

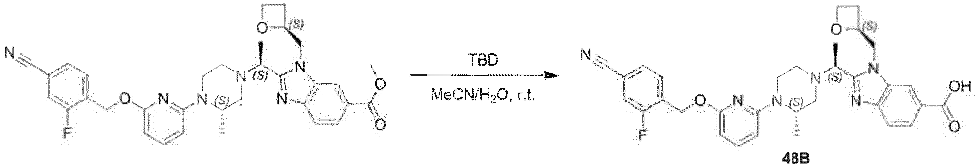

1,5,7-Triazabicyclo[4.4.0]dec-5-ene (77.8 mg, 0.56 mmol) in water (3.0 mL) was added dropwise into a solution of acetonitrile (12.0 mL) containing methyl 2-((S)-1-((S)-4-(6-(((4-cyano-2-fluorobenzyl)oxy)pyridin-2-yl)-3-methylpiperazin-1-yl)ethyl)-1-(((S)-oxetan-2-yl)methyl)-1*H-*benzo[*d*]imidazol-6-carboxylate (43.0 mg, 0.07 mmol) at room temperature. The reaction was carried out at room temperature for 2.0 h.

After the reaction was completed, the reaction solution was quenched by adding water, and the pH was adjusted to 6 by aqueous citric acid solution (0.5 mol/L). Then extracted with dichloromethane (30 mL × 3 times), and the organic phases were combined, washed with aqueous citric acid solution (30 mL × 2 times), and dried with anhydrous sodium sulfate. The crude product was purified by preparative high performance liquid chromatography. The separation conditions are as follows: chromatographic column: Agilent 5 Prep-C18 100 mm×30 mm 5 µM; mobile phase: water (containing 0.1% ammonia) and acetonitrile; flow rate: 20 mL/min; gradient: 36.3% acetonitrile, eluted at 8.23 min; detection wavelength: 254 nm. Upon purification and lyophilization, 9.0 mg of white solid 2-((S)-1-((S)-4-(6-(((4-cyano-2-fluorobenzyl)oxy)pyridin-2-yl)-3-methylpiperazin-1-yl)ethyl)-1-(((S)-oxetan-2-yl)methyl)-1*H* benzo[*d*]imidazol-6-carboxylic acid (**48B**) was obtained (yield: 22.1%). LC-MS: RT = 1.89 min, [M+H]⁺= 585.37.

¹H NMR (500 MHz, DMSO) δ 8.23 (s, 1H), 7.85 (dd, *J* = 10.0, 1.3 Hz, 1H), 7.79 (dd, *J* = 8.4, 1.5 Hz, 1H), 7.69-7.63 (m, 2H), 7.60 (t, *J* = 7.6 Hz, 1H), 7 43 (t, *J* = 8.0 Hz, 1H), 6 21 (d, *J* = 8.2 Hz, 1H), 6.07 (d, *J* = 78 Hz, 1H), 5.41 (d, *J* = 13.7 Hz, 1H), 5.32 (d, *J* = 13.7 Hz, 1H), 5.17-5.09 (m, 1H), 4.89 (dd, *J* = 155, 2.6 Hz, 1H), 4.71 (dd, *J* = 15.5, 5.7 Hz, 1H), 4.55-4.39 (m, 2H), 4.27 (s, 1H), 4.19 (d, *J* = 9.1 Hz, 1H), 3.88 (d, *J* = 12.1 Hz, 1H), 3.20 (s, 1H), 3.01-2.84 (m, 2H), 2.66-2.57 (m, 1H), 2.32 (dd, *J* = 31.7, 17.9 Hz, 3H), 1.43 (d, *J* = 6.7 Hz, 3H), 0.77 (d, *J=* 6.5 Hz, 3H).

### Example 49 2-((S)-1-(4-(4-chloro-6-((4-cyano-2-fluorobenzyl)oxy)pyridin-2-yl)piperazin-1-yl)ethyl)-1-(((S)-oxetan-2-yl)methyl)-1H-benzo[d]imidazol-6-carboxylic acid

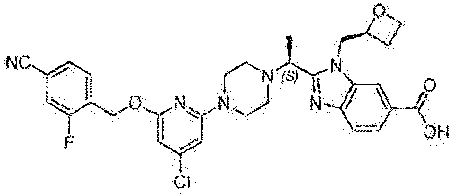

### Step A: Synthesis of tert-butyl 4-(6-bromo-4-chloropyridin-2-yl)piperazin-1-carboxylate

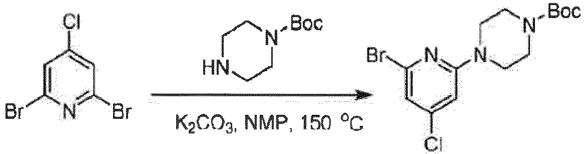

2,6-Dichloro-4-bromopyridine (200 mg, 1.1 mmol), N-Boc-piperazine (214 mg, 1.15 mmol) and potassium carbonate (302 mg, 2.2 mmol) were added to 10 mL of dried NMP, and the reaction was carried out at a temperature of 150°C for 1 h.

The reaction solution was cooled to room temperature after the reaction was completed, and was poured into 20 mL ice water solution. The mixed solution was stirred for 30 min and then filtered by suction. The filter cake was washed with water and dried to obtain 208 mg of white solid tert-butyl 4-(6-bromo-4-chloropyridin-2-yl)piperazin-1-carboxylate (yield: 50.5%). LC-MS: RT = 2.30 min, [M-55]⁺= 320.10.

### Step B: Synthesis of tert-butyl 4-(4-chloro-6-((4-cyano-2-fluorobenzyl)oxy)pyridin-2-yl)piperazin-1-carboxylate

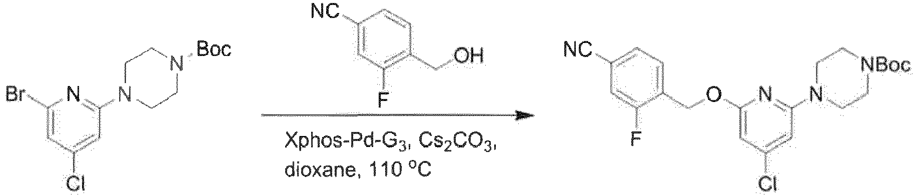

The intermediate tert-butyl 4-(6-bromo-4-chloropyridin-2-yl)piperazin-1-carboxylate (168 mg, 0.45 mmol) and 3-fluoro-4-hydroxymethyl benzonitrile (74.4 mg, 0.49 mmol), methanesulfonato(2-dicyclohexylphosphino-2',4',6'-triisopropyl-1,1'-biphenyl)(2'-amino-1,1'-biphenyl-2-yl)palladium (II) (19 mg, 0.02 mmol) and cesium carbonate (293.4 mg, 0.90 mmol) were added to 10 mL of dried dioxane at room temperature. The reaction was carried out at 110°C for 2 h under N₂ protection.

The reaction solution was evaporated after the reaction was completed, then extracted with 20 mL of ethyl acetate. The ethyl acetate layer was separated, dried with anhydrous sodium sulfate, and evaporated to obtain a pale yellow solid. After purification by column chromatography (n-hexane/ethyl acetate = 5/2), 82 mg of pale yellow solid tert-butyl 4-(4-chloro-6-((4-cyano-2-fluorobenzyl)oxy)pyridin-2-yl)piperazin-1-carboxylate was obtained (yield: 40.8%). LC-MS: RT = 2.29 min, [M-56+]⁺= 391.18.

### Step C: Synthesis of 4-(((4-chloro-6-(piperazin-1-yl)pyridin-2-yl)oxy)methyl)-3-fluorobenzonitrile

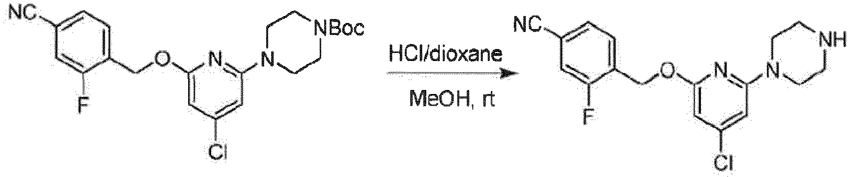

Tert-butyl 4-(4-chloro-6-((4-cyano-2-fluorobenzyl)oxy)pyridin-2-yl)piperazin-1-carboxylate (82 mg, 0.18 mmol) was dissolved in 10 mL of methanol solvent at room temperature, and 10 mL of hydrogen chloride in dioxane with a concentration of 4 M was added under an ice-bath. The reaction was carried out at room temperature for 2 h.

After the reaction was completed, the reaction solution was evaporated to obtain 85 mg of white solid 4-(((4-chloro-6-(piperazin-1-yl)pyridin-2-yl)oxy)methyl)-3-fluorobenzonitrile (yield: 140.5%). LC-MS: RT = 1.77 min, [M+H]⁺= 347.19.

### Step D: Synthesis of methyl 2-((S)-1-(4-(4-chloro-6-((4-cyano-2-fluorobenzyl)oxy)pyridin-2-yl)piperazin-1-yl)ethyl)-1-(((S)-oxetan-2-yl)methyl)-1H-benzo[d]imidazol-6-carboxylate

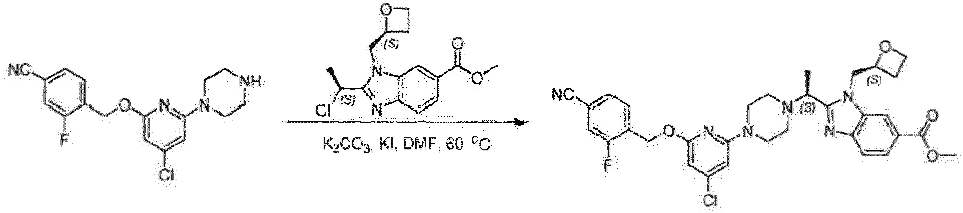

The intermediate 4-(((4-chloro-6-(piperazin-1-yl)pyridin-2-yl)oxy)methyl)-3-fluorobenzonitrile (85 mg, 0.19 mmol), methyl 2-((S)-1-chloroethyl)-1-(((S)-oxetan-2-yl)methyl)-1H-benzo[*d*]imidazol-6-carboxylate (59 mg, 0.19 mmol), potassium carbonate (52.4 mg, 0.38 mmol) and potassium iodide (63.0 mg, 0.38 mmol) were added to 10 mL of DMF at room temperature, and the reaction was carried out at a temperature of 60°C for 10 h.

The reaction solution was poured into ice water solution after the reaction was completed, and a white solid was precipitated. Then the mixture was filtered by suction, and the filter cake was washed with water to obtain 63 mg of white product methyl 2-((S)-1-(4-(4-chloro-6-((4-cyano-2-fluorobenzyl)oxy)pyridin-2-yl)piperazin-1 -yl)ethyl)-1 -(((S)-oxetan-2-yl)methyl)- 1*H*-benzo [d] imidazol-6-carboxylate (yield: 53.8%). LC-MS: RT = 1.98 min, [M+H]⁺= 619.30.

### Step E: Synthesis of 2-((S)-1-(4-(4-chloro-6-((4-cyano-2-tluorobenzyl)oxy)pyridin-2-yl)piperazin-1-yl)ethyl)-1-(((S)-oxetan-2-yl)methyl)-1H-benzo[d]imidazol-6-carboxylic acid

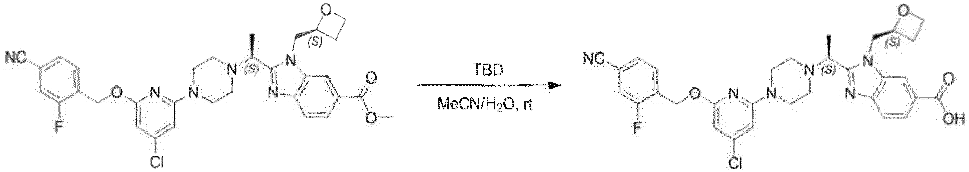

The intermediate methyl 2-((S)-1-(4-(4-chloro-6-((4-cyano-2-fluorobenzyl)oxy)pyridin-2-yl)piperazin-1-yl)ethyl)-1-(((S)-oxetan-2-yl)methyl)-1*H*-benzo[*d*]imidazol-6-carboxylate (63 mg, 0.10 mmol) was dissolved in 5 mL mixed solution of acetonitrile/water (4:1) at room temperature, and then 1,5,7-triazabicyclo[4.4.0]dec-5-ene (34.8 mg, 0.25 mmol) was added at room temperature. The reaction was carried out at room temperature for 4 h.

The reaction solution was poured into 15% aqueous citric acid after the reaction was completed, and the pH was adjusted to 3-4. The reaction solution was extracted with dichloromethane (20 mL), washed with water for 3 times, dried with anhydrous sodium sulfate, and evaporated to obtain a pale yellow solid. After purification by column chromatography (dichloromethane/methanol = 10/1), 30 mg of white solid 2-((S)-1-(4-(4-chloro-6-((4-cyano-2-fluorobenzyl)oxy)pyridin-2-yl)piperazin-1-yl)ethyl)-1-(((S)-oxetan-2-yl)methyl)-1*H*-benzo[*d*]imidazol-6-carboxylic acid was obtained (yield: 49.8%).

LC-MS: RT= 1.88min, [M+H]⁴= 605.37. ¹H NMR (400 MHz, DMSO) δ 8.24 (s, 1H), 7.87 (d, *J* = 10.1 Hz, 1H), 7.79 (dd, *J* = 8.4, 1.3 Hz, 1H), 766 (ddd, *J* = 15.1, 9.5, 7.9 Hz, 3H), 6.39 (s, 1H), 6.18 (d, *J* = 0 9 Hz, 1H), 5.38 (s, 2H), 5.13 (s, 1H), 4.77 (d, *J* = 12.8 Hz, 1H), 4.65 (dd, *J* = 15.5, 5.5 Hz, 1H), 4.45 (td, *J* = 13.4, 7.3 Hz, 2H), 4.16 (dt, *J* = 9 0, 5.7 Hz, 1H), 3.41 (m, 4H), 2.61 (dd, *J* = 16.7, 8.7 Hz, 1H), 2.50 (m, 4H), 2.29 (dd, *J* = 18.0, 7.8 Hz, 1H), 1.42 (d, *J* = 6.7 Hz,3H).

### Example 50 2-((S)-1-(4-(6-((4-cyano-2-fluorobenzyl)oxy)-4-(trifluoromethyl)pyridin-2-yl)piperazin-1-yl)ethyl)-3-(((S)-oxetan-2-yl)methyl)-3H-imidazo[4,5-b]pyridin-5-carboxylic acid

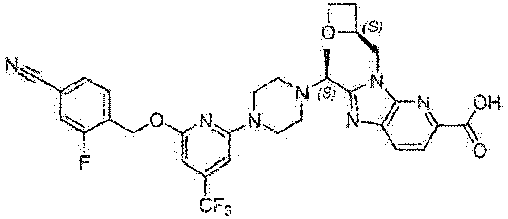

The specific synthesis route is as follows.

### Step A: Synthesis of methyl 2-((S)-1-(4-(6-((4-cyano-2-fluorobenzyl)oxy)-4-(trifluoromethyl)pyridin-2-yl)piperazin-1-yl)ethyl)-3-(((S)-oxetan-2-yl)methyl)-3H-imidazo[4,5-b]pyridin-5-carboxylate

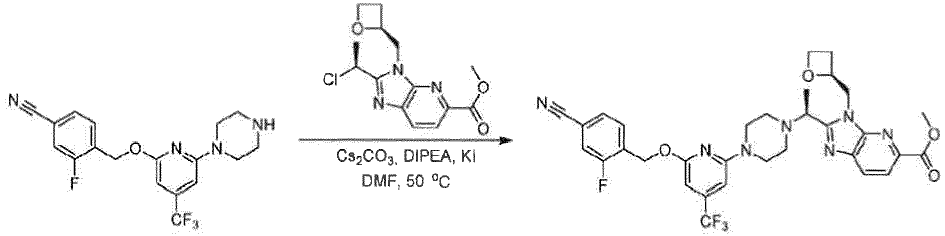

3-Fluoro-4-((((6-(piperazin-1-yl)-4-(trifluoromethyl)pyridin-2-yl)oxy)methyl)benzonitrile (50.0 mg, 0.13 mmol), methyl 2-((S)-1-chloroethyl)-1-(((S)-oxetan-2-yl)methyl)-1*H*-benzo[d]imidazol-6-carboxylate (44.0 mg, 0.13 mmol), cesium carbonate (40.4 mg, 0.13 mmol), and potassium iodide (22.0 mg, 0.13 mmol) were added to *N,N*-dimethylformamide (5.0 mL) at room temperature, and finally *N,N-*diisopropylethylamine (0.50 mL) was added. The reaction was carried out at 50°C for 4.0 h under N₂ protection.

After the reaction was completed, the reaction solution was quenched by adding water and extracted with ethyl acetate (50 mL × 3 times). The organic phases were combined, washed with saturated saline (30 mL × 3 times), then dried with anhydrous sodium sulfate, and finally purified by silica gel column chromatography (eluent: dichloromethane/methanol = 20 : 1) to obtain 37.2 mg of yellow solid methyl 2-((S)-1-(4-(6-((4-cyano-2-fluorobenzyl)oxy)-4-(trifluoromethyl)pyridin-2-yl)piperazin-1-yl)ethyl)-3-(((S)-oxetan-2-yl)methyl)-3*H-*imidazo[4,5-b]pyridin-5-carboxylate (yield: 43.7%). LC-MS: RT = 1.81 min, [M+H]⁺= 654.33.

### Step B: Synthesis of 2-((S)-1-(4-(6-((4-cyano-2-fluorobenzyl)oary)-4-(trifluoromethyl)pyridin-2-yl)piperazin-1-yl)ethyl)-3-(((S)-oxetan-2-yl)methyl)-3H-imidazo[4,5-blpyridin-5-carboxylic acid

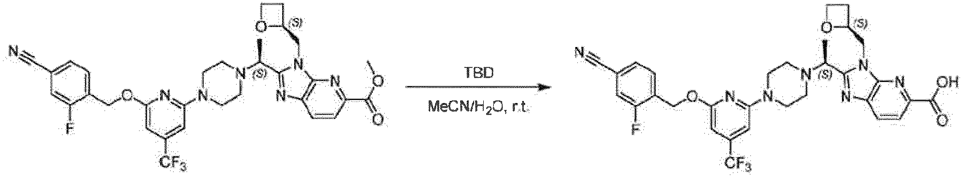

1,5,7-Triazabicyclo[4.4.0]dec-5-ene (66.7 mg, 0.48 mmol) in water (3.0 mL) was added dropwise into a solution of acetonitrile (12.0 mL) containing methyl 2-((S)-1-(4-(6-((4-cyano-2-fluorobenzyl)oxy)-4-(trifluoromethyl)pyridin-2-yl)piperazin-1-yl)ethyl)-3-(((S)-oxetan-2-yl)methyl)-3*H*-imidazo[4,5-b]pyridin-5-carboxylate (37.2 mg, 0.06 mmol) at room temperature. The reaction was carried out at room temperature for 3.0 h.

After the reaction was completed, the reaction solution was quenched by adding water, and the pH was adjusted to 6 by aqueous citric acid solution (0.5 mol/L). Then the mixture was extracted with dichloromethane (30 mL × 3 times), and the organic phases were combined, washed with aqueous citric acid solution (30 mL × 2 times), and dried with anhydrous sodium sulfate. The crude product was purified by preparative high performance liquid chromatography. The separation conditions are as follows: chromatographic column: Agilent 5 Prep-C18 100 mm×30 mm 5 µM; mobile phase: water (containing 0.1% ammonia) and acetonitrile; flow rate: 20 mL/min; gradient: 33.7% acetonitrile, eluted at 7.52 min; detection wavelength: 254 nm. Upon purification and lyophilization, 10.0 mg of white solid 2-((S)-1-(4-(6-((4-cyano-2-fluorobenzyl)oxy)-4-(trifluoromethyl)pyridin-2-yl)piperazin-1-yl)ethyl)-3-(((S)-oxetan-2-yl)methyl)-3*H*-imidazo[4,5-b]pyridin-5-carboxylic acid was obtained (yield: 26.1%). LC-MS: RT= 1.69 min, [M+H]⁺= 640.33.

### Example 51 2-((S)-1-(4-(6-((4-cyano-2-fluorobenzyl)oxy)pyridin-2-yl)piperazin-1-yl)ethyl)-3-(((S)-oxetan-2-yl)methyl)-3H-imidazo[4,5-b]pyridin-5-carboxylic acid (51A) and 2-((R)-1-(4-(6-((4-cyano-2-fluorobenzyl)oxy)pyridin-2-yl)piperazin-1-yl)ethyl)-3-(((S)-oxetan-2-yl)methyl)-3H-imidazo[4,5-b]pyridin-5-carboxylic acid (51B)

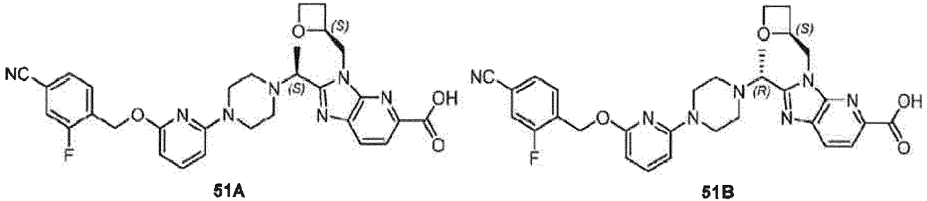

The specific synthesis route is as follows.

### Step A: Synthesis of methyl 2-(1-(4-(6-((4-cyano-2-fluorobenzyl)oxy)pyridin-2-yl)piperazin-1-yl)ethyl)-3-(((S)-oxetan-2-yl)methyl)-3H-imidazo[4,5-b]pyridin-5-carboxylate

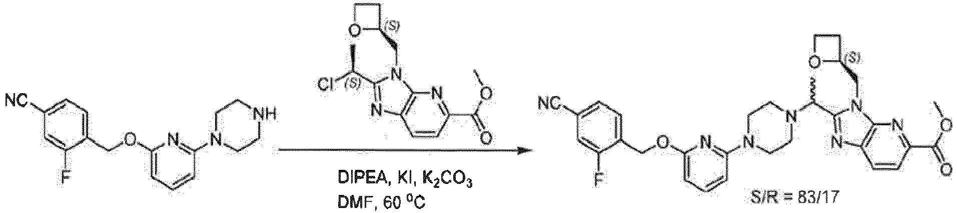

At 25°C, *N,N-*diisopropylethylamine (1.0 mL), potassium iodide (420 mg, 3.13 mmol), and potassium carbonate (577 mg, 4.18 mmol) were added into a solution of acetonitrile (8.0 mL) containing 4-(6-((4-cyano-2-fluorobenzyl)oxy)pyridin-2-yl)piperazine hydrochloride (652 mg, 2.09 mmol), and then N,N-dimethylformamide (10 mL) was added. Finally methyl 2-((S)-1-chloroethyl)-3-(((S)-oxetan-2-yl)methyl)-3*H*-imidazo[4,5-b]pyridin-5-carboxylate (650 mg, 2.09 mmol) was added. The reaction was carried out at 60°C for 3.0 h under N₂ protection.

After the reaction was completed, the reaction solution was quenched by adding water and extracted with ethyl acetate (30 mL × 2 times). The organic phases were combined, then washed with saturated saline (30 mL), dried with anhydrous sodium sulfate, and concentrated under reduced pressure. The residue was purified by silica gel column chromatography (eluent: ethyl acetate/n-hexane = 2/1). 690 mg of yellow solid, methyl 2-(1-(4-(6-((4-cyano-2-fluorobenzyl)oxy)pyridin-2-yl)piperazin-1-yl)ethyl)-3-(((S)-oxetan-2-yl)methyl)-3*H*-imidazo[4,5-b]pyridin-5-carboxylate, was obtained (yield: 56.4%, *dr =* 66%). LC-MS: RT = 1.93 min, [M+H]⁺= 586.26.

### Step B: Synthesis of 2-((S)-1-(4-(6-((4-cyano-2-fluorobenzyl)oxy)pyridin-2-yl)piperazin-1-yl)ethyl)-3-(((S)-oxetan-2-yl)methyl)-3H-imidazo[4,5-b]pyridin-5-carboxylic acid (51A) and 2-(R)-1-(4-(6-((4-cyano-2-fluorobenzyl)oxy)pyridin-2-yl)piperazin-1-yl)ethyl)-3-(((S)-oxetan-2-yl)methyl)-3H-imidazo[4,5-b]pyridin-5-carboxylic acid (51B)

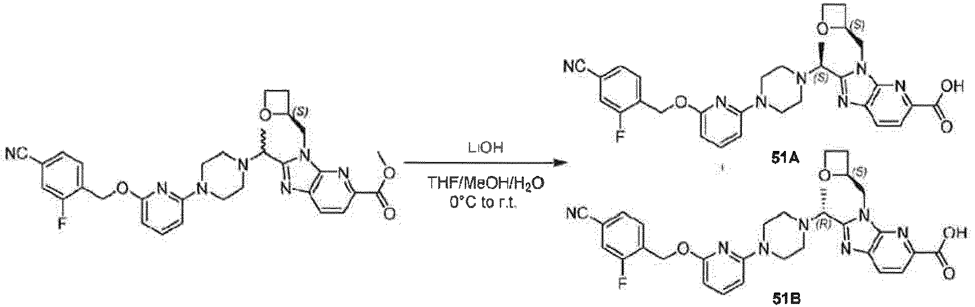

Lithium hydroxide (198 mg, 4.72 mmol) in water (1 mL) was added dropwise into a mixed solution of tetrahydrofuran (3 mL) and methanol (1 mL) containing methyl 2-(1-(4-(6-((4-cyano-2-fluorobenzyl)oxy)pyridin-2-yl)piperazin-1-yl)ethyl)-3-(((S)-oxetan-2-yl)methyl)-3*H*-imidazo[4,5-b]pyridin-5-carboxylate (690 mg, 1.18 mmol) at 0°C, and the reaction was carried out at 25°C for 30 min.

After the reaction was completed, the reaction solution was quenched by adding water and extracted with ethyl acetate (30 mL × 2 times). The organic phases were combined, then washed with saturated saline (20 mL), dried with anhydrous sodium sulfate, and concentrated under reduced pressure. The residue was purified by silica gel column chromatography (eluent: methanol/dichloromethane = 1/10). 290 mg of white solid 2-((S)-1-(4-(6-((4-cyano-2-fluorobenzyl)oxy)pyridin-2-yl)piperazin-1-yl)ethyl)-3-(((S)-oxetan-2-yl)methyl)-3*H*-imidazo[4,5-b]pyridin-5-carboxylic acid (**51A**) (yield: 43.0%) and 59 mg of white solid 2-((R)-1-(4-(6-((4-cyano-2-fluorobenzyl)oxy)pyridin-2-yl)piperazin-1-yl)ethyl)-3-(((S)-oxetan-2-yl)methyl)-3*H*-imidazo[4,5-b]pyridin-5-carboxylic acid (**51B**) (yield: 8.7%) were obtained. The crude products were purified by preparative high performance liquid chromatography. The separation conditions are as follows: chromatographic column: Agilent 5 Prep-C18 100 mm×30 mm 5 µM; mobile phase: eluted with water (containing 0.1% ammonia) and acetonitrile; flow rate: 20 mL/min; gradient: 51A and 51B were eluted by 21% acetonitrile at 10.12 min and 12.17 min, respectively; detection wavelength: 254 nm.

Compound **51A**: HPLC: RT = 10.12 min. LC-MS: RT = 1.79 min, [M+H]⁺=572.30. ¹H NMR (400 MHz, DMSO-*d₆*) δ 8.14 (d, *J* = 8.2 Hz, 1H), 7.97 (d, *J* = 8.2 Hz, 1H), 7.87 (dd, *J* = 10.0, 1.5 Hz, 1H), 7.69 (dd, *J* = 7.9, 1.4 Hz, 1H), 7.64 (t, *J* = 7.6 Hz, 1H), 7.45 (t, *J* = 8.0 Hz, 1H), 6.30 (d, *J* = 8.1 Hz, 1H), 6.10 (d, *J* = 7.8 Hz, 1H), 5.38 (s, 2H), 5.30-5.24 (m, 1H), 4.82-4.70 (m, 2H), 4.63-4.58 (m, 1H), 4.44-4 39 (m, 1H), 4.11-4.06 (m, 1H), 3.43-3.33 (m, 4H), 2.64-2.58 (m, 1H), 2.56 2.52 (m, 4H), 2.39-2.30 (m, 1H), 1.45 (d, *J* = 6.7 Hz, 3H). Compound 51B: HPLC: RT = 12.17 min. LC-MS: RT = 1.79 min, [M-H]⁺=572.30. ¹H NMR (400 MHz, DMSO) δ 8.09 (d, *J* = 8.2 Hz, 1H), 7.95 (d, *J* = 8.2 Hz, 1H), 7.87 (dd, *J* = 10.0, 1.4 Hz, 1H), 7.69 (dd, *J* = 7.9, 1.5 Hz, 1H), 7.67-7.61 (m, 1H), 7.46 (t, *J* = 8.0 Hz, 1H), 6.31 (d, *J* = 8.1 Hz, 1H), 6.11 (d, *J* = 7.8 Hz, 1H), 5.39 (s, 2H), 5.07-5.00 (m, 2H), 4.69-4.49 (m, 4H), 3.47-3.37 (m, 4H), 2.79-2.70 (m, 1H), 2.66-2.52 (m, 5H), 1.42 (d, *J* = 6.8 Hz, 3H).

### Example 52 2-((S)-1-(4-(6-((4-chloro-2-fluorobenzyl)oxy)pyridin-2-yl)piperazin-1-yl)ethyl)-3-(((S)-oxetan-2-yl)methyl)-3H-imidazo[4,5-b]pyridin-5-carboxylic acid (52A) and 2-((R)-1-(4-(6-((4-chloro-2-fluorobenzyl)oxy)pyridin-2-yl)piperazin-1-yl)ethyl)-3-(((S)-oxetan-2-yl)methyl)-3H-imidazo[4,5-b]pyridin-5-carboxylic acid (52B)

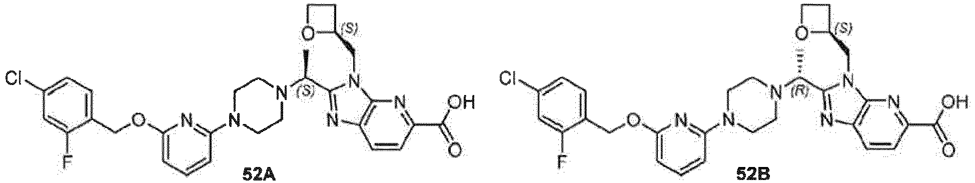

The specific synthesis route is as follows.

### Step A: Synthesis of 2-chloro-6-((4-chloro-2-fluorobenzyl)oxy)pyridine

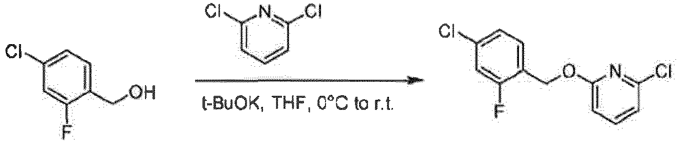

Potassium tert butoxide (4.88 g, 43.60 mmol) was added into a solution of tetrahydrofuran (50.0 mL) containing (4-chloro-2-fluorophenyl)methanol (5.0 g, 31.14mmol) at 0°C, and the mixed solution was stirred for 30 min. Then 2,6-dichloropyridine (4.2 g, 28.38 mmol) was added and the reaction was carried out overnight at room temperature.

After the reaction was completed, the reaction solution was quenched by adding water (50 mL) and extracted with ethyl acetate (50 mL × 3 times). The organic phases were combined, then washed with saturated saline (50 mL), dried with anhydrous sodium sulfate, and concentrated under reduced pressure. The residue was purified by silica gel column chromatography (eluent: ethyl acetate/n-hexane = 1/2). 7.53 g of yellow oily product 2-chloro-6-((4-chloro-2-fluorobenzyl)oxy)pyridine was obtained (yield: 88.9%). LC-MS: RT = 2.27 min, [M+H]⁺= 272.05.

### Step B: Synthesis of tert-butyl 4-(6-((4-chloro-2-fluorobenzyl)oxy)pyridin-2-yl)piperazin-1-carboxylate

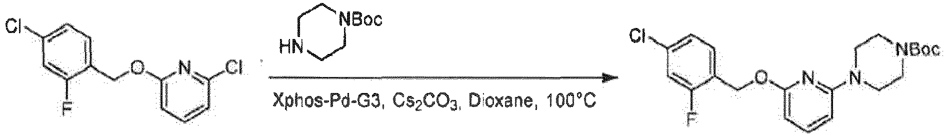

Tert-butyl piperazin-1-carboxylate (5.15 g, 27.68 mmol), cesium carbonate (18.05 g, 55.36 mmol) and methanesulfonato(2-dicyclohexylphosphino-2',4',6'-triisopropyl-1,1 '-biphenyl)(2'-amino-1,1 '-biphenyl-2-yl)palladium (II) (702.5 mg, 0.83 mmol) were added into a solution of 1,4-dioxane containing 2-chloro-6-((4-chloro-2-fluorobenzyl)oxy)pyridine (7.53 g, 27.68 mmol) at room temperature, and the reaction was carried out at 100°C for 3 h.

After the reaction was completed, the reaction solution was quenched by adding water and extracted with ethyl acetate (50 mL × 3 times). The organic phases were combined, then washed with saturated saline (50 mL × 2 times), dried with anhydrous sodium sulfate, and concentrated under reduced pressure. The residue was purified by silica gel column chromatography (eluent: ethyl acetate/n-hexane = 1/2). 4.7 g of white solid tert-butyl 4-(6-((4-chloro-2-fluorobenzyl)oxy)pyridin-2-yl)piperazin-1-carboxylate was obtained (yield: 40.2%). LC-MS: RT = 2.39 min, [M+H]⁺= 422.22.

### Step C: Synthesis of 4-(6-((4-chloro-2-fluorobenzyl)oxy)pyridin-2-yl)piperazine

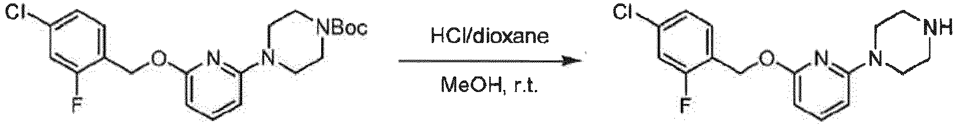

Hydrochloric acid in dioxane (20.0 mL, 4 mol/L) was added into a solution of methanol (30 mL) containing tert-butyl 4-(6-(((4-chloro-2-fluorobenzyl)oxy)pyridin-2-yl)piperazin-1-carboxylate (4.34 g, 10.28 mmol) at room temperature, and the reaction was carried out at room temperature for 3 h.

After the reaction was completed, the reaction solution was directly spin-dried to obtain 3.68 g of white solid 4-(6-((4-chloro-2-fluorobenzyl)oxy)pyridin-2-yl)piperazine hydrochloride, which was directly used in the next step. LC-MS: RT = 1.73 min, [M+H]⁺= 322.15.

### Step D: Synthesis of methyl 2-(1-(4-(6-((4-chloro-2-fluorobenzyl)oxy)pyridin-2-yl)piperazin-1-yl)ethyl)-3-(((S)-oxetan-2-yl)methyl)-3H-imidazo[4,5-b]pyridin-5-carbooylate

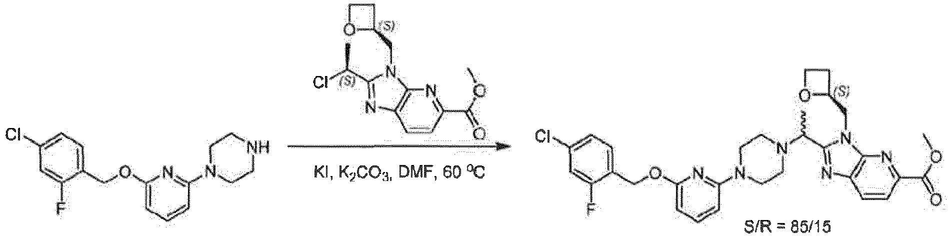

Methyl 2-(1-chloroethyl)-3-(((S)-oxetan-2-yl)methyl)-3*H*-imidazo[4,5-b]pyridin-5-carboxylate (650 mg, 2.09 mmol), potassium iodide (521 mg, 3.14 mmol) and potassium carbonate (577 mg, 4.18 mmol) were added into a solution of acetonitrile (8.0 mL) containing 4-(6-((4-chloro-2-fluorobenzyl)oxy)pyridin-2-yl)piperazine hydrochloride (750 mg, 2.09 mmol) at room temperature, and the reaction solution was carried out overnight at 60°C.

After the reaction was completed, the reaction solution was quenched by adding water and extracted with ethyl acetate (30 mL × 2 times). The organic phases were combined, then washed with saturated saline (30 mL), dried with anhydrous sodium sulfate, and concentrated under reduced pressure. The residue was purified by silica gel column chromatography (eluent: ethyl acetate/n-hexane = 2/1). 870 mg of yellow solid, methyl 2-(1-(4-(6-((4-chloro-2-fluorobenzyl)oxy)pyridin-2-yl)piperazin-1-yl)ethyl)-3-(((S)-oxetan-2-yl)methyl)-3*H*-imidazo[4,5-b]pyridin-5-carboxylate, was obtained (yield: 70.0%). LC-MS: RT= 1.93 min, [M+H]⁺= 595.25.

### Step E: Synthesis of 2-((S)-1-(4-(6-((4-chloro-2-fluorobenzyl)oxy)pyridin-2-yl)piperazin-1-yl)ethyl)-3-(((S)-oxetan-2-yl)methyl)-3H-imidazo[4,5-b]pyridin-5-carbonylic acid (52A) and 2-((R)-1-(4-(6-((4-chloro-2-fluorobenzyl)oxy)pyridin-2-yl)piperazin-1-yl)ethyl)-3-(((S)-oxetan-2-yl)methyl)-3H-imidazo[4,5-b]pyridin-5-carboxylic acid (52B)

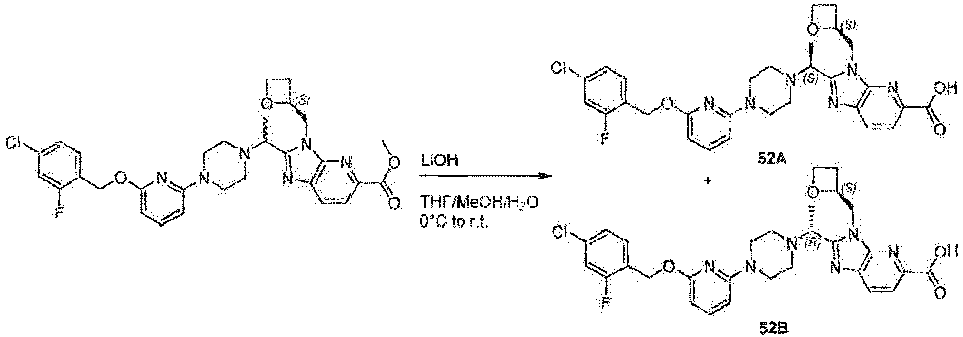

Lithium hydroxide (307 mg, 7.30 mmol) in water (1 mL) was added dropwise into a mixed solution of tetrahydrofuran (3 mL) and methanol (1 mL) containing methyl 2-(1-(4-(6-((4-chloro-2-fluorobenzyl)oxy)pyridin-2-yl)piperazin-1-yl)ethyl)-3-(((S)-oxetan-2-yl)methyl)-3*H*-imidazo[4,5-b]pyridin-5-carboxylate (870 mg, 1.46 mmol) at 0°C, and the reaction was carried out at room temperature for 30 min.

After the reaction was completed, the reaction solution was quenched by adding water and extracted with ethyl acetate (30 mL × 2 times). The organic phases were combined, then washed with saturated saline (20 mL), dried with anhydrous sodium sulfate, and concentrated under reduced pressure. The residue was purified by silica gel column chromatography (eluent: methanol/dichloromethane = 1/10). 300 mg of white solid 2-((S)-1-(4-(6-((4-chloro-2-fluorobenzyl)oxy)pyridin-2-yl)piperazin-1-yl)ethyl)-3-(((S)-oxetan-2-yl)methyl)-3*H*-imidazo[4,5-b]pyridin-5-carboxylic acid (**52A**) (yield: 35.4%) and 53 mg of white solid 2-((R)-1-(4-(6-((4-chloro-2-fluorobenzyl)oxy)pyridin-2-yl)piperazin-1-yl)ethyl)-3-(((S)-oxetan-2-yl)methyl)-3*H*-imidazo[4,5-b]pyridin-5-carboxylic acid (**52B**) (yield: 6.2%) were obtained. The crude products were purified by preparative high performance liquid chromatography. The separation conditions are as follows: chromatographic column: Agilent 5 Prep-C18 100 mm×30 mm 5 µM; mobile phase: eluted with water (containing 0.1% ammonia) and acetonitrile; flow rate: 20 mL/min; gradient: 52A and 52B were eluted by 25% acetonitrile at 14.09 min and 15.49 min, respectively; detection wavelength: 254 nm.

Compound **52A**: HPLC: RT = 14.09 min. LC-MS: RT = 1.91 min, [M+H]⁺ = 581.25. ¹H NMR (400 MHz, DMSO) δ 8.14 (d, *J* =8.2 Hz, 1H),7.97 (d, *J* = 8.2 Hz, 1H), 7.49 (t, *J* = 8.2 Hz, 1H), 7.46-7.41 (m, 2H), 7.28 (dd, *J* = 8.2, 2.0 Hz, 1H), 6.29 (d, *J* = 8.1 Hz, 1H), 6.07 (d, *J* = 7.8 Hz, 1H), 5.28 (s, 2H), 4.83-4.70 (m, 2H), 4.62 (q, *J* = 6.5 Hz, 1H), 4.44-4.39 (m, 1H), 4.11-4.06 (m, 1H), 3.42-3.19 (m, 5H), 2.64-2.55 (m, 5H), 2.39-2.32 (m, 1H), 1.47 (d, *J* = 6.8 Hz, 3H).

Compound **52B**: HPLC: RT = 15.49 min. LC-MS: RT = 1.91 min, [M+H]⁺ = 581.25. ¹H NMR (400 MHz, DMSO) δ 8.03 (d, *J* = 8.1 Hz, 1H), 7.92 (d, *J* = 8.2 Hz, 1H), 7.51 (d, *J* = 8.0 Hz, 1H), 7.49-7.41 (m, 2H), 7.28 (dd, *J* = 8.2, 2.1 Hz, 1H), 6.31 (d, *J* = 8.1 Hz, 1H), 6.07 (d, *J* = 7.8 Hz, 1H), 5.29 (s, 2H), 5.04-5.00 (m, 2H), 4.69-4.50 (m, 3H), 3.49-3.42 (m, 3H), 3.29-3.19 (m, 2H), 2.79-2.71 (m, 1H), 2.67-2.55 (m, 4H), 1.92-1.86 (m, 1H), 1.42 (d, *J* = 6.6 Hz, 3H).

### Example 53: Measurement of cAMP signal activation of the compounds in human GLP1R cells in vitro

### 1. Cells culture

A cell line stably expressing human GLP1 R (hGLPIR- U2OS) was used in this experiment. The cells were cultured in a growth medium (complete medium, DMEM+10% FBS+500 µg/mL G418 (Promega)) in an incubator at 37°C and has 5% CO₂ saturated humidity.

### 2. cAMP Determination

hGLP1R-U2OS was inoculated into 384-well plate with 1.0×10⁴ cells/well (5 µL) (cell working solution: DMEM+0.1% BSA+1 mM IBMX (available from Bide Pharmatech Ltd.)), and was placed in an incubator at 37°C and held for 30 min. The compounds to be tested were diluted stage-by-stage with Stimulation Buffer 1 (1X) in the kit, and the diluted compounds were added to the culture plate with 5 µL/well . They were incubated in an incubator at 37°C for 30 min. Then, 5 µL/well of cAMP-d2 prepared with Lysis & Detection buffer and 5µL/well of Anti cAMP-Cryptate working solution were added successively, and they were incubated at room temperature for 60 min before detection.

### 3. Data processing

The ratio of signal emitted by donor to receptor in each well was calculated as follows: HTRF rate = signal at 665 nm / signal at 620 nm × 104, and then the HTRF rate was converted into a reaction rate (%). The concentration-response curve of each Example compound was created by a 4-parameter equation as Y=Bottom+(Top-Bottom)/(1+10^((LogIC50-X)^{∗}HillSlope)), and the half maximal (50%) effective concentration (EC₅₀) was calculated. The results are shown in Table 1.

**Table 1: EC₅₀ of the compounds of the present invention on human GLP-1 R.**

| Example Compounds | EC₅₀ (nM) |
|---|---|
| 1A | 1.71 |
| 2A | 3.18 |
| 5 | 1.04 |
| 8 | 10.3 |
| 9 | 3.48 |
| 11 | 3.45 |
| 12 | 3.89 |
| 13 | 18.72 |
| 14 | 16.85 |
| 15 | 2.57 |
| 17 | 2.92 |
| 18 | 2.66 |
| 19 | 3.94 |
| 20A | 4.66 |
| 31 | 14.4 |
| 39A | 15.5 |
| 42 | 11.97 |
| 45 | 24.55 |
| 51A | 2.28 |
| 52A | 2.00 |

| | |
|---|---|
| Conclusion: The compounds of the present invention have significant activating activity on human GLP-1 R. | |

### Example 54: Pharmacokinetic study of the compounds of the present invention on rat

### 1. Experimental materials

SD rats: male, 180-250 g, purchased from Beijing Vital River Laboratory Animal Technology Co., Ltd.

Reagents: DMSO (dimethyl sulfoxide), PEG-400 (polyethylene glycol 400), physiological saline, heparin, acetonitrile, formic acid, and propranolol (internal standard), all of which are commercially available.

Instruments: Thermo Fisher Scientific LC-MS (U300 UPLC, TSQ QUANTUMN ULTRA triple quadrupole mass spectrometer).

### 2. Experimental procedure

A compound was weighed and dissolved in a DMSO-PEG-400-physiological saline (5:60:35, v/v/v) system. After the rats were administered with the compound intravenously or by gavage, 200 µL of venous blood samples were collected at 15 min, 30 min, 1 h, 2 h, 5 h, 7 h, and 24 h (for i.v. group an additional collection was performed at 5 min) in heparinized EP tubes respectively, which were centrifuged at 12,000 rpm for 2 min, and the plasma was frozen at -80°C for further testing. A certain amount of the test substance was precisely weighed and dissolved in DMSO to obtain a stock solution with a concentration of 2 mg/mL. An appropriate amount of the stock solution of the compound was accurately pipetted and diluted with acetonitrile to prepare a standard series solution. 20 µL of each of the above standard series solution was accurately pipetted, added with 180 µL of blank plasma, and vortexed mixed to prepare plasma samples corresponding to plasma concentrations of 0.3, 1, 3, 10, 30, 100, 300, 1000, 3000 ng/mL. Two-sample analysis was performed for each concentration to establish a standard curve. 30 µL of plasma (for samples that are obtained at 5 min, 15 min, 30 min, and 1 h after intravenous administration, the plasma were diluted 10 times) was taken, added with 200 µL of a solution of internal standard propranolol in acetonitrile (50 ng/mL), and vortexed mixed. The mixture was then added with 100 µL of pure water, and vortexed and mixed again. It was centrifuged at 4000 rpm for 5 min, and the supernatant was collected for LC-MS analysis. LC-MS detection conditions were as follows.

Chromatographic column: Thermo Scientific HYPERSIL GOLD C-18 UPLC column, 100*2.1 mm, 1.7 µm.

Mobile phase: water (with 0.1% formic acid)-acetonitrile; gradient elution was performed according to the table below.

| **Time (min)** | **Water (with 0.1% formic acid)** | **Acetonitrile** |
|---|---|---|
| 0 | 90% | 10% |
| 0.6 | 90% | 10% |
| 1 | 10% | 90% |
| 26 | 10% | 90% |
| 2.61 | 90% | 10% |
| 4 | 90% | 10% |

### 3. Data processing

After blood drug concentrations was detected by LC-MS, WinNonlin 6.1 software was used to calculate pharmacokinetic parameters based on non-compartmental model. The results are shown in Table 2.

**Table 2: Rat pharmacokinetic results of the compounds of the present invention**

| Example Compounds | Dose (mg/kg) Administration Route | Cmax (ng/ml) | AUClast (h*ng/ml) | T_{1/2}(h) | Cl (mL/min/kg) | F (%) |
|---|---|---|---|---|---|---|
| 1A | 1, i.v | 2310 | 2430 | 2.89 | 6.88 | 49.0 |
| | 5, p.o | 747 | 5950 | 3.4 | | |
| 2A | 1, i.v | 2330 | 1250 | 0.71 | 13.3 | 63.5 |
| | 5, p.o | 1290 | 3970 | 3.11 | | |
| 5 | 1, i.v | 4730 | 5770 | 3.87 | 2.88 | 72.8 |
| | 5, p.o | 3470 | 21000 | 2.76 | | |
| 12 | 1, i.v | 2250 | 1700 | 0.87 | 9.79 | 168.2 |
| | 5, p.o | 3780 | 14300 | 2.69 | | |
| 20A | 1, i.v | 1290 | 539 | 0.31 | 30.7 | 46.4 |
| | 5, p.o | 348 | 1250 | 1.17 | | |
| 44 | 1, i.v | 4360 | 2260 | 0.69 | 7.37 | 57.3 |
| | 5, p.o | 1410 | 6470 | 2.9 | | |
| 51A | 1, i.v | 1990 | 613 | 0.71 | 27.2 | 34.3 |
| | 5, p.o | 323 | 1050 | 1.06 | | |
| 52A | 1,i.v | 2050 | 1130 | 0.95 | 14.7 | 76.6 |
| | 5, p.o | 1160 | 4330 | 2.77 | | |
| Example 4A-1 (CN201780086550.1) | 1, i.v | 3510 | 1560 | 1.56 | 11 | 2.6 |
| | 5, p.o | 49 | 202 | 1.51 | | |
| Example 10A-77 (CN20 1780086550.1) | 1, i.v | 1130 | 388 | 0.422 | 41.9 | 3.8 |
| | 5, p.o | 28 | 74.3 | 1.39 | | |

Conclusion: It can be seen from Table 2 that the compounds of the present invention have an improved oral absorption in rats, and have improved exposure and bioavailability.

### Example 55: Study on the effect of the compounds of the present invention on hERG potassium ion channel

### 1. Cells Culture

A cell line CHO-hERG from ovary of Chinese hamster that stably expresses hERG potassium channel was used in this experiment. The cells were cultured in F12 complete medium containing 10% FBS, 1% antibiotics (G418), and 89 µg/mL hygromycin. The recovery medium was a F12 medium containing 10% FBS. Culture was performed at 37°C and under 5% CO₂.

### 2. hERG determination

The inhibitory activity of the compounds of the present invention in series gradient concentrations on the potassium channel in CHO-hERG was determined by manual patch clamp system.

### 3. Data processing

The current value of hERG is imported into EXCEL for data analysis, and the measured IC₅₀ values are shown in Table 3.

**Table 3: Inhibitory effect of the compounds of the present invention on hERG potassium ion channel**

| **Example compound** | IC₅₀(µM) |
|---|---|
| 20A | >30 |
| **Example** 4A-1 (CN201780086550.1) | 11.87 |

Conclusion: It can be seen from Table 3 that the compounds of the present invention have weak inhibitory effects on hERG. Besides, the IC₅₀ values of some compounds tested according to the above experimental method are even greater than 100 µM, which indicate that the cardiovascular-related side effects caused by hERG can be significantly reduced.

The above-mentioned examples are preferred examples of the present invention. However, the embodiments of the present invention are not limited by the above-mentioned examples, and any other changes, modifications, substitutions, combinations, and simplifications that do not deviate from the spirit and principle of the present invention should be regarded as equivalent replacements, and are all included within the protection scope of the present invention.

## Claims

1. A compound of Formula (I): 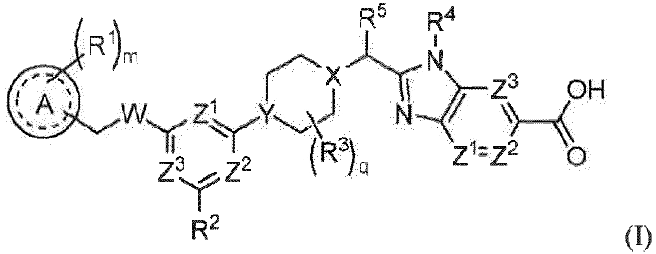
or a stereoisomer, tautomer, or pharmaceutically acceptable salt thereof, wherein,
A is selected from the group consisting of phenyl ring, heteroaromatic ring, and 8- to 10-membered fused aromatic ring;
R¹ is selected from the group consisting of hydrogen, halogen, cyano, alkyl, alkoxy, haloalkyl, haloalkoxy, alkoxyalkyl, cycloalkyl, heterocycloalkyl, alkyl substituted by one or more R⁷, aryl substituted by one or more R⁸, and heteroaryl substituted by one or more R⁸;
m is 0, 1, 2, or 3;
W is O or NH;
R² is selected from the group consisting of hydrogen, halogen, cyano, alkyl, haloalkyl, alkoxy, and alkoxyalkyl;
R³ is selected from the group consisting of fluorine, hydroxyl, cyano, oxo (=O), C₁₋₃ alkyl, OC₁₋₃ alkyl, C₃₋₄ cycloalkyl, and C₃₋₄ spiroalkyl formed by cyclization of two R³ together, wherein the C₁₋₃ alkyl, OC₁₋₃ alkyl, C₃₋₄ cycloalkyl, and C₃₋₄ spiroalkyl can be substituted by 0 to 3 fluorine atoms or 0 to 1 hydroxyl where the valence allows;
q is 0, 1, or 2;
X is CH or N;
Y is CH or N;
R⁵ is selected from the group consisting of halogen, hydroxyl, alkyl, haloalkyl, alkoxy, haloalkoxy, alkoxyalkyl, cycloalkyl, heterocycloalkyl, alkylcycloalkyl, and alkylheterocycloalkyl;
R⁴ is -C₁₋₃ alkyl, -C₀₋₃ alkylene-C₃₋₆ cycloalkyl, -C₀₋₃ alkylene-R⁹, or -C₁₋₃ alkylene-R⁶, wherein the alkyl can be substituted by 0 to 3 substituents independently selected from the group consisting of 0 to 3 F atoms or by 0 to 1 substituent selected from the group consisting of -C₀₋₁ alkylene-CN, -C₀₋₁ alkylene-OR^{O} and -N(R^{N})₂ where the valence allows, and wherein the alkylene and cycloalkyl can be independently substituted by 0 to 2 substituents independently selected from the group consisting of 0 to 2 F atoms or by 0 to 1 substituent selected from the group consisting of -C₀₋₁ alkylene-CN, -C₀₋₁ alkylene-OR^{O} and -N(R^{N})₂ where the valence allows;
R⁹ is 4- to 6-membered heterocycloalkyl, wherein, where the valence allows, the heterocycloalkyl can be substituted by 0 to 2 substituents independently selected from the group consisting of
0 to 1 oxo (=O),
0 to 1 -CN,
0 to 2 F atoms, and
0 to 2 substituents independently selected from the group consisting of -C₁₋₃ alkyl and -OC₁₋₃ alkyl, wherein, where the valence allows, the alkyl of the -C₁₋₃ alkyl and -OC₁₋₃ alkyl can be substituted by 0 to 3 substituents independently selected from the group consisting of
0 to 3 F atoms,
0 to 1 -CN, and
0 to 1 -OR^{O};
R⁶ is 5- to 6-membered heteroaryl, wherein, where the valence allows, the heteroaryl can be substituted by 0 to 2 substituents independently selected from the group consisting of
0 to 2 halogen,
0 to 1 substituent selected from the group consisting of -OR^{O} and -N(R^{N})₂, and
0 to 2 -C₁₋₃ alkyl, wherein, where the valence allows, the alkyl can be substituted by 0 to 3 substituents independently selected from the group consisting of
0 to 3 F atoms, and
0 to 1 -OR^{O};
each R^{O} is independently H or -C₁₋₃ alkyl, wherein the -C₁₋₃ alkyl can be substituted by 0 to 3 F atoms;
each R^{N} is independently H or -C₁₋₃ alkyl;
Z¹, Z², and Z³ are each independently -CR^{Z} or N, and each R^{Z} is independently H, F, Cl, or -CH₃;
R⁷ is selected from the group consisting of halogen, cyano, hydroxyl, cycloalkyl, heterocycloalkyl, and sulfonyl; and
R⁸ is selected from the group consisting of halogen, cyano, alkyl, haloalkyl, alkoxy, haloalkoxy, alkoxyalkyl, cycloalkyl, heterocycloalkyl, and sulfonyl.

2. The compound according to claim 1 or the stereoisomer, tautomer, or pharmaceutically acceptable salt thereof, wherein the compound is represented by Formula (III): 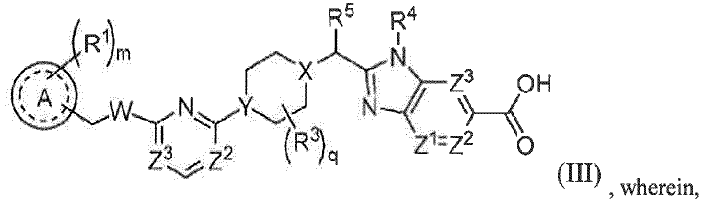
A is selected from the group consisting of phenyl ring, heteroaromatic ring, and 8- to 10-membered fused aromatic ring;
R¹ is selected from the group consisting of hydrogen, halogen, cyano, alkyl, alkoxy, haloalkyl, haloalkoxy, alkoxyalkyl, cycloalkyl, heterocycloalkyl, alkyl substituted by one or more R⁷, aryl substituted by one or more R⁸, and heteroaryl substituted by one or more R⁸;
m is 0, 1, 2, or 3;
W is O or NH;
R³ is selected from the group consisting of fluorine, hydroxyl, cyano, oxo (=O), C₁₋₃ alkyl, OC₁₋₃ alkyl, C₃₋₄ cycloalkyl, and C₃₋₄ spiroalkyl formed by cyclization of two R³ together, wherein the C₁₋₃ alkyl, OC₁₋₃ alkyl, C₃₋₄ cycloalkyl, and C₃₋₄ spiroalkyl can be substituted by 0 to 3 fluorine atoms or 0 to 1 hydroxyl where the valence allows;
q is 0, 1, or 2;
X is CH or N;
Y is CH or N;
R⁵ is selected from the group consisting of halogen, hydroxyl, alkyl, haloalkyl, alkoxy, haloalkoxy, alkoxyalkyl, cycloalkyl, heterocycloalkyl, alkylcycloalkyl, and alkylheterocycloalkyl;
R⁴ is -C₁₋₃ alkyl, -C₀₋₃ alkylene-C₃₋₆ cycloalkyl, -C₀₋₃ alkylene-R⁹, or -C₁₋₃ alkylene-R⁶, wherein the alkyl can be substituted by 0 to 3 substituents independently selected from the group consisting of 0 to 3 F atoms or by 0 to 1 substituent selected from the group consisting of -C₀₋₁ alkylene-CN, -C₀₋₁ alkylene-OR^{O} and -N(R^{N})₂ where the valence allows, and wherein the alkylene and cycloalkyl can be independently substituted by 0 to 2 substituents independently selected from the group consisting of 0 to 2 F atoms or by 0 to 1 substituent selected from the group consisting of -C₀₋₁ alkylene-CN, -C₀₋₁ alkylene-OR^{O} and -N(R^{N})₂ where the valence allows;
R⁹ is 4- to 6-membered heterocycloalkyl, wherein, where the valence allows, the heterocycloalkyl is substituted by 0 to 2 substituents independently selected from the group consisting of
0 to 1 oxo (=O),
0 to 1 -CN,
0 to 2 F atoms, and
0 to 2 substituents independently selected from the group consisting of -C₁₋₃ alkyl and -OC₁₋₃ alkyl, wherein, where the valence allows, the alkyl of the -C₁₋₃ alkyl and -OC₁₋₃ alkyl can be substituted by 0 to 3 substituents independently selected from the group consisting of
0 to 3 F atoms,
0 to 1 -CN, and
0 to 1 -OR^{O};
R⁶ is 5- to 6-membered heteroaryl, wherein, where the valence allows, the heteroaryl can be substituted by 0 to 2 substituents independently selected from the group consisting of
0 to 2 halogen,
0 to 1 substituent selected from the group consisting of -OR^{O} and -N(R^{N})₂, and
0 to 2 -C₁₋₃ alkyl, wherein, where the valence allows, the alkyl can be substituted by 0 to 3 substituents independently selected from the group consisting of
0 to 3 F atoms, and
0 to 1 -OR^{O};
each R^{O} is independently H or -C₁₋₃ alkyl, wherein the C₁₋₃ alkyl can be substituted by 0 to 3 F atoms;
each R^{N} is independently H or -C₁₋₃ alkyl;
Z¹, Z², and Z³ are each independently -CR^{Z} or N, and each R^{Z} is independently H, F, Cl, or -CH₃;
R⁷ is selected from the group consisting of halogen, cyano, hydroxyl, cycloalkyl, heterocycloalkyl, and sulfonyl; and
R⁸ is selected from the group consisting of halogen, cyano, alkyl, haloalkyl, alkoxy, haloalkoxy, alkoxyalkyl, cycloalkyl, heterocycloalkyl, and sulfonyl.

3. The compound according to claim 1 or the stereoisomer, tautomer, or pharmaceutically acceptable salt thereof, wherein the compound is represented by Formula (II): 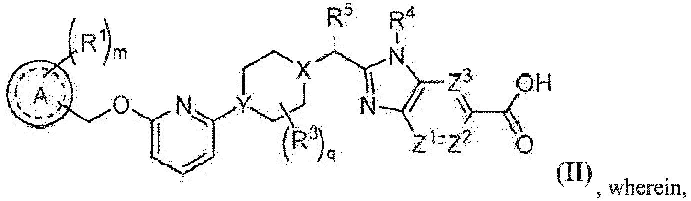
A is selected from the group consisting of phenyl ring, heteroaromatic ring, and 8- to 10-membered fused aromatic ring;
R¹ is selected from the group consisting of hydrogen, halogen, cyano, alkyl, alkoxy, haloalkyl, haloalkoxy, alkoxyalkyl, cycloalkyl, heterocycloalkyl, alkyl substituted by one or more R⁷, aryl substituted by one or more R⁸, and heteroaryl substituted by one or more R⁸;
m is 0, 1, 2, or 3;
R³ is selected from the group consisting of fluorine, hydroxyl, cyano, C₁₋₃ alkyl, OC₁₋₃ alkyl, C₃₋₄ cycloalkyl, and C₃₋₄ spiroalkyl formed by cyclization of two R³ together, wherein the C₁₋₃ alkyl, OC₁₋₃ alkyl, C₃₋₄ cycloalkyl, and C₃₋₄ spiroalkyl can be substituted by 0 to 3 fluorine atoms or 0 to 1 hydroxyl where the valence allows;
q is 0, 1, or 2;
X is CH or N;
Y is CH or N;
R⁵ is selected from the group consisting of halogen, hydroxyl, alkyl, haloalkyl, alkoxy, haloalkoxy, alkoxyalkyl, cycloalkyl, heterocycloalkyl, alkylcycloalkyl, and alkylheterocycloalkyl;
R⁴ is -C₁₋₃ alkyl, -C₀₋₃ alkylene-C₃₋₆ cycloalkyl, -C₀₋₃ alkylene-R⁹, or -C₁₋₃ alkylene-R⁶, wherein the alkyl can be substituted by 0 to 3 substituents independently selected from the group consisting of 0 to 3 F atoms or by 0 to 1 substituent selected from the group consisting of -C₀₋₁ alkylene-CN, -C₀₋₁ alkylene-OR^{O} and -N(R^{N})₂ where the valence allows, and wherein the alkylene and cycloalkyl can be independently substituted by 0 to 2 substituents independently selected from the group consisting of 0 to 2 F atoms or by 0 to 1 substituent selected from the group consisting of -C₀₋₁ alkylene-CN, -C₀₋₁ alkylene-OR^{O} and -N(R^{N})₂ where the valence allows;
R⁹ is 4- to 6-membered heterocycloalkyl, wherein, where the valence allows, the heterocycloalkyl can be substituted by 0 to 2 substituents independently selected from the group consisting of
0 to 1 oxo (=O),
0 to 1 -CN,
0 to 2 F atoms, and
0 to 2 substituents independently selected from the group consisting of -C₁₋₃ alkyl and -OC₁₋₃ alkyl, wherein, where the valence allows, the alkyl of the -C₁₋₃ alkyl and -OC₁₋₃ alkyl can be substituted by 0 to 3 substituents independently selected from the group consisting of
0 to 3 F atoms,
0 to 1 -CN, and
0 to 1 -OR^{O};
R⁶ is 5- to 6-membered heteroaryl, wherein, where the valence allows, the heteroaryl can be substituted by 0 to 2 substituents independently selected from the group consisting of
0 to 2 halogen,
0 to 1 substituent selected from the group consisting of -OR^{O} and -N(R^{N})₂, and
0 to 2 -C₁₋₃ alkyl, wherein, where the valence allows, the alkyl can be substituted by 0 to 3 substituents independently selected from the group consisting of
0 to 3 F atoms, and
0 to 1 -OR^{O};
each R^{O} is independently H or -C₁₋₃ alkyl, wherein the C₁₋₃ alkyl can be substituted by 0 to 3 F atoms;
each R^{N} is independently H or -C₁₋₃ alkyl;
Z¹, Z², and Z³ are each independently -CR^{Z} or N, and each R^{Z} is independently H, F, Cl, or -CH₃;
R⁷ is selected from the group consisting of halogen, cyano, hydroxyl, cycloalkyl, heterocycloalkyl, and sulfonyl; and
R⁸ is selected from the group consisting of halogen, cyano, alkyl, haloalkyl, alkoxy, haloalkoxy, alkoxyalkyl, cycloalkyl, heterocycloalkyl, and sulfonyl.

4. The compound according to any one of claims 1-3 or the stereoisomer, tautomer, or pharmaceutically acceptable salt thereof, wherein the alkyl is a C₁₋₆ alkyl selected from the group consisting of methyl, ethyl, propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl.

5. The compound according to any one of claims 1-3 or the stereoisomer, tautomer, or pharmaceutically acceptable salt thereof, wherein the alkoxy is a C₁₋₆ alkoxy selected from the group consisting of methoxy, ethoxy, propoxy, isopropoxy, n-butoxy, isobutoxy, sec-butoxy, tert-butoxy, n-pentyloxy, sec-pentyloxy, 1-ethylpropoxy, 2-methylbutoxy, tert-pentyloxy, 1,2-dimethylpropoxy, isopentyloxy, neopentyloxy, n-hexyloxy, isohexyloxy, sec-hexyloxy, tert-hexyloxy, neohexyloxy, 2-methylpentyloxy, 1,2-dimethylbutoxy, and 1-ethylbutoxy; and/or the alkoxyalkyl is a C₁₋₄ alkoxy-C₁₋₄ alkyl, preferably selected from methoxymethyl, methoxyethyl, methoxypropyl, methoxybutyl, ethoxymethyl, ethoxyethyl, ethoxypropyl, ethoxybutyl, propoxymethyl, propoxyethyl, propoxypropyl, propoxybutyl, butoxymethyl, butoxyethyl, butoxypropyl, butoxybutyl, and the like.

6. The compound according to any one of claims 1-3 or the stereoisomer, tautomer, or pharmaceutically acceptable salt thereof, wherein the halogen is selected from the group consisting of fluorine, chlorine, bromine, and iodine; haloalkyl refers to an alkyl on which one or more hydrogen atoms are substituted by halogen; haloalkoxy refers to an alkoxy on which one or more hydrogen atoms are substituted by halogen; or heterocycloalkyl refers to an alkyl on which one or more hydrogen atoms are substituted by heterocyclyl.

7. The compound according to any one of claims 1-3 or the stereoisomer, tautomer, or pharmaceutically acceptable salt thereof, wherein the fused aromatic ring is selected from the group consisting of naphthalene and fused heteroaromatic ring, wherein the fused heteroaromatic ring is formed by fusing a heteroaromatic ring with an aromatic ring or a heteroaromatic ring, and wherein the heteroaromatic ring comprises one or more heteroatoms independently selected from the group consisting of nitrogen, oxygen, and sulfur.

8. The compound according to claim 7 or the stereoisomer, tautomer, or pharmaceutically acceptable salt thereof, wherein the fused heteroaromatic ring is selected from the group consisting of indazole, quinoline, isoquinoline, quinoxaline, indole, isoindole, cinnoline, quinazoline, phthalazine, purine, naphthyridine, pteridine, benzofuran, benzothiophene, benzoxazole, benzothiazole, benzisoxazole, benzisothiazole, benzoxadiazole, benzothiadiazole, benzotriazole, benzotriazine, benzimidazole, pyrazolopyrazine, pyrazinopyrimidine, pyrazinopyridazine, pyrazinotriazine, pyrazolopyrimidine, imidazolopyrimidine, triazolopyrimidine, pyrimidotriazine, pyrimidopyridazine, imidazolopyridazine, pyrazolopyridazine, triazolopyridazine, pyridazinotriazine, imidazolotriazine, pyrazolotriazine, triazolotriazine, imidazolopyridine, pyridopyridazine, pyrazolopyridine, pyridopyrimidine, pyridotriazine, oxazolopyridine, thiazolopyridine, isoxazolopyridine, isothiazolopyridine, oxadiazolopyridine, thiadiazolopyridine, furanopyridine, pyrrolopyridine, imidazolopyrazine, triazolopyrazine, oxazolopyrazine, thiazolopyrazine, isoxazolopyrazine, isothiazolopyrazine, oxadiazolopyrazine, thiadiazolopyrazine, furanopyrazine, pyrrolopyrazine, oxazolopyrimidine, thiazolopyrimidine, isoxazolopyrimidine, isothiazolopyrimidine, oxadiazolopyrimidine, thiadiazolopyrimidine, furanopyrimidine, pyrrolopyrimidine, oxazolopyridazine, thiazolopyridazine, isoxazolopyridazine, isothiazolopyridazine, oxadiazolopyridazine, thiadiazolopyridazine, furanopyridazine, pyrrolopyridazine, oxazolotriazine, thiazolotriazine, isoxazolotriazine, isothiazolotriazine, oxadiazolotriazine, thiadiazolotriazine, furanotriazine, and pyrrolotriazine.

9. The compound according to claim 8 or the stereoisomer, tautomer, pharmaceutically acceptable salt thereof, wherein the naphthyridine is selected from the group consisting of 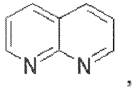 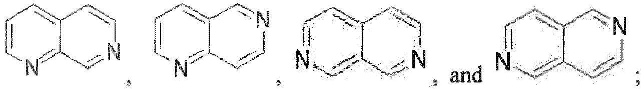 the imidazolopyridine is selected from the group consisting of 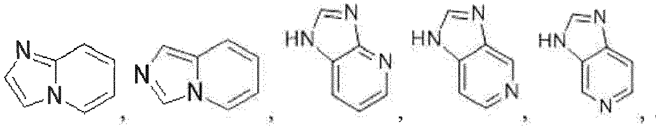 and 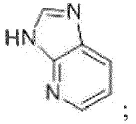 the imidazolopyrazine is selected from the group consisting of 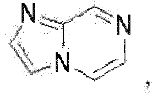 and 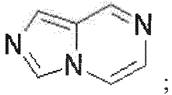 the triazolopyrazine is selected from the group consisting of 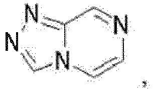 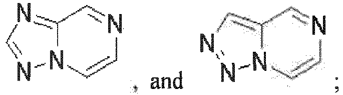 the pyrazolopyrimidine is selected from the group consisting of 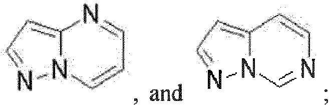 the imidazolopyrimidine is selected from the group consisting of 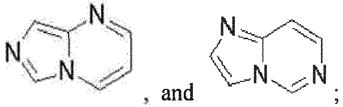 the triazolopyrimidine is selected from the group consisting of 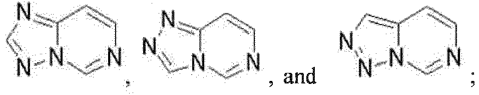 the imidazolopyridazine is selected from the group consisting of 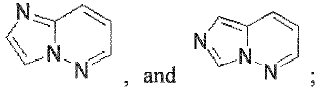 the thiazolopyridazine is selected from the group consisting of 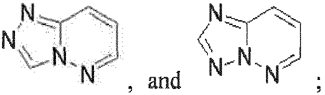 the imidazolotriazine is selected from the group consisting of 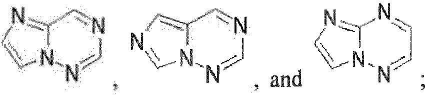 the pyridopyridazine is selected from the group consisting of 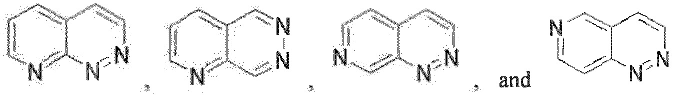 ; the pyrazolopyridine is selected from the group consisting of 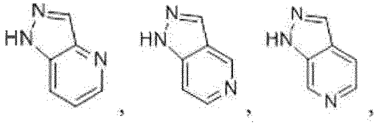 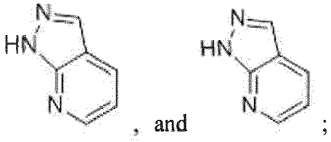 the pyridopyrimidine is selected from the group consisting of 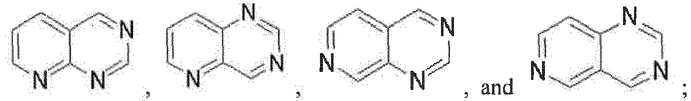 the pyridotriazine is selected from the group consisting of 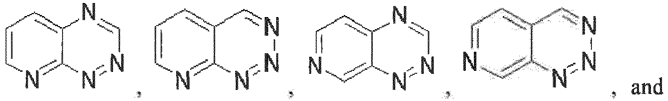 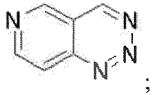 and/or the pyrimidotriazine is selected from the group consisting of

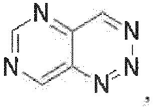

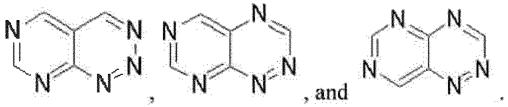

10. The compound according to any one of claims 1-3 or the stereoisomer, tautomer, or pharmaceutically acceptable salt thereof, wherein the heterocycle of the heterocycloalkyl is a 4- to 10-membered heterocycle selected from the group consisting of 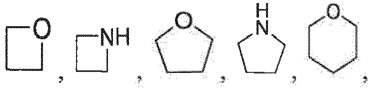 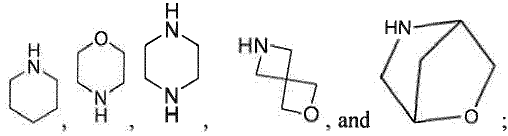 the aryl is phenyl; and/or the heteroaryl is a 5- to 12-membered heteroaryl selected from the group consisting of

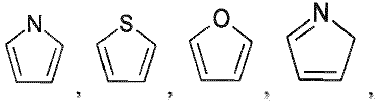

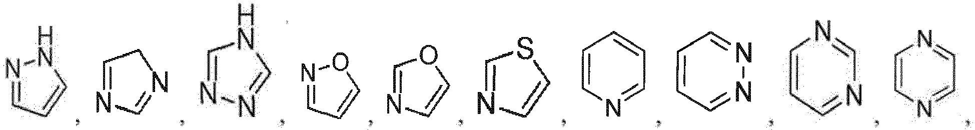

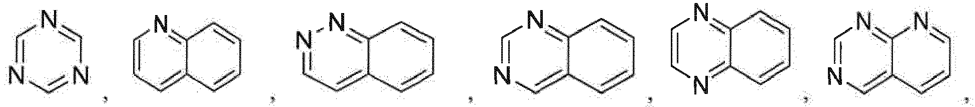

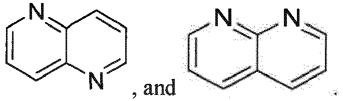

11. The compound according to any one of claims 1-3 or the stereoisomer, tautomer, or pharmaceutically acceptable salt thereof, wherein the cycloalkyl is a C₃₋₆ cycloalkyl selected from the group consisting of cyclopropyl, cyclobutyl, cyclopentyl, and cyclohexyl.

12. The compound according to any one of claims 1-3 or the stereoisomer, tautomer, or pharmaceutically acceptable salt thereof, wherein A is selected from the group consisting of phenyl ring,

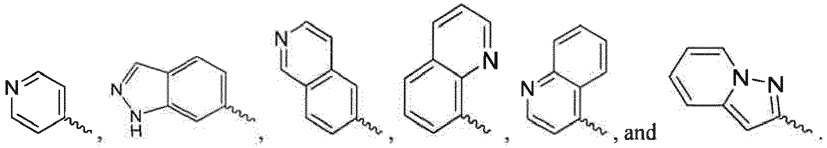

13. The compound according to claim 1 or the stereoisomer, tautomer, or pharmaceutically acceptable salt thereof, wherein A is selected from the group consisting of phenyl ring, 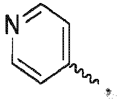 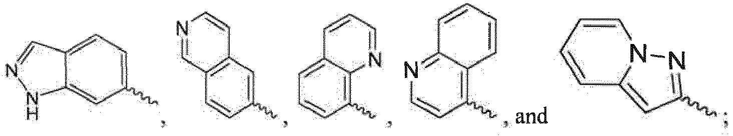
R¹ is selected from the group consisting of hydrogen, fluorine, chlorine, bromine, cyano, difluoroethyl, and trifluoroethyl;
m is 0, 1, or 2;
W is O or NH;
R² is selected from the group consisting of chlorine, cyano, trifluoromethyl, methoxy, and methoxymethyl;
q is 0 or 1;
R³ is fluorine, methyl, oxo (=O), hydroxyl, fluoromethyl, or methoxyethyl;
R⁴ is oxetan-2-yl-methyl;
X is CH or N;
Y is CH or N;
R⁵ is selected from the group consisting of methyl, ethyl, cyclopropylmethyl, fluoroethyl, and methoxyethyl;
Z¹ is CH;
Z² is CH or CF; and
Z³ is CH, N, or CF.

14. The compound according to claim 2 or the stereoisomer, tautomer, or pharmaceutically acceptable salt thereof, wherein A is selected from the group consisting of phenyl ring, 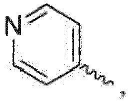 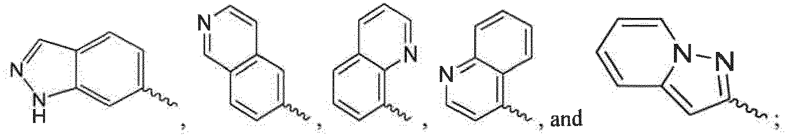
R¹ is selected from the group consisting of hydrogen, fluorine, chlorine, bromine, cyano, difluoroethyl, and trifluoroethyl;
m is 0, 1, or 2;
W is O or NH;
q is 0 or 1;
R³ is fluorine, methyl, oxo (=O), hydroxyl, fluoromethyl, or methoxyethyl;
R⁴ is oxetan-2-yl-methyl;
X is CH or N;
Y is CH or N;
R⁵ is selected from the group consisting of methyl, ethyl, cyclopropylmethyl, fluoroethyl, and methoxyethyl;
Z¹ is CH;
Z² is CH or CF; and
Z³ is CH, N, or CF.

15. The compound according to claim 3 or the stereoisomer, tautomer, or pharmaceutically acceptable salt thereof, wherein A is selected from the group consisting of phenyl ring, 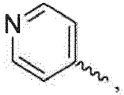 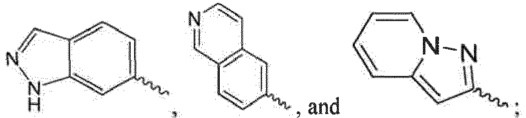
R¹ is selected from the group consisting of hydrogen, fluorine, chlorine, bromine, cyano, difluoroethyl, and trifluoroethyl;
m is 0, 1, or 2;
q is 0;
R⁴ is oxetan-2-yl-methyl;
X is CH or N;
Y is CH or N;
R⁵ is selected from the group consisting of methyl, ethyl, cyclopropylmethyl, fluoroethyl, and methoxyethyl;
Z¹ is CH;
Z² is CH or CF; and
Z³ is CH, N, or CF.

16. The compound according to any one of claims 1-15 or the stereoisomer, tautomer, or pharmaceutically acceptable salt thereof, wherein when the carbon atom connected with R⁵ is a chiral carbon atom, the chiral carbon atom is in S configuration and/or R configuration, preferably in S configuration.

17. The compound according to any one of claims 1-3 or the stereoisomer, tautomer, or pharmaceutically acceptable salt thereof, wherein the compound is selected from the group consisting of
| | **Structural formula** | | **Structural formula** |
|---|---|---|---|
| 1 | 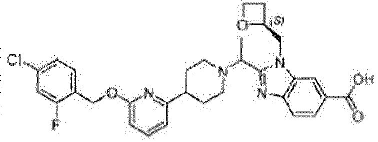 | 27 | 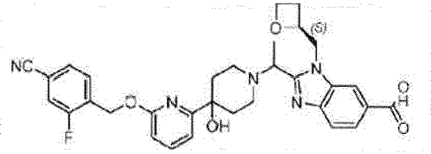 |
| 2 | 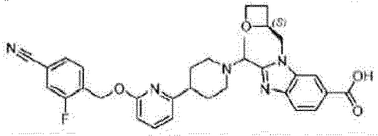 | 28 | 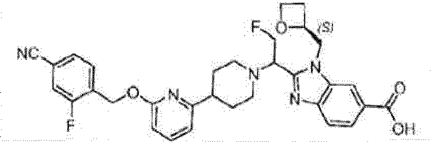 |
| 3 | 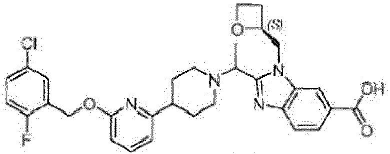 | 29 | 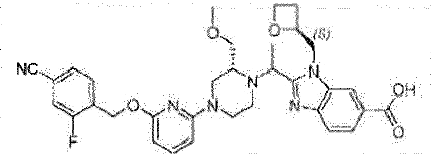 |
| 1 | 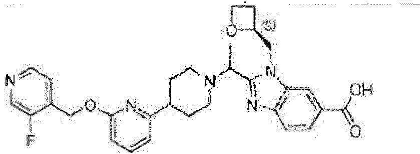 | 30 | 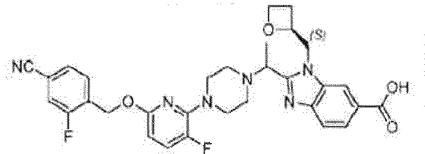 |
| 5 | 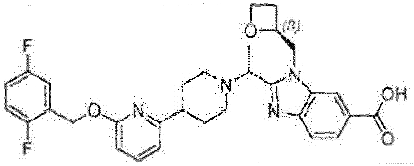 | 31 | 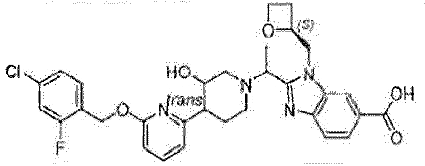 |
| 6 | 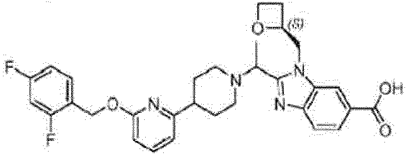 | 32 | 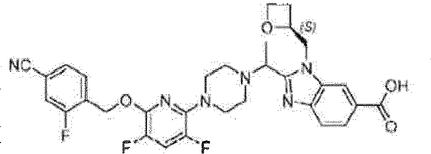 |
| 7 | 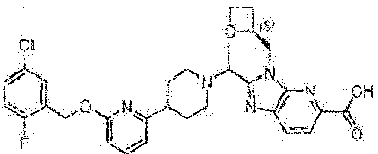 | 33 | 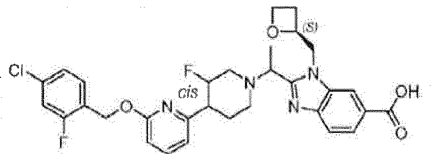 |
| 8 | 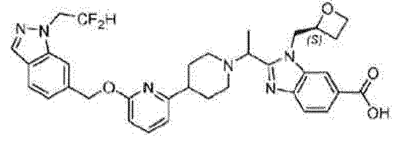 | 34 | 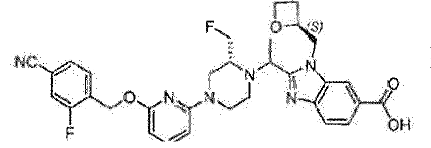 |
| 9 | 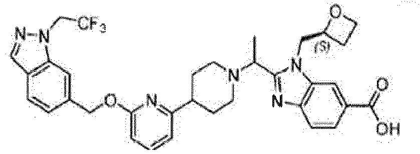 | 35 | 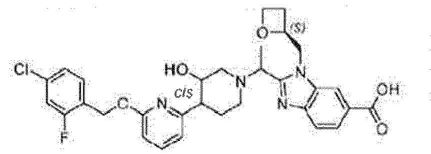 |
| 10 | 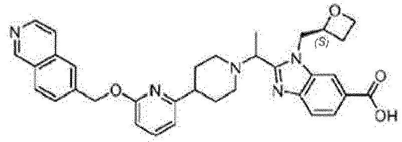 | 36 | 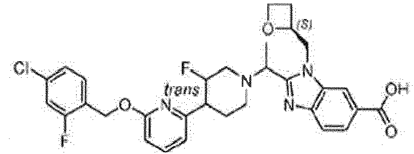 |
| 11 | 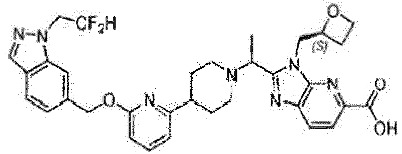 | 37 | 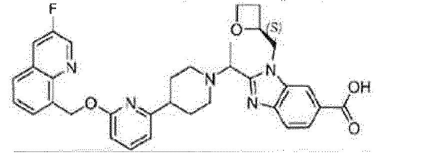 |
| 12 | 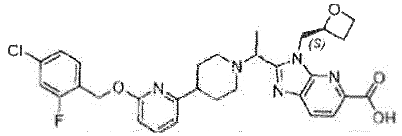 | 38 | 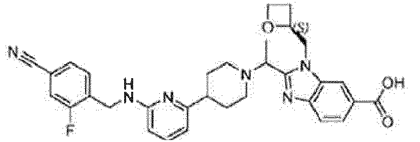 |
| 13 | 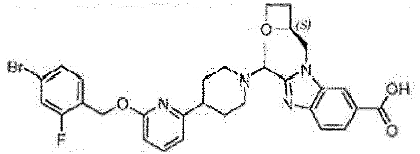 | 39 | 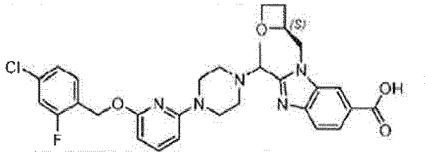 |
| 14 | 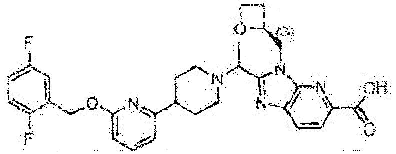 | 40 | 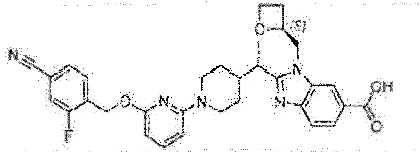 |
| 15 | 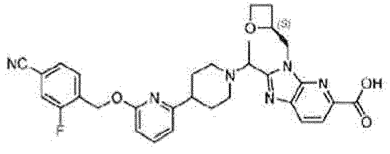 | 41 | 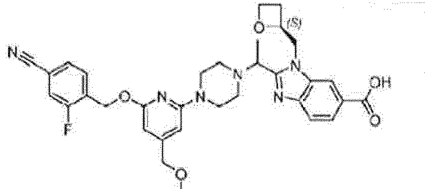 |
| 16 | 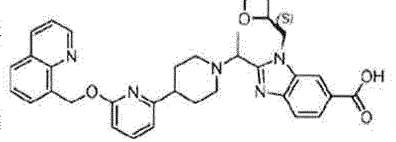 | 42 | 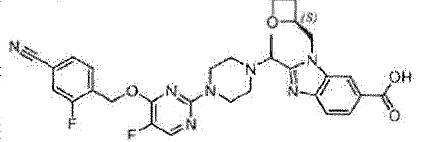 |
| 17 | 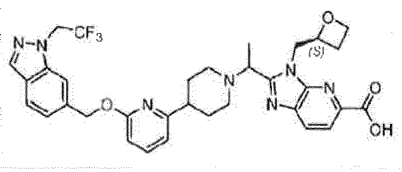 | 43 | 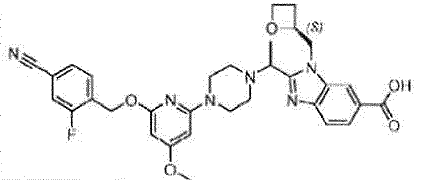 |
| 18 | 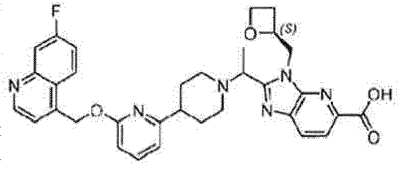 | 44 | 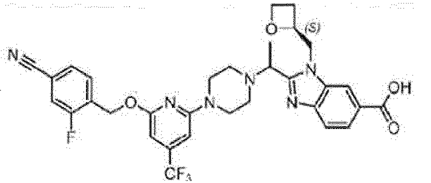 |
| 19 | 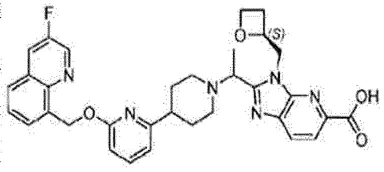 | 45 | 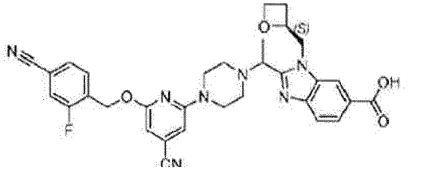 |
| 20 | 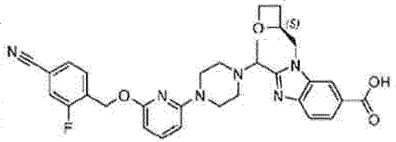 | 46 | 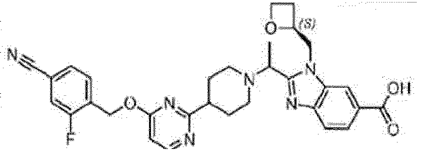 |
| 21 | 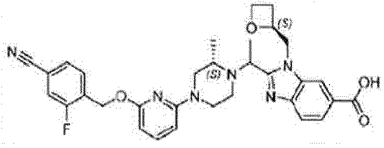 | 47 | 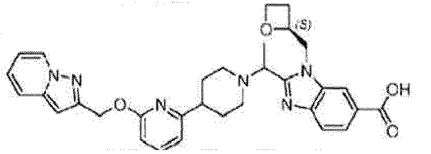 |
| 22 | 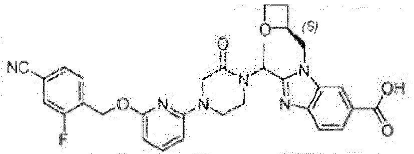 | 48 | 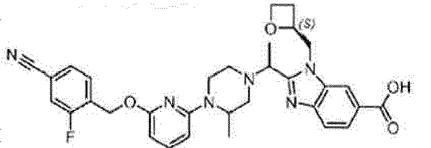 |
| 23 | 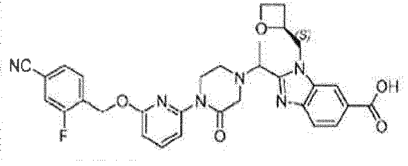 | 49 | 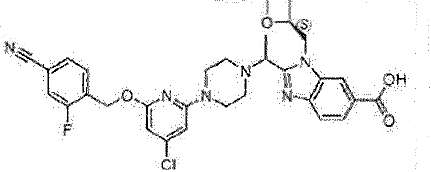 |
| 24 | 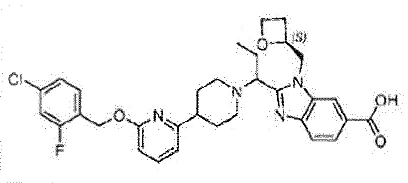 | 50 | 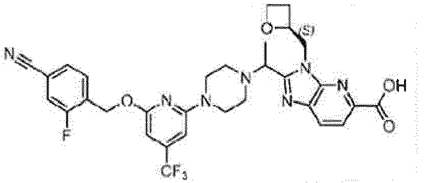 |
| 25 | 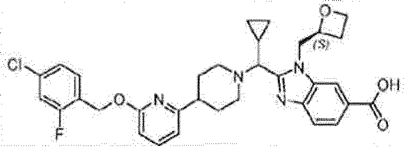 | 51 | 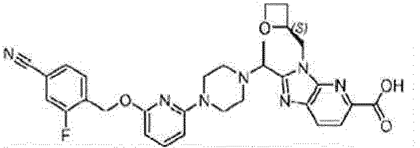 |
| 26 | 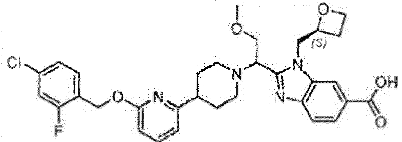 | 52 | 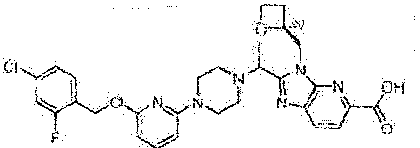 |

18. The compound according to any one of claims 1-3 or the stereoisomer, tautomer, or pharmaceutically acceptable salt thereof, wherein the compound is selected from the group consisting of
| | **Structural formula** | | **Structural formula** |
|---|---|---|---|
| 1A | 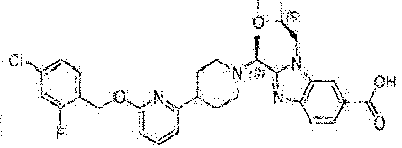 | 27 | 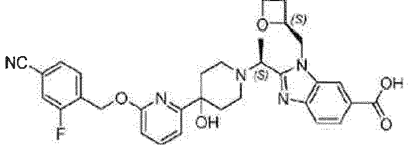 |
| 1B | 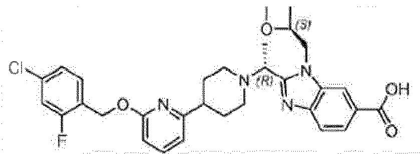 | 28 | 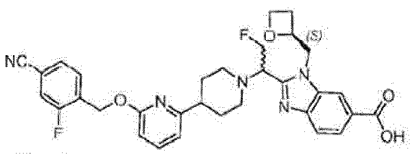 |
| 2A | 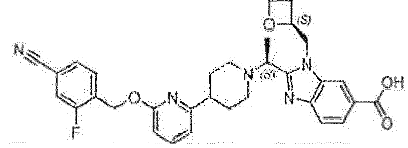 | 29 | 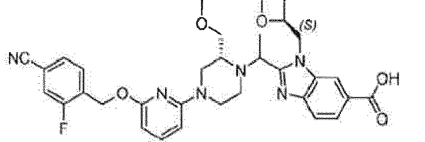 |
| 2B | 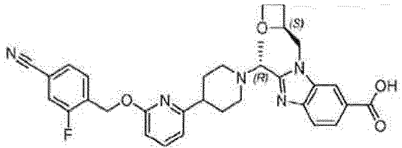 | 30 | 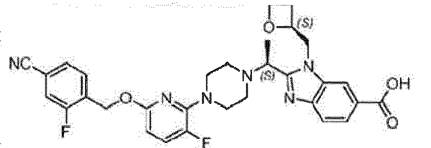 |
| 3 | 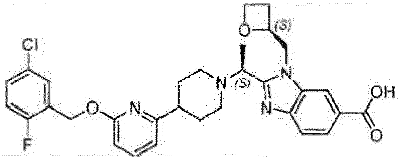 | 31 | 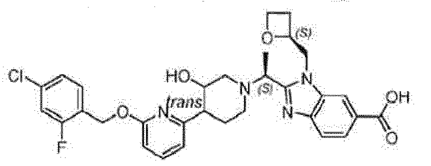 |
| 4 | 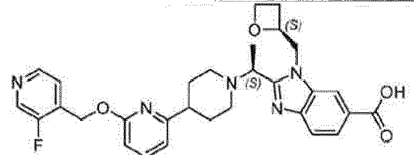 | 32 | 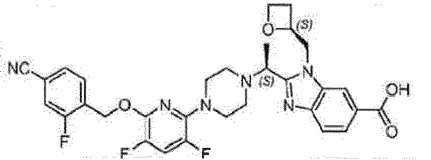 |
| 5 | 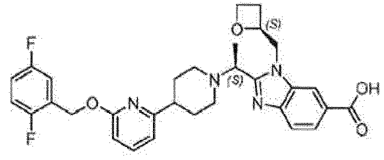 | 33 | 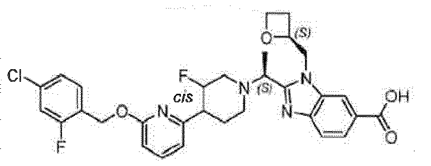 |
| 6 | 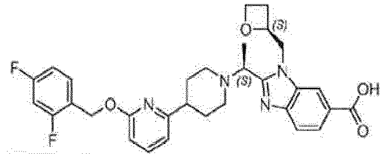 | 34 | 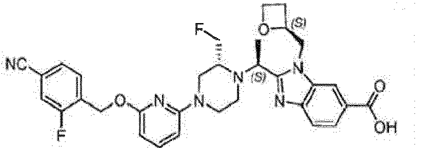 |
| 7 | 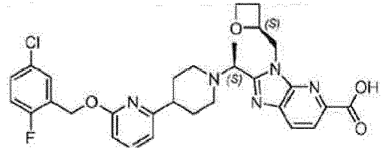 | 35 | 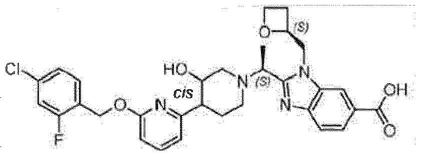 |
| 8 | 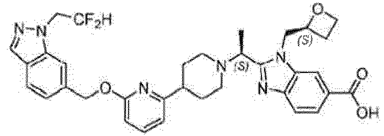 | 36 | 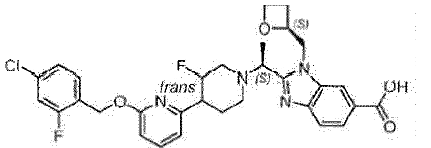 |
| 9 | 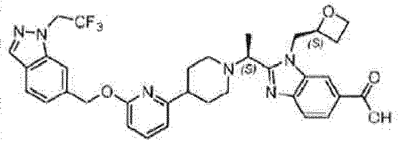 | 37 | 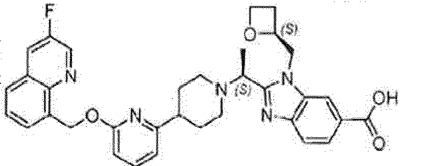 |
| 10 | 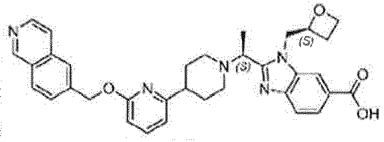 | 38 | 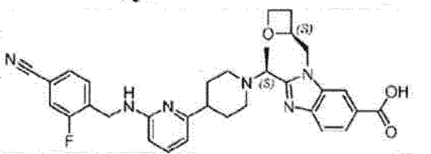 |
| 11 | 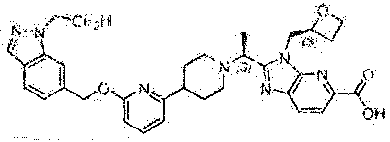 | 39A | 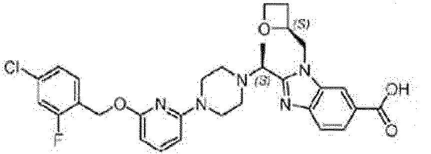 |
| 12 | 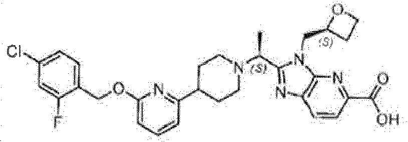 | 39B | 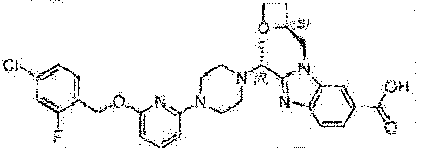 |
| 13 | 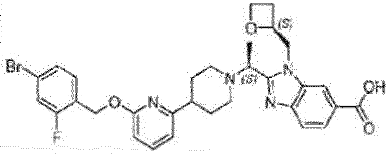 | 40 | 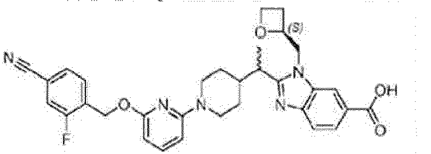 |
| 14 | 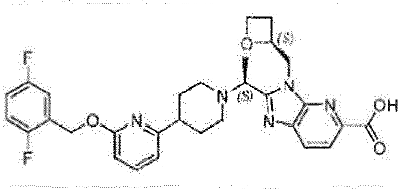 | 41 | 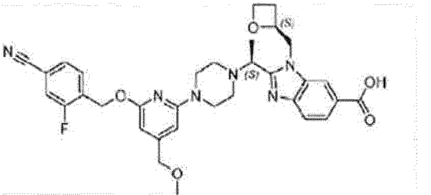 |
| 15 | 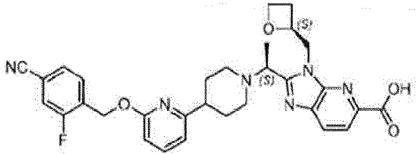 | 12 | 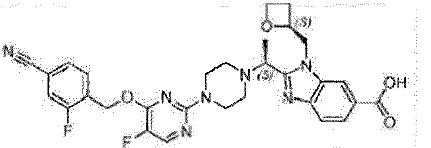 |
| 16 | 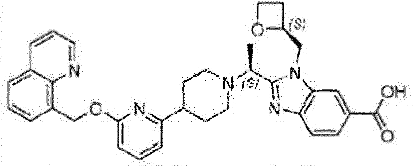 | 43 | 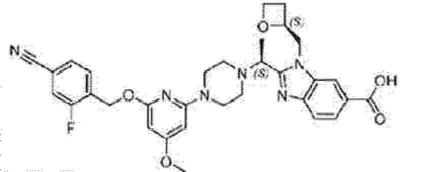 |
| 17 | 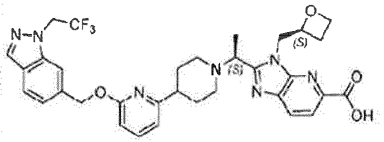 | 44 | 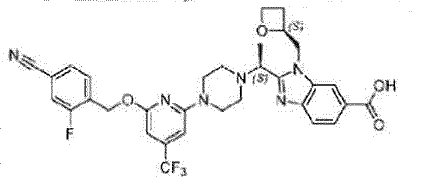 |
| 18 | 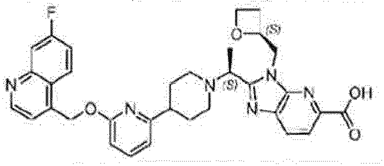 | 45 | 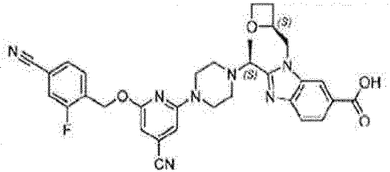 |
| 19 | 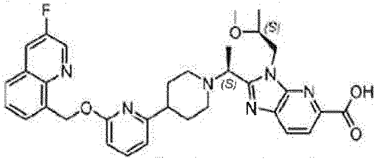 | 46 | 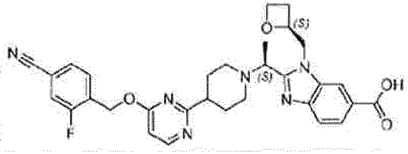 |
| 20A | 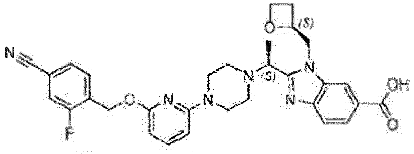 | 47 | 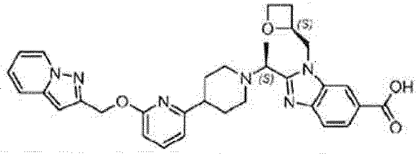 |
| 20B | 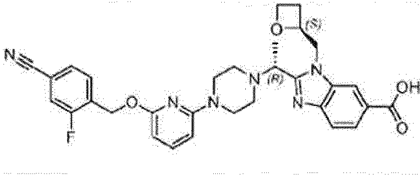 | 48A | 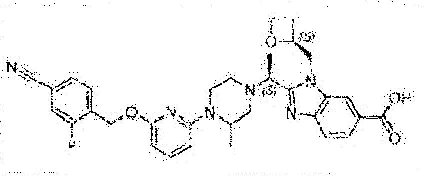 |
| 21 | 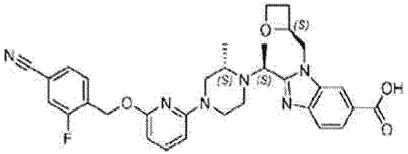 | 48B | 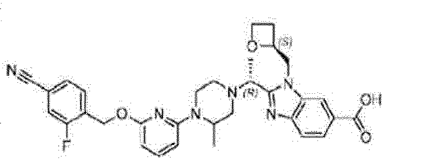 |
| 22 | 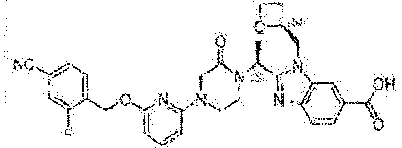 | 49 | 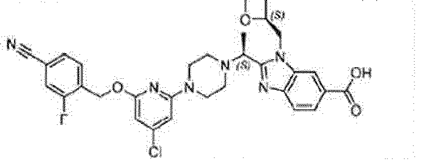 |
| 23 | 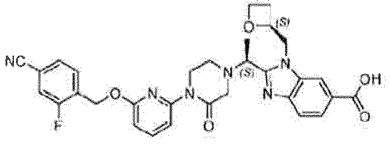 | 50 | 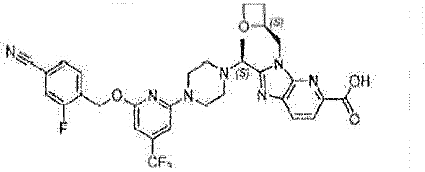 |
| 24A | 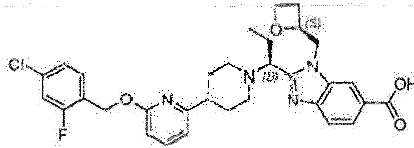 | 51A | 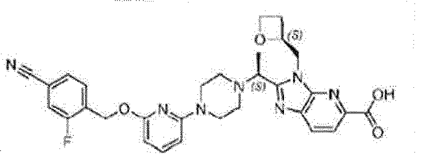 |
| 24B | 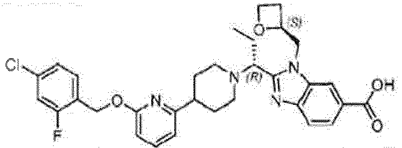 | 51B | 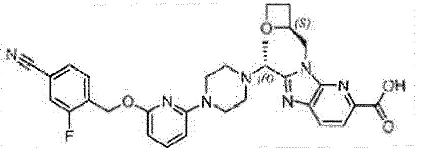 |
| 25 | 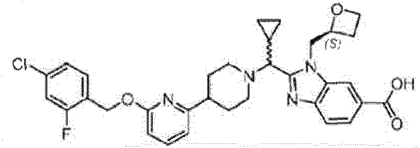 | 52A | 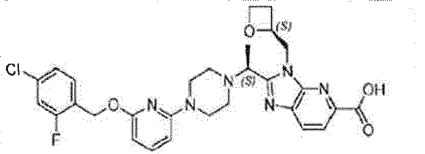 |
| 26 | 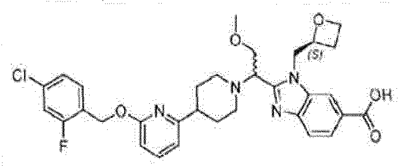 | 52B | 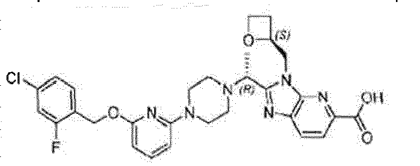 |

19. The compound according to any one of claims 1-3 or the stereoisomer, tautomer, or pharmaceutically acceptable salt thereof, wherein the pharmaceutically acceptable salt refers to a salt that is prepared from the compound and a pharmaceutically acceptable acid or base.

20. The compound according to any one of claims 1-18 or the stereoisomer, tautomer, or pharmaceutically acceptable salt thereof, wherein one or more hydrogen atoms of the compound are substituted with isotopic deuterium.

21. A pharmaceutical composition, comprising the compound according to any one of the preceding claims 1-20 or the stereoisomer, tautomer, or pharmaceutically acceptable salt thereof, and one or more pharmaceutically acceptable carriers.

22. Use of the compound according to any one of the preceding claims 1-20 or the stereoisomer, tautomer, or pharmaceutically acceptable salt thereof in the manufacture of a medicament for treating GLP-1-related diseases, preferably diabetes mellitus-related diseases.
